# EUROPÄISCHE PATENTSCHRIFT

(11) **EP 1 572 628 B1**
(45) Veröffentlichungstag und Bekanntmachung des Hinweises auf die Patenterteilung: **25.07.2007**
(21) Anmeldenummer: 03782248.3
(22) Anmeldetag: 28.11.2003
(51) Int. Cl.: C07C 235/62, C07D 211/34, C07D 211/62, C07D 319/06, C07D 205/04, C07D 307/42, C07D 317/54, C07D 319/08, C07D 211/60, A61K 31/16, A61K 31/445, A61P 9/00

(54) **ISOPHTHALSÄUREDERIVATE**
ISOPHTALIC ACID DERIVATIVES
DERIVES D'ACIDE ISOPHTALIQUE

(30) Priorität: 11.12.2002 DE 10257785
(43) Veröffentlichungstag der Anmeldung: 14.09.2005
(73) Patentinhaber: Bayer HealthCare AG, 51368 Leverkusen (DE)
(72) Erfinder: HÄRTER, Michael, 51375 Leverkusen (DE); ERGÜDEN, Jens, 42489 Wülfrath (DE); WUNDER, Frank, 42117 Wuppertal (DE); TINEL, Hanna, 42111 Wuppertal (DE); KÖBBERLING, Johannes, 41516 Grevenborich (DE); BECKER, Eva-Maria, 42117 Wuppertal (DE); MÜNTER, Klaus, 42489 Wülfrath (DE); SCHLEMMER, Karl-Heinz, 42113 Wuppertal (DE); KAST, Raimund, 42349 Wuppertal (DE); KOLKHOF, Peter, 42133 Wuppertal (DE); KARIG, Gunter, 51069 Köln (DE); BÄRFACKER, Lars, 46047 Oberhausen (DE); HÜBSCH, Walter, 42113 Wuppertal (DE); SCHUHMACHER, Joachim, 42109 Wuppertal (DE); ZULEGER, Susanne, 50931 Köln (DE); CONCEPCION, Arnel, Kyoto-fu, 611-0002 (JP); SHIMIZU, Haruka, Tokyo, 113-0033 (JP)
(86) Internationale Anmeldenummer: PCT/EP2003/013433
(87) Internationale Veröffentlichungsnummer: WO 2004/052839

(56) Entgegenhaltungen:
- EP-A- 0 791 576
- WO-A-98/40364
- WO-A-98/40370
- US-A1- 2002 045 751

## Beschreibung

Die vorliegende Erfindung betrifft Isophthalsäurederivate, ein Verfahren zu ihrer Herstellung sowie ihre Verwendung zur Herstellung von Arzneimitteln zur Behandlung und/oder Prophylaxe von Krankheiten bei Menschen und Tieren, insbesondere von kardiovaskulären Erkrankungen.

Cysteinyl-Leukotriene sind wichtige Mediatoren für eine Vielzahl pathologischer Krankheitszustände. Sie werden bei Aktivierung inflammatorischer Zellen, wie zum Beispiel polymorphkerniger Leukozyten, Makrophagen und Mastzellen, mit Hilfe von 5-Lipoxygenase aus Arachidonsäure gebildet. Dabei entsteht zunächst Leukotrien A4 (LTA4), welches dann in weiteren Reaktionschritten durch Addition von Glutathion in Leukotrien C4 (LTC4) umgewandelt wird. Bei der weiteren Metabolisierung entstehen dann Leukotrien D4 (LTD4) und Leukotrien E4 (LTE4). LTC4, LTD4 und LTE4 werden zusammenfassend als Cysteinyl-Leukotriene bezeichnet.

Die physiologischen Effekte der Cysteinyl-Leukotriene werden über G-Protein gekoppelte Rezeptoren vermittelt. Pharmakologisch und molekularbiologisch charakterisiert sind zwei Cysteinyl-Leukotrien Rezeptoren:

Der Cysteinyl-Leukotrien Rezeptor 1 (CysLTl) wird vor allem durch LTD4, schwächer auch durch LTC4 und LTE4 aktiviert. Er trägt daher auch die Bezeichnung LTD4-Rezeptor. Der Rezeptor konnte 1999 kloniert und charakterisiert werden (Lynch et. al. (1999) Nature 399; 789-793). Der CysLTl-Rezeptor weist eine starke Expression in Milz, peripheren Leukozyten und Lunge auf. Eine Expression des CysLT1 Rezeptors im humanen Herzen konnte bisher nicht nachgewiesen werden. CysLT1 spezifische Rezeptor-Antagonisten, wie beispielsweise Pranlukast, Zafirlukast und Montelukast führen zur Relaxation der glatten Muskulatur der Bronchien und sind für die Behandlung bronchialen Asthmas entwickelt worden.

Der Cysteinyl-Leukotrien Rezeptor 2 (CysLT2) wird vor allem durch LTC4, schwächer auch durch LTD4 und LTE4 aktiviert. Er wird daher auch als LTC4-Rezeptor bezeichnet. Der Rezeptor konnte im Jahr 2000 identifiziert und charakterisiert werden (Heise et. al. (2000) Journal of Biological Chemistry 275; 30531-30536; Takasaki et. al. (2000) Biochem. Biophys. Res. Commun. 274; 316-322; Nothacker et. al. (2000) Mol. Pharmacol. 58; 1601-1608). Der humane CysLT2-Rezeptor weist eine sehr starke Expression in Herz, Plazenta, Milz und peripheren Blut-Leukozyten (PBL) auf. Mit Hilfe von PCR-Untersuchungen und *in-situ* Hybridisierungen konnte gezeigt werden, dass dieser Rezeptor im Herz in glatten Muskelzellen von Koronararterien, in Myozyten und sehr stark auch in Purkinjefasern exprimiert wird (Kamohara et. al. (2001) Biochem. Biophys. Res. Commun. 287; 1088-1092; Hui et. al. (2001) Journal of Biological Chemistry 276; 47489-47495). Bei der Aktivierung des CysLT2 Rezeptors kommt es, wie beim CysLT1 Rezeptor, zu einem Anstieg der intrazellulären Calcium-Konzentration.

Cysteinyl-Leukotriene sind vasoaktive Substanzen, d.h. sie führen zu einer starken Konstriktion von Koronararterien. Zusätzlich senken sie die Kontraktilität des Herzens, induzieren Änderungen im Elektrokardiogramm, beeinflussen den Blutdruck, erhöhen die mikrovaskuläre Permeabilität, fördern die Ödembildung und induzieren eine starke Bronchokonstriktion (Letts et. al (1987) Cardiovasc. Clin. 18; 101-113; Fauler und Frölich (1989) Cardiovasc. Drugs and Therapy 3; 499-505; Piper et. al. (1990) Adv. Prostaglandin Thromboxane Leukotr. Res. 20; 146-152). Daher bilden Antagonisten der Cysteinyl-Leuktrien Rezeptoren einen therapeutischen Ansatz für die Behandlung von kardiovaskulären Erkrankungen.

In EP-A 516 069 sind Leukotrien B4-Antagonisten zur Behandlung von allergischen und antiinflammatorischen Erkrankungen beschrieben. EP-A 791 576 und EP-A 341 551 offenbaren Leukotrien-Antagonisten zur Behandlung von Asthma.

Gegenstand der vorliegenden Erfindung sind Verbindungen der Formel (I) 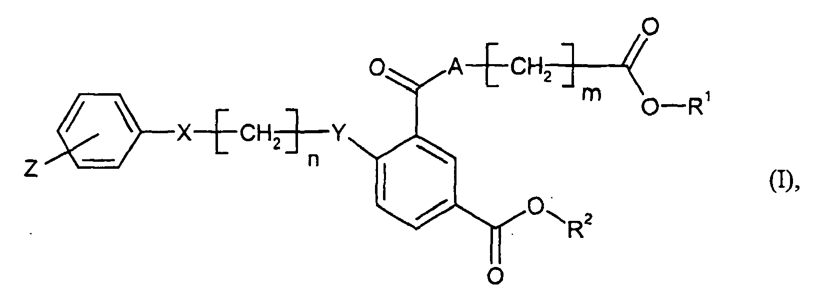 worin
- A: einen 4- bis 7-gliedrigen stickstoffhaltigen gesättigten Heterocyclus, der über das Stickstoffatom an die Ketogruppe gebunden ist und der gegebenenfalls in Nachbarschaft zu einem Stickstoffatom eine Carbonylgruppe trägt,
oder
einen Rest 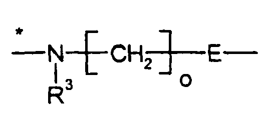 bedeutet,
worin
E für (C₃-C₇)-Cycloalkandiyl, (C₅-C₇)-Cycloalkendiyl oder für 5- bis 10-gliedriges Heterocyclyl, das über ein Kohlenstoffatom an die [CH₂]ₒ-Gruppe gebunden ist, steht,
o für 0, 1 oder 2 steht,
R³ für Wasserstoff oder (C₁-C₆)-Alkyl steht und
* für die Anknüpfstelle an die Ketogruppe steht,
- m: 0, 1 oder 2 bedeutet,
- n: 1, 2, 3 oder 4 bedeutet,
- R¹: Wasserstoff oder (C₁-C₆)-Alkyl bedeutet,
- R²: Wasserstoff oder (C₁-C₆)-Alkyl bedeutet,
- X: eine Bindung, -CH=CH-, -C≡C- oder O bedeutet,
- Y: 0, *-NH-C(=O)- oder NH bedeutet,
worin
* für die Anknüpfstelle an den Phenylring steht
und
- Z: sich in meta oder para Stellung zum Substituenten X befindet und entweder (C₆-C₁₀)-Alkoxy, das 1 oder 2 weitere Sauerstoffatome in der Kette enthalten kann,
oder
einen Rest 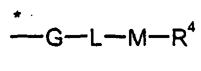 bedeutet,
worin
G für eine Bindung, 0 oder S steht,
L für (C₁-C₆)-Alkandiyl, (C₃-C₆)-Alkendiyl oder (C₃-C₆)-Alkindiyl steht,
M für eine Bindung, O oder S steht,
R⁴ für (C₆-C₁₀)-Aryl, Biphenyl, Phenoxyphenyl, Benzyloxyphenyl, (E)-Phenylvinylphenyl, 2-Phenylethylphenyl, Tetrahydronaphthyl, Benzyl, Heteroaryl, 5- bis 10-gliedriges Heterocyclyl, (C₃-C₇)-Cycloalkyl oder (C₃-C₇)-Cycloalkylmethyl steht, wobei Aryl, Biphenyl, Phenoxyphenyl, Benzyloxyphenyl, (E)-Phenylvinylphenyl, 2-Phenylethylphenyl, Tetrahydronaphthyl, Benzyl, Heteroaryl, Heterocyclyl, Cycloalkyl und Cycloalkylmethyl ihrerseits bis zu dreifach unabhängig voneinander durch Halogen, Cyano, Nitro, Trifluormethyl, Trifluormethoxy, (C₁-C₆)-Alkyl, (C₁-C₆)-Alkoxy, (C₂-C₆)-Alkenyl, (C₃-C₇)-Cycloalkyl, (C₃-C₇)-Cycloalkylmethoxy, (C₅-C₇)-Cycloalkenyl, (C₃-C₇)-Cycloalkoxy oder (C₅-C₇)-Cycloalkenyloxy substituiert sein können, und
* für die Anknüpfstelle an den Phenylring steht,
und ihre Salze, Hydrate, Hydrate der Salze und Solvate.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen.

Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen' Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure,

Apfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B.

Natrium- und Kaliumsalze), Erdalkalisalze (z.B. Calcium- und Magnesiumsalze) und Ammoniumsalze, abgeleitet von Ammoniak oder organischen Aminen mit 1 bis 16C-Atomen, wie beispielhaft und vorzugsweise Ethylamin, Diethylamin, Triethylamin, Ethyldiisopropylamin, Monoethanolamin, Diethanolamin, Triethanolamin, Dicyclo-hexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
Alkyl per se und "Alk" in Alkoxy stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.
Alkoxy steht beispielhaft und vorzugsweise für Methoxy, Ethoxy, n-Propoxy, Isopropoxy, tert.-Butoxy, n-Pentoxy und n-Hexoxy.
Alkandiyl steht für einen geradkettigen oder verzweigten gesättigten Alkandiylrest mit 1 bis 6 Kohlenstoffatomen. Bevorzugt ist ein geradkettiger oder verzweigter Alkandiylrest mit 1 bis 4 Kohlenstoffatomen: Beispielhaft und vorzugsweise seien genannt Methylen, Ethan-1,2-diyl, Ethan-1,1-diyl, Propan-1,3-diyl, Propan-1,2-diyl, Propan-2,2-diyl, Butan-1,4-diyl, Butan-1,3-diyl, Butan-2,4-diyl, Pentan-1,5-diyl, Pentan-2,4-diyl, 2-Methyl-pentan-2,4-diyl.
Alkenyl steht für einen geradkettigen oder verzweigten Alkenylrest mit 2 bis 6 Kohlenstoffatomen. Bevorzugt ist ein geradkettiger oder verzweigter Alkenylrest mit 2 bis 4 Kohlenstoffatomen. Beispielhaft und vorzugsweise seien genannt: Vinyl, Allyl, n-Prop-1-en-1-yl, n-But-2-en-1-yl und 2-Methyl-2-buten-1-yl.
Alkendiyl steht für einen geradkettigen oder verzweigten Alkendiylrest mit 2 bis 6 Kohlenstoffatomen. Bevorzugt ist ein geradkettiger oder verzweigter Alkendiylrest mit 3 bis 6, besonders bevorzugt mit 4 oder 5 Kohlenstoffatomen. Beispielhaft und vorzugsweise seien genannt: Allyl-1,3-diyl, 2-Buten-1,4-diyl und 1-Penten-1,5-diyl.
Alkindiyl steht für einen geradkettigen oder verzweigten Alkindiylrest mit 2 bis 6 Kohlenstoffatomen. Bevorzugt ist ein geradkettiger oder verzweigter Alkindiylrest mit 2 bis 4, besonders bevorzugt mit 3 oder 4 Kohlenstoffatomen. Beispielhaft und vorzugsweise seien genannt: Propin-1,3-diyl und 2-Butin-1,4-diyl.
Cycloalkyl per se und "Cycloalk" in Cycloalkoxy und Cycloalkandiyl steht für eine Cycloalkylgruppe mit in der Regel 3 bis 8, bevorzugt 5 bis 7 Kohlenstoffatomen, beispielhaft und vorzugsweise für Cyclopropyl, Cyclobutyl, Cyclopentyl, Cyclohexyl und Cycloheptyl.
Cycloalkoxy steht beispielhaft und vorzugsweise für Cyclopropyloxy, Cyclobutyloxy, Cyclopentyloxy, Cyclohexyloxy und Cycloheptyloxy.
Cycloalkandiyl steht beispielhaft und vorzugsweise für Cyclopropan-1,2-diyl, Cyclobutan-1,2-diyl, Cyclobutan-1,3-diyl, Cyclopentan-1,2-diyl, Cyclopentan-1,3-diyl, Cyclohexan-1,2-diyl, Cyclohexan-1,3-diyl, Cyclohexan-1,4-diyl, Cycloheptan-1,2-diyl, Cycloheptan-1,3-diyl und Cycloheptan-1,4-diyl.
Cycloalkenyl per se und "Cycloalken" in Cycloalkendiyl und in Cycloalkenyloxy steht für eine Cycloalkenylgruppe mit in der Regel 5 bis 7 Kohlenstoffatomen; beispielhaft und vorzugsweise für Cyclopent-2-en-1-yl, Cyclopent-3-en-1-yl, Cyclohex-2-en-1-yl, Cyclohex-3-en-1-yl, Cyclohept-2-en-1-yl, Cyclohept-3-en-1-yl und Cyclohept-4-en-1-yl.
Cycloalkendiyl steht beispielhaft und vorzugsweise für Cyclopent-4-en-1,3-diyl, Cyclohex-2-en-1,4-diyl, Cyclohex-4-en-1,3-diyl und Cyclohept-5-en-1,3-diyl.
Cycloalkenyloxy steht beispielhaft und vorzugsweise für Cyclopent-2-en-1-yloxy, Cyclopent-3-en-1-yloxy, Cyclohex-2-en-1-yloxy, Cyclohex-3-en-1-yloxy, Cyclohept-2-en-1-yloxy, Cyclohept-3-en-1-yloxy und Cyclohept-4-en-1-yloxy.
Aryl steht für einen mono- bis tricyclischen aromatischen, carbocyclischen Rest mit in der Regel 6 bis 10 Kohlenstoffatomen; beispielhaft und vorzugsweise für Phenyl und Naphthyl.
Heteroaryl steht für einen aromatischen, mono- oder bicyclischen, gegebenenfalls benzokondensierten, Rest mit in der Regel 5 bis 10, vorzugsweise 5 bis 6 Ringatomen und bis zu 5, vorzugsweise bis zu 4 Heteroatomen aus der Reihe S, 0 und N, beispielhaft und vorzugsweise für Thienyl, Furyl, Pyrrolyl, Thiazolyl, Oxazolyl, Imidazolyl, Pyridyl, Pyrimidyl, Pyridazinyl, Indolyl; Indazolyl, Benzofuranyl, Benzothiophenyl, Chinolinyl, Isochinolinyl, Benzimidazolyl und Benzoxazolyl.
Heterocyclyl steht für einen mono- oder polycyclischen, vorzugsweise mono- oder bicyclischen, gegebenenfalls benzokondensierten, nicht-aromatischen heterocyclischen Rest mit in der Regel 5 bis 10, vorzugsweise 5 bis 8, insbesondere 5 oder 6 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Bevorzugt sind 5- oder 6-gliedrige, monocyclische gesättigte Heterocyclylreste mit bis zu zwei Heteroatomen aus der Reihe O, N und S, die gegebenenfalls benzokondensiert sein können, wie beispielhaft und vorzugsweise Tetrahydrofuran-2-yl, Pyrrolidin-2-yl, Pyrrolidin-3-yl, Pyrrolinyl, Piperidinyl, Morpholinyl, Perhydroazepinyl, 1,3-Benzodioxolyl, Tetrahydro-2H-pyranyl, 1,3-Dioxanyl, 1,4-Dioxanyl, 2,3-Dihydro-1,4-dioxinyl, 2,3-Dihydro-1,4-benzodioxinyl und 4H-1,3-Benzodioxinyl.
Halogen steht für Fluor, Chlor, Brom und Jod.

Wenn Reste in den erfindungsgemäßen Verbindungen substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt. Ganz besonders bevorzugt ist die Substitution mit einem Substituenten.

Bevorzugt sind Verbindungen der Formel (I),
worin
- A: einen 4- bis 6-gliedrigen stickstoffhaltigen gesättigten Heterocyclus, der über das Stickstoffatom an die Ketogruppe gebunden ist,
oder
einen Rest 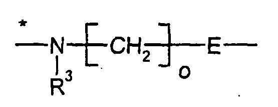 bedeutet,
worin
E für (C₅-C₆)-Cycloalkandiyl steht,
o für 0 oder 1 steht,
R³ für Wasserstoff steht und
* für die Anknüpfstelle an die Ketogruppe steht,
- m: 0 oder 1 bedeutet,
- n: 1, 2 oder 3 bedeutet,
- R¹: Wasserstoff bedeutet,
- R²: Wasserstoff bedeutet,
- X: eine Bindung oder O bedeutet,
- Y: 0 oder *-NH-C(=O)- bedeutet,
worin
* für die Anknüpfstelle an den Phenylring steht
und
- Z: sich in meta oder para Stellung zum Substituenten X befindet und entweder (C₇-C₉)-Alkoxy, das 1 weiteres Sauerstoffatom in der Kette enthalten kann,
oder
einen Rest 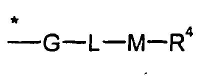 bedeutet,
worin
G für eine Bindung oder O steht,
L für (C₁-C₆)-Alkandiyl oder (C₃-C₆)-Alkendiyl steht,
M für eine Bindung, 0 oder S steht,
R⁴ für Phenyl, Naphthyl, Biphenyl, Phenoxyphenyl, Benzyloxyphenyl, (E)-Phenylvinylphenyl, 2-Phenylethylphenyl, Tetrahydronaphthyl, Benzyl, 1,3-Dioxanyl, 1,4-Dioxanyl, Dimethyl-1,3-dioxanyl, Tetrahydro-2H-pyranyl, (C₃-C₇)-Cycloalkyl oder (C₃-C₇)-Cycloalkymethyl steht, wobei Phenyl, Naphthyl, Biphenyl, Phenoxyphenyl, Benzyloxyphenyl, (E)-Phenylvinylphenyl, 2-Phenylethylphenyl, Tetrahydronaphthyl, Benzyl, Cycloalkyl und Cycloalkylmethyl ihrerseits bis zu dreifach unabhängig voneinander durch Halogen, Cyano, Nitro, Trifluormethyl, Trifluormethoxy, (C₁-C₆)-Alkyl, (C₁-C₆)-Alkoxy, (C₃-C₇)-Cycloalkyl, (C₃-C₇)-Cycloalkylmethoxy oder (C₃-C₇)-Cycloalkoxy substituiert sein können, und
* für die Anknüpfstelle an den Phenylring steht,
und ihre Salze, Hydrate, Hydrate der Salze und Solvate.

Besonders bevorzugt sind Verbindungen der Formel (I), worin
- n: 3 bedeutet.

Ebenfalls besonders bevorzugt sind Verbindungen der Formel (I), worin
- X: eine Bindung bedeutet.

Ebenfalls besonders bevorzugt sind Verbindungen der Formel (I), worin
- Y: O bedeutet.

Ebenfalls besonders bevorzugt sind Verbindungen der Formel (I), worin
- Z: sich in para-Stellung zum Substituenten X befindet.

Ebenfalls besonders bevorzugt sind Verbindungen der Formel (I),
worin

A-[CH₂]ₘ-CO₂R¹

einen Rest 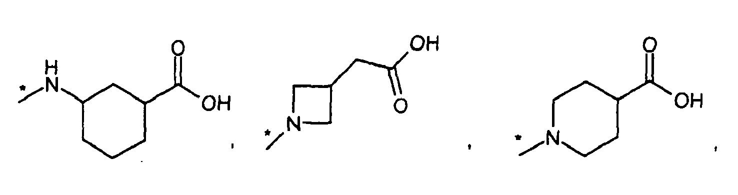 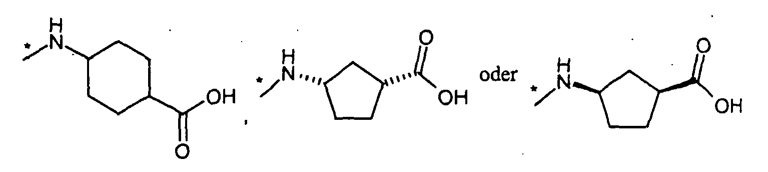 bedeutet,
worin
- *: für die Anknüpfstelle an die Ketogruppe steht,

- n: 3 bedeutet,
- R²: Wasserstoff bedeutet,
- X: eine Bindung bedeutet,
- Y: O bedeutet,
und
- Z: sich in para Stellung zum Substituenten X befindet und entweder n-Octyloxy, n-Heptyloxy,
oder
einen Rest 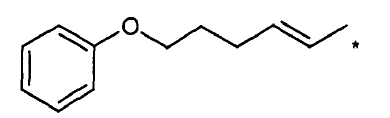 worin
* für die Anknüpfstelle an den Phenylring steht oder
einen Rest 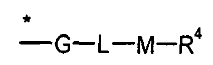 bedeutet,
worin
G für O steht,
L für Methandiyl, n-Propandiyl oder n-Butandiyl steht,
M für eine Bindung oder O steht,
R⁴ für Phenyl, 4-Biphenyl, 4-Phenoxyphenyl, 4-Benzyloxyphenyl, 1,2,3,4-Tetrahydronaphth-6-yl, 5,5-Dimethyl-1,3-dioxan-2-yl oder Cyclohexyl steht, wobei Phenyl seinerseits einmal durch Halogen, Trifluormethoxy, (C₃-C₄)-Alkyl, (C₃-C₄)-Alkoxy, Cyclopentyl, Cyclohexyl oder (C₃-C₆)-Cycloalkylmethoxy substituiert sein kann, und
* für die Anknüpfstelle an den Phenylring steht,
und ihre Salze, Hydrate, Hydrate der Salze und Solvate.

Ganz besonders bevorzugt sind Verbindungen der Formel (I),
worin

A-[CH₂]ₘ-CO₂R¹

einen Rest 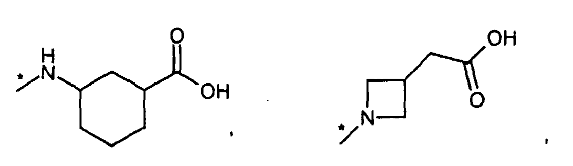 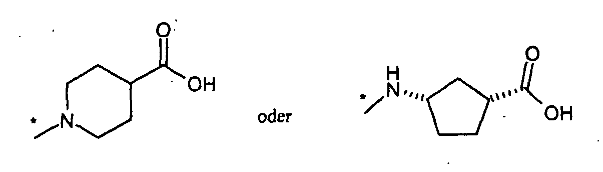 bedeutet,
worin
- *: für die Anknüpfstelle an die Ketogruppe steht,

- n: 3 bedeutet,
- R²: Wasserstoff bedeutet,
- X: eine Bindung bedeutet,
- Y: 0 bedeutet,
und
- Z: sich in para Stellung zum Substituenten X befindet und entweder
n-Octyloxy, n-Heptyloxy,
oder
einen Rest 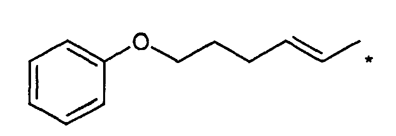 worin
* für die Anknüpfstelle an den Phenylring steht oder
einen Rest

*-O-CH₂-R⁴,

worin
R⁴ für Phenyl, 4-Biphenyl, 4-Phenoxyphenyl, 4-Benzyloxyphenyl oder 1,2,3,4-Tetrahydronaphth-6-yl, wobei Phenyl seinerseits einmal durch Trifluormethoxy, n-Propyl, n-Butyl, tert-Butyl, n-Propyloxy, iso-Propyloxy, iso-Butyloxy, Cyclohexyl oder Cyclopropylmethoxy substituiert sein kann, und
* für die Anknüpfstelle an den Phenylring steht,
oder
einen Rest

*-O-CH₂-CH₂-CH₂-R⁴,

worin
R⁴ für 4-Chlorphenyl, 5,5-Dimethyl-1,3-dioxan-2-yl oder Cyclohexyl steht und
* für die Anknüpfstelle an den Phenylring steht, oder
einen Rest

*-O-CH₂-CH₂-CH₂-CH₂-O-R⁴

bedeutet,
worin
R⁴ für Phenyl oder Cyclohexyl steht und
* für die Anknüpfstelle an den Phenylring steht,
und ihre Salze, Hydrate, Hydrate der Salze und Solvate.

Insbesondere bevorzugt sind Verbindungen der Formel (I),
worin

A-[CH₂]ₘ-CO₂R¹

einen Rest 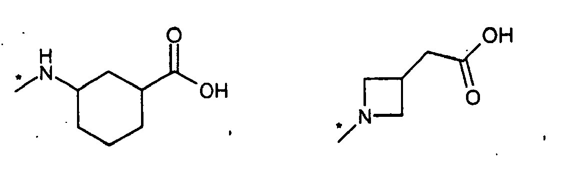 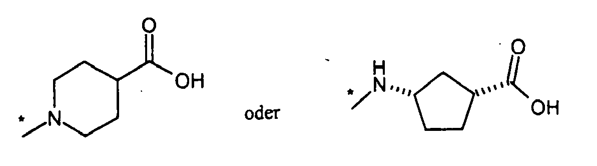 bedeutet,
worin
- *: für die Anknüpfstelle an die Ketogruppe steht,

- n: 3 bedeutet,
- R²: Wasserstoff bedeutet,
- X: eine Bindung bedeutet,
- Y: O bedeutet,
und
- Z: sich in para Stellung zum Substituenten X befindet und einen Rest 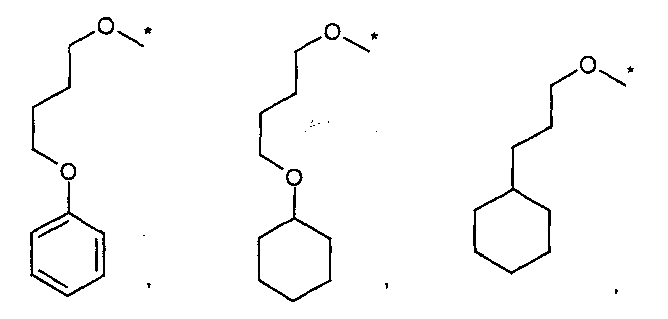 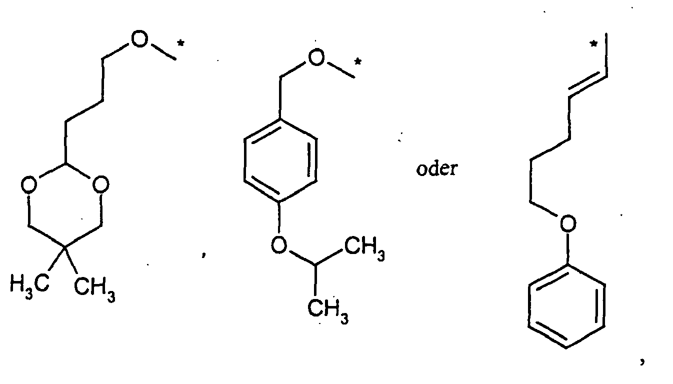 bedeutet,
worin
* für die Anknüpfstelle an den Phenylring steht,
und ihre Salze, Hydrate, Hydrate der Salze und Solvate.

Insbesondere ganz besonders bevorzugt sind die folgenden Verbindungen der Formel (I):
3-{[(3-Carboxycyclohexyl)amino]carbonyl}-4-{3-[4-(4-phenoxybutoxy)phenyl]-propoxy}benzoesäure,
3-{[(3-Carboxycyclohexyl)amino]carbonyl}-4-{3-[4-(3-cyclohexylpropoxy)phenyl]-propoxy}benzoesäure,
3-{[(3-Carboxycyclohexyl)amino]carbonyl}-4-(3-{4-[4-(cyclohexyloxy)butoxy]-phenyl}propoxy)benzoesäure,
1-(5-Carboxy-2-{3-[4-(3-cyclohexylpropoxy)phenyl]propoxy}benzoyl)piperidin-4-carbonsäure,
3-{[(3-Carboxycyclohexyl)amino]carbonyl}-4-(3-{4-[(4-isopropoxybenzyl)oxy]-phenyl}propoxy)benzoesäure,
3-{[3-(Carboxymethyl)azetidin-1-yl]carbonyl}-4-{3-[4-(3-cyclohexylpropoxy)-phenyl]- propoxy}benzoesäure,
3-{[(3-Carboxycyclohexyl)amino]carbonyl}-4-(3-{4-[(1E)-5-phenoxypent-1-en-1-yl]-phenyl}propoxy)benzoesäure.

Ebenfalls bevorzugt sind Verbindungen der Formel (I),
worin
- A: einen 4- bis 7-gliedrigen stickstoffhaltigen gesättigten Heterocyclus, der über das Stickstoffatom an die Ketogruppe gebunden ist, der ein weiteres Stickstoffatom im Ring enthalten kann und der gegebenenfalls in Nachbarschaft zu einem Stickstoffatom eine Carbonylgruppe trägt,
oder
einen Rest 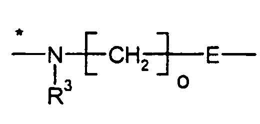 bedeutet,
worin
E für (C₃-C₇)-Cycloalkandiyl, (C₅-C₇)-Cycloalkendiyl oder für 5- bis 10-gliedriges Heterocyclyl, das über ein Kohlenstoffatom an die [CH₂]ₒ-Gruppe gebunden ist, steht,
o für 0, 1 oder 2 steht,
R³ für Wasserstoff oder (C₁-C₆)-Alkyl steht und
* für die Anknüpfstelle an die Ketogruppe steht,
- m: 0, 1 oder 2 bedeutet,
- n: 1, 2, 3 oder 4 bedeutet,
- R¹: Wasserstoff oder (C₁-C₆)-Alkyl bedeutet,
- R²: Wasserstoff oder (C₁-C₆)-Alkyl bedeutet,
- X: eine Bindung, O, NH, N-Methyl oder N-Acetyl bedeutet,
- Y: O, *-NH-C(=O)- oder NH bedeutet,
worin
* für die Anknüpfstelle an den Phenylring steht und
- Z: einen Rest 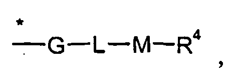 der sich in meta oder para Stellung zum Substituenten X befindet, bedeutet,
worin
G für eine Bindung, O oder S steht,
L für (C₁-C₆)-Alkandiyl, (C₃-C₆)-Alkendiyl oder (C₃-C₆)-Alkindiyl steht,
M für eine Bindung, O oder S steht,
R⁴ für (C₆-C₁₀)-Aryl, Biphenyl, Heteroaryl, 5- bis 10-gliedriges Heterocyclyl oder (C₃-C₇)-Cycloalkyl steht, wobei Aryl, Biphenyl, Heteroaryl, Heterocyclyl und Cycloalkyl ihrerseits bis zu dreifach unabhängig voneinander durch Halogen, Cyano, Nitro, Trifluormethyl, Trifluormethoxy, (C₁-C₆)-Alkyl, (C₁-C₆)-Alkoxy, (C₂-C₆)-Alkenyl, (C₃-C₇)-Cycloalkyl, (C₅-C₇)-Cycloalkenyl, (C₃-C₇)-Cycloalkoxy oder (C₅-C₇)-Cycloalkenyloxy substituiert sein können,
* für die Anknüpfstelle an den Phenylring steht,
und ihre Salze, Hydrate, Hydrate der Salze und Solvate.

Ebenfalls bevorzugt sind Verbindungen der Formel (I),
worin
- A: einen Rest 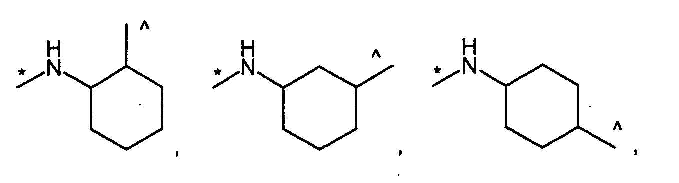 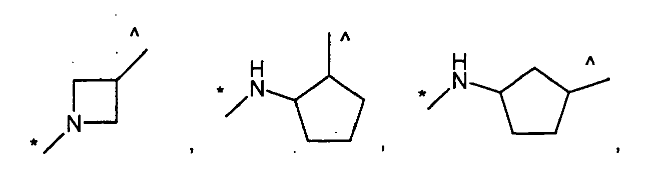 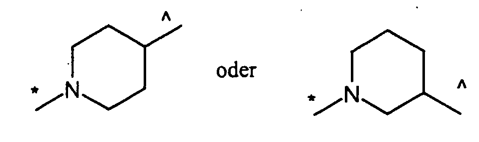 bedeutet,
worin
* für die Anknüpfstelle an die Ketogruppe steht und
^ für die Anknüpfstelle an die 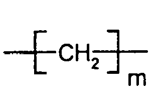 -Gruppe steht,
- m: 0 oder 1 bedeutet,
- n: 2 oder 3 bedeutet,
- R¹: Wasserstoff bedeutet,
- R²: Wasserstoff bedeutet,
- X: eine Bindung bedeutet,
- Y: O oder *-NH-C(=O)- bedeutet,
worin
* für die Anknüpfstelle an den Phenylring steht und
- Z: einen Rest 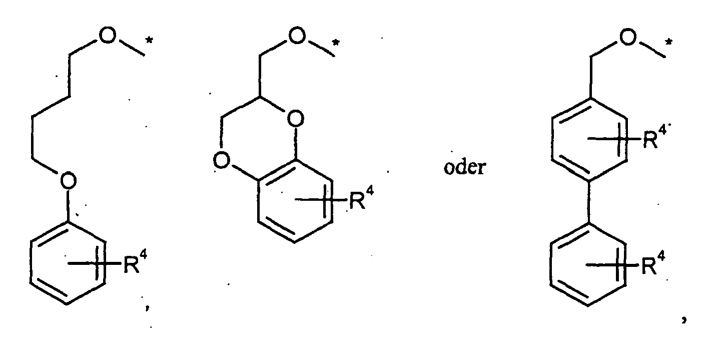 der sich in meta oder para Stellung zum Substituenten X befindet, bedeutet,
worin
R⁴ und R^{4'} unabhängig voneinander für Fluor, Chlor, Brom, Trifluormethyl, Trifluormethoxy, (C₁-C₆)-Alkyl oder (C₁-C₆)-Alkoxy stehen und
* für die Anknüpfstelle an den Phenylring steht,
und ihre Salze, Hydrate, Hydrate der Salze und Solvate.

Ganz besonders bevorzugt sind Kombinationen von zwei oder mehreren der oben genannten Vorzugsbereiche.

Gegenstand der vorliegenden Erfindung ist auch ein Verfahren zur Herstellung der Verbindungen der Formel (I), das dadurch gekennzeichnet ist, dass man
entweder
[**A**] Verbindungen der Formel (II) 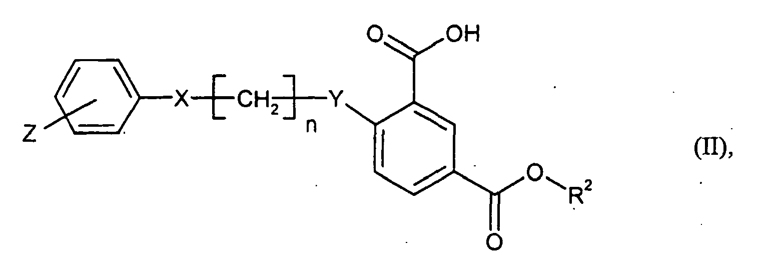 worin
   - R²: für (C₁-C₆)-Alkyl steht und
   - n, X, Y und Z: die oben angegebene Bedeutung haben,
   mit Verbindungen der Formel (III) 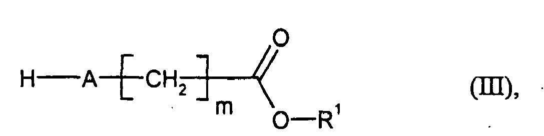 worin
   - R¹: für (C₁-C₆)-Alkyl steht und
   - m und A: die oben angegebene Bedeutung haben,
   oder
[**B1**] Verbindungen der Formel (IVa) 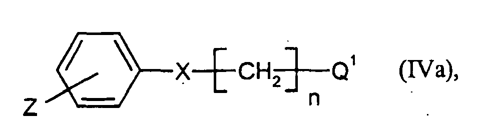 worin
   - Q¹: für eine geeignete Abgangsgruppe wie beispielsweise Halogen, Mesylat oder Tosylat steht und
   - n, X und Z: die oben angegebene Bedeutung haben,
   mit Verbindungen der Formel (Va) 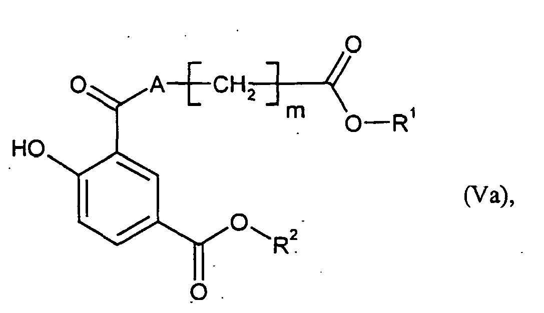 worin
   - R¹ und R²: für (C₁-C₆)-Akyl stehen und
   - A und m: die oben angegebene Bedeutung haben,
   oder
[**B2**] Verbindungen der Formel (IVb) 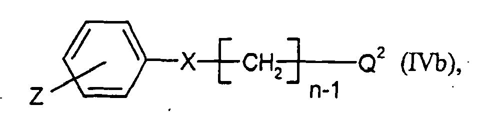 worin
   - Q²: für eine Säurechloridgruppe steht und
   - n, X und Z: die oben angegebene Bedeutung haben,
   mit Verbindungen der Formel (Vb) 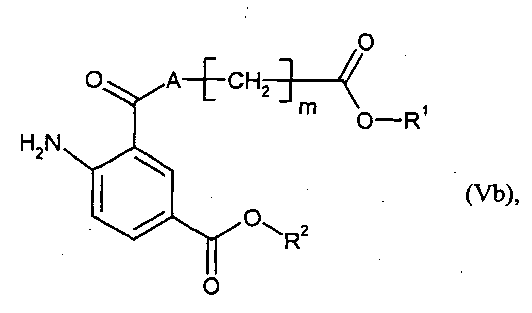 worin
   - R¹ und R²: für (C₁-C₆)-Alkyl stehen und
   - A und m: die oben angegebene Bedeutung haben,
   oder
[**B3**] Verbindungen der Formel (IVa) 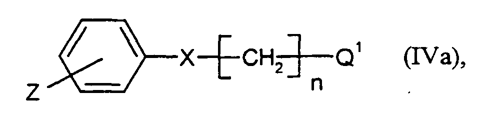 worin
   - Q¹: für eine geeignete Abgangsgruppe wie beispielsweise Halogen, Mesylat oder Tosylat steht und
   - n, X und Z: die oben angegebene Bedeutung haben,
   mit Verbindungen der Formel (Vb) 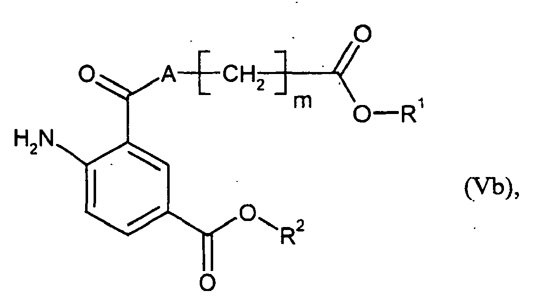 worin
   - R¹ und R²: für (C₁-C₆)-Alkyl stehen und
   - A und m: die oben angegebene Bedeutung haben,
   oder
[**C**] Verbindungen der Formel (XII) 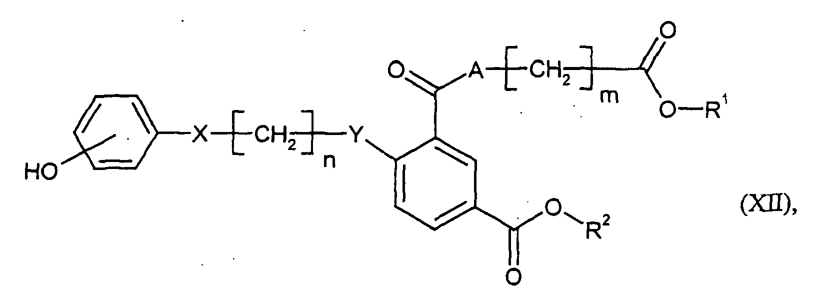 worin
   - R¹ und R²: für (C₁-C₆)-Alkyl stehen und
   - n, m, X, Y und A: die oben angegebene Bedeutung haben,
   mit Verbindungen der Formel (XIII)

   R⁴-M-L-Q³ (XIII),

   worin
   - Q³: für eine geeignete Abgangsgruppe wie beispielsweise Halogen, bevorzugt Brom, Chlor oder Iod, oder Mesylat oder Tosylat steht und
   - R⁴, M und L: die oben angegebene Bedeutung haben,
   umsetzt
   oder
[**D**] in Verbindungen, die gemäß Verfahrensschritt [A], [B1], [B2], [B3] oder [C] hergestellt werden, die beiden Estergruppen verseift.

Das erfindungsgemäße Verfahren kann durch folgendes Formelschema beispielhaft erläutert werden:

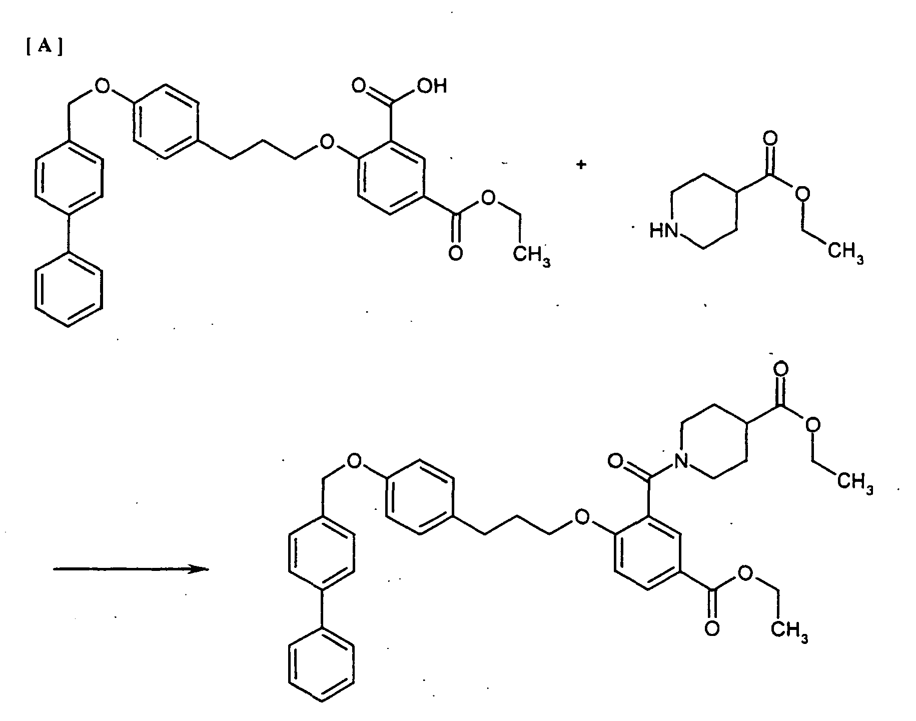

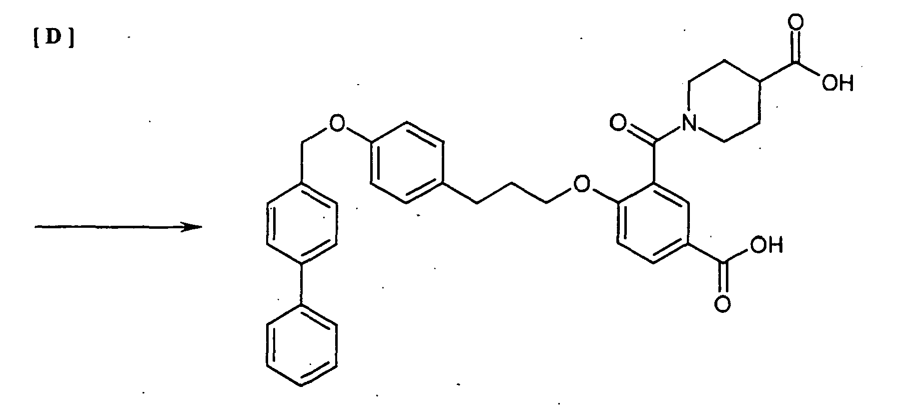

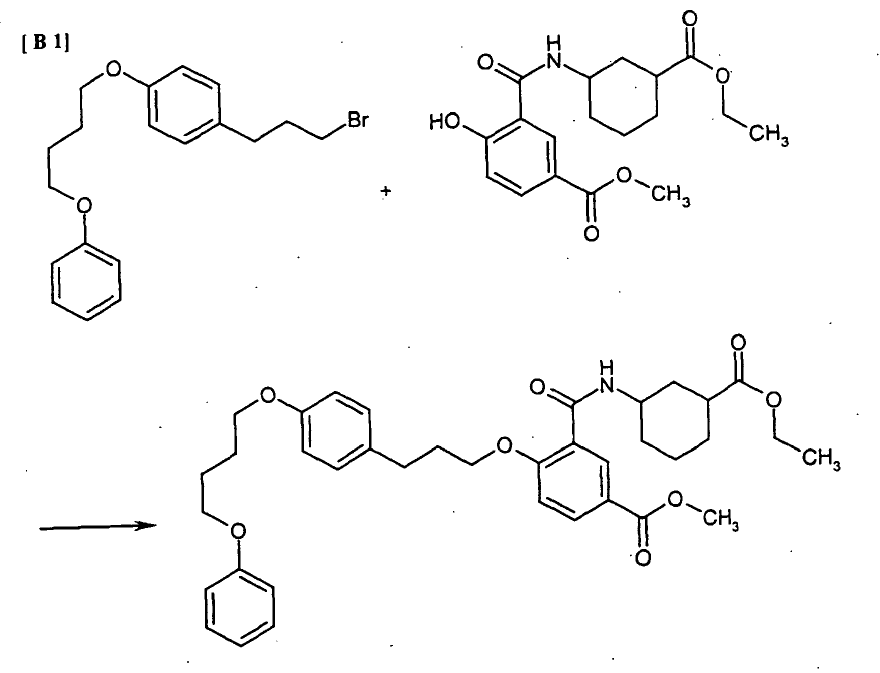

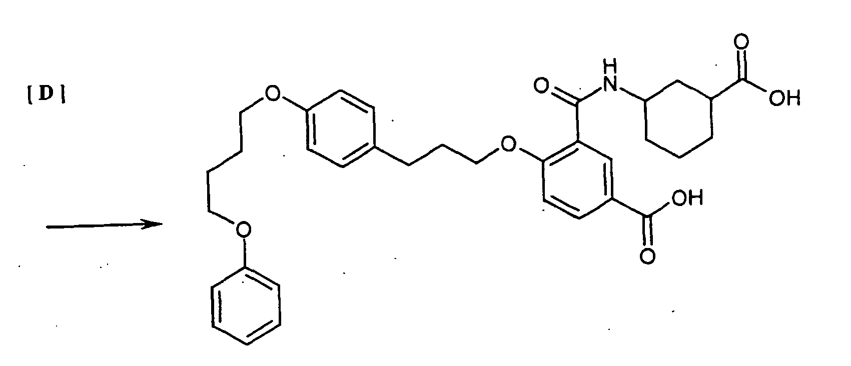

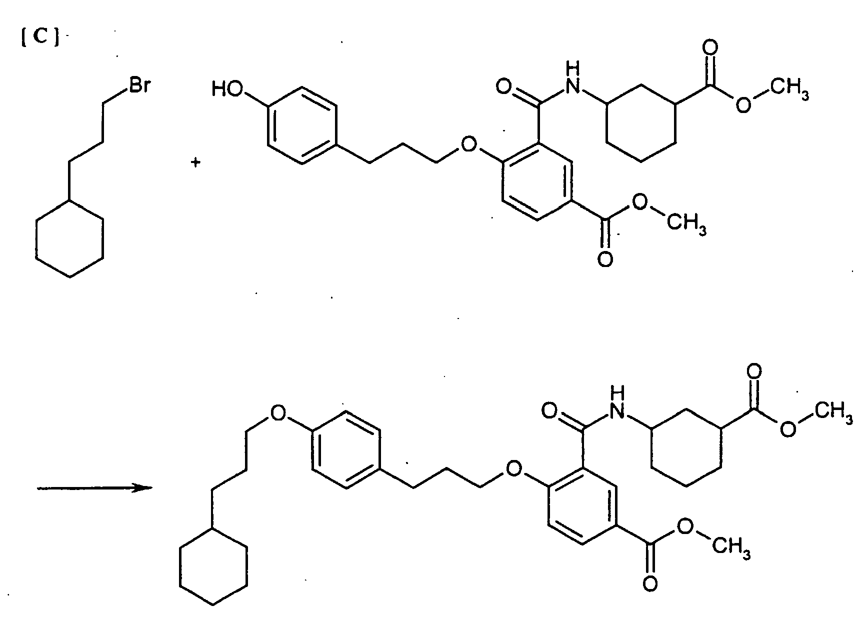

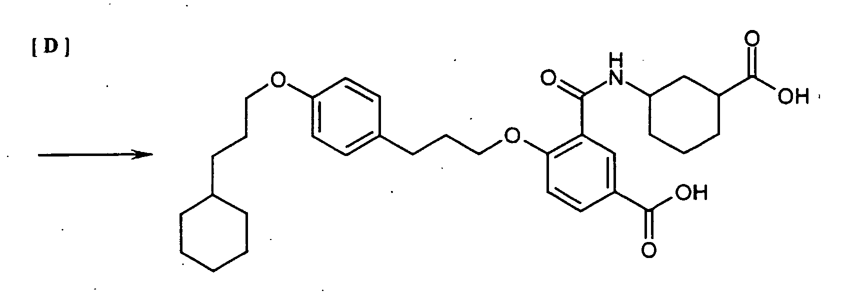

Verbindungen der Formel (II) können beispielsweise hergestellt werden, indem man Verbindungen der Formel (IVa) mit Verbindungen der Formel (VI) 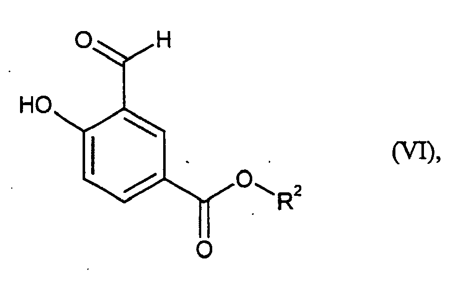 worin
- R²: für (C₁-C₆)-Alkyl steht,
zu Verbindungen der Formel (VII) 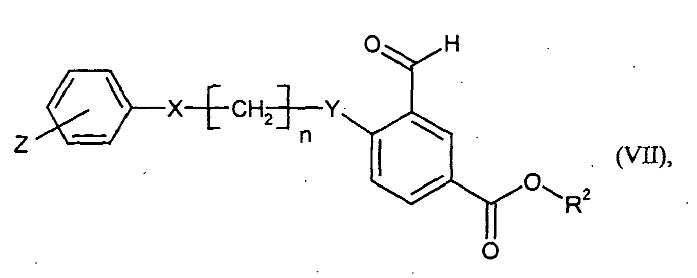 worin
- R²: für (C₁-C₆)-Alkyl steht,
- Y: O bedeutet und
- n, X und Z: die oben angegebene Bedeutung haben,
umsetzt und anschließend die Aldehydgruppe oxidiert.

Verbindungen der Formel (IVa) können beispielsweise hergestellt werden, indem man Verbindungen der Formel (VIII) 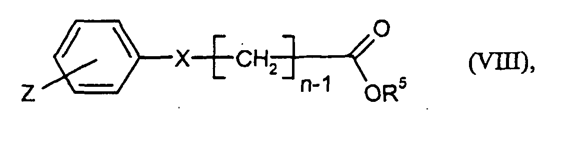 worin
- R⁵: für Wasserstoff oder einen Alkylrest steht und
- n, X und Z: die oben angegebene Bedeutung haben,
durch Reduktion der Carbonsäure- bzw. der Estergruppe in die entsprechenden Alkohole der Formel (IX) 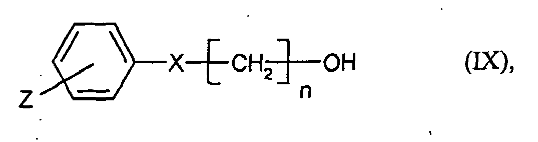 worin
n, X und Z die oben angegebene Bedeutung haben,
überführt und abschließend die Hydroxygruppe in eine Abgangsgruppe wie beispielsweise Halogen, Mesylat oder Tosylat umwandelt.

Verbindungen der Formel (IVb) können beispielsweise aus Verbindungen der Formel (VIII) hergestellt werden, indem man für den Fall, dass R⁵ in Verbindungen der Formel (VIII) für Wasserstoff steht, die Carbonsäuregruppe in das entsprechende Säurechlorid überführt oder im Fall von Verbindungen der Formel (VIII), in denen R⁵ für eine Alkylgruppe steht, in einem vorgeschalteten Schritt die entsprechende Estergruppe zunächst zur Carbonsäuregruppe verseift.

Verbindungen der Formel (Va) können beispielsweise hergestellt werden, indem man Verbindungen der Formel (III) mit Verbindungen der Formel (X) 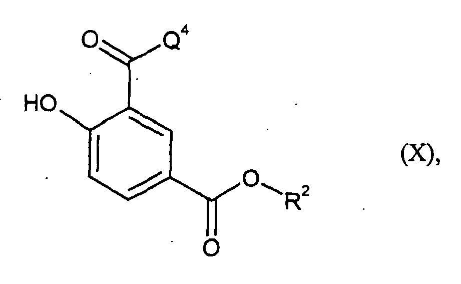 worin
- Q⁴: für Hydroxy oder Chlor steht und
- R²: für (C₁-C₆)-Alkyl steht,
umsetzt.

Verbindungen der Formel (Vb) können beispielsweise hergestellt werden, indem man in einer ersten Amidkupplungsreaktion Verbindungen der Formel (III) mit Verbindungen der Formel (XI) 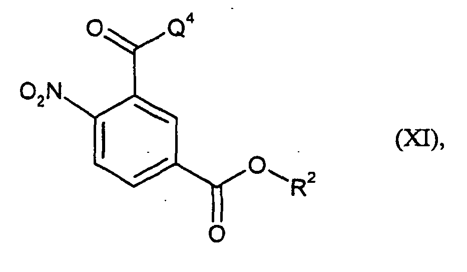 worin
- Q⁴: für Hydroxy oder Chlor steht und
- R²: für (C₁-C₆)-Alkyl steht,
umsetzt und abschließend die Nitrogruppe zur entsprechenden Aminogruppe reduziert.

Verbindungen der Formel (VIIIa), entsprechend Verbindungen der Formel (VIII), in denen X für ein Sauerstoffatom und R⁵ für einen Alkylrest steht, können beispielsweise hergestellt werden, indem man Verbindungen der Formel (XIV) 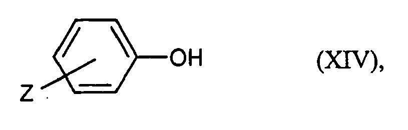 worin
- Z: die oben angegebene Bedeutung hat,
mit Verbindungen der Formel (XV) 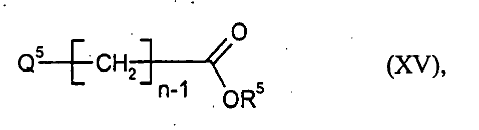 worin
- Q⁵: für eine geeignete Abgangsgruppe wie beispielsweise Halogen, Mesylat oder Tosylat steht und
- R⁵: für einen Alkylrest steht,
umsetzt.

Verbindungen der Formel (VIIIb), entsprechend Verbindungen der Formel (VIII), in denen X für eine Bindung oder -CH=CH-, Z für *-O-L-M-R⁴ und R⁵ für einen Alkylrest steht, können beispielsweise hergestellt werden, indem man Verbindungen der Formel (XIII) mit Verbindungen der Formel (XVI) 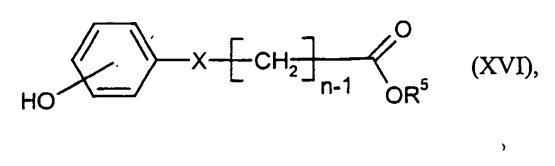 worin
- R⁵: für einen Alkylrest steht,
- X: für eine Bindung oder -CH=CH- steht, und
- n: die oben angegebene Bedeutung hat,
umsetzt.

Verbindungen der Formel (VIIIc), entsprechend Verbindungen der Formel (VIII), in denen X für eine Bindung, Z für *-L-M-R⁴ und R⁵ für einen Alkylrest steht, können beispielsweise hergestellt werden, indem man Verbindungen der Formel (XVII)

R⁴-M-L-PPh₃⁺Br⁻ (XVII),

worin
- R⁴, M und L: die oben angegebene Bedeutung haben,
mit Verbindungen der Formel (XVIII) 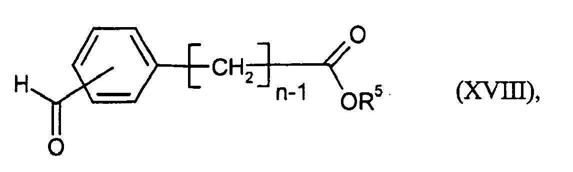 worin
- R⁵: für einen Alkylrest steht und
- n: die oben angegebene Bedeutung hat,
umsetzt.

Verbindungen der Formel (IXa), entsprechend Verbindungen der Formel (IX), in denen X für -C≡C- und n für 1 steht, können beispielsweise hergestellt werden, indem man Verbindungen der Formel (XIX) 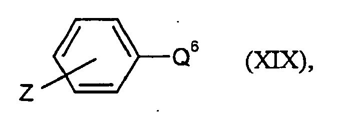 worin
- Q⁶: für eine geeignete Abgangsgruppe wie beispielsweise Brom, Iod oder Trifluormethansulfonat steht und
- Z: die oben angegebene Bedeutung hat,
mit Propargylalkohol umsetzt.

Verbindungen der Formel (XII) können beispielsweise hergestellt werden, indem man Verbindungen der Formel (Va) mit Verbindungen der Formel (XX) 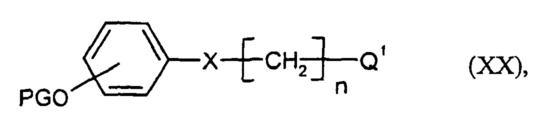 worin
- Q¹: für eine geeignete Abgangsgruppe wie beispielsweise Halogen, Mesylat oder Tosylat steht,
- PG: für eine geeignete Schutzgruppe wie beispielsweise eine Silyl-Schutzgruppe steht und
- n und X: die oben angegebene Bedeutung haben,
umsetzt und anschließend die Schutzgruppe nach üblichen, dem Fachmann bekannten Methoden abspaltet.

Verbindungen der Formel (XX) können beispielsweise hergestellt werden, indem man Verbindungen der Formel (XVI) mit üblichen, dem Fachmann bekannten Schutzgruppenreagenzien, wie beispielsweise Triisopropylsilylchlorid, umsetzt und anschließend die Carbonsäure- bzw. der Estergruppe in die Abgangsgruppe Q¹, wie für Verbindungen der Formel (IVa) beschrieben, überführt.

Verbindungen der Formel (I), in denen R¹ Wasserstoff bedeutet, können statt aus den entsprechenden Alkylestern der Verbindungen der Formeln (III), (Va) oder (Vb) (R¹ = Alkyl) auch aus den entsprechenden Benzylestem (R¹ = Benzyl) hergestellt werden.

Bei den Amidkupplungen der Verfahrensschritte (II) + (III) -> (I); (III) + (X) -> (Va) (für Q⁴ = Hydroxy) sowie des ersten Teilschritts (III) + (XI) -> (Vb) (für Q⁴ = Hydroxy) werden die Amine vorzugsweise in Form ihrer Hydrochloride eingesetzt.

Die Umsetzungen erfolgen bevorzugt unter Standardbedingungen in Gegenwart von allgemein üblichen Reagenzien zur Amid- bzw. Peptidkupplung wie beispielsweise N-[(3-Dimethylamino)propyl]-N'-ethylcarbodiimid-Hydrochlorid (EDC) und 1-Hydroxy-1H-benzotriazol-Hydrat (HOBT) in Gegenwart von Hilfsbasen wie Triethylamin oder Diisopropylethylamin, in Lösemitteln wie Dichlormethan oder Dimethylformamid bei Raumtemperatur.

Bei den Amidkupplungen der Verfahrensschritte (III) + (X) -> (Va) (für Q⁴ = Chlor) sowie des ersten Teilschritts (III) + (XI) -> (Vb) (für Q⁴ gleich Chlor) werden die Amine vorzugsweise in Form ihrer Hydrochloride eingesetzt. Die Umsetzungen erfolgen bevorzugt unter Standardbedingungen in Gegenwart von Hilfsbasen wie Triethylamin oder Diisopropylethylamin, in Lösemitteln wie Diethylether, Tetrahydrofuran oder Methylenchlorid bei Temperaturen zwischen 0°C und Raumtemperatur.

Die Esterverseifung bei der Herstellung von Verbindungen (I) nach Verfahren [D], bei denen R¹ und R² für Wasserstoff stehen, sowie beim ersten Teilschritt der Reaktion (VIII) -> (IVb) erfolgt vorzugsweise in Gegenwart wässriger AlkalihydroxidLösung wie zum Beispiel 2-molarer Natronlauge bei Temperaturen zwischen Raumtemperatur und 70°C unter Zusatz von mit Wasser mischbaren organischen Lösemitteln wie zum Beispiel Methanol oder Tetrahydrofuran oder Gemischen daraus.

Die Verfahrensschritte (IVa) + (Va) -> (I); (IVa) + (Vb) -> (I); (IVa) + (VI) -> (VII); (XII) + (XIII) -> (I); (XIV) + (XV) -> (VIIIa); (XIII) + (XVI) -> (VIIIb) sowie (Va) + (XX) -> (XII) erfolgen vorzugsweise in inerten Lösemitteln wie beispielsweise Tetrahydrofuran, Dioxan, Dimethylformamid, N-Methylpyrrolidon, Acetonitril, Aceton oder Butyronitril, in Gegenwart von Hilfsbasen wie beispielsweise Kaliumcarbonat, Natriumcarbonat, Caesiumcarbonat, Triethylamin, Ethyldiisopropylamin oder Pyridin in einem Temperaturbereich zwischen Raumtemperatur und dem Siedepunkt des jeweiligen Lösemittels.

Alternativ kann die Herstellung von Verbindung (VII) auch durch Umsetzung des Alkohols (IX) mit Verbindung (VI) unter Bedingungen der Mitsunobu-Reaktion erfolgen. Als Lösemittel eignen sich dafür besonders Ether wie zum Beispiel Tetrahydrofuran oder Chlorkohlenwasserstoffe wie zum Beispiel Dichlormethan. Die Reaktion erfolgt in Gegenwart von Triphenylphosphin und Azoverbindungen wie zum Beipiel Azodiacarbonsäure-diethylester (DEAD) oder Azodicarbonsäure-diisopropylester (DIAD). Der bevorzugte Temperaturbereich für die Durchführung der Reaktion liegt zwischen -10°C und Raumtemperatur.

Der Verfahrensschritt (IVb) + (Vb) -> (I) erfolgt vorzugsweise in einem inerten Lösemittel wie beispielsweise in Ether, Tetrahydrofuran, Dichlormethan oder Chloroform in Gegenwart einer Hilfsbasen wie beispielsweise Triethylamin, Diisopropylethylamin oder Pyridin bei Temperaturen zwischen 0°C und Raumtemperatur.

Der Verfahrensschritt (VII) -> (II) erfolgt unter üblichen, dem Fachmann für die Oxidation von Aldehyd- zu Carbonsäuregruppen bekannten Bedingungen. Gut geeignet ist dabei beispielsweise die Oxidation mit Natriumchlorit in Gegenwart von Wasserstoffperoxid und Natriumdihydrogenphosphat oder in Gegenwart von Amidoschwefelsäure in einem Lösemittelgemisch aus Wasser mit Acetonitril oder Tetrahydrofuran oder Dioxan im Temperaturbereich zwischen 0°C und Raumtemperatur.

Der Verfahrensschritt (VIII) -> (IX) erfolgt unter üblichen, dem Fachmann für die Reduktion von Carbonsäure- bzw. Estergruppen zu den entsprechenden Alkoholgruppen bekannten Bedingungen, wie beispielsweise mit komplexen Metallhydriden wie Lithiumaluminiumhydrid in inerten Lösemitteln wie zum Beispiel Tetrahydrofuran im Temperaturbereich zwischen 0°C und dem Siedepunkt des jeweiligen Lösemittels.

Die Umwandlung der Alkoholfunktion in eine Abgangsgruppe Q¹ im Verfahrensschritt (IX) -> (IVa) kann auf vielfältige, dem Fachmann bekannte Art und Weise erfolgen. Vorzugsweise erfolgt die Reaktion zum entsprechenden Bromid in Tetrahydrofuran als Lösemittel bei Raumtemperatur mit einem Gemisch aus Triphenylphosphin und Tetrabrommethan oder in Dichlormethan als Lösemittel im Temperaturbereich zwischen 0°C und Raumtemperatur mit Phosphortribromid gegebenenfalls in Gegenwart von Pyridin. Vorzugsweise erfolgt die Reaktion zum entsprechenden Mesylat oder Tosylat in Dichlormethan als Lösemittel im Temperaturbereich zwischen 0°C und Raumtemperatur mit Methan-sulfonsäurechlorid bzw. para-Toluolsulfonsäurechlorid in Gegenwart von tertiären Aminen wie beispielsweise Triethylamin oder Diisopropylethylamin.

Die im ersten Teilschritt der Reaktion (VIII) -> (IVb) erhaltene Carbonsäuregruppe wird durch Chlorierung in das entsprechende Säurechlorid überführt. Bevorzugte Chlorierungsreagenzien sind Thionylchlorid oder Oxalylchlorid. Die Umsetzung erfolgt gegebenenfalls in Gegenwart katalytischer Mengen Dimethylformamid, wobei als Lösemittel halogenierte Kohlenwasserstoffe wie zum Beispiel Dichlormethan oder Chloroform zugesetzt werden können. Die Reaktionstemperatur liegt dabei zwischen 0°C und dem Siedepunkt des jeweiligen Lösemittels oder Chlorierungsreagenzes.

Die Reduktion der Nitrogruppe im zweiten Teilschritt der Reaktion (III) + (XI) -> (Vb) kann beispielsweise mit Zinn(II)-chlorid erfolgen. Bevorzugt ist die Reduktion mit Wasserstoff an Edelmetallkatalysatoren, wie zum Beispiel Palladium auf Kohle als Trägermaterial, bei einem Wasserstoffdruck von einem bis vier bar und Raumtemperatur in Methanol, Ethanol oder Essigsäureethylester als Lösemittel.

Der Verfahrensschritt (XVII) + (XVIII) -> (VIIIc) erfolgt unter üblichen, dem Fachmann für die Wittig-Reaktion bekannten Bedingungen. Die Reaktion erfolgt vorzugsweise in einem inerten Lösemittel wie beispielsweise in Hexan, Diethylether, Toluol oder Tetrahydrofuran, besonders bevorzugt in Tetrahydrofuran, in Gegenwart einer starken Basen wie beispielsweise n-Butyllithium oder Natrium-bis-(trimethylsilyl)amid bei Temperaturen zwischen -50°C und Raumtemperatur.

Der Verfahrensschritt (XIX) -> (IXa) erfolgt vorzugsweise in Triethylamin als Lösemittel in Gegenwart von Kupfer(I)iodid, einem Palladium(II)-Salz wie beispielsweise Dichlorbis(triphenylphosphin)-palladium ([Pd(PPh₃)₂]Cl₂) und Triphenylphosphin bei Temperaturen zwischen 50°C und 70°C.

Die Verbindungen der Formel (III), (VI), (VIII), (X), (XI), (XIII), (XIV), (XV), (XVI), (XVII), (XVIII), (XIX) und (XX) sind dem Fachmann an sich bekannt oder lassen sich nach üblichen literaturbekannten Verfahren herstellen.

Darüber hinaus können Verbindungen der Formel (I),
worin.
- A: 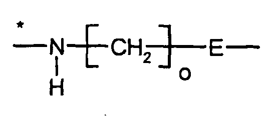 bedeutet,
- R¹: Wasserstoff bedeutet,
- R²: Wasserstoff bedeutet,
- n: 3 bedeutet,
- X: eine Bindung bedeutet,
- Y: O bedeutet,
- Z: sich in para Stellung zum Substituenten X befindet,
- G: O bedeutet und
- E, L, M, R⁴, m und o: die oben angegebene Bedeutung haben
auch mit Hilfe der Festphasensynthese hergestellt werden:

An eine Festphase aus der Gruppe von mit zwischen 1 und 30 % Divinylbenzol (DVB) quervernetztem micro- oder macroporösem Polystyrol (PS), Polystyrol/Polyethylenglycol (PS/PEG) Propf- oder Blockcopolymeren und funktionalisierten Glasoberflächen (controlled pore glass, CPG) werden über eine an der Festphase vorhandene Benzaldehydfunktionalität primäre Amine als Imin angebunden und anschließend zum sekundären Amin reduziert. Vorzugsweise wird hierzu 4-(4-Formyl-3-methoxyphenoxy)butyrylaminomethylharz, basierend auf mit 2 % DVB quervernetztem PS ("Pol-CHO", Nova Biochem), mit einem zwei- bis fünffachen Überschuss einer an der Säurefunktionalität *t*-Butyl oder *t*-Hexyl geschützten cyclischen oder acyclischen β-, γ- oder δ-Aminosäure vorzugsweise als deren Hydrochloride in Dimethylformamid, N-Methylpyrrolidon, Dichlormethan, Dioxan Tetrahydrofuran, Toluol oder anderen geeigneten Lösungsmitteln mit oder ohne wasserentziehende Mittel wie Trimethylorthoformiat, Natriumsulfat oder Magnesiumsulfat, gegebenenfalls in Gegenwart von Hilfsbasen wie Kaliumcarbonat, Natriumcarbonat, Triethylamin, Ethyldiisopropylamin und Pyridin bei Temperaturen zwischen Raumtempertur und 50°C für 2 bis 24 Stunden umgesetzt (Reaktion a):

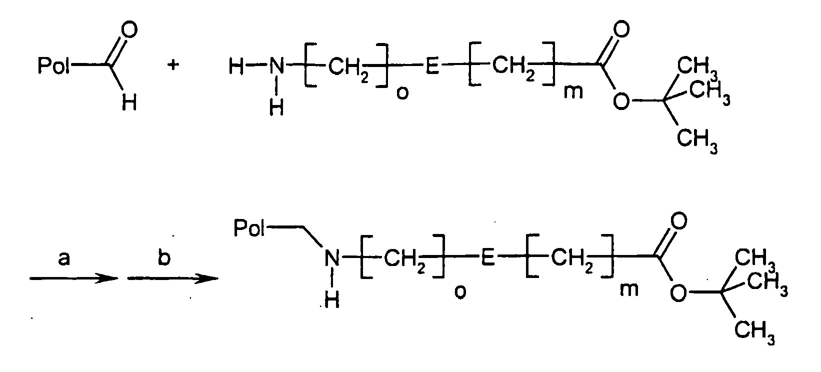

Anschließend wird die Festphase entweder ein oder mehrfach mit verschiedenen Lösungsmitteln wie Dimethylformamid, N-Methylpyrrolidon, Dichlormethan, Dioxan Tetrahydrofuran, Toluol, Acetonitril oder Methanol gewaschen oder direkt weiter in der anschließenden Reduktion umgesetzt. Hierzu wird die Festphase mit einem zwei- bis zehnfachen Überschuss an Reduktionsmitteln wie Natriumborhydrid, Natriumcyanoborhydrid, Natriumtriacetoxyborhydrid oder Tetrabutylammoniumborhydrid in Lösungsmitteln wie Dichlormethan, Dimethylformamid, Tetrahydrofuran oder Methanol bzw. auch Gemischen dieser bei Temperaturen zwischen -78°C und Raumtemperatur gegebenenfalls unter Zugabe von bis zu 100 Äquivalenten Essigsäure für 0,5 bis 18 Stunden umgesetzt (Reaktion b).

Nach Waschen und Trocknen der Festphase nach üblichen, dem Fachmann bekannten Verfahren wird diese nun mit 2-Hydroxy-5-methoxycarbonylbenzoesäure zur Reaktion gebracht. Dazu werden die dem Fachmann bekannten Reagenzien zur Amid- bzw. Peptidkupplung eingesetzt. Beispielsweise handelt es sich dabei um N-[(3-Dimethylamino)propyl]-N'-ethylcarbodiimid-Hydrochlorid (EDC) und 1-Hydroxy-1H-benzotriazol-Hydrat (HOBT), die in Gegenwart von Hilfsbasen wie Triethylamin oder Diisopropylethylamin in Lösemitteln wie Dichlormethan oder Dimethylformamid bei Raumtemperatur eingesetzt werden (Reaktion c).

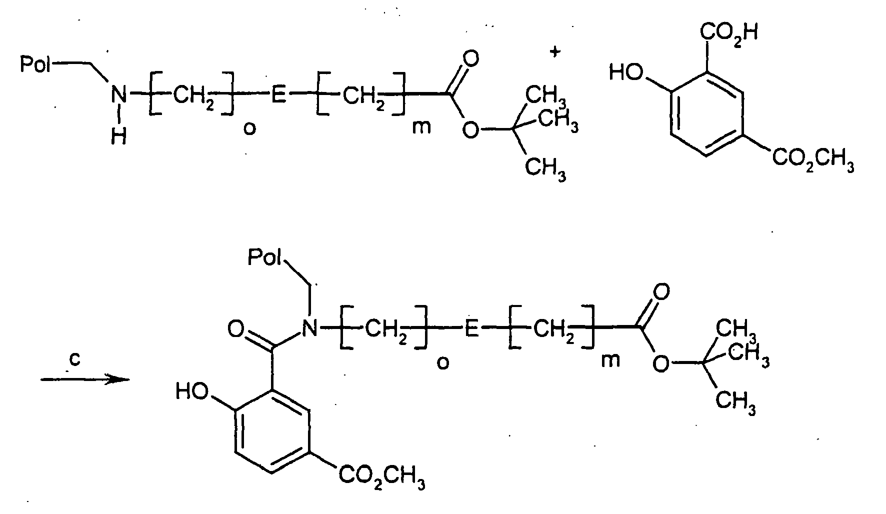

Nach Waschen und Trocknen der Festphase nach üblichen, dem Fachmann bekannten Verfahren wird diese nun mit 1 bis 5 Äquivalenten 3-Brom-(4-*tert*.-butyldimethylsilyloxyphenyl)-propan oder 3-Brom-(4-tri-*iso*-propylsilyloxyphenyl)-propan und einer Base aus der Gruppe von Natriumcarbonat, Natriumhydrid, Kaliumcarbonat und Caesiumcarbonat in polaren aprotischen Lösungsmitteln wie Tetrahydrofuran, Dioxan, Dimethylformamid, N-Methylpyrrolidon gegebenenfalls unter Zugabe von 18-Krone-6 oder Tetrabutylamoniumiodid bei Temperaturen zwischen Raumtemperatur und dem Siedepunkt des entsprechenden Lösungsmittels für 1-24 Stunden umgesetzt (Reaktion d). Nach Waschen und Trocknen der Festphase nach üblichen, dem Fachmann bekannten Verfahren wird diese nun mit 2 bis 10 Äquivalenten Tetrabutylammoniumfluorid in Tetrahydrofuran bei Raumtemperatur für 2-24 Stunden zur Reaktion gebracht und so die Silylschutzgruppe abgespalten (Reaktion e).

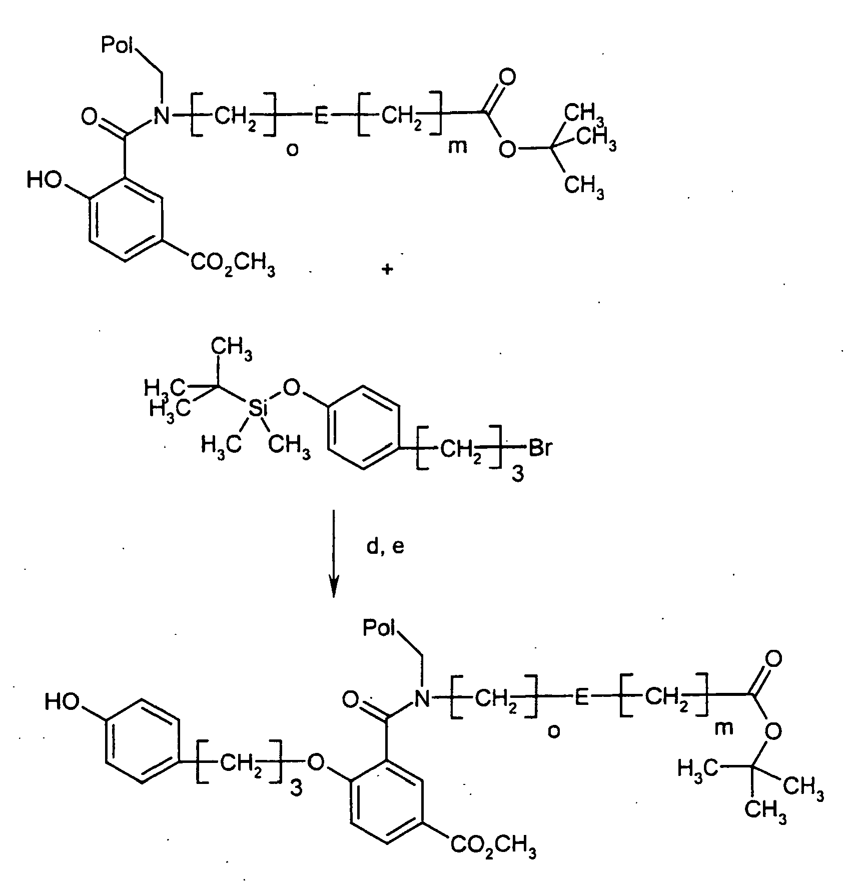

Nach Waschen und Trocknen der Festphase nach üblichen, dem Fachmann bekannten Verfahren wird diese nun mit Verbindungen der Formel R⁴-M-L-Q³ umgesetzt, wobei Q³ für Chlor, Brom, Jod, Mesylat, Tosylat, oder eine andere, dem Fachmann bekannte Abgangsgruppe in nucleophilen Substitutionsreaktionen steht. Die Reaktionen werden unter Verwendung einer Base aus der Gruppe von Natriumcarbonat, Natriumhydrid, Kaliumcarbonat und Caesiumcarbonat in polaren aprotischen Lösungsmitteln wie Tetrahydrofuran, Dioxan, Dimethylformamid, N-Methylpyrrolidon, gegebenefalls unter Zugabe von 18-Krone-6 oder Tetrabutylamoniumiodid, bei Temperaturen zwischen Raumtemperatur und dem Siedepunkt des jeweiligen Lösungsmittels für 1-24 Stunden durchgeführt (Reaktion f). Nach Waschen und Trocknen der Festphase nach üblichen, dem Fachmann bekannten Verfahren wird an dieser nun der Methylester mit Basen wie Natriumcarbonat, Kaliumcarbonat, Caesiumcarbonat, Natriumhydroxid oder Kaliumhydroxid in Gemischen aus polaren Lösungsmitteln wie Tetrahydrofuran, Dioxan oder N-Methylpyrrolidon mit Wasser oder Methanol bei Temperaturen zwischen 0°C und 60°C verseift (Reaktion g).

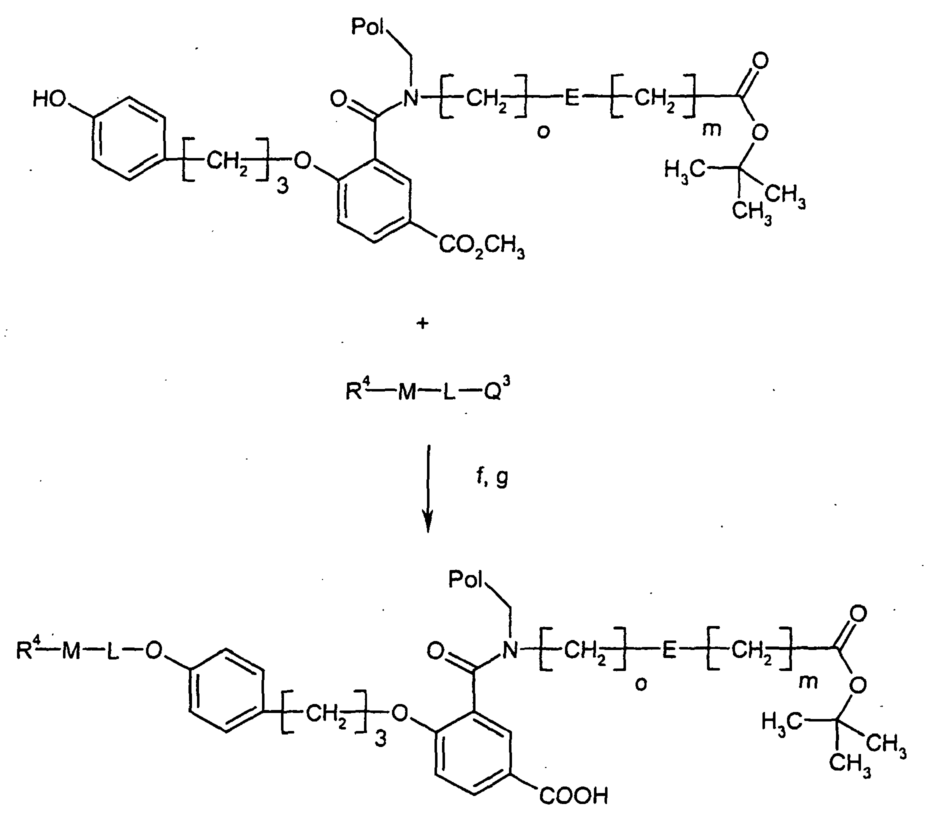

Nach gründlichem Waschen der Festphase mit gängigen Lösungsmitteln wie Methanol, Dichlormethan, Wasser, Dimethylformamid, N-Methylpyrrolidon, Dioxan, Tetrahydrofuran, Acetonitril oder Toluol wird diese getrocknet. Anschließend wird das Produkt mit einem Gemisch aus 20-70 % Trifluoressigsäure in Dichlormethan von der Festphase abgespalten, wobei gleichzeitig die *t*-Butylschutzgruppe entfernt wird (Reaktion h). Nach Filtration zur Abtrennung der Festphase und Einengen im Vakuum bei Temperaturen zwischen 0°C und 100°C liegen die Produkte in Reinheiten zwischen 75 % und 100 % vor.

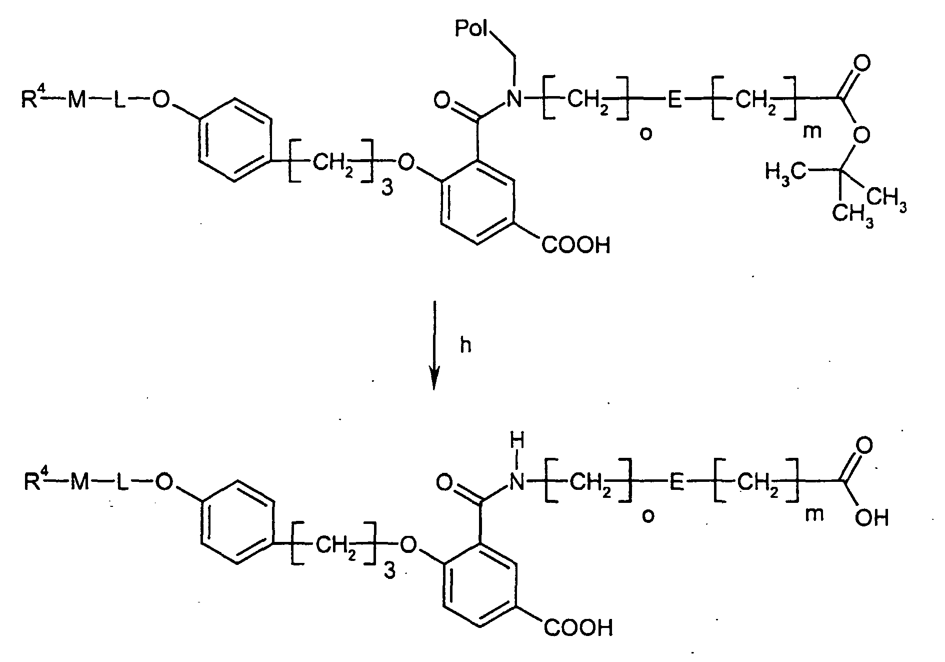

Die erfindungsgemäßen Verbindungen zeigen ein nicht vorhersehbares, wertvolles pharmakologisches und pharmakokinetisches Wirkspektrum. Sie eignen sich daher zur Verwendung als Arzneimittel zur Behandlung und/oder Prophylaxe von Krankheiten bei Menschen und Tieren.

Die pharmazeutische Wirksamkeit der erfindungsgemäßen Verbindungen der Formel (I) lässt sich durch ihre Wirkung als selektive Antagonisten des Cysteinyl-Leukotrien-Rezeptors 2 erklären.

Als "selektiv" werden im Rahmen der vorliegenden Erfindung solche Cysteinyl-Leukotrien-Rezeptor Antagonisten bezeichnet, welche die Aktivität des Cysteinyl-Leukotrien-Rezeptor 2 bei einer um mehr als den Faktor 10, vorzugsweise mehr als den Faktor 100, insbesondere mehr als den Faktor 1000, geringeren Konzentration inhibieren, als eine äquivalente Aktivität des Cysteinyl-Leukotrien-Rezeptors 1. Bezüglich der Testmethoden für die Bestimmung der Selektivität sei auf die in Abschnitt B 1. bzw. B 2. beschriebenen Testmethoden verwiesen.

Als "Antagonisten" werden im Rahmen der vorliegenden Erfindung solche Modulatoren des Cysteinyl-Leukotrien-Rezeptors bezeichnet, die antagonistisch wirksam sind und lediglich eine partielle, vorzugsweise keine messbare agonistische Komponente enthalten.

Die Verbindungen der Formel (I) sind geeignet für die Prophylaxe und/oder Behandlung von verschiedenen Erkrankungen, insbesondere von kardiovaskulären Erkrankungen.

Beispielhaft und vorzugsweise seien genannt: atriale und ventrikuläre Arrhythmien, Myokardinfarkt, Arteriosklerose, Herzinsuffizienz, stabile und instabile Angina pectoris, myokardiale Ischämie, transitorische und ischämische Attacken, Hirnschlag, entzündliche kardiovaskuläre Erkrankungen, koronare Herzerkrankung, periphere und kardiale Gefässerkrankungen, periphere Durchblutungsstörungen, Restenosen wie nach Thrombolysetherapien, percutan transluminalen Angioplastien (PTA) und transluminalen Koronarangioplastien (PTCA), pulmonale Hypertonie, Koronarspasmen, Thrombosen, thromboembolische Erkrankungen, Bypass-Operationen, Herztransplantationen, Ödembildung, Schock, Bluthochdruck, akutes Nierenversagen, entzündliche Erkrankungen, asthmatische Erkrankungen, Chronic Obstructive Airways Disease (COPD), Schmerzzustände, Prostatahypertrophie, entzündliche Hauterkrankungen, Plazentainsuffizienz, Plazentationsstörungen, Inkontinenz, Blasenentzündung, hyperaktive Blase, Erkrankungen der Nebenniere wie zum Beispiel Phäochromozytom und Nebennierenapoplexie, Erkrankungen des Darms wie zum Beispiel Morbus Crohn oder Diarrhoe.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge der erfindungsgemäßen Verbindungen.

Weiterer Gegenstand der vorliegenden Erfindung sind Arzneimittel, enthaltend mindestens eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: Cysteinyl-Leukotrien-Rezeptor-1-Antagonist, Cysteinyl-Leukotrien-Biosynthese-Inhibitor, Thrombolytika, ein Thrombozytenaggregationshemmer, β-Blocker, Nitrate, Ca-Kanal-Blocker und/oder ein anti-inflammatorischer Wirkstoff, wie zum Beispiel ein Cyclooxygenase-Hemmer.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Bevorzugt ist die parenterale, insbesondere die intravenöse Applikation, z.B. als iv Bolus Injektion (d.h. als Einzelgabe, z.B. per Spritze), Kurzzeitinfusion (d.h. Infusion über einen Zeitraum von bis zu einer Stunde) oder Langzeitinfusion (d.h. Infusion über einen Zeitraum von mehr als einer Stunde). Das applizierte Volumen kann dabei in Abhängigkeit von den speziellen Bedingungen zwischen 0,5 bis 30, insbesondere 1 bis 20 ml bei der iv Bolus Injektion, zwischen 25 bis 500, insbesondere 50 bis 250 ml bei der Kurzzeitinfusion und zwischen 50 bis 1000, insbesondere 100 bis 500 ml bei der Langzeitinfusion betragen. Hierzu kann es vorteilhaft sein, den Wirkstoff in fester Form bereitzustellen (z.B. als Lyophilisat oder als Salz) und erst unmittelbar vor der Applikation im Lösungsmedium zu lösen.

Hierbei müssen die Applikationsformen steril und pyrogenfrei sein. Sie können auf wässrigen oder Mischungen aus wässrigen und organischen Lösungsmitteln basieren. Dazu zählen z.B. wässrige Lösungen, Mischungen aus wässrigen und organischen Lösungsmitteln (insbesondere Ethanol, Polyethylenglykol (PEG) 300 oder 400), wässrige Lösungen enthaltend Cyclodextrine oder wässrige Lösungen enthaltend Emulgatoren (grenzflächenaktive Lösungsvermittler, z.B. Lecithin oder Pluronic F 68, Solutol HS 15, Cremophor). Bevorzugt sind hierbei wässrige Lösungen.

Für die parenterale Applikation eignen sich weitgehend isotone und euhydrische Formulierungen, z.B. solche mit einem pH-Wert zwischen 3 und 11, vorzugsweise zwischen 6 und 8, insbesondere um 7,4.

Die Verpackung der Injektionslösungen erfolgt in geeigneten Behältnissen aus Glas oder Kunststoff, z.B. in Durchstichflaschen (Vials). Aus diesen kann die Lösung direkt entnommen und appliziert werden. Im Falle eines Lyophilisats wird sie in dem Vial aufgelöst durch Zuspritzen eines geeigneten Lösungsmittels und dann entnommen. Die Verpackung der Infusionslösungen erfolgt in geeigneten Behältnissen aus Glas oder Kunststoff, z.B. in Flaschen oder kollabierenden Kunststoffbeuteln.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise , mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich sowohl in der Human- als auch in der Veterinärmedizin als vorteilhaft erwiesen, den erfindungsgemäßen Wirkstoff in Gesamtmengen von etwa 0,01 bis etwa 700, vorzugsweise 0,01 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den erfindungsgemäßen Wirkstoff vorzugsweise in Mengen von etwa 0,1 bis etwa 80, insbesondere 0,1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- DC: Dünnschichtchromatographie
- DCI: direkte chemische Ionisation (bei MS)
- DCM: Dichlormethan
- DIEA: *N,N-* Diisopropylethylamin
- DMSO: Dimethylsulfoxid
- DMF: *N,N* Dimethylformamid
- d. Th.: der Theorie
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- ges.: gesättigt
- H: Stunde
- HPLC: Hochdruck-, Hochleistungsflüssigchromatographie
- konz.: konzentriert
- Kp.: Siedepunkt
- LC-MS: Flüssigchromatographie-gekoppelte Massenspektroskopie
- LDA: Lithium-Diisopropylamid
- MPLC: Mitteldruck-, Mittelleistungsflüssigchromatographie
- MS: Massenspektroskopie
- NMR: Kernresonanzspektroskopie
- proz.: prozentig
- RP-HPLC: Reverse Phase HPLC
- RT: Raumtemperatur
- R_{f}: Retentionsindex (bei DC)
- Rₜ: Retentionszeit (bei HPLC)
- TFA: Trifluoressigsäure
- THF: Tetrahydrofuran

### HPLC- und LC-MS-Methoden:

### Methode 1 (HPLC)

Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2%B, 0.5 min 2%B, 4.5 min 90%B, 6.5 min 90%B; Fluss: 0.75 ml/min; Ofen: 30°C; UV-Detektion: 210 nm.

### Methode 2 (HPLC)

Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2%B, 0.5 min 2%B, 4.5 min 90%B, 9 min 90%B; Fluss: 0.75 ml/min; Ofen: 30°C; UV-Detektion: 210 nm.

### Methode 3 (LC-MS)

Gerätetyp MS: Micromass ZQ; Gerätetyp HPLC: Waters Alliance 2790; Säule:

Grom-Sil 120 ODS-4 HE 50 mm x 2 mm, 3.0 µm; Eluent B: Acetonitril + 0.05% Ameisensäure, Eluent A: Wasser + 0.05% Ameisensäure; Gradient: 0.0 min 5%B → 2.0 min 40%B → 4.5 min 90%B → 5.5 min 90%B; Ofen: 45°C; Fluss: 0.0 min 0.75 ml/min → 4.5 min 0.75 ml/min → 5.5 min 1.25 ml/min; UV-Detektion: 210 nm.

### Methode 4 (HPLC)

Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2%B, 0.5 min 2%B, 4.5 min 90%B, 15 min 90%B; Fluss: 0.75ml/min; Ofen: 30°C; UV-Detektion: 210 nm.

### Methode 5 (HPLC)

Instrument: Symmetry TM C18 3.9*150 mm; flow 1,5 ml/min; eluent water (A)/acetonitrile (B); gradient -0.6 min 10% of B, -3.8 min 100% B, -5.0 min 100% B,-5.5 min 10% B; stop time 6.0 min; injection volume 10 µl; diode array detector signal 214 nM and 254 nm.

### Methode 6 (LC-MS)

Gerätetyp MS: Micromass ZQ; Gerätetyp HPLC: Waters Alliance 2795; Säule: Phenomenex Synergi 2µ Hydro-RP Mercury 20 mm x 4mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

### Methode 7 (LC-MS)

Gerätetyp MS: Micromass ZQ; Gerätetyp HPLC: HP 1100 Series; UV DAD; Säule: Phenomenex Synergi 2µ Hydro-RP Mercury 20 mm x 4 mm ; Eluent A: 11 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

### Methode 8 (LC-MS)

Gerätetyp MS: Micromass ZQ; Gerätetyp HPLC: HP 1100 Series; UV DAD; Säule: Grom-Sil 120 ODS-4 HE 50 mm x 2 mm, 3.0 µm; Eluent A: Wasser + 500 µl 50%ige Ameisensäure / l, Eluent B: Acetonitril + 500 µl 50%ige Ameisensäure / l; Gradient: 0.0 min 0%B → 2.9 min 70%B → 3.1 min 90%B → 4.5 min 90%B; Ofen: 50 °C; Fluss: 0.8 ml/min; UV-Detektion: 210 nm.

### Methode 9 (LC-MS)

Gerätetyp MS: Micromass ZQ; Gerätetyp HPLC: Waters Alliance 2795; Säule: Merck Chromolith SpeedROD RP-18e 50 mm x 4.6 mm; Eluent A: Wasser + 500 µl 50%ige Ameisensäure / l; Eluent B: Acetonitril + 500 µl 50%ige Ameisensäure / l; Gradient: 0.0 min 10%B → 3.0 min 95%B → 4.0 min 95%B; Ofen: 35°C; Fluss: 0.0 min 1.0 ml/min → 3.0 min 3.0 ml/min → 4.0 min 3.0 ml/min; UV-Detektion: 210 nm.

### Methode 10 (LC-MS)

Instrument: Micromass Platform LCZ mit HPLC Agilent Serie 1100; Säule: Grom-SIL120 ODS-4 HE, 50 mm x 2.0 mm, 3 µm ; Eluent A: 1 1 Wasser + 1 ml 50%ige Ameisensäure, Eluent B: 1 1 Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 210 nm.

### Methode 11 (LC-MS)

Gerätetyp MS: Micromass ZQ; Gerätetyp HPLC: Waters Alliance 2790; Säule: Grom-Sil 120 ODS-4 HE, 50mm x 2 mm, 3.0 µm; Eluent A: 1 1 Wasser + 500 µl 50%ige Ameisensäure; Eluent B: 1 1 Acetonitril + 500 µl 50%ige Ameisensäure; Gradient: 0.0 min 0%B→ 0.2 min 0%B→ 2.9 min 70%B→ 3.1 min 90%B→ 4.5 min 90%B; Ofen: 45°C; Fluss: 0.8 ml/min; UV-Detektion: 210 nm.

### Methode 12 (HPLC)

Instrument: HP 1100 mit DAD-Detektion; Säule: Kromasil 100 RP-18, 125 mm x 4 mm, 5µm; Eluent A: 1 1 Wasser + 4 Flaschen PIC B7, Eluent B: Acetonitril; Gradient: 0.0 min 2%B, 1.0 min 2%B, 9.0 min 90%B, 13 min 90%B, 13.5 min 2%B, 15.5 min 2%B; Fluss: 2 ml/min; Ofen: 30°C; UV-Detektion: 210 nm.
(PIC B7: Heptan sulfonic acid von Millipore/Waters Corp.)

### Methode 13 (LC-MS)

Instrument: Micromass Platform LCZ mit HPLC Agilent Serie 1100; Säule: Grom-SIL120 ODS-4 HE, 50 mm x 2.0 mm, 3 µm; Eluent A: 1 l Wasser + 1 ml 50%ige Ameisensäure, Eluent B: 1 l Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.

### Methode 14 (LC-MS)

Instrument: Micromass Quattro LCZ, mit HPLC Agilent Serie 1100; Säule: Grom-SIL120 ODS-4 HE, 50 mm x 2.0 mm, 3 µm; Eluent A: 1 l Wasser + 1 ml 50%ige Ameisensäure, Eluent B: 1 l Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.

### Ausgangsverbindungen

### Beispiel I

### 3-[4-(4-Phenoxybutoxy)phenyl]propansäure-methylester

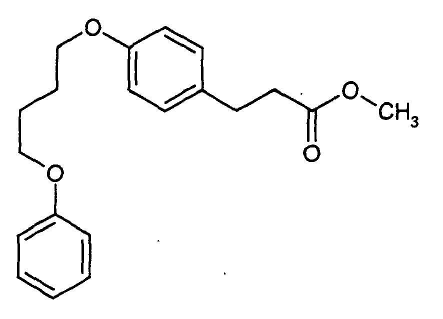

Eine Lösung von 10.0 g 3-(4-Hydroxyphenyl)propansäure-methylester und 12.7 g 4-Phenoxy-1-butylbromid in 100 ml Acetonitril wird mit 11.5 g Kaliumcarbonat versetzt und 15 Stunden zum Rückfluss erhitzt. Anschließend wird das Lösemittel am Rotationsverdampfer entfernt und der Rückstand mit Ethylacetat und Wasser aufgenommen. Nach Phasentrennung wird die wässrige Phase mit Ethylacetat extrahiert. Die vereinigten organischen Phasen werden mit gesättigter Natriumchlorid-Lösung gewaschen und über wasserfreiem Natriumsulfat getrocknet. Nach Einrotieren wird das Rohprodukt durch Saugfiltration über Kieselgel mit Cyclohexan/Ethylacetat 19:1 gereinigt. Es werden 12.6 g Produkt erhalten.
DC: R_{f}-Wert: 0.36 (Cyclohexan/Ethylacetat 1:1).
HPLC (Methode 1): Rₜ: 5.41 min.
¹H-NMR (200 MHz, DMSO-d₆, δ/ppm): 7.32-7.23 (m, 2H), 7.11 (d, 2H), 6.96-6.82 (m, 5H), 3.98 (m, 4H), 3.57 (s, 3H), 2.77 (t, 3H), 2.57 (t, 2H), 1.85 (m, 4H).
MS (ESI+): m/z = 329 (M+H⁺), 351 (M+Na⁺).

### Beispiel II

### 3-[4-(4-Phenoxybutoxy)phenyl]-1-propanol

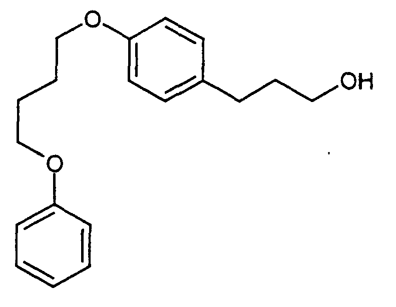

20.1 ml einer 1-molaren Lösung von Lithiumaluminiumhydrid in Tetrahydrofuran wird vorgelegt. Unter Rühren wird eine Lösung von 12.0 g 3-[4-(4-Phenoxybutoxy)-phenyl]propansäure-methylester in 40 ml THF so zugetropft, dass das Gemisch gerade anfängt zu sieden. Es wird 30 Minuten bei Raumtemperatur nachgerührt. Zu der Suspension wird vorsichtig 1 ml Methanol zugegeben, um überschüssiges Lithiumaluminiumhydrid zu hydrolysieren. Anschließend wird der Ansatz auf 1-molare Salzsäure gegeben und mit Ethylacetat extrahiert. Die organische Phase wird abgetrennt, mit gesättigter Natriumchlorid-Lösung gewaschen und über wasserfreiem Natriumsulfat getrocknet. Nach Filtrieren und Einrotieren werden 10.9 g Produkt erhalten.
HPLC (Methode 1): Rₜ: 4.94 min.
¹H-NMR (300 MHz, DMSO-d₆, δ/ppm): 7.30-7.24 (m, 2H), 7.08 (d, 2H), 6.95-6.88 (m, 3H), 6.83 (d, 2H), 4.39 (t, 1H), 4.01 (m, 4H), 3.38 (quart, 2H), 2.53 (t, 2H), 1.83 (m, 4H), 1.67 (m, 2H).
MS (ESI+): m/z = 301 (M+H⁺), 323 (M+Na⁺).

### Beispiel III

### 3-[4-(1,1'-Biphenyl-4-ylmethoxy)phenyl]-1-propanol

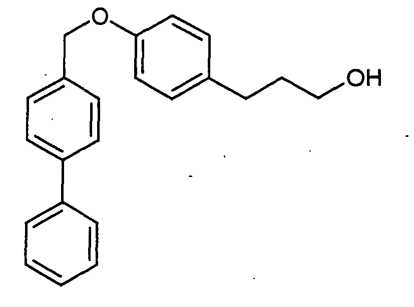

12.8 g (41.4 mmol) 4-Phenylbenzylbromid, 6.94 g (45.6 mmol) 4-Hydroxyphenylpropanol und 6.87 g (49.7 mmol) Kaliumcarbonat werden in 50 ml Butyronitril 6 Stunden bei 120 °C gerührt. Nach dem Abkühlen auf Raumtemperatur wird von den anorganischen Salzen abgesaugt und im Vakuum eingeengt. Das Rohprodukt wird über Kieselgel 60 (Laufmittelgradient Cyclohexan --> Cyclohexan/Ethylacetat 60:40) chromatographisch gereinigt. Man erhält 4.90 g (37 % d. Th.) Produkt.
Fp.: 128 °C.
HPLC (Methode 1): Rₜ: 5.12 min.
¹H-NMR (300 MHz, DMSO-d₆, δ/ppm): 7.70-7.64 (m, 4H), 7.55-7.42 (m, 4H), 7.40-7.33 (m, 1H), 7.11 (d, 2H), 6.93 (d, 2H), 5.11 (s, 2H), 4.39 (t, 1H), 3.39 (m, 2H), 2.53 (m, 2H), 1.67 (quint, 2H).
MS (DCI): m/z = 336 (M+NH₄⁺).

### Beispiel IV

### 1-(3-Brompropyl)-4-(4-phenoxybutoxy)benzol

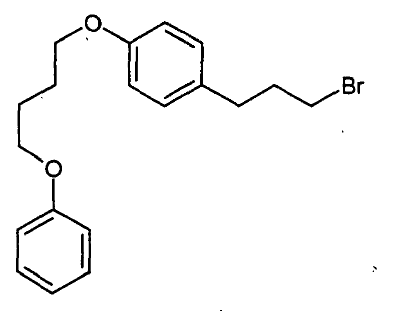

10.0 g 3-[4-(4-Phenoxybutoxy)phenyl]-1-propanol werden in 50 ml Tetrahydrofuran gelöst und mit 10.5 g festem Triphenylphophin versetzt. Zu der Lösung gibt man 13.2 g festes Tetrabrommethan. Nach etwa 5 Minuten beginnt das Gemisch trübe zu werden. Nach einer Stunde ist die Reaktion beendet. Es wird vom Niederschlag abfiltriert und das Filtrat eingeengt. Das Rohprodukt wird durch Saugfiltration an Kieselgel mit Cyclohexan/Ethylacetat 15:1 als Laufmittel gereinigt. Es werden 8.5 g Produkt erhalten.
HPLC (Methode 1): Rₜ: 6.01 min.
¹H-NMR (400 MHz, DMSO-d₆, δ/ppm): 7.29-7.24 (m, 2H), 7.12 (d, 2H), 6.94-6.89 (m, 3H), 6.86 (d, 2H), 4.00 (m, 4H), 3.48 (t, 2H), 2.63 (t, 2H), 2.05 (m, 2H), 1.84 (m, 4H).
MS (DCI, NH₃): m/z = 380/382 (M+NH₄⁺).

### Beispiel V

### 4-{[4-(3-Brompropyl)phenoxy]methyl}-1,1'-biphenyl

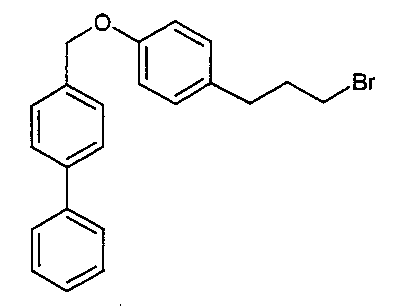

Analog zu dem unter Beispiel IV beschriebenen Verfahren wird 4-{[4-(3-Brompropyl)phenoxy]methyl}-1,1'-biphenyl durch Bromierung von 3-[4-(1,1'-Biphenyl-4-ylmethoxy)phenyl]-1-propanol erhalten.
HPLC (Methode 1): Rₜ: 6.10 min
¹H-NMR (300 MHz, DMSO-d₆, δ/ppm): 7.70-7.64 (m, 4H), 7.55-7.43 (m, 4H), 7.40-7.33 (m, 1H), 7.14 (d, 2H), 6.96 (d, 2H), 5.12 (s, 2H), 3.48 (t, 2H), 2.64 (t, 2H), 2.05 (quint, 2H).
MS (DCI): m/z = 400 (M+NH₄⁺).

### Beispiel VI

### 3-[4-(4-Phenoxybutoxy)phenyl]propansäure

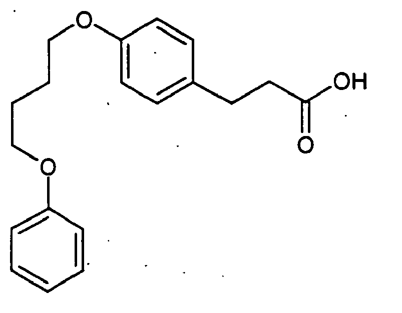

Eine Lösung von 10 g 3-[4-(4-Phenoxybutoxy)phenyl]propansäure-methylester in 100 ml Tetrahydrofuran wird mit je 100 ml Methanol und 100 ml 2-molarer Natronlauge versetzt. Es wird zwei Stunden lang auf 60°C erwärmt. Dann wird mit 2-molarer Salzsäure angesäuert und der Grossteil der organischen Lösemittel am Rotationsverdampfer entfernt. Es fällt ein Niederschlag aus, der bei Raumtemperatur abgesaugt und mit Wasser gewaschen wird. Es werden 7.7 g Produkt erhalten.
HPLC (Methode 1): Rₜ: 4.77 min.
¹H-NMR (200 MHz, DMSO-d₆, δ/ppm): 12.10 (s, breit, 1H), 7.32-7.23 (m, 2H), 7.12 (d, 2H), 6.95-6.81 (m, 5H), 4.00 (m, 4H), 2.73 (t, 2H), 2.48 (t, 2H), 1.85 (m, 4H).
MS (DCI, NH₃): m/z = 332.2 (M+NH₄⁺).

### Beispiel VII

### 3-[4-(4-Phenoxybutoxy)phenyl]propansäurechlorid

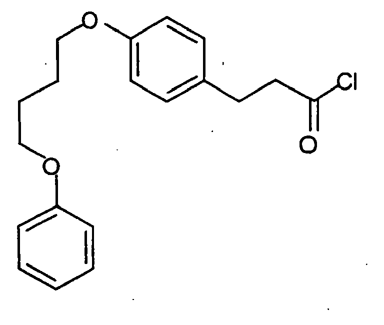

Eine Suspension von 250 mg 3-[4-(4-Phenoxybutoxy)phenyl]propansäure in 10 ml Chloroform wird auf ca. 40°C erwärmt. Dabei bildet sich eine Lösung. Es wird mit 1.4 ml Oxalylchlorid versetzt (Gasentwicklung). Das Gemisch wird zum Rückfluss erhitzt. Nach zwei Stunden wird der Ansatz zur Trockene einrotiert und das Produkt am Hochvakuum getrocknet. Es werden 264 mg Produkt erhalten.
MS (DCI, NH₃): m/z = 350/352 (M+NH₄⁺).

### Beispiel VIII

### 5-(Methoxycarbonyl)-2-nitrobenzoesäure

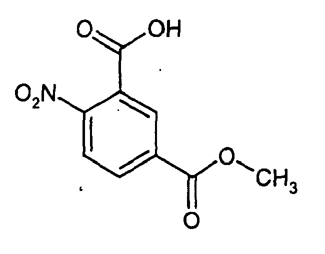

Eine Lösung von 2.0 g 3-Formyl-4-nitrobenzoesäure-methylester [M.G. Vetelino et al., Tetrahedron Lett. 35, 219-222 (1994).] in 20 ml Acetonitril wird bei 10°C mit einer Lösung von 320 mg Natriumdihydrogenphosphat in 10 ml Wasser und 1 ml Wasserstoffperoxid (35%ig) versetzt. Dann läßt man eine Lösung von 1.5 g Natriumchlorit in 10 ml Wasser im Verlauf von 15 Minuten zutropfen. Anschließend wird das Gemisch noch weitere 2 Stunden bei 10°C gerührt. Die Reaktion wird durch Zusatz von 400 mg Natriumsulfit beendet. Es wird mit 2-molarer Salzsäure verdünnt und mit Ethylacetat extrahiert. Der organische Extrakt wird mit gesättigter Natriumchlorid-Lösung gewaschen und über wasserfreiem Natriumsulfat getrocknet. Nach Filtrieren und Einrotieren werden 225 mg Produkt erhalten.
HPLC (Methode 1): Rₜ: 3.68 min.
¹H-NMR (300 MHz, DMSO-d₆, δ/ppm): 14.22 (s breit, 1H), 8.34 (d, 1H), 8.29 (dd, 1H), 8.09 (d, 1H), 3.92 (s, 3H).
MS (DCI, NH₃): m/z = 243 (M+NH₄⁺), 260 (M+N₂H₇⁺).

### Beispiel IX

### 4-Hydroxy-3-({[3(methoxycarbonyl)cyclohexyl]amino}carbonyl)benzoesäure-methylester

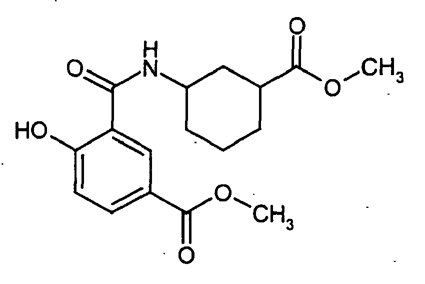

### Methode 1:

10 g 3-Aminocyclohexan-1-carbonsäure-Hydrochlorid werden zusammen mit 15.5 ml Trimethylsilylchlorid in 55 ml Methanol bei Raumtemperatur über Nacht gerührt. Durch Anschließendes Entfernen des Lösemittels und aller weiteren flüchtigen Komponenten am Rotationsverdampfer werden 8 g 3-Aminocyclohexan-1-carbonsäuremethylester-Hydrochlorid erhalten. Eine Lösung von 7.37 g 2-Hydroxy-5-carbomethoxybenzoesäure [CAS-Nr. 79128-78-2] in 1000 ml Dichlormethan wird nacheinander mit 8.0 g 3-Aminocyclohexan-1-carbonsäuremethylester-Hydrochlorid, 14.4 g N-[(3-Dimethylamino)propyl]-N'-ethylcarbodiimid-Hydrochlorid (EDC), 5.1 g 1-Hydroxy-1H-benzotriazol-Hydrat (HOBT) und 11 ml Triethylamin versetzt. Nach 15 Stunden bei Raumtemperatur wird das Reaktionsgemisch mit 250 ml Wasser versetzt. Nach Phasentrennung wird die wässrige Phase mit Ethylacetat extrahiert. Die vereinigten Dichlormethan- und Ethylacetat-Phasen werden mit gesättigter Natriumchlorid-Lösung gewaschen und über wasserfreiem Natriumsulfat getrocknet. Nach Filtrieren und Einengen wird ein Öl erhalten, das zunächst mittels-Saugfiltration über Kieselgel mit Cyclohexan/Ethylacetat 4:1 als Laufmittel vorgereinigt wird. Das so erhaltene Produkt enthält ein Gemisch aller vier möglichen Stereoisomere: cis-Racemat und trans-Racemat. Die Abtrennung des Grossteils eines der beiden Racemate gelingt durch Kristallisation aus Cyclohexan, dem etwa 5 % Ethylacetat beigemischt sind. Dabei werden 4.18 g Produkt erhalten (Racemat A).

Das nach Einrotieren der Mutterlauge verbleibende Produkt wird mittels präparativer HPLC an achiraler RP-Säule getrennt. Dabei werden weitere 0.2 g des Materials der Fraktion 1 erhalten und mit dieser vereinigt. Darüber hinaus werden 1.55 g des anderen Racemats gewonnen (Racemat B).
Racemat A:
   HPLC (Methode 1): Rₜ: 4.50 min.
   ¹H-NMR (300 MHz, DMSO-d₆, δ/ppm): 13.34 (s breit, 1H), 8.92 (d breit, 1H), 8.57 (d, 1H), 7.97 (dd, 1H), 6.99 (d, 1H), 3.88 (m, 1H), 3.83 (s, 3H), 3.60 (s, 3H), 2.49 (m, 1H), 2.07 (m, 1H), 1.92-1.78 (m, 3H), 1.56-1.15 (m, 4H).
   MS (DCI, NH₃): m/z = 336.1 (M+H⁺), 353 (M+NH₄⁺).
Racemat B:
   HPLC (Methode 1): Rₜ: 4.42 min.
   ¹H-NMR (300 MHz, DMSO-d₆, δ/ppm): 13.13 (s breit, 1H), 8.76 (d breit, 1H), 8.53 (d, 1H), 7.97 (dd, 1H), 7.00 (d, 1H), 4.12 (m, 1H), 3.83 (s, 3H), 3.62 (s, 3H), 2.82 (m, 1H), 1.93 (m, 1H), 1.83-1.45 (m, 7H).
   MS (DCI, NH₃): m/z = 336.1 (M+H⁺), 353 (M+NH₄⁺).

### Methode 2:

Das Racemat B kann in präparativem Massstab chromatographisch in seine optischen Antipoden aufgespalten werden.
Methode: Chiraler Kieselgelselektor KBD 8361 (Partikelgröße 10µm) basierend auf dem Selektor Poly(N-methacryloyl-L-leucin-1-menthylamid); Säule: 420 mm x 100 mm; Elutionsmittelgemisch aus tert.-Butylmethylether/Ethylacetat 9:1 (vol/vol); Fluss: 100 ml/min; UV-Detektion: 254 nm; Probe: 10 g gelöst in 1000 ml Elutionsmittel; Probenaufgabe: 80 ml; Temperatur: 24 °C.

### (+)-Enantiomer von Racemat B [(+)-B-Enantiomer]:

HPLC (Säule KBD 8361 wie oben, 250 mm x 20 mm, Isohexan/Ethylacetat 4:1, Fluss: 25 ml/min, UV-Detektion: 280 nm, 24°C): Rₜ: 15.8 min.
Spezifischer Drehwert (Methanol, 589 nm, 20°C): +36.8°.

### (-)-Enantiomer von Racemat B [(-)-B-Enantiomer]:

HPLC (Säule KBD 8361 wie oben, 250 mm x 20 mm, Isohexan/Ethylacetat 4:1, Fluss: 25 ml/min, UV-Detektion: 280 nm, 24°C): Rₜ: 9.6 min.
Spezifischer Drehwert (Methanol, 589 nm, 20°C): -37.6°.

### Methode 3:

Das Racemat B kann gezielt auch auf die folgende Weise erhalten werden:
Eine Lösung von 5.85 g 2-Hydroxy-5-(methoxycarbonyl)benzoesäure in 128 ml wasserfreiem THF wird bei 0 °C tropfenweise mit 21.8 ml Thionylchlorid versetzt. Man lässt das Reaktionsgemisch über Nacht bei Raumtemperatur rühren. Dann werden das Lösemittel und überschüssiges Thionylchlorid am Rotationsverdampfer, komplett entfernt. Der verbleibende Rückstand wird in 120 ml wasserfreiem Dichlormethan aufgenommen, mit 5.5 g trans-3-Aminocyclohexancarbonsäuremethylester-Hydrochlorid und bei -20°C mit 10.4 ml Triethylamin versetzt. Anschließend lässt man das Reaktionsgemisch auf Raumtemperatur kommen und rührt weitere 1.5 Stunden bei dieser Temperatur. Anschließend wird mit ca. 50 ml 0.01-molarer Salzsäure versetzt. Nach Phasentrennung wird die wässrige Phase noch zweimal mit Ethylacetat extrahiert. Die vereinigten organischen Extrakte werden über wasserfreiem Natriumsulfat getrocknet. Es wird filtriert und einrotiert. Das Produkt wird durch Saugfiltration über Kieselgel mit Cyclohexan/Ethylacetat 4:1 als Laufmittel gereinigt. Es werden 4.06 g Produkt erhalten. Das Produkt besteht zu 87% aus Racemat B und zu 13% aus Racemat A. Eine weitergehende Aufreinigung kann, wie unter Beispiel IX, Methode 1, beschrieben, erfolgen.

### Beispiel X

### 1-[2-Hydroxy-5-(methoxycarbonyl)benzoyl]-4-piperidincarbonsäure-methylester

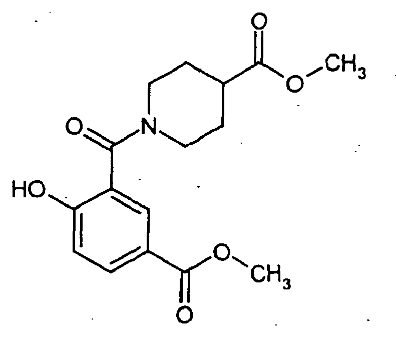

### Methode 1:

Analog zu dem unter Beispiel IX, Methode 1, beschriebenen Verfahren wird 1-[2-Hydroxy-5-(methoxycarbonyl)benzoyl]-4-piperidincarbonsäure-methylester aus 2-Hydroxy-5-carbomethoxybenzoesäure [CAS-Nr. 79128-78-2] und Piperidin-4-carbonsäure-methylester-Hydrochlorid hergestellt.
HPLC (Methode 1): Rₜ: 3.64 min.
¹H-NMR (400 MHz, DMSO-d₆, δ/ppm): 10.78 (s, 1H), 7.83 (dd, 1H), 7.69 (d, 1H), 7.96 (d, 1H), 4.37 (breit, 1H), 3.79 (s, 3H), 3.61 (s, 3H), 3.36 (breit, 1H), 2.98 (m breit, 2H), 2.63 (m, 1H), 1.83 (m, 2H), 1.50 (m, 2H).
MS (DCI, NH₃): m/z = 322 (M+H⁺), 339 (M+NH₄⁺).

### Methode 2:

Eine Lösung von 10.0 g 2-Hydroxy-5-carbomethoxybenzoesäure in 600 ml THF wird bei einer Temperatur von ca. 0°C tropfenweise mit 37 ml Thionylchlorid versetzt. Man lässt das Gemisch auf Raumtemperatur kommen und rührt 15 Stunden lang. Anschließend wird der Einsatz am Rotationsverdampfer komplett eingeengt. Es verbleibt ein Öl, das in 400 ml wasserfreiem Dichlormethan gelöst wird. Anschließend werden bei ca. -20°C 11 ml Triethylamin und 8.76 g Methylisonipecotat hinzugefügt. Man lässt das Reaktionsgemisch auf Raumtemperatur kommen. Nach zwei Stunden wird mit 0.1-molarer Salzsäure angesäuert (pH ca. 3) und mit Ethylacetat extrahiert. Der organische Extrakt wird nacheinander mit Wasser und gesättigter Natriumchlorid-Lösung gewaschen. Trocknen über wasserfreiem Natriumsulfat, Filtrieren, Einrotieren. Das Rohprodukt wird zunächst per Saugfiltration über Kieselgel mit Cyclohexan/Ethylacetat 1:1 als Laufmittel vorgereinigt. Die Produkt enthaltenden Fraktionen werden vereinigt, einrotiert und der erhaltene Rückstand mit wenig Ethylacetat verrührt. Es werden 13.4 g eines Feststoffs erhalten.

### Beispiel XI

### 1-[5-(Methoxycarbonyl)-2-nitrobenzoyl]-4-piperidincarbonsäure-methylester

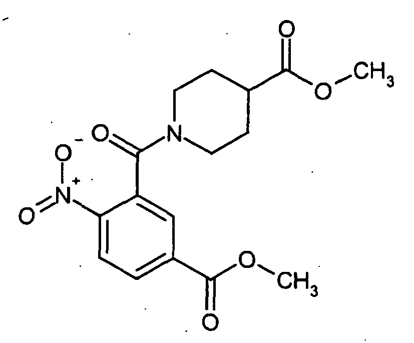

Analog zu dem unter Beispiel IX beschriebenen Verfahren wird 1-[5-(Methoxycarbonyl)-2-nitrobenzoyl]-4-piperidincarbonsäure-methylester aus 5-(Methoxycarbonyl)-2-nitrobenzoesäure und Piperidin-4-carbonsäure-methylester-Hydrochlorid hergestellt.
DC: R_{f}-Wert: 0.21 (Cyclohexan/Ethylacetat 1:1).
HPLC (Methode 1): Rₜ: 3.96 min.
¹H-NMR (200 MHz, DMSO-d₆; δ/ppm): 8.32 (d, 1H), 8.19 (dd, 1H), 8.00 (d, 1H), 4.34 (m, 1H), 3.92 (s, 3H), 3.62 (s, 3H), 3.18-2.94 (m, 2H), 2.77-2.63 (m, 1H), 1.97 (m, 1H), 1.80-1.48 (m, 4H).

### Beispiel XII

### 1-[2-Amino-5-(methoxycarbonyl)benzoyl]-4-piperidincarbonsäure-methylester

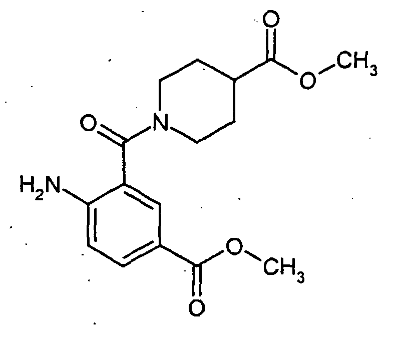

Eine Lösung von 600 mg 1-[5-(Methoxycarbonyl)-2-nitrobenzoyl]-4-piperidincarbonsäure-methylester in 200 ml Methanol wird mit einer Spatelspitze Palladium (10 %ig auf Aktivkohle) versetzt und bei Raumtemperatur und einem Wasserstoffdruck von 3.5 bar in einer Parr-Apparatur hydriert. Nach drei Stunden wird das Reaktionsgemisch über wenig Kieselgul filtriert und einrotiert. Es werden 474 mg Produkt erhalten.
HPLC (Methode 1): Rₜ: 3.68 min.
¹H-NMR (300 MHz, DMSO-d₆, δ/ppm): 7.67 (dd, 1H), 7.57 (d, 1H), 6.72 (d, 1H), 5.98 (s breit, 2H), 3.82 (m, 1H), 3.76 (s, 3H), 3.62 (s, 3H), 3.02 (m, 2H), 2.63 (m, 1H), 1.85 (m, 2H), 1.63-1.48 (m, 3H).
MS (DCI, NH₃): m/z = 321.2 (M+H⁺), 338 (M+NH₄⁺).

### Beispiel XIII

### 3-Formyl-4-{3-[4-(4-phenoxybutoxy)phenyl]propoxy}benzoesäure-ethylester

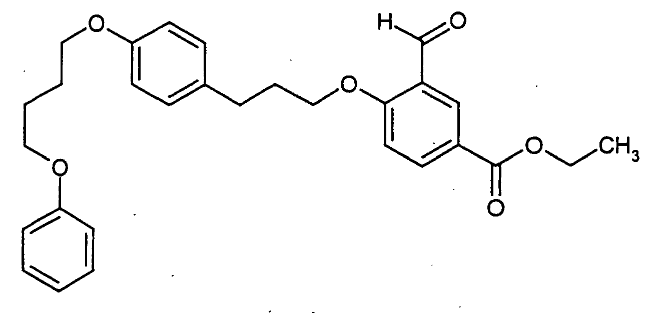

1.37 g (3.76 mmol) 1-(3-Brompropyl)-4-(4-phenoxybutoxy)benzol, 0.79 g (3.76 mmol) 3-Formyl-4-hydroxybenzoesäureethylester [CAS-Nr. 82304-99-2] und 0.78 g (5.64 mmol) Kaliumcarbonat werden in 20 ml DMF gelöst und der Ansatz 16 Stunden bei 40°C gerührt. Anschließend wird eingeengt, der Rückstand in 200 ml Wasser aufgenommen und mit Dichlormethan (dreimal 100 ml) extrahiert. Die vereinigten organischen Phasen werden getrocknet (Natriumsulfat), eingeengt und das Rohprodukt über Kieselgel chromatographisch (Laufmittelgradient Cyclohexan --> Cyclohexan/Ethylacetat 5:1) gereinigt. Man erhält 1.45 g Produkt.
HPLC (Methode 2): Rₜ: 5.62 min.
¹H-NMR (300 MHz, CDCl₃, δ/ppm): 10.48 (s, 1H), 8.49 (s, 1H), 8.20 (dd, 1H), 7.29-7.23 (m, 2H), 7.12-7.06 (d, 2H), 6.98-6.80 (m, 6H), 4.36 (q, 2H), 4.13 (t, 2H), 4.07-3.96 (m, 4H), 2.78 (t, 2H), 2.17 (quint, 2H), 1.97 (m, 4H), 1.38 (t, 3H).
MS (ESI+): m/z = 477 (M+H⁺).

### Beispiel XIV

### 4-{3-[4-(1,1'-Biphenyl-4-ylmethoxy)phenyl]propoxy}-3-formylbenzoesäureethylester

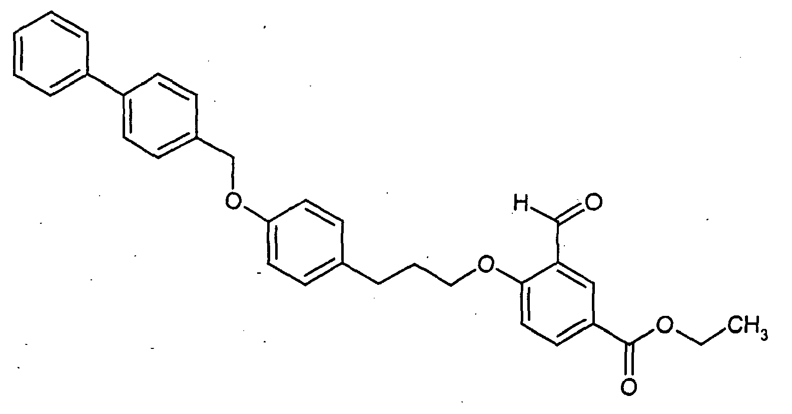

Die Darstellung erfolgt analog Beispiel XIII aus 1,1'-Biphenyl-4-ylmethyl-4-(3-brompropyl)phenylether und 3-Formyl-4-hydroxybenzoesäureetllylester.
HPLC (Methode 2): Rₜ: 6.04 min.
¹H-NMR (200 MHz, DMSO-d₆, δ/ppm): 10.33 (s, 1H), 8.86 (d, 1H), 8.22 (dd, 1H), 7.65-7.55 (m, 4H), 7.53-7.34 (m, 5H), 7.16-6.90 (m, 5H), 5.08 (s, 2H), 4.38 (q, 2H), 4.29 (t, 2H), 2.80 (t, 2H), 2.24 (quint, 2H), 1.39 (t, 3H).
MS (DCI): m/z = 512 (M+NH₄⁺).

### Beispiel XV

### 2-{3-[4-(1,1'-Biphenyl-4-ylmethoxy)phenyl]propoxy}-5-(ethoxycarbonyl)-benzoesäure

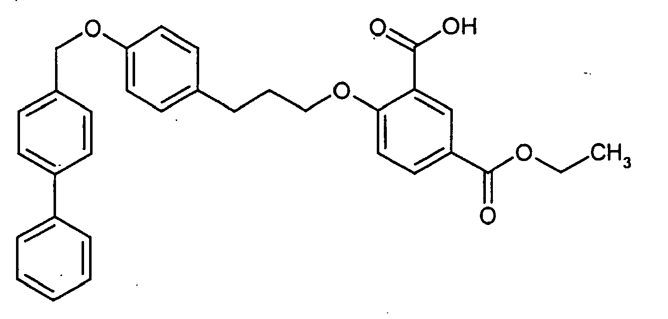

3.10 g (6.28 mmol) 4-{3-[4-(1,1'-Biphenyl-4-ylmethoxy)phenyl]propoxy}-3-formylbenzoesäureethylester werden in 50 ml THF vorgelegt. Bei ca. 0°C (Innentemperatur) werden gleichzeitig Lösungen von 2.13 g (18.8 mmol) Natriumchlorit in 2.5 ml Wasser und 1.83 g (18.8 mmol) Amidoschwefelsaeure in 9 ml Wasser zugetropft. Anschließend wird 15 Minuten bei 0°C gerührt. Das Reaktionsgemisch wird auf 120 ml Wasser gegeben und mit Ethylacetat (viermal 60 ml) ausgeschüttelt. Die vereinigten organischen Phasen werden mit gesättigter Natriumchlorid-Lösung gewaschen (zweimal 50 ml). In der organischen Phase ist ein feinverteilter Niederschlag zu erkennen. Man gibt 300 ml Dichlormethan zu, trocknet über Natriumsulfat und engt ein. Das erhaltene Produkt (2.96 g) wird ohne weitere Aufreinigung umgesetzt.
HPLC (Methode 2): Rₜ: 6.13 min.
¹H-NMR (200 MHz, CDCl₃, δ/ppm): 8.86 (d, 1H), 8.22 (dd, 1H), 7.64-7.55 (m, 4H), . 7.53-7.33 (m, 5H), 7.15-6.92 (m, 5H), 5.08 (s, 2H), 4.38 (q, 2H), 4.29 (t, 2H), 2.80 (t, 2H), 2.24 (quint., 2H), 1.39 (t, 3H).
LC-MS (Methode 3): Rₜ: 4.99 min.
MS (ESI+): m/z = 511 (M+H⁺).

### Beispiel XVI

### 5-(Ethoxycarbonyl)-2-{3-[4-(4-phenoxybutoxy)phenyl]propoxy}benzoesäure

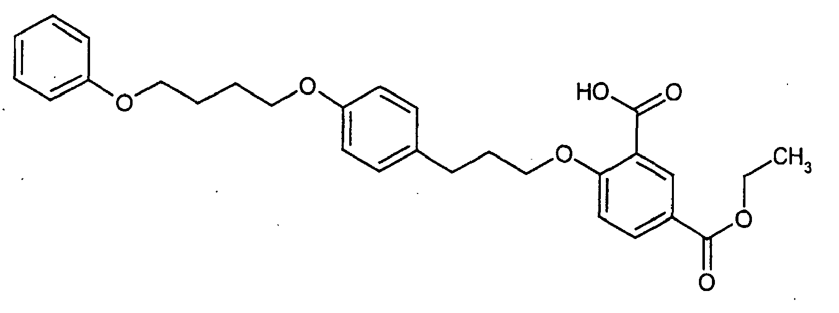

Die Darstellung erfolgt analog Beispiel XV aus 3-Formyl-4-{3-[4-(4-phenoxybutoxy)phenyl]propoxy}benzoesäureethylester.
¹H-NMR (300 MHz, CDCl₃, δ/ppm): 10.46 (s, breit, 1H), 8.85 (d, 1H), 8.22 (dd, 1H), 7.32-7.21 (m, 3H), 7.12-6.80 (m, 7H), 4.38 (q, 2H), 4.28 (t, 2H), 4.08-3.97 (m, 4H), 2.78 (t, 2H), 2.24 (quint, 2H), 1.97 (m, 4H), 1.39 (t, 3H).
LC-MS (Methode 3): Rₜ: 4.20 min.
MS (ESI+): m/z = 493 (M+H⁺).

### Beispiel XVII

### 3-[4-(3-Cyclohexylpropoxy)phenyl]propansäure-methylester

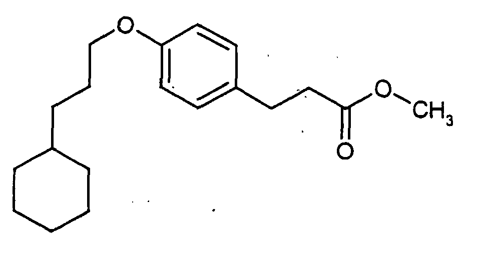

Eine Lösung von 32.21 g 3-(4-Hydroxyphenyl)propansäure-methylester und 44.0 g 1-Brom-3-cyclohexylpropan in 100 ml wasserfreiem DMF wird mit 69.9 g Cäsiumcarbonat versetzt und 6 Stunden bei einer Temperatur von 50°C gerührt. Anschließend wird das Reaktionsgemisch auf ca. 800 ml Wasser gegossen, mit 12.3 ml Eisessig versetzt und dreimal mit Ethylacetat extrahiert. Die vereinigten organischen Extrakte werden nacheinander mit Wasser und gesättigter Natriumchlorid-Llösung gewaschen. Nach Trocknen über wasserfreiem Natriumsulfat wird das Lösemittel am Rotationsverdampfer entfernt. Das Produkt wird durch Saugfiltration an Kieselgel mit Cyclohexan/Ethylacetet 20:1 als Laufmittel gereinigt.
Es werden 51.52 g Produkt erhalten.
DC: R_{f}-Wert: 0.47 (Cyclohexan/Ethylacetat 1:1).
HPLC (Methode 2): Rₜ: 6.09 min.
¹H-NMR (300 MHz, DMSO-d₆, δ/ppm): 7.10 (d, 2H), 6.81 (d, 2H), 3.88 (t, 2H), 3.57 (s, 3H), 2.77 (t, 2H), 2.57 (t, 2H), 1.72-1.64 (m, 7H), 1.37-1.09 (m, 6H), 0.93-0.81 (m, 2H).
MS (DCI, NH₃): m/z = 321.9 (M+NH₄⁺).

### Beispiel XVIII

### 3-[4-(3-Cyclohexylpropoxy)phenyl]propan-1-ol

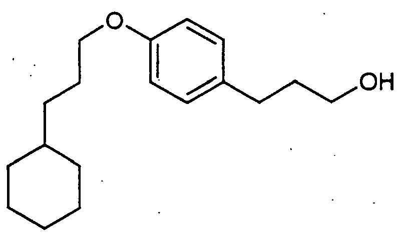

Analog zu dem unter Beispiel II beschriebenen Verfahren wird 3-[4-(3-Cyclohexylpropoxy)phenyl]propan-1-ol durch Reduktion von 3-[4-(3-Cyclohexylpropoxy)-phenyl]propansäure-methylester mit Lithiumaluminiumhydrid erhalten.
DC: R_{f}-Wert: 0.30 (Cyclohexan/Ethylacetat 1:1).
HPLC (Methode 1): Rₜ: 5.60 min.
¹H-NMR (300 MHz, DMSO-d₆, δ/ppm): 7.08 (d, 2H), 6.80 (d, 2H), 4.38 (t, 1H), 3.88 (t, 2H), 3.39 (quart., 2H), 2.51 (t, 2H), 1.71-1.61 (m, 9H), 1.32-1.12 (m, 6H), 0.93-0.81 (m, 2H).
MS (DCI, NH₃): m/z = 394.1 (M+NH₄⁺).

### Beispiel XIX

### 1-(3-Brompropyl)-4-(3-cyclohexylpropoxy)benzol

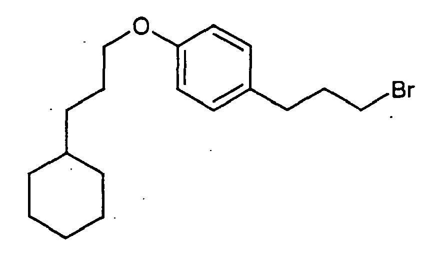

Analog zu dem unter Beispiel IV beschriebenen Verfahren wird 1-(3-Brompropyl)-4-, (3-cyclohexylpropoxy)benzol durch Bromierung mit Triphenylphosphin und Tetrabrommethan aus 3-[4-(3-Cyclohexylpropoxy)phenyl]propan-1-ol erhalten.
DC: R_{f}-Wert: 0.69 (Cyclohexan/Ethylacetat 1:1).
HPLC (Methode 4): Rₜ: 7.27 min.
¹H-NMR (300 MHz, DMSO-d₆, δ/ppm): 7.10 (d, 2H), 6.82 (d, 2H), 3.89 (t, 2H), 3.48 (t, 2H), 2.62 (t, 2H), 2.03 (quint., 2H), 1.73-1.60 (m, 7H), 1.30-1.12 (m, 6H), 0.93-0.81 (m, 2H).
MS (DCI, NH₃): m/z = 338 und 340 (M⁺), 356 und 358 (M+NH₄⁺).

### Beispiel XX

### 3-[4-(3-Cyclohexylpropoxy)phenyl]propylmethansulfonat

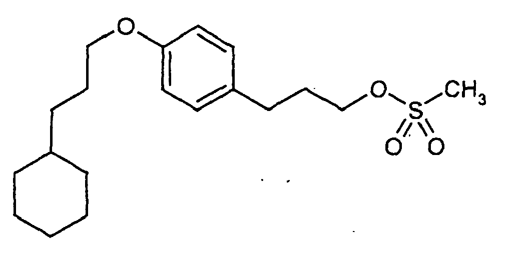

Eine Lösung von 30.89 g 3-[4-(3-Cyclohexylpropoxy)phenyl]propan-1-ol in 250 ml wasserfreiem THF wird bei 0°C mit 43.3 g Diisopropylethylamin und 25.6 g Methansulfonsäurechlorid versetzt. Man lässt eine Stunde bei Raumtemperatur rühren. Anschließend werden je 50 ml Wasser und Ethylacetat zugesetzt. Die Phasen werden getrennt und die organische Phase wird nacheinander mit Wasser und gesättigter Natriumchloridlösung gewaschen. Nach Trocknen über wasserfreiem Magnesiumsulfat wird das Lösemittel am Rotationsverdampfer entfernt. Der Rückstand wird mit Petrolether verrieben. Nach Absaugen und Trocknen werden 37.8 g Produkt erhalten.
HPLC (Methode 5): Rₜ: 5.57 min.
¹H-NMR (200 MHz, CDC1₃, δ/ppm): 7.08 (d, 2H), 6.83 (d, 2H), 4.22 (t, 2H), 3.90 (t, 2H), 3.01 (s, 3H), 2.70 (t, 2H), 2.04 (d quart., 2H), 1.89-0.78 (m, 15H).
MS (DCI, NH₃): m/z = 372.2 (M+NH₄⁺).

### Beispiel XXI

### 3-{4-[4-(Cyclohexyloxy)butoxy]phenyl}propansäure-methylester

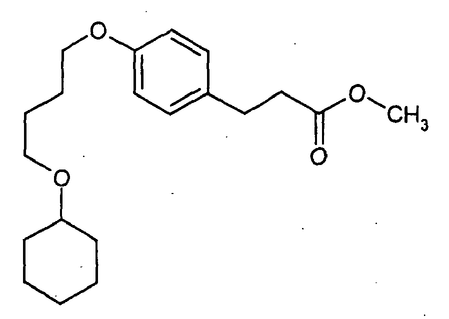

Analog zu dem unter Beispiel I beschriebenen Verfahren wird 3-{4-[4-(Cyclohexyloxy)butoxy]phenyl}propansäure-methylester durch Alkylierung von 3-(4-Hydroxyphenyl)propansäure-methylester mit (4-Brombutoxy)cyclohexan erhalten.
¹H-NMR (200 MHz, CDCl₃, δ/ppm): 7.09 (d, 2H), 6.82 (d, 2H), 3.94 (t, 2H), 3.67 (s, 3H), 3.49 (t, 2H), 3.30-3.15 (m, 1H), 2.88 (t, 2H), 2.59 (t., 2H), 1.94-1.17 (m, 14H).
LC-MS (Methode 6): Rₜ: 3.01 min, m/z = 335 (M+H⁺).

### Beispiel XXII

### 3-{4-[4-(Cyclohexyloxy)butoxy]phenyl}propan-1-ol

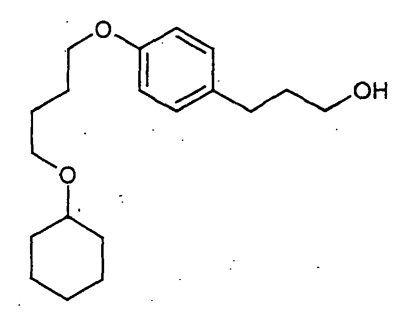

Analog dem unter Beispiel II beschriebenen Verfahren wird 3-{4-[4-(Cyclohexyloxy)butoxy]phenyl}propan-1-ol durch Reduktion von 3-{4-[4-(Cyclohexyloxy)-butoxy]phenyl}propansäure-methylester mit Lithiumaluminiumhydrid erhalten.
¹H-NMR (400 MHz, CDCl₃, δ/ppm): 7.10 (d, 2H), 6.82 (d, 2H), 3.95 (t, 2H), 3.67 (t, 2H), 3.49 (t, 2H), 3.23-3.19 (m, 1H), 2.63 (t, 2H), 1.92-1.20 (16H).
LC-MS (Methode 6): Rₜ: 2.67 min, m/z = 307 (M+H⁺).

### Beispiel XXIII

### 1-(3-Brompropyl)-4-[4-(cyclohexyloxy)butoxy]benzol

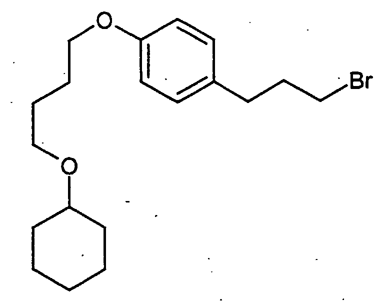

Analog dem unter Beispiel IV beschriebenen Verfahren wird 1-(3-Brompropyl)-4-[4-(cyclohexyloxy)butoxy]benzol durch Bromierung von 3-{4-[4-(Cyclohexyloxy)-butoxy]phenyl}propan-1-ol mit Triphenylphosphin und Tetrabrommethan erhalten.
¹H-NMR (400 MHz, CDCl₃, δ/ppm): 7.09 (d, 2H), 6.82 (d, 2H), 3.97 (t, 2H), 3.50 (t, 2H), 3.38 (t, 2H), 3.23-3.18 (m, 1H), 2.71 (t, 2H), 2,12 (quint, 2H), 1.92-1.82 (m, 4H), 1.77-1.70 (m, 4H), 1.57-1.19 (6H).
MS (EI+): m/z = 369 und 371 (M+H⁺).

### Beispiel XXIV

### 3-{4-[(4-Isopropoxybenzyl)oxy]phenyl}propansäure-methylester

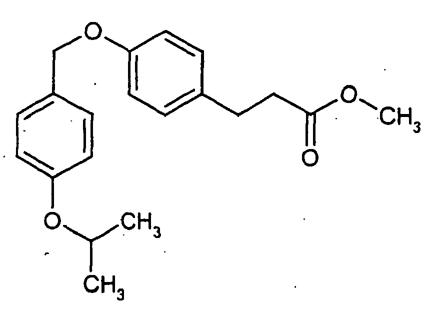

Eine Lösung von 11.0 g 4-Isopropoxybenzylchlorid und 10.7 g 3-(4-Hydroxyphenyl)propansäure-methylester in 120 ml Butyronitril wird mit 12.4 g Kaliumcarbonat versetzt und 15 Stunden zum Rückfluss erhitzt. Anschließend wird das Lösemittel am Rotationsverdampfer abdestilliert. Der Rückstand wird in Ethylacetat aufgenommen und nacheinander mit Wasser und gesättigter Natriumchlorid-Lösung gewaschen. Nach Trocknen über Natriumsulfat wird die organische Phase eingedampft und das Produkt durch Saugfiltration über Kieselgel mit Cyclohexan/Ethylacetat 9:1 gereinigt. Es werden 9.4 g Produkt erhalten.
HPLC (Methode 1): Rₜ: 5.33 min.
¹H-NMR (300 MHz, DMSO-d₆, δ/ppm): 7.32 (d, 2H), 7.11 (d, 2H), 6.90 (d, 2H), 6.89 (d, 2H), 4.94 (s, 2H), 4.60 (sep, 1H), 3.57 (s, 3H), 2.78 (t, 2H), 2.57 (t, 2H), 1.27 (d, 6H).
MS (DCI, NH₃): m/z = 346.2 (M+NH₄⁺).

### Beispiel XXV

### 3-{4-[(4-Isopropoxybenzyl)oxy]phenyl}propan-1-ol

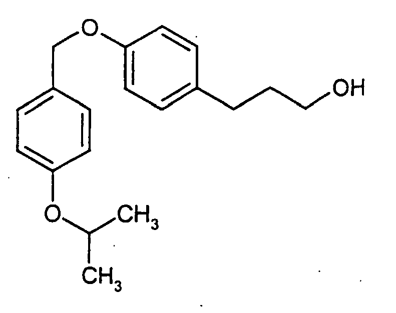

Analog zu dem unter Beispiel II beschriebenen Verfahren wird 3-{4-[(4-Isopropoxybenzyl)oxy]phenyl}propan-1-ol durch Reduktion von 3-{4-[(4-Isopropoxybenzyl)-oxy]phenyl}propansäure-methylester mit Lithiumaluminiumhydrid erhalten.
DC: R_{f}-Wert: 0.48 (Cyclohexan/Ethylacetat 1:1).
HPLC (Methode 1): Rₜ: 4.87 min.
¹H-NMR (300 MHz, CDCl₃, δ/ppm): 7.32 (d, 2H), 7.11 (d, 2H), 6.90 (d, 2H), 6.89 (d, 2H), 4.93 (s, 2H), 4.54 (sep, 1H), 3.68 (t breit, 2H), 2.64 (t, 2H), 1.93-1.79 (m, 2H), 1.33 (d, 6H), 1.26 (s breit, 1H).
MS (DCI, NH₃): m/z = 318 (M+NH₄⁺).

### Beispiel XXVI

### 1-(3-Brompropyl)-4-[(4-isopropoxybenzyl)oxy]benzol

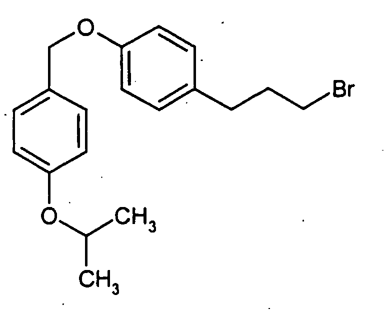

Analog zu dem unter Beispiel IV beschriebenen Verfahren wird 1-(3-Brompropyl)-4-[(4-isopropoxybenzyl)oxy]benzol durch Bromierung mit Triphenylphosphin und Tetrabrommethan aus 3-{4-[(4-Isopropoxybenzyl)oxy]phenyl}propan-1-ol erhalten.
DC: R_{f}-Wert: 0.48 (Cyclohexan/Ethylacetat 20:1).
HPLC (Methode 5): Rₜ: 4.87 min.
¹H-NMR (300 MHz, CDCl₃, δ/ppm): 7.32 (d, 2H), 7.10 (d, 2H), 6.90 (d, 2H), 6.89 (d, 2H), 4.94 (s, 2H), 4.53 (sep, 1H), 3.38 (t, 2H), 2.71 (t, 2H), 2.12 (pent, 2H), 1.33 (d, 6H).
MS (DCI; NH₃): m/z = 380 und 382 (M+NH₄⁺).

### Beispiel XXVII

### 4-(Cyclopropylmethoxy)benzoesäure-methylester

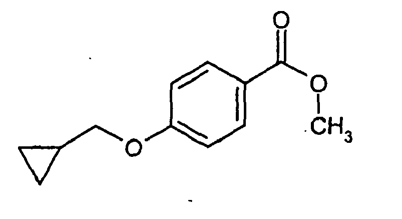

Eine Lösung von 3.0 g 4-Hydroxybenzoesäure-methylester und 2.93 g Cyclopropylmethylbromid in 60 ml wasserfreiem Acetonitril wird mit 3.27 g Kaliumcarbonat versetzt und 15 Stunden zum Rückfluss erhitzt. Anschließend wird das Lösemittel am Rotationsverdampfer entfernt. Der Rückstand wird mit Ethylacetat aufgenommen und nacheinander mit Wasser und gesättigter Natriumchlorid-Lösung gewaschen. Nach Trocknen über festem Natriumsulfat wird zur Trockene eingedampft. Das Produkt wird durch Saugfiltration über Kieselgel mit Cyclohexan/Ethylacetat 5:1 als Laufmittel gereinigt. Es werden 4.0 g eines Feststoffs erhalten.
DC: R_{f}-Wert: 0.29 (Cyclohexan/Ethylacetat 5:1).
HPLC (Methode 1): Rₜ: 4.77 min.
¹H-NMR (400 MHz, DMSO-d₆, δ/ppm): 7.89 (d, 2H), 7.02 (d, 2H), 3.90 (d, 2H), 3.81 (s, 3H), 1.28-1.19 (m, 1H), 0.61-0.57 (m, 2H), 0.36-0.32 (m, 2H).
MS (DCI, NH₃): m/z = 207.1 (M+H⁺), 224.1 (M+NH₄⁺).

### Beispiel XXVIII

### [4-(Cyclopropylmethoxy)phenyl]methanol

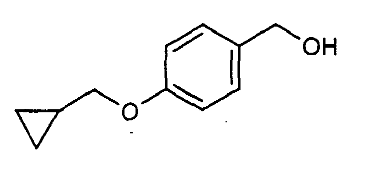

Analog zu dem unter Beispiel II beschriebenen Verfahren wird [4-(Cyclopropylmethoxy)phenyl]methanol durch Reduktion von 4-(Cyclopropylmethoxy)-benzoesäure-methylester mit Lithiumaluminiumhydrid erhalten.
DC: R_{f}-Wert: 0.38 (Cyclohexan/Ethylacetat 1:1).
HPLC (Methode 1): Rₜ: 3.91 min.
¹H-NMR (300 MHz, DMSO-d₆, δ/ppm): 7.20 (d, 2H), 6.86 (d, 2H), 4.98 (t, 1H), 4.40 (d, 2H), 3.78 (d, 2H), 1.27-1.12 (m, 1H), 0.58-0.52 (m, 2H), 0.32-0.27 (m, 2H).
MS (DCI, NH₃): m/z = 161 (M+H⁺), 178 (M+NH₄⁺), 195 (M+N₂H₇⁺).

### Beispiel XXIX

### 1-(Chlormethyl)-4-(cyclopropylmethoxy)benzol

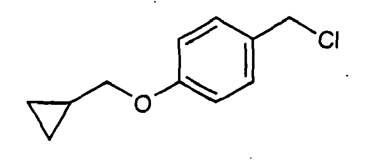

2.54 g [4-(Cyclopropylmethoxy)phenyl]methanol werden in 30 ml wasserfreiem Dichlormethan gelöst und bei Raumtemperatur tropfenweise mit 1.56 ml Thionylchlorid versetzt. Nach beendeter Zugabe lässt man noch 30 Minuten bei Raumtemperatur rühren. Dann wird der Ansatz am Rotationsverdampfer zur Trockene eingeengt. Es werden 2.77 g Produkt erhalten.
DC: R_{f}-wert: 0.60 (Cyclohexan/Ethylacetat 5:1).
¹H-NMR (300 MHz, DMSO-d₆, δ/ppm): 7.33 (d, 2H), 6.91 (d, 2H), 4.70 (s, 2H), 3.81 (d, 2H), 1.28-1.13 (m, 1H), 0.59-0.52 (m, 2H), 0.33-0.28 (m, 2H).
MS (EI+)::m/z = 196 (M⁺).

### Beispiel XXX

### 1-(Chlormethyl)-2-isopropoxybenzol

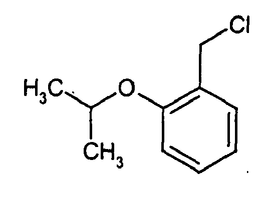

Analog zu dem unter Beispiel XXIX beschriebenen Verfahren wird 1-(Chlormethyl)-2-isopropoxybenzol aus (2-Isopropoxyphenyl)methanol durch Umsetzen mit Thionylchlorid erhalten. Die Reinigung des Produktes erfolgt durch Vakuumdestillation.
Kp. 54 °C (0.16 mbar).
¹H-NMR (200 MHz, DMSO-d₆, δ/ppm): 7.38 (d, 1H), 7.29 (d, 1H), 7.05 (d, 1H), 6.91 (t, 1H), 4.68 (s, 2H), 4.67 (hep, 1H), 1.29 (d, 6H).

### Beispiel XXXI

### [(4-Brombutoxy)methyl]cyclopropan

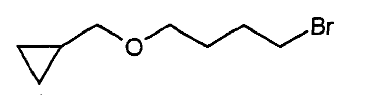

Eine Lösung von 5.0 g Hydroxymethylcyclopropan in 25 ml Toluol wird bei Raumtemperatur portionsweise mit 2.8 g einer 60%igen Suspension von Natriumhydrid in Mineralöl versetzt. Anschließend wird das Gemisch 2 Stunden lang auf 100 °C erhitzt. Danach lässt man auf Raumtemperatur kommen und versetzt mit einer Lösung von 14.9 g 1,4-Dibrombutan in 15 ml Toluol. Man lässt weitere 15 Stunden bei 100 °C rühren. Dann werden bei Raumtemperatur 10 ml Wasser zugesetzt, die Phasen getrennt, und das Toluol am Rotationsverdampfer entfernt. Das Produkt wird durch Vakuumdestillation gewonnen.
Kp. 58-63 °C (0.56 mbar).
¹H-NMR (300 MHz, DMSO-d₆, δ/ppm): 3,53 (t, 2H), 3,38 (t, 2H), 3.18 (d, 2H), 1.89-1.79 (m, 2H), 1.63-1.54 (m, 2H), 1.02-0.91 (m, 1H), 0.47-0.41 (m, 2H), 0.14-0.10 (m, 2H).

### Beispiel XXXII

### cis-3-Hydroxycyclohexancarbonsäure-methylester

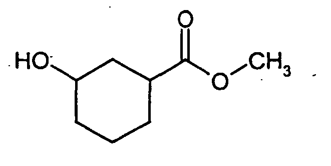

Eine Lösung von 20.7 g 3-Oxocyclohexancarbonsäure-methylester in 520 ml Methanol wird bei einer Temperatur von -78 °C portionsweise mit insgesamt 2.51 g festem Natriumborhydrid versetzt. Nach 2.5 Stunden bei -78 °C wird mit 5 ml Wasser versetzt, und man lässt das Reaktionsgemisch auf Raumtemperatur kommen. Anschließend wird das Lösemittel am Rotationsverdampfer entfernt. Es wird mit Ethylacetat gegen Wasser extrahiert. Nach Trocknen der organischen Phase über wasserfreiem Natriumsulfat, Filtrieren und Entfernen des Ethylacetats werden 19.0 g eines Öls erhalten. Laut NMR besteht das Produkt aus einem Gemisch von zwei Diastereomeren im Verhältnis 94:6. Aus Analogie zu ähnlichen, in der Literatur beschriebenen Reduktionen von in der Position 3 substituierten Cyclohexanonen mit Natriumborhydrid wird geschlossen, dass es sich bei dem Hauptprodukt um das cis-Isomere handeln muss.
¹H-NMR (300 MHz, DMSO-d₆, δ/ppm, Haupisomer): 4.60 (d, 1H), 3.58 (s, 3H), 3.43-3.32 (m, 1H), 2.32 (tt, 1H), 2.03-1.97 (m, 1H), 1.82-1.67 (m, 3H), 1.31-0.96 (m, 4H).
MS (DCI, NH₃): m/z = 159 (M+H⁺), 176 (M+NH₄⁺).

### Beispiel XXXIII

### cis-3-{[(4-Methylphenyl)sulfonyl]oxy}cyclohexancarbonsäure-methylester

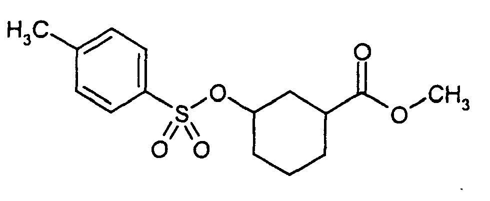

Eine Lösung von 18.97 g cis-3-Hydroxycyclohexancarbonsäure-methylester und 97 ml Pyridin in 410 ml wasserfreiem Dichlormethan wird bei einer Temperatur von 0°C mit 32 g para-Toluolsulfonsäurechlorid versetzt. Das Kältebad wird entfernt, und man lässt das Reaktionsgemisch über Nacht bei Raumtemperatur rühren. Anschließend werden das Lösemittel und überschüssiges Pyridin am Rotationsverdampfer entfernt. Der dabei erhaltene Rückstand wird in Ethylacetat aufgenommen. Es wird vom Ungelösten abfiltriert und das Filtrat eingeengt. Der feste Rückstand wird insgesamt sechsmal mit je ca. 500 ml Petrolether verrührt. Dabei werden 29.7 g eines Feststoffs erhalten.
HPLC (Methode 1): Rₜ: 4.67 min.
¹H-NMR (300 MHz, DMSO-d₆, δ/ppm): 7.79 (d, 2H), 7.48 (d, 2H), 4.53-4.44 (m, 1H), 3.57 (s, 3H), 2.42 (s, 3H), 2.07-1.99 (m, 1H), 1.78-1.69 (m, 3H), 1.49 (quart., 1H), 1.41-1.13 (m, 3H).
MS (DCI, NH₃): m/z = 330 (M+NH₄⁺).

### Beispiel XXXIV

### trans-3-Azidocyclohexancarbonsäure-methylester

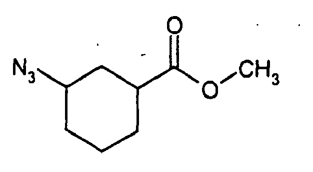

Eine Lösung von 29.6 g cis-3-{[(4-Methylphenyl)sulfonyl]oxy}cyclohexancarbonsäure-methylester in 660 ml DMF wird mit 6.49 g Natriumazid versetzt und 15 Stunden bei 80 °C gerührt. Bei Raumtemperatur werden 1000 ml Wasser zugesetzt und das Produkt dreimal mit je ca. 300 ml Diethylether extrahiert. Der organische Extrakt wird mit gesättigter Natriumchlorid-Lösung gewaschen und über wasserfreiem Natriumsulfat getrocknet. Nach Filtrieren und Einrotieren bei Raumtemperatur werden 16.8 g eines Öls erhalten. Die Zuordnung, dass es sich bei dem Produkt um das trans-Isomere handelt, erfolgt aus der Erfahrung, dass Reaktionen dieses Typs unter Inversion der Stereochemie am Reaktionszentrum erfolgen.
¹H-NMR (200 MHz, DMSO-d₆, δ/ppm): 3.94 (quint., 1H), 3.60 (s, 3H), 2.68-2.56 (m, 1H), 1.82-1.72 (m, 3H), 1:62-1.43 (m, 5H).
MS (DCI, NH₃): m/z = 201 (M+NH₄⁺); 218 (M+N₂H₇⁺).

### Beispiel XXXV

### trans-3-Aminocyclohexancarbonsäure-methylester - Hydrochlorid

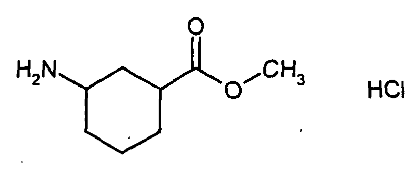

### Methode 1:

Eine Lösung von 16.45 g trans-3-Azidocyclohexancarbonsäure-methylester in 502 ml Methanol wird zunächst mit 1.68 g 10% Palladium auf Kohle und 17.29 g Ammoniumformiat versetzt und dann 1 Stunde zum Rückfluss erhitzt. Anschließend wird über wenig Tonsil filtriert und einrotiert. Der Rückstand wird in wenig Ethylacetat gelöst und mit 30 ml 4-molarer Lösung von Chlorwasserstoff in Dioxan versetzt. Nach 20 Minuten wird zur Trockene eingedampft. Es werden 16.3 g eines Feststoffs erhalten.
¹H-NMR (400 MHz, DMSO-d₆, δ/ppm): 8.23 (s breit, 3H), 3.61 (s, 3H), 3.07-3.00 (m, 1H), 2.51-2.42 (m, 1H), 2.17 (d breit, 1H), 1.93-1.74 (m, 3H), 1.42-1.11 (m,4H). MS (DCI, NH₃): m/z = 158 (M+H⁺).

### Methode 2:

trans-3-Aminocyclohexancarbonsäure-methylester - Hydrochlorid kann auch nach dem unter Beispiel XXXV, Methode 1, beschriebenen Verfahren aus trans-3-Azidocyclohex-4-encarbonsäure-methylester erhalten werden. Dabei wird lediglich die Menge des eingesetzten Ammoniumformiats verdoppelt.

### Beispiel XXXVI

### trans-3-Azidocyclohex-4-encarbonsäure

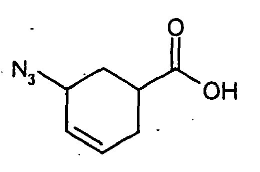

Eine Lösung von 18.4 g racemischem cis-6-Oxabicyclo[3.2.1]oct-3-en-7-on in 175 ml THF wird mit einer Lösung von 10.6 g Natriumazid in 70 ml Wasser versetzt.. Dann wird das Reaktionsgemisch 15 Stunden zum Rückfluss erhitzt. Anschließend wird das THF bei einer Badtemperatur von 30°C am Rotationsverdampfer abgezogen. Die verbleibende wässrige Phase wird mit 165 ml 2-molarer Natronlauge versetzt und zweimal mit je 110 ml Toluol und einmal mit Diethylether extrahiert. Die wässrige Phase wird dann bei ca. 10°C mit konzentrierter Salzsäure angesäuert. Das Produkt wird mit Dichlormethan extrahiert. Der organische Extrakt wird über wasserfreiem Natriumsulfat getrocknet, filtriert und eingeengt. Es werden 19.7 g eines Öls erhalten, das beim Lagern im Kühlschrank fest wird. Die Zuordnung, dass es sich bei dem Produkt um das trans-Isomere handelt, erfolgt aus der Erfahrung, dass Reaktionen dieses Typs unter Inversion der Stereochemie am Reaktionszentrum erfolgen.
HPLC (Methode 1): Rₜ: 3.62 min.
¹H-NMR (300 MHz, DMSO-d₆, δ/ppm): 12.38 (s, breit, 1H), 6.09-6.03 (m, 1H), 5.82-5.77 (m, 1H), 4.14 (pseudo-d, 1H), 2.59-2.48 (m, 1H, teilweise überdeckt durch DMSO-Signal), 2.37-2.28 (m, 1H), 2.18-2.07 (m, 1H), 2.01-1.96 (m, 1H), 1.82-1.73 (m, 1H).
MS (ESI-): m/z = 166 (M-H⁻), 333 (2M-H⁻).

### Beispiel XXXVII

### trans-3-Azidocyclohex-4-encarbonsäure-methylester

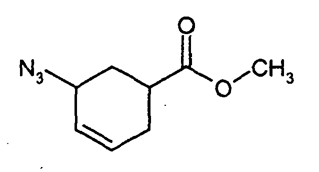

Eine Lösung von 19.5 g trans-3-Azidocyclohex-4-encarbonsäure in 1.5 l wasserfreiem Methanol wird bei 0°C tropfenweise mit 32.6 ml Trimethylsilylchlorid versetzt. Nach einer Stunde bei 0°C wird noch eine weitere Stunde bei Raumtemperatur gerührt. Dann wird zur Trockene eingedampft. Es werden 19.1 g eines Öls erhalten.
DC (Cyclohexan/Ethylacetat 1:1): R_{f}: 0.75.
HPLC (Methode 1): Rₜ: 4.24 min.
¹H-NMR (300 MHz, DMSO-d₆, δ/ppm): 6.08-6.02 (m, 1H), 5.82-5.77 (m, 1H), 4.17 (pseudo-d, 1H), 3.62 (s, 3H), 2.69-2.59 (m,-1H), 2.38-2:29 (m, 1H), 2.20-2.08 (m, 1H), 2.02-1.94 (m, 1H), 1.85-1.76 (m, 1H).
MS (DCI, NH₃): m/z = 199 (M+NH₄⁺).

### Beispiel XXXVIII

### 3-{4-[(Triisopropylsilyl)oxy]phenyl}propansäure-methylester

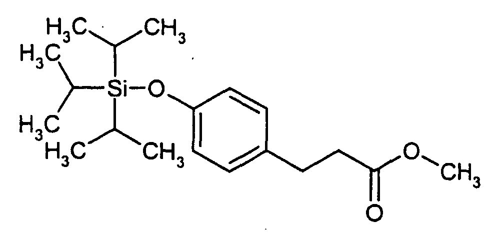

Bei Raumtemperatur wird eine Lösung von 10 g 3-(4-Hydroxyphenyl)propansäure-methylester und 11.77 g Imidazol in 22 ml DMF mit 13 ml Triisopropylsilylchlorid versetzt. Man lässt bei Raumtemperatur rühren. Nach 15 Stunden wird der Ansatz auf 200 ml 5%ige, wässrige Natriumdihydrogenphosphat-Lösung gegossen und mit Ethylacetat extrahiert. Die organische Phase wird mit Wasser und gesättigter Natriumchlorid-Lösung gewaschen. Trocknen über wasserfreiem Natriumsulfat, Filtrieren, Einrotieren. Das Rohprodukt wird durch Saugfiltration an Kieselgel mit Cyclohexan/Ethylacetat 30:1 gereinigt. Es werden 19.38 g eines Öls erhalten.
DC (Cyclohexan/Ethylacetat 9:1): R_{f}: 0.53.
HPLC (Methode 4): Rₜ: 6.27 min.
¹H-NMR (200 MHz, DMSO-d₆, δ/ppm): 7.09 (d, 2H), 6.77 (d, 2H), 3.56 (s, 3H), 2.78 (t, 2H), 2.58 (t, 2H), 1.28-1.13 (m, 3H), 1.05 (d, 18H).
MS (DCI, NH₃): m/z = 354 (M+NH₄⁺).

### Beispiel XXXIX

### 3-{4-[(Triisopropylsilyl)oxy]phenyl}propan-1-ol

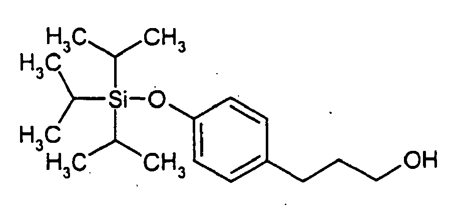

14.4 ml einer 1-molaren Lösung von Lithiumaluminiumhydrid in THF wird vorgelegt. Unter Rühren wird eine Lösung von 19.22 g 3-{4-[(Triisopropylsilyl)-oxy]phenyl}propansäure-methylester in 58 ml THF so zugetropft, dass das Gemisch gerade anfängt zu sieden. Nach beendeter Zugabe lässt man noch 30 Minuten bei Raumtemperatur rühren. Dann wird überschüssiges Lithiumaluminiumhydrid durch vorsichtige Zugabe von 1 ml Methanol hydrolysiert. Der Ansatz wird auf 20%ige, wässrige Natrium-kalium-tartrat-Lösung gegossen und mit Ethylacetat extrahiert. Die organische Phase wird nacheinander mit Wasser und gesättigter Natriumchlorid-Lösung gewaschen. Trocknen über wasserfreiem Natriumsulfat. Nach Filtrieren und Einrotieren wird das Produkt durch MPLC an Kieselgel mit Cyclohexan/Ethylacetat 9:1 als Laufmittel gereinigt. Es werden 10.7 g eines Öls erhalten.
DC (Cyclohexan/Ethylacetat 9:1): R_{f}: 0.17.
HPLC (Methode 4): Rₜ: 5.78 min.
¹H-NMR (300 MHz, DMSO-d₆, δ/ppm): 7.05 (d, 2H), 6.77 (d, 2H), 4.39 (t, 1H), 3.38 (quart, 2H), 2.53 (t, 2H), 1:67 (m, 2H), 1.28-1.16 (m, 3H), 1.05 (d, 18H).
MS (DCI, NH₃): m/z = 326 (M+NH₄⁺), 634 (2M+NH₄⁺).

### Beispiel XL

### [4-(3-Brompropyl)phenoxy](triisopropyl)silan

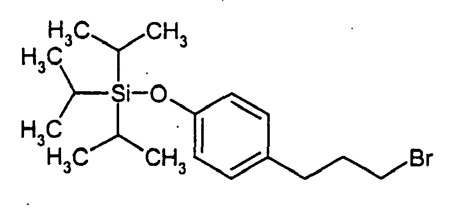

Eine Lösung von 16.85 g 3-{4-[(Triisopropylsilyl)oxy]phenyl}propan-1-ol in 15 ml THF wird mit 17.19 g Triphenylphosphin versetzt. Sobald es gelöst ist, werden 21.73 g Tetrabrommethan zugegeben und das Ganze bei Raumtemperatur gerührt. Das Reaktionsgemisch erwärmt sich dabei zunächst etwas und nach ca. 5 Minuten beginnt sich ein Feststoff abzuscheiden. Nach 2 Stunden wird vom ausgefallenen Niederschlag abfiltriert und das Lösemittel am Rotationsverdampfer entfernt. Der Rückstand wird mittels MPLC über Kieselgel mit Cyclohexan/Ethylacetat 50:1 als Laufmittel gereinigt. Es werden 17.53 g eines Öls erhalten.
DC (Cyclohexan/Ethylacetat 4:1): R_{f}: 0.65.
HPLC (Methode 4): Rₜ: 8.19 min.
¹H-NMR (300 MHz, DMSO-d_{6,} δ/ppm): 7.09 (d, 2H), 6.79 (d, 2H), 3.48 (t, 2H), 2.63 (t, 2H), 2.07 (m, 2H), 1.28-1.17 (m, 3H), 1.07 (d, 18H).
MS (DCI, NH₃): m/z = 388 und 390 (M+NH₄⁺).

### Beispiel XLI

### 3-({[3-(Methoxycarbonyl)cyclohexyl]amino}carbonyl)-4-(3-{4-[(triisopropylsilyl)-oxy]phenyl}propoxy)benzoesäure-methylester

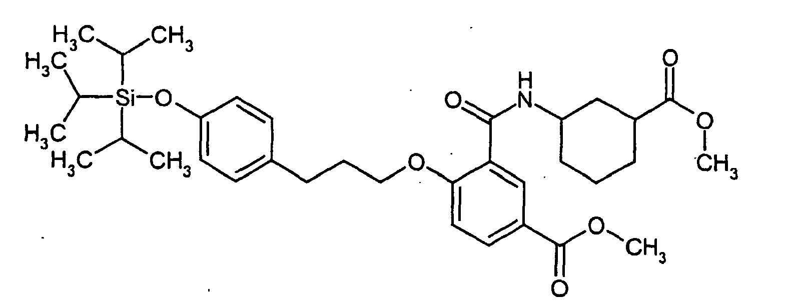

Eine Lösung von 2.66 g [4-(3-Brompropyl)phenoxy](triisopropyl)silan und 2.40 g des (+)-B-Enantiomers von 4-Hydroxy-3-({[3(methoxycarbonyl)cyclohexyl]amino}carbonyl)benzoesäure-methylester (siehe Beispiel IX, Methode 2) in 100 ml Butyronitril wird mit 1.19 g Kaliumcarbonat versetzt und 16 Stunden bei 120 °C Badtemperatur gerührt. Anschließend wird das Lösemittel abrotiert und der Rückstand zwischen Ethylacetat und wässriger Natriumdihydrogenphosphat-Lösung verteilt. Die organische Phase wird nacheinander mit Wasser und gesättigter Natriumchlorid-Lösung gewaschen. Trocknen über wasserfreiem Natriumsulfat. Nach Filtrieren und Einrotieren wird das Produkt durch Saugfiltration an Kieselgel mit Cyclohexan/Ethylacetat 2:1 als Laufmittel gereinigt. Es werden 3.91 g eines Öls erhalten.
DC (Cyclohexan/Ethylacetat 1:1): R_{f}: 0.51.
HPLC (Methode 4): Rₜ: 7.14 min.
¹H-NMR (300 MHz, DMSO-d₆, δ/ppm): 8.21 (d, 1H), 8.08 (d breit, 1H), 8.01 (dd, 1H), 7.21 (d, 1H), 7.09 (d, 2H), 6.78 (d, 2H), 4.14 (pseudo-t, 3H), 3.82 (s, 3H), 3.57 (s, 3H), 2.70 (pseudo-t, 3H), 2.07 (m, 2H), 1.82 (m, 2H), 1.70-1.48 (m, 6H), 1.27-1.17 (m, 3H), 1.05 (d, 18H).
MS (DCI, NH₃): m/z = 626 (M+H⁺), 643 (M+NH₄⁺).

### Beispiel XLII

### 4-[3-(4-Hydroxyphenyl)propoxy]-3-({[3-(methoxycarbonyl)cyclohexyl]amino}carbonyl)benzoesäure-methylester

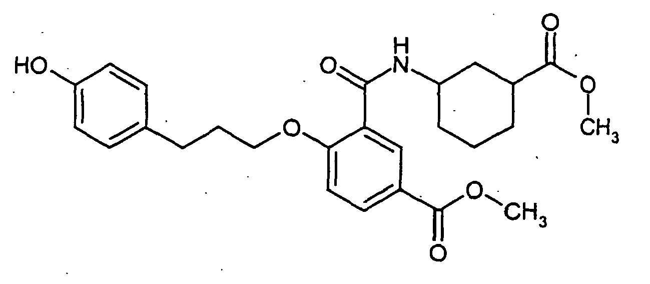

Eine Lösung von 3.90 g 3-({[3-(Methoxycarbonyl)cyclohexyl]amino}carbonyl)-4-(3-{4-[(triisopropylsilyl)oxy]phenyl}propoxy)benzoesäure-methylester in 40 ml THF wird bei 0 °C mit 6.5 ml einer 1-molaren Lösung von Tetra-n-butylammoniumfluorid in THF versetzt. Es wird 10 Minuten bei Raumtemperatur nachgerührt. Dann wird der Ansatz zur Trockene einrotiert, mit gesättigter Ammoniumchlorid-Lösung versetzt und mit Ethylacetat extrahiert. Nach Trocknen über wasserfreiem Natriumsulfat wird filtriert und einrotiert. Das erhaltene Rohprodukt wird durch Saugfiltration an Kieselgel mit Cyclohexan/Ethylacetat 1:1 als Laufmittel gereinigt. Es werden 2.64 g eines Öls erhalten.
DC (Cyclohexan/Ethylacetat 1:1): R_{f}: 0.17.
HPLC (Methode 1): Rₜ: 4.48 min.
¹H-NMR (300 MHz, DMSO-d_{6,} δ/ppm): 9.11 (s, 1H), 8.22 (d, 1H), 8.08 (d breit, 1H), 8.01 (dd, 1H), 7.22 (d, 1H), 7.00 (d, 2H), 6.68 (d, 2H), 4.13 (pseudo-t, 3H), 3.83 (s, 3H), 3.58 (s, 3H), 2.75-2.62 (m, 3H), 2.05 (quint, 2H), 1.82 (m, 2H), 1.69-1.49 (m, 6H).
MS (DCI, NH₃): m/z = 470 (M+H⁺), 487 (M+NH₄⁺).

### Beispiel XLIII

### (2E)-3-{4-[5-Phenoxypent-1-en-1-yl]phenyl}acrylsäureethylester (E/Z-Gemisch)

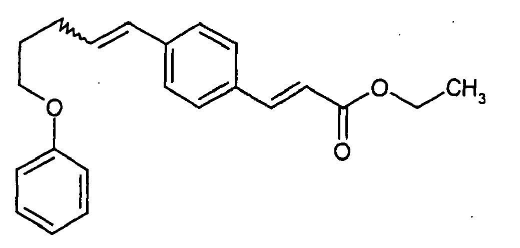

Unter Argon werden 3.16 g (4-Phenoxybutyl)(triphenyl)phosphoniumbromid in 25 ml THF suspendiert und auf -40 °C gekühlt. Man tropft 2.36 ml n-Butyllithium (1.6-molar in Hexan) zu, erwärmt nach 5 Minuten auf 0 °C und kühlt nach 15 Minuten wieder auf -40 °C. Eine Lösung von 0.70 g 4-Formylzimtsäureethylester in THF wird zugetropft und die Reaktionsmischung nach 5 Minuten auf Raumtemperatur erwärmt. Das Lösungsmittel wird am Rotationsverdampfer entfernt. Man erhält 0.75 g Produkt als E/Z-Gemisch.
HPLC (Methode 5): Rₜ: 5.44 min.
LC-MS (Methode 8): Rₜ: 4.0 min, m/z (EI+) = 336.

### Beispiel XLIV

### (2E)-3-{4-[5-Phenoxypent-1-en-1-yl]phenyl}prop-2-en-1-ol (E/Z-Gemisch)

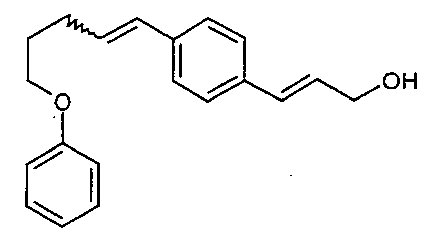

Eine Lösung von 457 mg (2E)-3-{4-[5-Phenoxypent-1-en-1-yl]phenyl}acrylsäureethylester (E/Z-Gemisch) in 4 ml Toluol wird auf -30°C gekühlt und 0.53 ml einer Lösung von Diisobutylaluminiumhydrid (ca. 5.5-molar in Hexan) zugetropft. Nach 30 Minuten erwärmt man auf 0°C, gibt gesättigte Ammoniumchlorid-Lösung hinzu, extrahiert mit Dichlormethan und trocknet die organische Phase über Natriumsulfat. Das Lösungsmittel wird am Rotationsverdampfer entfernt. Man erhält 85 mg Produkt.
HPLC (Methode 5): Rₜ: 4.91 min.
¹H-NMR (200 MHz, CDCl₃, δ/ppm): 7.40-7.19 (m, 6H), 6.98-6.83 (m, 3H), 6.67-6.29 (m, 3H), 5.70 (dt, 1H), 4.33 (t, 2H), 3.98 (t, 2H), 2.55 (dq, 2H), 2.05-1.86 /m, 2H), 1.42 (t, 1H).
MS (DCI, NH₃): m/z = 312 (M+NH₄⁺).

### Beispiel XLV

### 3-[4-(5-Phenoxypentyl)phenyl]propan-1-ol

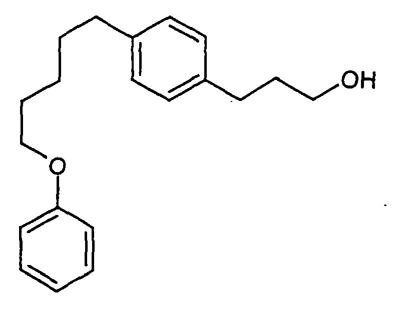

Zu einer Lösung von 98 mg (2E)-3-{4-[5-Phenoxypent-1-en-1-yl]phenyl}prop-2-en-1-ol in ca. 5 ml Ethylacetat werden ca. 5 mg Palladium auf Kohle (10%) gegeben und über Nacht bei Raumtemperatur unter 1 bar Wasserstoff hydriert. Man filtriert vom Katalysator ab, engt ein und reinigt den Rückstand über Kieselgel mit Cyclohexan/Ethylacetat 3:1. Es werden 53 mg Produkt erhalten.
HPLC (Methode 5):-Rₜ: 5.07 min.
¹H-NMR (400 MHz, CDCl₃, δ/ppm): 7.31-7.23 (m, 5H), 7.21-7.19 (m, 1H), 6.96-6.86 (m, 3H), 3.95 (t, 2H), 3.68 (q, 2H), 2.68 (t, 2H); 2.62 (t, 2H), 1.94-1.87 (m, 4H), 1.73-1,63 (m, 2H), 1.56-1.47 (m, 2H), 1.37-1.30 (m, 1H).
LC-MS (Methode 9): Rₜ: 2.72 min, m/z (EI+) = 298.

### Beispiel XLVI

### 1-(3-Iodpropyl)-4-(5-phenoxypentyl)benzo

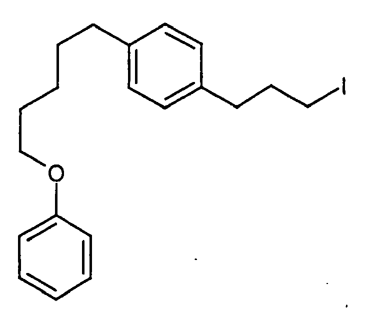

Eine Lösung von 53 mg 3-[4-(5-Phenoxypentyl)phenyl]propan-1-ol in Diethylether/Acetonitril 3:1 wird auf 0°C gekühlt und unter Argon 24 mg Imidazol, 70 mg Triphenylphosphin und 68 mg Iod zugegeben. Man rührt 30 Minuten und beendet die Reaktion durch Zugabe von konzentrierter Natriumthiosulfat-Lösung. Es wird mit Ethylacetat extrahiert, die organische Phase mit gesättigter Natriumchlorid-Lösung gewaschen, über Natriumsulfat getrocknet und eingeengt. Man reinigt den Rückstand über Kieselgel mit Cyclohexan/Ethylacetat 40:1. Es werden 34 mg Produkt erhalten.
MS (DCI, NH₃): m/z = 426 (M+NH₄⁺).

### Beispiel XLVII

### 4-(Biphenyl-4-ylmethoxy)phenylacetat

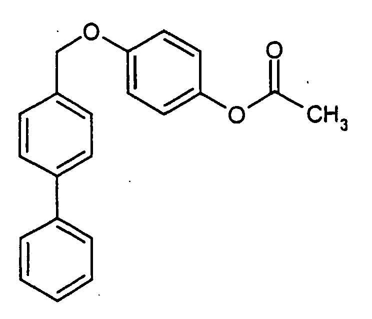

Zu einer Lösung von 2.80 g 4-Biphenylmethylbromid und 1.72 g 4-Hydroxyphenylacetat in 75 ml Butyronitril werden 2.35 g wasserfreies Kaliumcarbonat gegeben und die Reaktionsmischung über Nacht bei 120°C gerührt. Man saugt ab, wäscht mit Acetonitril und engt ein. Der Rückstand wird in Ethylacetat aufgenommen, mit Wasser und gesättigter Natriumchlorid-Lösung gewaschen, über Natriumsulfat getrocknet, abfiltriert und das Lösungsmittel am Rotationsverdampfer entfernt. Anschließend rührt man mit Ethylacetat aus, saugt ab und wäscht die erhaltenen Kristalle mehrfach mit Ethylacetat. Es werden 1.36 g Produkt erhalten: Aus der Mutterlauge lassen sich nach Einengen und Chromatographie an Kieselgel mit Dichlormethan weitere 0.36 g Produkt isolieren.
HPLC (Methode 5): Rₜ: 5.04 min.
¹H-NMR (200 MHz, DMSO-d₆, δ/ppm): 7.73-7.62 (m, 4H), 7.58-7.32 (m, 5H), 7.05 (s, 4H), 5.15 (s, 2H), 2.24 (s, 3H).
MS (EI): m/z = 318 (M⁺).

### Beispiel XLVIII

### 4-(Biphenyl-4-ylmethoxy)phenol

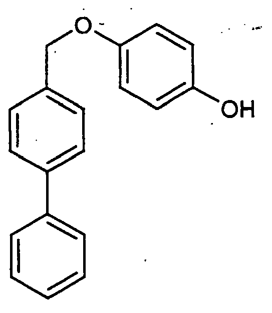

Man legt 4.5 ml einer Lösung von Lithiumaluminiumhydrid (1-molar in THF) vor und tropft 1.30 g 4-(Biphenyl-4-ylmethoxy)phenylacetat in 25 ml THF langsam zu. Nach 30 Minuten ist der Umsatz vollständig, und es wird Salzsäure (1-molar) zugetropft, bis sich der anfangs ausgefallene Niederschlag wieder löst. Man extrahiert mit Dichlormethan, wäscht die organische Phase je einmal mit Wasser und gesättigter Natriumchlorid-Lösung, trocknet über Natriumsulfat, filtriert ab und entfernt das Lösungsmittel am Rotationsverdampfer. Es werden 1.11 g Produkt erhalten.
HPLC (Methode 5): Rₜ: 4.75 min.
¹H-NMR (200 MHz, CDCl₃, δ/ppm): 8.89 (s, 1H), 7.66. (d, 4H), 7.53-7.33 (m, 5H), 6.84 (d, 2H), 6.68 (d, 2H), 5.04 (s, 2H).
MS (DCI, NH₃): m/z = 294 (M+NH₄⁺).

### Beispiel XLIX

### [4-(Biphenyl-4-ylmethoxy)phenoxy]essigsäure-methylester

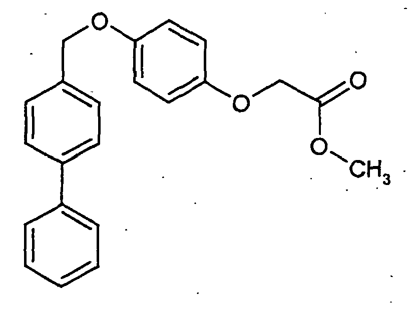

Zu einer Lösung von 1.10 g 4-(Biphenyl-4-ylmethoxy)phenol und 0.41 ml Bromessigsäuremethylester in 20 ml DMF werden 1.95 g Cäsiumcarbonat gegeben, die Reaktionsmischung über Nacht bei Raumtemperatur und anschließend noch 4 Stunden bei 80°C gerührt. Man entfernt das Lösungsmittel am Rotationsverdampfer, verreibt den Rückstand mit Wasser, saugt die ausgefallenen Kristalle ab und trocknet über Nacht bei 40°C im Hochvakuum. Es werden 1.36 g Produkt erhalten.
HPLC (Methode 5): Rₜ: 4.98 min.
¹H-NMR (200 MHz, CDCl₃, δ/ppm): 7.66-7.41 (m, 9H), 6.98-6.81 (m, 4H), 5.05 (s, 2H), 4.58 (s, 2H), 3.81 (s, 3H).
MS (DCI, NH₃): m/z = 366 (M+NH₄⁺).

### Beispiel L

### 2-[4-(Biphenyl-4-ylmethoxy)phenoxy]ethanol

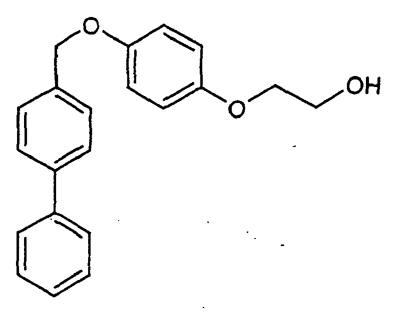

Eine Lösung von 1.21 g [4-(Biphenyl-4-ylmethoxy)phenoxy]essigsäuremethylester in 40 ml THF wird vorgelegt und 3.82 ml Lithiumaluminiumhydrid (1-molar in THF) langsam zugetropft. Man rührt bei Raumtemperatur über Nacht. Nach Zugabe von Salzsäure (1-molar), Dichlormethan und Wasser werden die unlöslichen Kristalle abgesaugt und über Nacht bei 40°C im Hochvakuum getrocknet (221 mg Produkt). Aus der organischen Phase erhält man nach Waschen mit Wasser und gesättigter Natriumchlorid-Lösung, Trocknen über Natriumsulfat, Filtration und Entfernen des Lösungsmittels im Vakuum weitere 700 mg Produkt.
HPLC (Methode 5): Rₜ: 4.73 min.
¹H-NMR (200 MHz, DMSO-d₆, δ/ppm): 7.77-7.32 (m, 9H), 6.98-6.80 (m, 4H), 5.07 (s, 2H), 4.84 (t, 1H), 3.92 (s, 2H), 3.77-3.60 (m, 2H).
MS (DCI, NH₃): m/z = 338 (M+NH₄⁺).

### Beispiel LI

### 4-{[4-(2-Bromethoxy)phenoxy]methyl}biphenyl

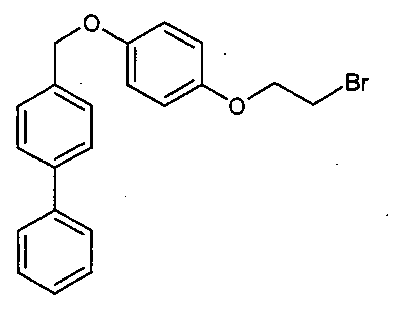

Unter Argon werden 800 mg 2-[4-(Biphenyl-4-ylmethoxy)phenoxy]ethanol in 25 ml Dichlormethan vorgelegt, 1.16 g Tetrabrommethan zugegeben und 10 Minuten bei Raumtemperatur gerührt. Man kühlt auf 0°C ab, gibt 1.83 g Triphenylphosphin zum Ansatz, rührt eine Stunde bei 0°C und 2 Stunden bei Raumtemperatur. Es wird eingeengt, der Rückstand in wenig Dichlormethan aufgenommen und über Kieselgel abfiltriert. Man erhält 480 mg Produkt.
HPLC (Methode 5): Rₜ: 5.30 min.
¹H-NMR (300 MHz, DMSO-d₆, δ/ppm): 7.73-7.63 (m, 4H), 7.56-7.33 (m, 5H), 7.02-6.88 (m, 4H), 5.11 (s, 2H), 4.26 (t, 2H), 3.77 (t, 2H).
MS (EI): m/z = 383 und 385 (M⁺).

### Beispiel LII

### {4-[5-Phenoxypent-1-en-1-yl]phenoxy}essigsäure-methylester (E/Z-Gemisch)

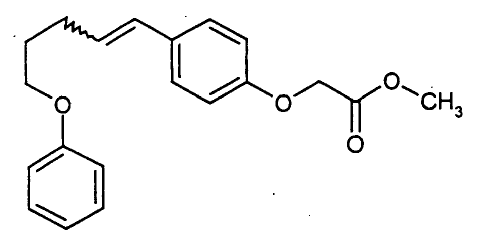

Die Herstellung erfolgt analog Beispiel XLIII aus (4-Formylphenoxy)-essigsäuremethylester und (4-Phenoxybutyl)(triphenyl)phosphoniumbromid. Man erhält das Produkt als E/Z-Gemisch.
HPLC (Methode 5): Rₜ: 5.03 min.
¹H-NMR (300 MHz, CDCl₃, δ/ppm): 7.32-7.18 (m, 4H), 6.96-6.81 (m, 5H), 6.43-6.34 (m, 1H), 6.12 (dt, 0.5H), 5.63 (dt, 0.5H), 4.62 (d, 2H), 4.00 (q, 2H), 3.81 (d, 3H), 2.50 (dq, 1H), 2.38 (dq, 1H), 2.01-1.87 (m, 2H).
LC-MS (Methode 10): Rₜ: 3.49 min, m/z = 233 (M- C₆H₅O).

### Beispiel LIII

### 2-{4-[(1Z)-5-Phenoxypent-1-en-1-yl]phenoxy}ethanol

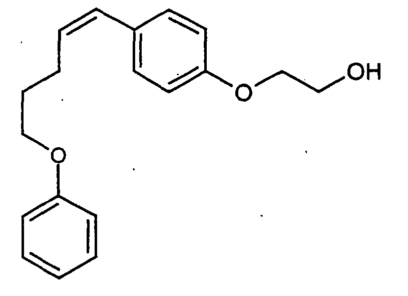

Die Herstellung erfolgt analog Beispiel XLIV aus {4-[5-Phenoxypent-1-en-1-yl]-phenoxy}essigsäuremethylester (E/Z-Gemisch). Die Trennung der Doppelbindungsisomere erfolgt durch HPLC (Stability C-30 5 µm 250 mm x 20 mm Nr. 20101; Acetonitril/Wasser 3:1; Fluss: 25 ml/min; UV-Detektion: 210 nm).
HPLC (Methode 5): Rₜ: 4.80 min.
¹H-NMR (300 MHz, CDCl₃, δ/ppm): 7.30-7.20 (m, 4H), 6.93 (t, 1H), 6.60-6.85 (m, 4H), 6.39 (d, 1H), 5.62 (dt, 1H), 4.11-4.07 (m, 2H), 4.01-3.95 (m, 4H), 2.51 (dq, 2H), 2.00-1.91 (m, 3H).
LC-MS (Methode 9): Rₜ: 2.51 min, m/z = 205 (M - C₆H₅O).

### Beispiel LIV

### 1-(2-Iodethoxy)-4-[(1Z)-5-phenoxypent-1-en-1-yl]benzol

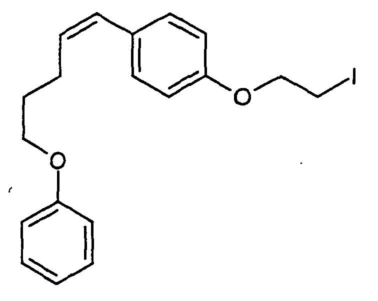

Die Herstellung erfolgt analog Beispiel XLVI aus 2-{4-[(1Z)-5-Phenoxypent-1-en-1-yl]phenoxy}ethanol.
HPLC (Methode 5): Rₜ: 5.55 min.

### Beispiel LV

### 1-Iod-4-(4-phenoxybutoxy)benzol

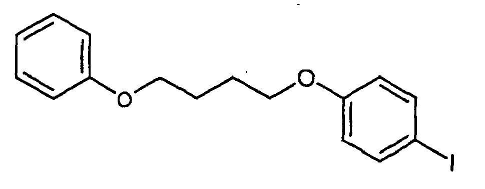

Die Herstellung erfolgt analog Beispiel XVII aus 4-Phenoxybutylbromid und 4-Iodphenol.
HPLC (Methode 5): Rₜ: 5.56 min.
¹H-NMR (200 MHz, CDCl₃, δ/ppm): 7.60-7.49 (m, 2H), 7.37-7.20 (m, 2H), 7.00-6.82 (m, 3H), 6.74-6.62 (m, 2H), 4.10-3.90 (m, 4H), 2.06-1.89 (m, 4H).
MS (DCI, NH₃): m/z = 386 (M+NH₄⁺).

### Beispiel LVI

### 3-[4-(4-Phenoxybutoxy)phenyl]prop-2-in-1-ol

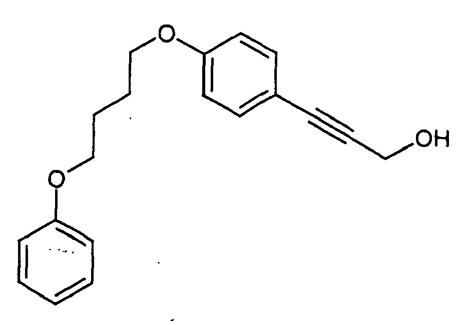

Unter Argon wird eine Lösung von 1.00 g 1-Iod-4-(4-phenoxybutoxy)benzol in 15 ml Triethylamin vorgelegt und 155 mg Kupfer(I)iodid, 57 mg Triphenylphosphin und 152 mg Bis(triphenylphosphin)palladium(II)chlorid zugegeben. Man tropft 457 mg Propargylalkohol zum Ansatz und rührt eine Stunde bei 60°C. Die Reaktionsmischung wird über Celite abfiltriert und mehrfach mit Ethylacetat und Dichlormethan nachgewaschen. Man wäscht die vereinigten Phasen dreimal mit Wasser, zweimal mit verdünnter Salzsäure und einmal mit gesättigter Natriumchlorid-Lösung, trocknet über Natriumsulfat, filtriert und engt ein. Der Rückstand wird über Kieselgel mit Cyclohexan/Ethylacetat 5:1 gereinigt. Man erhält 737 mg Produkt.
¹H-NMR (300 MHz, CDCl₃, δ/ppm): 7.40-7.23 (m, 4H), 6.96-6.79 (m, 5H), 4.48 (d, 2H), 4.08-3.98 (m, 4H), 2.03-1.94 (m, 4H), 1.57 (t, 1H).
MS (DCI, NH₃): m/z = 297 (M+NH₄⁺).

### Beispiel LVII

### 1-(3-Bromprop-1-in-1-yl)-4-(4-phenoxybutoxy)benzol

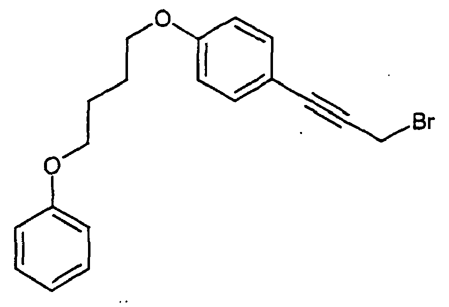

Die Herstellung erfolgt analog Beispiel IV aus 3-[4-(4-Phenoxybutoxy)phenyl]prop-2-in-1-ol.
HPLC (Methode 5): Rₜ: 5.32 min.
¹H-NMR (300 MHz, CDCl₃, δ/ppm): 7.40-7.23 (m, 4H), 6.97-6.79 (m, 5H), 4.16 (s, 2H), 4.10-3.96 (m, 4H), 2.03-1.92 (m, 4H).
MS (DCI, NH₃): m/z = 376 und 378 (M+NH₄⁺).

### Beispiel LVIII

### 2-[4-(5-Phenoxypentyl)phenoxy]ethanol

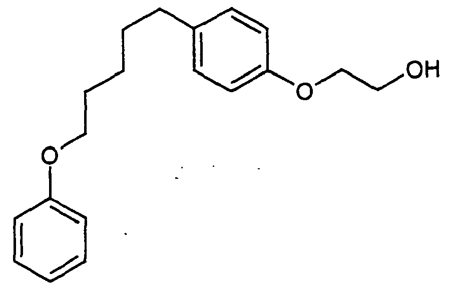

Die Herstellung erfolgt analog Beispiel XLV aus 2-{4-[5-Phenoxypent-1-en-1-yl]phenoxy}ethanol.
HPLC (Methode 5): Rₜ: 4.88 min.
¹H-NMR (300 MHz, CDCl₃, δ/ppm): 7.30-7.23 (m, 2H), 7.12-7.07 (m, 2H), 6.95-6.82 (m, 5H), 4.09-4.05 (m, 2H), 3.98-3.91 (m, 4H), 2.59 (t, 2H), 1.99 (t, 1H), 1.85-1.75 (m, 2H), 1.72-1.61 (m, 2H), 1.55-1.43 (m, 2H).
LC-MS (Methode 10): Rₜ: 3.35 min, m/z (EI+) = 300.

### Beispiel LIX

### 1-(2-Iodethoxy)-4-(5-phenoxypentyl)benzol

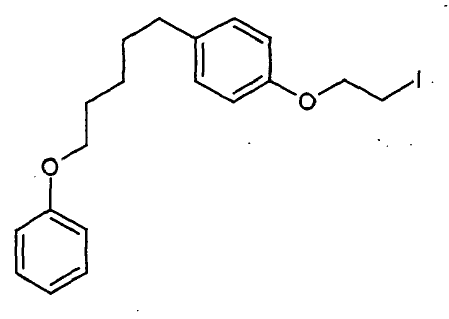

Die Herstellung erfolgt analog Beispiel XLVI aus 2-[4-(5-Phenoxypentyl)-phenoxy]ethanol.
HPLC (Methode 5): Rₜ: 5.58 min.
MS (DCI, NH₃): m/z = 428 (M+NH₄⁺).

### Beispiel LX

### 3-{4-[(4-Phenoxybut-2-in-1-yl)oxy]phenyl}propansäure-methylester

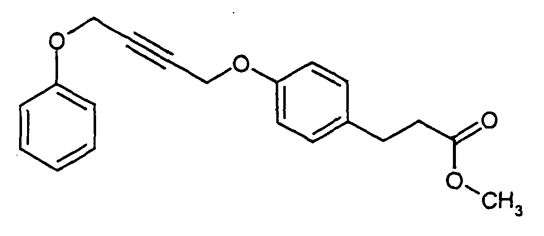

Die Herstellung erfolgt analog Beispiel XVII aus [(4-Brombut-2-in-1-yl)oxy]benzol und 3-(4-Hydroxyphenyl)propansäuremethylester.
HPLC (Methode 5): Rₜ: 4.77 min.
¹H-NMR (300 MHz, CDCl₃, δ/ppm): 7.27 (dd, 2H), 7.09 (d, 2H), 7.03-6.90 (m, 3H), 6.86 (d, 2H), 4.70 (dd, 4H), 3.67-(s, 3H), 2.89 (t, 2H), 2.59 (t, 2H).
MS (DCI, NH₃): m/z = 342 (M+NH₄⁺).

### Beispiel LXI

### 3-{4-[(4-Phenoxybut-2-in-1-yl)oxy]phenyl}propan-1-ol

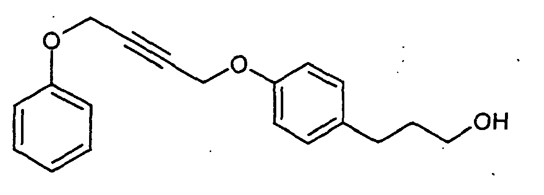

Die Herstellung erfolgt analog Beispiel II aus 3-{4-[(4-Phenoxybut-2-in-1-yl)oxy]-phenyl}propansäure-methylester.
HPLC (Methode 5): Rₜ: 4.42 min.
¹H-NMR (300 MHz, CDCl_{3,} δ/ppm): 7.31-7.24 (m, 2H), 7.10 (d, 2H), 7.00 (d, 1H), 6.94 (dd, 2H), 6.86 (d, 2H), 4.71 (dd, 4H), 3.71-3.62 (m, 2H), 2.66 (t, 2H), 1.92-1.82 (m, 2H), 1.22 (t, 1H).
MS (DCI, NH₃): m/z = 314 (M+NH₄⁺).

### Beispiel LXII

### 1-(3-Brompropyl)-4-[(4-phenoxybut-2-in-1-yl)oxy]benzol

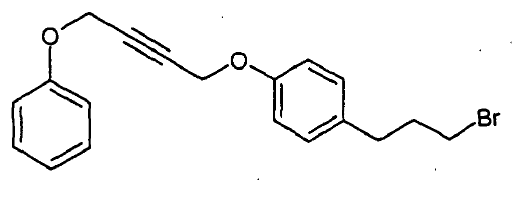

Die Herstellung erfolgt analog Beispiel IV aus 3-{4-[(4-Phenoxybut-2-in-1-yl)oxy]-phenyl}propan-1-ol.
HPLC (Methode 5): Rₜ: 5.17 min.
¹H-NMR (300 MHz, CDCl₃, δ/ppm): 7.32-7.24 (m, 2H), 7.09 (d, 2H), 7.00 (d, 1H), 6.94 (dd, 2H), 6.87 (d, 2H), 4.71 (dd, 4H), 3.38 (t, 2H), 2.72 (t, 2H), 2.13 (quint, 2H). LC-MS (Methode 9): Rₜ: 2.88 min, m/z (EI+) = 358 und 360.

### Beispiel LXIII

### 3-{4-[5-Phenoxypent-1-en-1-yl]phenyl}propansäure-ethylester (E/Z-Gemisch)

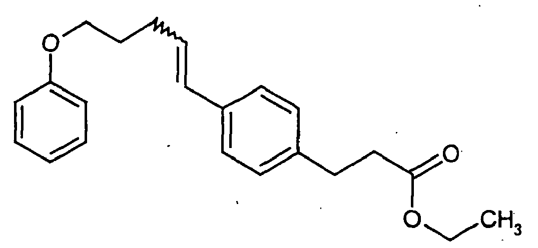

Die Herstellung erfolgt analog Beispiel XLIII aus (4-Phenoxybutyl)-(triphenyl)phosphoniumbromid und 3-(4-Formylphenyl)propansäureethylester. Man erhält das Produkt als E/Z-Gemisch.
HPLC (Methode 5): Rₜ: 5.38 min.
¹H-NMR (300 MHz, CDCl₃, δ/ppm): 7.31-7.10 (m, 6H), 6.96-6.83 (m, 3H), 6.46-6.36 (m, 1H), 6.20 (dt, 0.37H), 5.66 (dt, 0.63H), 4.12 (dq, 2H), 3.99 (q, 2H), 2.98-2.88 (m, 2H), 2.64-2.56 (m, 2H), 2.51 (dq, 1.25H), 2.39 (q, 0.75H), 2.00-1.88 (m, 2H), 1.23 (t, 3H).
LC-MS (Methode 9): Rₜ: 3.01 min, m/z (EI+) = 338.

### Beispiel LXIV

### 3-{4-[(1Z)-5-Phenoxypent-1-en-1-yl]phenyl}propan-1-ol

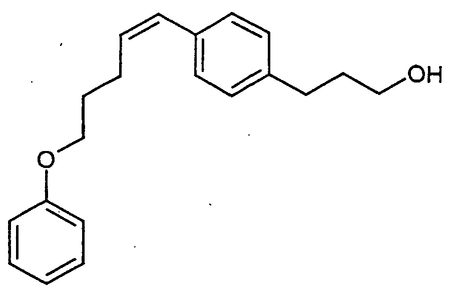

Die Herstellung erfolgt analog Beispiel XLIV aus 3-{4-[5-Phenoxypent-1-en-1-yl]phenyl}-propansäureethylester (E/Z-Gemisch). Die Trennung der Doppelbindungsisomere erfolgt durch HPLC (Kromasil 100 C-18 5 µm 250 mm x 20 mm; Methanol/Wasser 3:1; Fluss: 25 ml/min; UV-Detektion: 210 nm).
¹H-NMR (400 MHz, CDCl₃, δ/ppm): 7.30-7.24 (m, 2H), 7.22 (d, 2H), 7.15 (d, 2H), 6.93 (t, 1H), 6.87 (d, 2H), 6.43 (d, 1H), 5.66 (dt, 1H), 3.98 (t, 2H), 3.68 (t, 2H), 2.70 (t, 2H), 2.53 (dq, 2H), 1.98-1.86 (m, 4H), 1.23 (s, breit, 1H).
LC-MS (Methode 9): Rₜ: 2.61 min, m/z = 203 (M - OC₆H₅).

### Beispiel LXV

### (4Z)-5-[4-(3-Brompropyl)phenyl]pent-4-en-1-yl-phenyl-ether

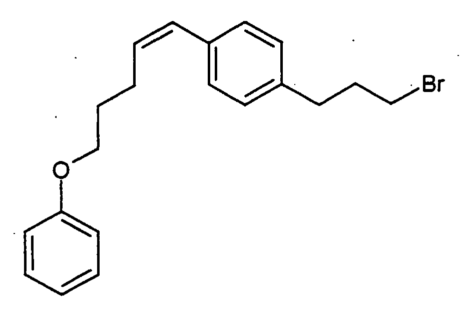

Die Herstellung erfolgt analog Beispiel IV aus 3-{4-[(1Z)-5-Phenoxypent-1-en-1-yl]phenyl}propan-1-ol.
HPLC (Methode 5): Rₜ: 5.69 min.

### Beispiel LXVI

### 3-{4-[(1E)-5-Phenoxypent-1-en-1-yl]phenyl}propan-1-ol

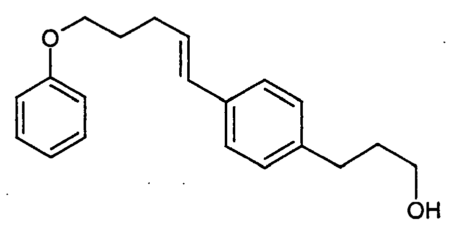

Die Herstellung erfolgt analog Beispiel XLIV aus 3-{4-[5-Phenoxypent-1-en-1-yl]phenyl}-propansäureethylester (E/Z-Gemisch). Die Trennung der Doppelbindungsisomere erfolgt durch HPLC (Kromasil 100 C-18 5 µm 250 mm x 20 mm; Methanol/Wasser 3:1; Fluss: 25 ml/min; UV-Detektion: 210 nm].
¹H-NMR (400 MHz, CDCl₃, δ/ppm): 7.30-7.24 (m, 4H), 7.13 (d, 2H), 6.96-6.88 (m, 3H), 6.41 (d, 1H), 6.21 (dt, 1H), 4.01 (t, 2H), 3.68 (t, 2H), 2.69 (t, 2H), 2.39 (q, 2H), 2.00-1.84 (m, 4H), 1.23 (s, breit, 1H).
LC-MS (Methode 9): Rₜ: 2.63 min, m/z = 203 (M- OC₆H₅).

### Beispiel LXVII

### (4E)-5-[4-(3-Brompropyl)phenyl]pent-4-en-1-yl-phenylether

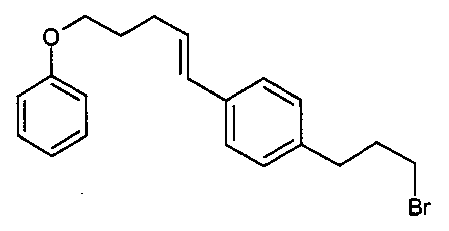

Die Herstellung erfolgt analog Beispiel IV aus 3-{4-[(lE)-5-Phenoxypent-1-en-1-yl]phenyl}propan-1-ol.
HPLC (Methode 5): Rₜ: 5.68 min.

### Beispiel LXVIII

### (2E)-3-[4-(4-Phenoxybutoxy)phenyl]acrylsäuremethylester

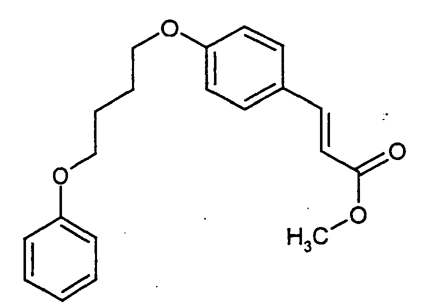

Die Herstellung erfolgt analog Beispiel I aus (2E)-3-(4-Hydroxyphenyl)-acrylsäuremethylester und (4-Brombutoxy)benzol.
HPLC (Methode 2): Rₜ: 5.4 min.
¹H-NMR (200 MHz, DMSO-d₆, δ/ppm): 7.73-7.61 (m, 3H), 7.34-7.22 (m, 2H), 7.04-6.86 (m, 5H), 6.48 (d, 1H), 4.15-3.95 (m, 4H), 3.70 (s, 3H), 1.93.1.81 (m, 4H).
MS (DCI, NH₃): m/z = 327 (M+H⁺), 344 (M+NH₄⁺).

### Beispiel LXIX

### (2E)-3-[4-(4-Phenoxybutoxy)phenyl]prop-2-en-1-ol

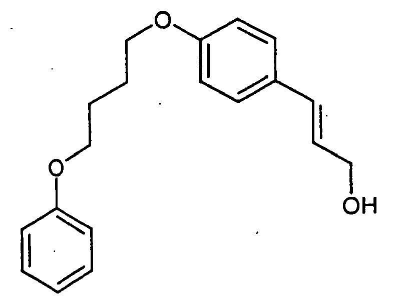

Die Herstellung erfolgt analog Beispiel II aus (2E)-3-[4-(4-Phenoxybutoxy)phenyl]-acrylsäuremethylester.
¹H-NMR (300 MHz, CDCl₃, δ/ppm): 7.34-7.24 (m, 4H), 6.97-6.82 (m, 5H), 6.56 (d, 1H), 6.23 (dt, 1H), 4.30 (d,2H), 4.07-4.01 (m, 4H), 2.01-1.95 (m, 4H), 1.36 (s, breit, 1H).

### Beispiel LXX

### 1-[(1E)-3-Bromprop-1-en-1-yl]-4-(4-phenoxybutoxy)benzol

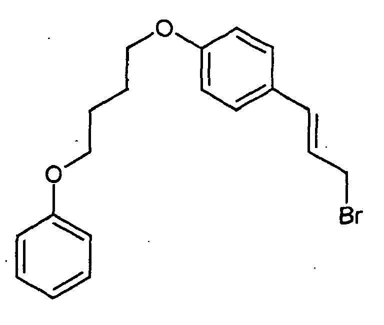

Eine Lösung von 250 mg (2E)-3-[4-(4-Phenoxybutoxy)phenyl]prop-2-en-1-ol in 10 ml Dichlormethan wird auf -10°C gekühlt und 82 mg Phosphortribromid in 0.5 ml Dichlormethan zugetropft. Man rührt eine Stunde und erwärmt dabei auf Raumtemperatur. Der Ansatz wird mit gesättigter Natriumhydrogencarbonat-Lösung versetzt und mehrfach mit Diethylether extrahiert, die organische Phase über Natriumsulfat getrocknet, abfiltriert und eingeengt. Man erhält 164 mg Produkt.
HPLC (Methode 5): Rₜ: 5.20 min.
¹H-NMR (300 MHz, CDCl₃, δ/ppm): 7.35-7.21 (m, 4H), 6.98-6.80 (m, 5H), 6.58 (d, 1H), 6.25 (dt, 1H), 4.17 (d,2H), 4.08-3.96 (m, 4H), 2.06-1.92 (m, 4H).
MS (DCI, NH₃): m/z = 378 und 380 (M+NH₄⁺), 281 (M-Br⁻).

### Beispiel LXXI

### [2-(Methoxycarbonyl)cyclohexyl]-methanammoniumbromid

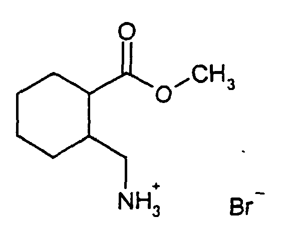

Man rührt 2.90 g Octahydro-1H-isoindol-1-on in 80 ml konzentrierter Bromwasserstoffsäure bei 100°C über Nacht. Nach Zugabe von ca. 5 ml Methanol wird für weitere 5 Stunden erhitzt und die Reaktionsmischung nach dem Erkalten eingeengt. Der Rückstand wird mit Impfkristallen versetzt oder angerieben, anschließend mit Ethylacetat ausgerührt und vom Lösungsmittel abgesaugt. Zum vollständigen Umsatz nimmt man in ca. 5 ml Methanol auf, gibt einige Tropfen konzentrierte Schwefelsäure hinzu und rührt ca. zwei Stunden unter Rückfluss. Die Reaktionsmischung wird nach dem Erkalten eingeengt, mit Ethylacetat ausgerührt, abgesaugt und im Vakuum getrocknet.
HPLC (Methode 5):Rₜ: 3.15 min.
¹H-NMR (400 MHz, DMSO-d₆, δ/ppm): 7.75 (s, breit, 3H), 3.61 (s, 3H), 2.95-2.73 (m, 3H), 2.05-1.92 (m, 1H), 1.86-1.73 (m, 1H), 1.66-1.22 (7H).
MS (DCI, NH₃): m/z =172 (M+H⁺).

### Beispiel LXXII

### 1-[5-(Methoxycarbonyl)-2-(3-{4-[(triisopropylsilyl)oxy]phenyl}propoxy)benzoyl]-piperidin-4-carbonsäure-methylester

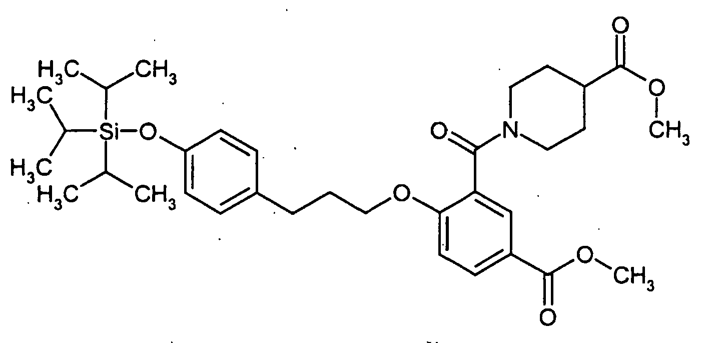

Nach dem in Beispiel XLI beschriebenen Verfahren wird 1-[5-(Methoxycarbonyl)-2-(3-{4-[(triisopropylsilyl)oxy]phenyl}propoxy)benzoyl]piperidin-4-carbonsäuremethylester aus [4-(3-Brompropyl)phenoxy](triisopropyl)silan und 1-[2-Hydroxy-5-(methoxycarbonyl)benzoyl]-4-piperidincarbonsäure-methylester hergestellt.
HPLC (Methode 4): Rₜ: 6.63 min.
MS (ESI): m/z = 612 (M+H⁺).

### Beispiel LXXIII

### 1-[2-[3-(4-Hydroxyphenyl)propoxy]-5-(methoxycarbonyl)benzoyl]piperidin-4-carbonsäure-methylester

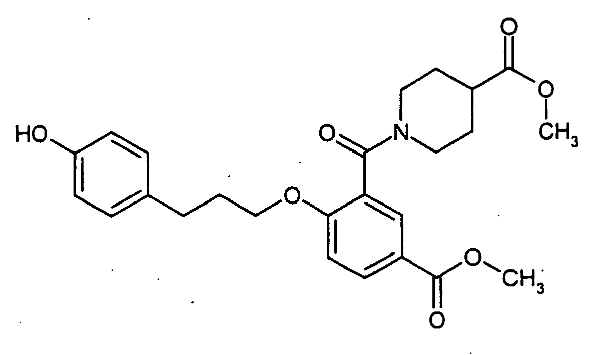

Analog dem in Beispiel XLII beschriebenen Verfahren wird 1-[2-[3-(4-Hydroxyphenyl)propoxy]-5-(methoxycarbonyl)benzoyl]piperidin-4-carbonsäuremethylester aus 1-[5-(Methoxycarbonyl)-2-(3-{4-[(triisopropylsilyl)oxy]phenyl}-propoxy)benzoyl]piperidin-4-carbonsäure-methylester hergestellt.
HPLC (Methode 1): Rₜ: 4.30 min.
¹H-NMR (200 MHz, CDCl₃, δ/ppm): 8.02 (dd, 1H), 7.93 und 7.90 (2 d, zusammen 1H), 7.00 (d, 2H), 6.88 (d, 1H), 6.75 (d, 2H), 5.67 (d, 1H), 4.62 (m, 1H), 4.02 (m, 2H), 3.87 (s, 3H), 3.70 und 3.63 (2 s, zusammen 3H), 3.49 (m, 1H), 3.27 (m, 1H), 3.12-2.95 (m, 2H), 2.72-2.52 (m, 3H), 2.11-1.97 (m, 3H), 1.84-1.63 (m, 2H).
MS (DCI, NH₃): m/z = 456 (M+H⁺).

### Ausführungsbeispiele:

### Beispiel 1

### 1-[5-(Methoxycarbonyl)-2-({3-[4-(4-phenoxybutoxy)phenyl]propanoyl}amino)-benzoyl]-4-piperidincarbonsäure-methylester

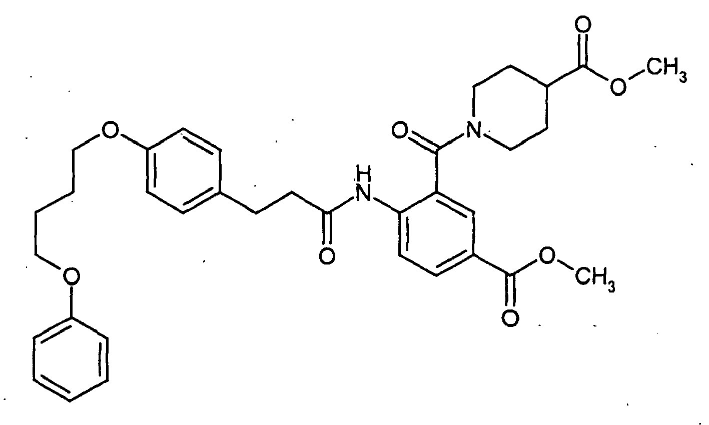

Eine Lösung von 450 mg 1-[2-Amino-5-(methoxycarbonyl)benzoyl]-4-piperidincarbonsäure-methylester in 7.5 ml Dichlormethan wird mit 2 ml Pyridin und einer Lösung von 470 mg 3-[4-(4-Phenoxybutoxy)phenyl]propansäurechlorid in 7.5 ml Dichlormethan versetzt. Man lässt das Reaktionsgemisch 15 Stunden bei Raumtemperatur rühren. Anschliessend wird mit 2-molarer Salzsäure sauer gestellt und mit Ethylacetat extrahiert. Der organische Extrakt wird mit gesättigter Kochsalz-Lösung gewaschen und über wasserfreiem Natriumsulfat getrocknet. Nach Filtrieren und Einrotieren wird ein Rohprodukt erhalten, das durch Flash-Chromatographie (Kieselgel, Cyclohexan/Ethylacetat 2:1) gereinigt wird. Es werden 617 mg Produkt erhalten.
DC: R_{f}-Wert: 0.55 (Cyclohexan/Ethylacetat 1:9).
HPLC (Methode 1): Rₜ: 5.08 min.
¹H-NMR (300 MHz, DMSO-d₆, δ/ppm): 9.76 (s, 1H), 7.97 (dd, 1H), 7.82-7.77 (m, 2H), 7.30-7.23 (m, 2H), 7.13 (d, 2H), 6.93-6.89 (m, 3H), 6.84 (d, 2H), 4.32 (m 1H), 4.00 (m, 4H), 3.83 (s, 3H), 3.60 (s, 3H), 2.92-2.77 (m, 4H), 2.64-2.56 (m, 3H), 1.93-1.42 (m, 9H).
MS (ESI+): m/z = 617 (M+H⁺).

### Beispiel 2

### 3-({[3-(Methoxycarbonyl)cyclohexyl]amino}carbonyl)-4-{3-[4-(4-phenoxybutoxy)-phenyl]propoxy}benzoesäure-methylester (Racemat B)

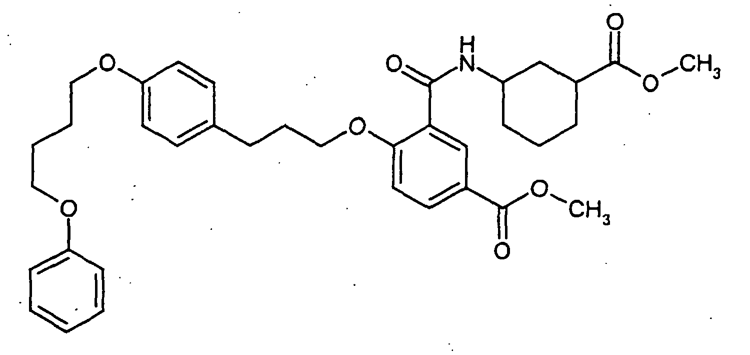

Eine Lösung von 542 mg 1-(3-Brompropyl)-4-(4-phenoxybutoxy)benzol und 500 mg 4-Hydroxy-3-({[3-(methoxycarbonyl)cyclohexyl]amino}carbonyl)benzoesäure-methylester (Racemat B) in 50 ml Butyronitril wird mit 248 mg Kaliumcarbonat versetzt und 15 Stunden zum Rückfluss erhitzt. Anschliessend wird das Reaktionsgemisch mit 5%iger Natriumdihydrogenphospat-Lösung versetzt, und es wird mit Ethylacetat extrahiert. Der organische Extrakt wird mit gesättigter Kochsalzlösung gewaschen und über wasserfreiem Natriumsulfat getrocknet. Nach Filtrieren und Einrotieren wird ein Rohprodukt erhalten, das durch Saugfiltration über Kieselgel mit Cyclohexan/Ethylacetat 2:1 als Laufmittel gereinigt wird. Es werden 672 mg Produkt erhalten.
DC: R_{f}-Wert: 0.33 (Cyclohexan/Ethylacetat 1:1).
HPLC (Methode 1): Rₜ: 5.68 min.
¹H-NMR (300 MHz, DMSO-d₆, δ/ppm): 8.21 (d, 1H), 8.08 (d breit, 1H), 8.00 (dd, 1H), 7.30-7.20 (m, 3H), 7.12 (d, 2H), 6.94-6.88 (m, 3H), 6.84 (d, 2H), 4.17-4.12 (m, 3H), 4.01 (m, 4H), 3.83 (s, 3H), 3.57 (s, 3H), 2.73-2.68 (m, 3H), 2.08 (m, 1H), 1.88-1.81 (m, 6H), 1.69-1.49 (m, 6H).
MS (ESI+): m/z = 618 (M+H⁺), 640 (M+Na⁺).

### Beispiel 3

### 3-({[3-(Methoxycarbonyl)cyclohexyl]amino}carbonyl)-4-{3-[4-(4-phenoxybutoxy)-phenyl]propoxy}benzoesäure-methylester (Racemat A)

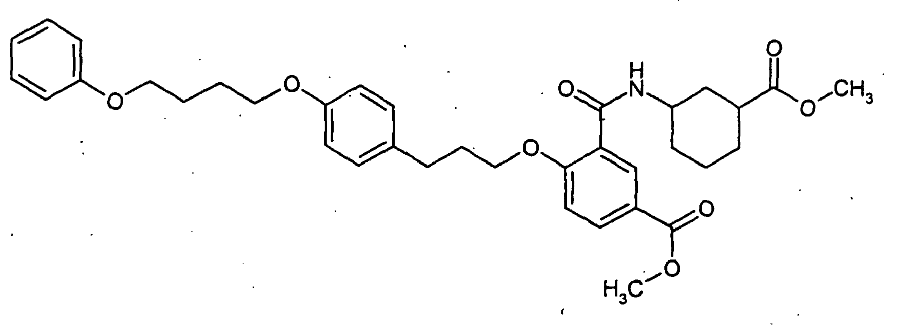

Analog dem unter Beispiel 2 beschriebenen Verfahren wird 3-({[3-(Methoxycarbonyl)cyclohexyl]amino}carbonyl)-4-{3-[4-(4-phenoxybutoxy)phenyl]propoxy}-benzoesäure-methylester. (Racemat A) aus 1-(3-Brompropyl)-4-(4-phenoxybutoxy)benzol und 4-Hydroxy-3-({[3-(methoxycarbonyl)cyclohexyl]amino}-carbonyl)benzoesäure-methylester (Racemat A) hergestellt.
DC: R_{f}-Wert: 0.38 (Cyclohexan/Ethylacetat 1:1).
HPLC (Methode 1): Rₜ: 5.67 min.
¹H-NMR (300 MHz, DMSO-d₆, δ/ppm): 8.20 (d, 1H), 8.07 (d breit, 1H), 8.00 (dd, 1H), 7.30-7.18 (m, 3H), 7.12 (d, 2H), 6.94-6.88 (m, 3H), 6.84 (d, 2H), 4.13 (t, 2H), 4.01 (m, 4H), 3.84 (m, 1H), 3.83 (s, 3H), 3.57 (s, 3H), 2.70 (t, 2H), 2.48 (m, 1H), 2.17 (m, 1H), 2.03 (m, 2H), 1.92-1.73 (m, 7H), 1.43-1.12 (m, 4H).
MS (ESI): m/z = 618 (M+H⁺), 640 (M+Na⁺).

### Beispiel 4

### 1-(5-(Methoxycarbonyl)-2-{3-[4-(4-phenoxybutoxy)phenyl]propoxy}benzoyl)-4-piperidincarbonsäure-methylester

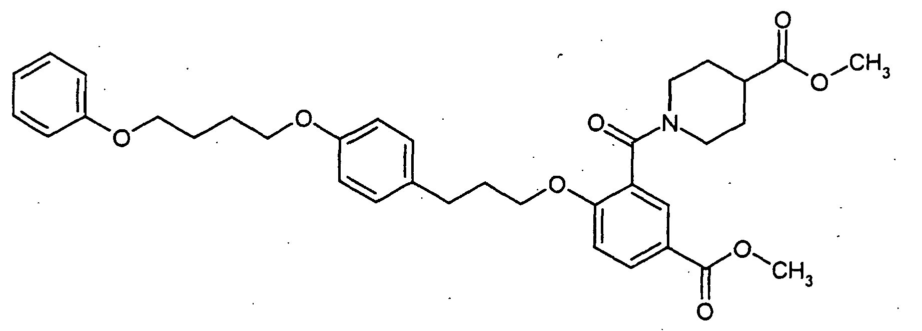

Analog dem unter Beispiel 2 beschriebenen Verfahren wird 1-(5-(Methoxycarbonyl)-2-{3-[4-(4-phenoxybutoxy)phenyl]propoxy}benzoyl)-4-piperidincarbonsäure-methylester aus 1-(3-Brompropyl)-4-(4-phenoxybutoxy)benzol und 1-[2-Hydroxy-5-(methoxycarbonyl)benzoyl]-4-piperidincarbonsäure-methylester hergestellt.
HPLC (Methode 1): Rₜ: 5.42 min.
¹H-NMR (400 MHz, DMSO-d₆, δ/ppm): 7.97 (d, 1H), 7.73 (dd, 1H), 7.29-7.25 (m, 2H), 7.17 (d, 1H), 7.12-7.09 (m, 2H), 6.93-6.90 (m, 3H), 6.86-6.83 (m, 2H), 4.43 (m, 1H), 4.16-3.97 (m, 6H), 3.82 (s, 3H), 3.60 und 3.54 (2 s, 3H), 3.29 (m, 1H), 3.11-2.98 (m, 1H), 2.92 (m, 1H), 2.71-2.60 (m, 3H), 2.03-1.72 (m, 8), 1.64-1.50 (m, 2H).
MS (DCI, NH₃⁺): m/z = 604.3 (M+H⁺), 621.3 (M+NH₄⁺).

### Beispiel 5

### 1-[2-{3-[4-(1,1'-Biphenyl-4-ylmethoxy)phenyl]propoxy}-5-(ethoxycarbonyl)-benzoyl]-4-piperidincarbonsäure-methylester

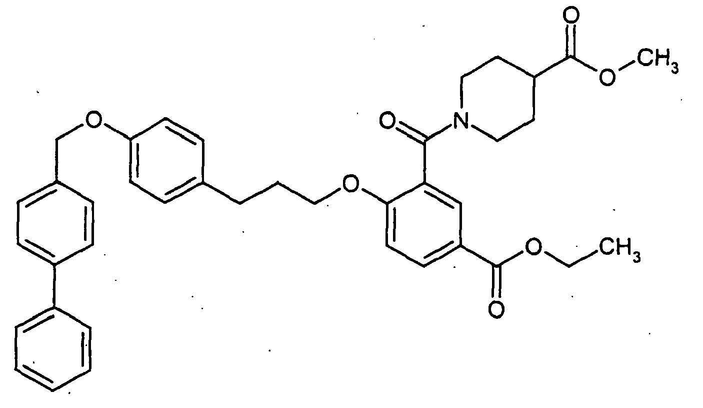

Eine Suspension von 100 mg 2-{3-[4-(1,1'-Biphenyl-4-ylmethoxy)phenyl]propoxy}-5-(ethoxycarbonyl)benzoesäure und 39 mg Piperidin-4-carbonsäure-methylester-Hydrochlorid in 30 ml Dichlormethan wird nacheinander mit 75 mg N-[(3-Dimethylamino)propyl]-N'-ethylcarbodiimid-Hydrochlorid (EDC), 27 mg 1-Hydroxy-1H-benzotriazol-Hydrat (HOBT) und 40 mg Triethylamin versetzt. Nach 15 Stunden bei Raumtemperatur wird das Reaktionsgemisch mit Wasser versetzt. Nach Phasentrennung wird die wässrige Phase mit Ethylacetat extrahiert. Die vereinigten Dichlormethan- und Ethylacetat-Phasen werden mit gesättigter Kochsalzlösung gewaschen und über wasserfreiem Natriumsulfat getrocknet. Nach Filtrieren und Einengen wird ein Rohprodukt erhalten, das mittels Saugfiltration über Kieselgel mit Cyclohexan/Ethylacetat 1:1 als Laufmittel gereinigt wird. Es werden 120 mg Produkt erhalten.
HLPC (Methode 1): Rₜ: 5.75 min.
¹H-NMR (200 MHz, DMSO-d₆, δ/ppm): 7.97 (dd, 1H), 7.75-7.66 (m, 5H), 7.53-7.37 (m, 5H), 7.19-7.11 (m, 3H), 6.96-6.92 (m, 2H), 5.12 (s, 2H), 4.48-4.37 (m, 1H), 4.28 (quart, 2H), 4.13-3.98 (m, 2H), 3.60 und 3.53 (2 s, 3H), 3.28 (m, 1H), 3.14-2.86 (m, 2H), 2.71-2.59 (m, 3H), 2.02-1.88 (m, 3H), 1.79-1.54 (m, 3H), 1.31 (t, 3H).
MS (ESI+): m/z = 636.1 (M+H⁺).

### Beispiel 6

### 3-({cis-[2-(Ethoxycarbonyl)cyclohexyl]amino}carbonyl)-4-{3-[4-(4-phenoxybutoxy)-phenyl]propoxy}benzoesäure-ethylester

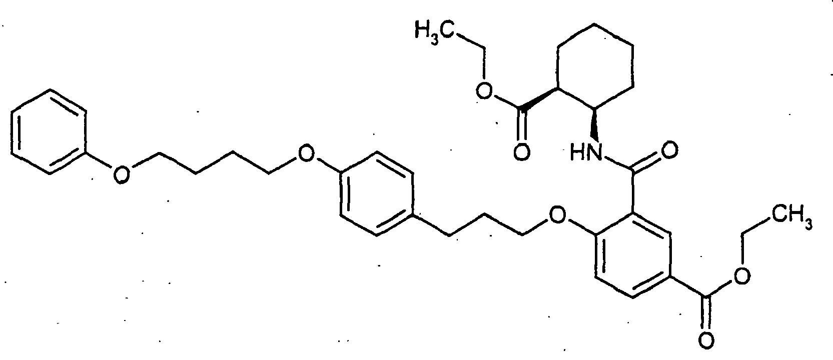

Eine Suspension von 100 mg (0.20 mmol) 5-(Ethoxycarbonyl)-2-{3-[4-(4-phenoxybutoxy)-phenyl]propoxy}benzoesäure in 30 ml Dichlormethan wird bei RT nacheinander mit 39 mg (0.20 mmol) N-[(3-Dimethylamino)propyl]-N'-ethylcarbodiimid-Hydrochlorid (EDC) und 27.4 mg (0.20 mmol) 1-Hydroxy-1H-benzotriazol Hydrat (HOBT) versetzt. Nach 30 min werden.41 mg (0.41 mmol) Triethylamin und 38 mg (0.18 mmol) cis-2-Amino-1-cyclohexancarbonsäure-ethylester-Hydrochlorid zugegeben und der Ansatz über Nacht gerührt. Anschließend werden 10 ml Wasser zugegeben, die Phasen getrennt und die wässrige Phase mit Dichlormethan (dreimal 25 ml) extrahiert. Die vereinigten organischen Phasen werden mit gesättigter Natriumchlorid-Lösung gewaschen, getrocknet (Natriumsulfat) und am Rotationsverdampfer eingeengt. Das Rohprodukt wird durch Säulenchromatographie (Kieselgel 60, Laufmittelgradient Cyclohexan --> Cyclohexan-Ethylacetat 5:1) gereinigt. Man erhält 102 mg Produkt.
HPLC (Methode 2): Rₜ: 6.22 min
¹H-NMR (300MHz, CDCl₃, δ/ppm): 8.33 (d, 1H), 8.43 (d, 1H), 8.07 (dd, 1H), 7.31-7.23 (m, 2H), 7.11 (d, 2H), 6.96-6.86 (m, 4H), 6.83 (d, 2H), 4.47 (m, 1H), 4.35 (m, 2H), 4.23-3.96 (m, 8H), 2.93-2.70 (m, 3H), 2.38-2.15 (m, 2H), 2.12-1.90 (m, 6H), 1.81-1.61 (m, 3H), 1.37 (t, 3H), 1.19 (t, 3H).
MS (ESIpos): m/z = 646 (M+H)⁺

### Beispiel 7

### 3-{[4-(2-Ethoxy-2-oxoethyl)-1-piperidinyl]carbonyl}-4-{3-[4-phenoxybutoxy)-phenyl]-propoxy}benzoesäure-ethylester

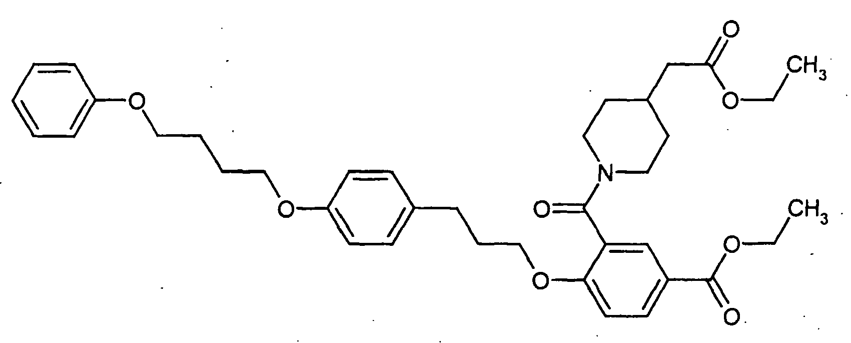

Analog dem unter Beispiel 6 beschriebenen Verfahren wird die Verbindung aus 5-(Ethoxycarbonyl)-2-{3-[4-(4-phenoxybutoxy)-phenyl]propoxy}benzoesäure und 4-Piperidinessigsäureethylester [CAS-Nr. 59184-90-6] hergestellt. Die Reinigung des Rohproduktes erfolgt durch HPLC [YMC GEL ODS-AQ-S 5/15µm, Gradient: Acetonitril/(Wasser + 0.2 % TFA) 10:90...95:5].
LC-MS (Methode 3): Rₜ: 5.12 min.
MS (ESI+): m/z = 646 (M+H⁺).

### Beispiel 8

### 1-(5-(Ethoxycarbonyl)-2-{3-[4-(4-phenoxybutoxy)phenyl]propoxy}benzoyl)-3-azetidincarbonsäure-methylester

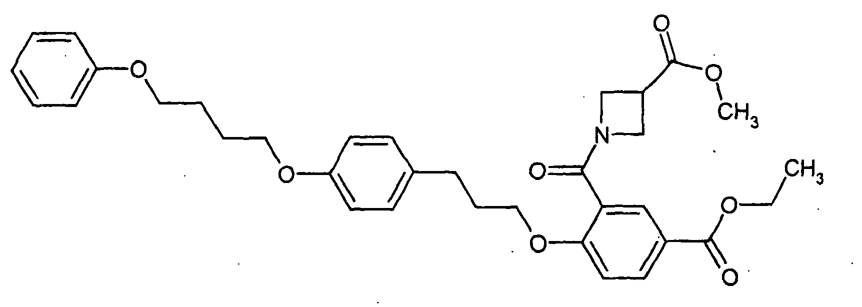

Analog dem unter Beispiel 6 beschriebenen Verfahren wird die Verbindung aus 5-(Ethoxycarbonyl)-2-{3-[4-(4-phenoxybutoxy)-phenyl]propoxy}benzoesäure und 3-Azetidincarbonsäuremethylester [CAS-Nr. 343238-58-4] hergestellt. Das Rohprodukt wird ohne Reinigung direkt weiter umgesetzt.
LC-MS (Methode 3): Rₜ: 4.84 min.
MS (ESI+): m/z = 590 (M+H⁺).

### Beispiel 9

### 4-{3-[4-(1,1'-Biphenyl-4-ylmethoxy)phenyl]propoxy}-3-({cis-[2-(ethoxycarbonyl)-cyclohexyl]amino}carbonyl)benzoesäure-ethylester

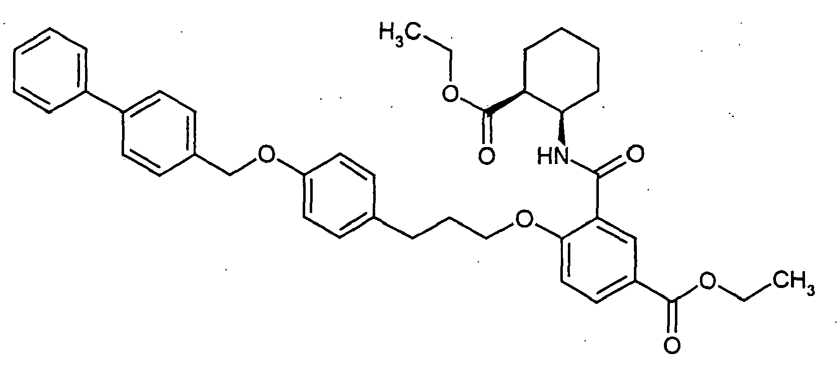

Analog dem unter Beispiel 6 beschriebenen Verfahren wird die Verbindung aus 2-{3-[4-(1,1'-Biphenyl-4-ylmethoxy)phenyl]propoxy}-5-(ethoxycarbonyl)benzoesäure und cis-2-Amino-1-cyclohexanecarbonsäure-ethylester-Hydrochlorid hergestellt.
HPLC (Methode 4): Rₜ: 6.42 min.
¹H-NMR (200 MHz, DMSO-d₆, δ/ppm): 8.43-8.33 (m, 2H), 8.03 (dd, 1H), 7.71-7.63 (m, 4H), 7,56-7.11 (m, 8H), 6.95 (d, 2H), 5.12 (s, 2H), 4.48-4.14 (m, 5H), 3.99 (m, 2H), 2.86-2.65 (m, 3H), 2.22-2.03 (m, 2H), 1.92-1.24 (m, 8H), 1.31 (t, 3H), 1.08 (t, 3H).
MS (ESI+): m/z = 664 (M+H⁺).

### Beispiel 10

### 4-{3-[4-(1,1'-Biphenyl-4-ylmethoxy)phenyl]propoxy}-3-({cis-[4-(methoxycarbonyl)-cyclohexyl]amino}carbonyl)benzoesäure-ethylester

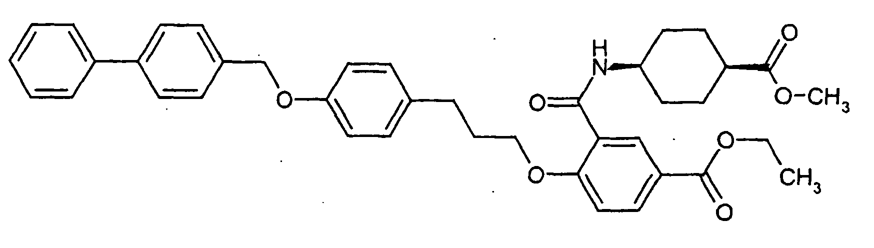

Analog dem unter Beispiel 6 beschriebenen Verfahren wird die Verbindung aus 2-{3-[4-(1,1'-Biphenyl-4-ylmethoxy)phenyl]propoxy}-5-(ethoxycarbonyl)benzoesäure und cis-4-Amino-1-cyclohexancarbonsäure-ethylester hergestellt.
HPLC (Methode 2): Rₜ: 5.93 min.
¹H-NMR (300 MHz, DMSO-d₆, δ/ppm): 8.21 (d, 1H), 8.08 (d, 1H), 7.99 (dd, 1H), 7.70-7.64 (m, 4H), 7.54-7.43 (m, 4H), 7.40-7.33 (m, 1H), 7.20 (d, 1H), 7.14 (d, 2H), 6.94 (d, 2H), 5.11 (s, 2H), 4.30 (q, 2H), 4.14 (t, 2H), 3.97 (m, breit, 1H), 3.53 (s, 3H), 2.69 (t, 2H), 2.50 (m, verdeckt, 1H), 2.06 (m, 2H), 2.90-1.76 (m, 2H), 1.75-1.54 (m, 6H), 1.31 (t, 3H).
MS (ESI+): m/z = 650 (M+H⁺).

### Beispiel 11

### 3-{[(3-Carboxycyciohexyl)amino]carbonyl}-4-{3-[4-(4-phenoxybutoxy)phenyl]-propoxy}benzoesäure (Racemat B)

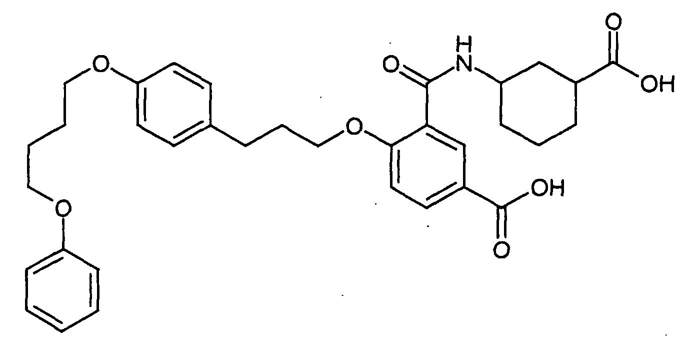

Eine Lösung von 638 mg 3-({[3-(Methoxycarbonyl)cyclohexyl]amino}carbonyl)-4-{3-[4-(4-phenoxybutoxy)phenyl]propoxy}benzoesäure-methylester (Racemat B) in 4 ml Tetrahydrofuran (THF) und 4 ml Methanol wird mit 4 ml 2-molarer Natronlauge versetzt und eine Stunde auf 60°C erwärmt. Anschliessend wird der pH-Wert mit 2-molarer Salzsäure auf einen Wert von 3-4 eingestellt, und es wird mit Ethylacetat extrahiert. Der organische Extrakt wird mit gesättigter Natriumchlorid-Lösung gewaschen und über wasserfreiem Natriumsulfat getrocknet. Nach Filtrieren und Einrotieren wird ein Rohprodukt erhalten, das durch Umkristallisieren aus Diethylether gereinigt wird. Es werden 589 mg Produkt isoliert.
Schmelzpunkt: 182-183°C.
HPLC (Methode 1): Rₜ: 4.95 min.
¹H-NMR (300 MHz, DMSO-d₆, δ/ppm): 12.38 (s breit, 2H), 8.21 (d, 1H), 8.05 (d, 1H), 7.98 (dd, 1H), 7.29-7.23 (m, 2H), 7.19-7.11 (m, 3H), 6.93-6.88 (m, 3H), 6.85 (d, 2H), 4.15-4.10 (m, 3H), 4.01 (m, 4H), 2.71 (t, 2H), 2.62 (m, 1H), 2.07 (m, 2H), 1.91-1.70 (m, 6H), 1.62-1.16 (m, 6H).
MS (ESI+): m/z = 590 (M+H⁺), 612 (M+Na⁺).

### Beispiel 12

### 3-{[(3-Carboxycyclohexyl)amino]carbonyl}-4-{3-[4-(4-phenoxybutoxy)phenyl]-propoxy}benzoesäure (Racemat A)

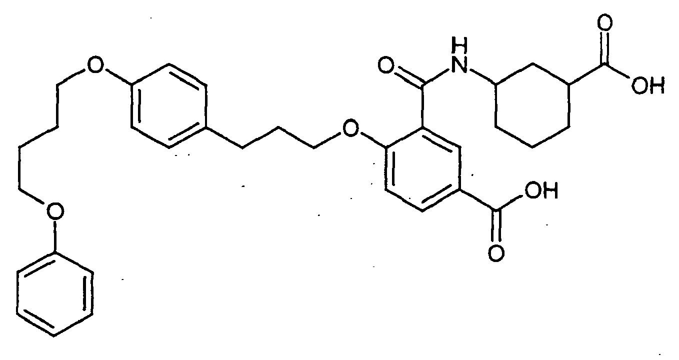

Analog dem unter Beispiel 11 beschriebenen Verfahren wird 3-{[(3-Carboxycyclohexyl)amino]carbonyl}-4-{3-[4-(4-phenoxybutoxy)phenyl]propoxy}-benzoesäure (Racemat A) aus 3-({[3-(Methoxycarbonyl)cyclohexyl]amino}-carbonyl)-4-{3-[4-(4-phenoxybutoxy)phenyl]propoxy}benzoesäure-methylester (Racemat A) hergestellt.
Schmelzpunkt: >210°C.
HPLC (Methode 1): Rₜ: 4.91 min.
¹H-NMR (200 MHz, DMSO-d₆, δ/ppm): 12.48 (s breit, 2H), 8.18 (d, 1H), 8.09 (d, 1H), 7.98 (dd, 1H), 7.32-7.11 (m, 5H), 6.96-6.83 (m, 5H), 4.12 (t, 2H), 4.01 (m, 4H), 3.82 (m, 1H), 2.70 (t, 2H), 2.38 (m, 1H), 2.19-1.73 (m, 10H), 1.41-1.10 (m, 4H).
MS (ESI+): m/z = 590 (M+H⁺), 612 (M+Na⁺).

### Beispiel 13

### 1-(2-{3-[4-(1,1'-Biphenyl-4-ylmethoxy)phenyl]propoxy}-5-carboxybenzoyl)-4-piperidincarbonsäure

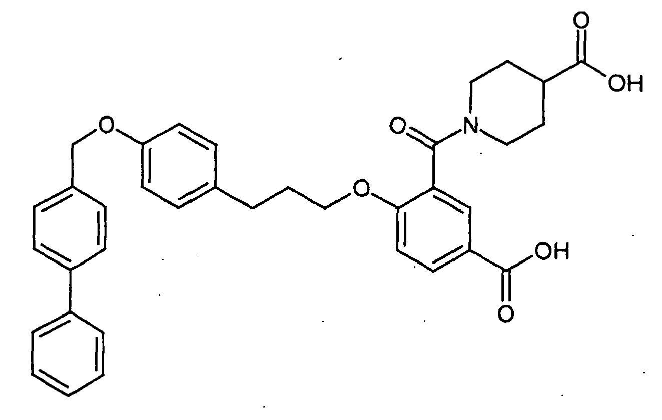

Analog dem unter Beispiel 11 beschriebenen Verfahren wird 1-(2-{3-[4-(1,1'-Biphenyl-4-ylmethoxy)phenyl]propoxy}-5-carboxybenzoyl)-4-piperidincarbonsäure aus 1-[2-{3-[4-(1,1'-Biphenyl-4-ylmethoxy)phenyl]propoxy}-5-(ethoxycarbonyl)-benzoyl]-4-piperidincarbonsäure-methylester hergestellt.
Schmelzpunkt: 193-194°C.
HLPC (Methode 1): Rₜ: 4.93 min.
¹H-NMR (200 MHz,DMSO-d₆, δ/ppm): 12.31 (s breit, 2H), 7.93 (dd, 1H), 7.72-7.64 (m, 5H), 7.55-7.32 (m, 5H), 7.17-7.12 (m, 3H), 6.98-6.92 (m, 2H), 5.12 (s, 2H), 4.48-4.37(m, 1H), 4.15-3.97 (m, 2H), 3.29 (m, 1H), 3.13-2.83 (m, 2H), 2.70-2.59 (m, 3H), 2.02-1.89 (m, 3H), 1.80-1.70 (m, 1H), 1.62-1.47 (m, 2H).
MS (ESI+): 594 (M+H⁺), 616 (M+Na⁺).

### Beispiel 14

### 1-[5-Carboxy-2-({3-[4-(4-phenoxybutoxy)phenyl]propanoyl}amino)benzoyl]-4-piperidincarbonsäure

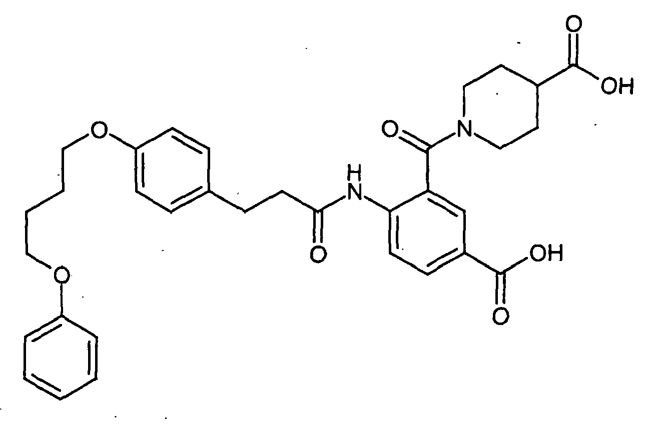

Analog dem unter Beispiel 11 beschriebenen Verfahren wird 1-[5-Carboxy-2-({3-[4-(4-phenoxybutoxy)phenyl]propanoyl}amino)berizoyl]-4-piperidincarbonsäure aus 1-[5-(Methoxycarbonyl)-2-({3-[4-(4-phenoxybutoxy)phenyl]propanoyl}amino)-benzoyl]-4-piperidincarbonsäure-methylester hergestellt.
HPLC (Methode 1): Rₜ: 4.6 min.
¹H-NMR (300 MHz, DMSO-d₆, δ/ppm): 12.63 (s breit, 2H), 9.73 (s, 1H), 7.93 (dd, 1H), 7.77-7.73 (m, 2H), 7.29-7.25 (m, 2H), 7.14 (d, 2H), 6.93-6.90 (m, 3H), 6.84 (d, 2H), 4.32 (m 1H), 4.00 (m, 4H), 3.30 (1H), 2.90-2.78 (m, 4H), 2.62 (m, 2H), 2.48 (m, 1H), 1.93-1.82 (m, 5H), 1.69 (m, 1H), 1.59-1.39 (m, 2H).
MS (ESI-): m/z = 587.2 (M-H⁻).

### Beispiel 15

### 1-(5-Carboxy-2-{3-[4-(4-phenoxybutoxy)phenyl]propoxy}benzoyl)-4-piperidincarbonsäure

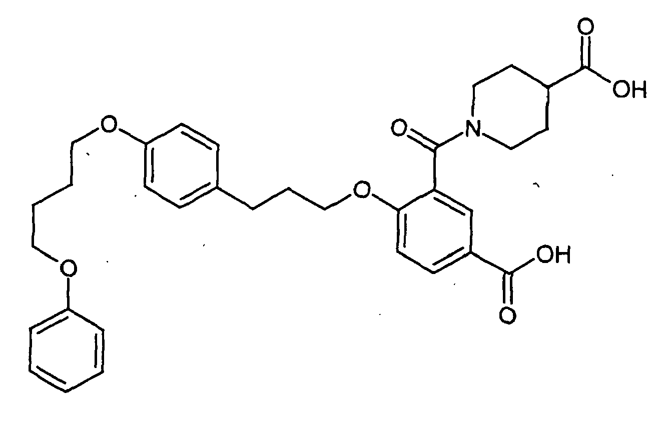

Analog dem unter Beispiel 11 beschriebenen Verfahren wird 1-(5-Carboxy-2-{3-[4-(4-phenoxybutoxy)phenyl]propoxy}benzoyl)-4-piperidincarbonsäure aus 1-(5-(Methoxycarbonyl)-2-{3-[4-(4-phenoxybutoxy)phenyl]propoxy}benzoyl)-4-piperidincarbonsäure-methylester hergestellt.
Schmelzpunkt: 142-143°C.
HPLC (Methode 1): Rₜ: 4.70 min.
¹H-NMR (300 MHz, DMSO-d₆, δ/ppm): 7.93 (dd, 1H), 7.70 (dd, 1H), 7.29-7.24 (m, 2H), 7.17-7.11 (m, 3H), 6.93-6.82 (m, 5H), 4.41 (m, 1H), 4.13-3.96 (m, 6H), 3.31 (m, 1H), 3.11-2.89 (m, 2H), 2.63 (t, 2H), 2.53 (m, 1H), 1.99-1.83 (m, 7), 1.76 (m, 1H), 1.67-1.48 (m, 2H).
MS (DCI, NH₃⁺): m/z = 576.3 (M+H⁺), 593.2 (M+NH₄⁺).

### Beispiel 16

### 3-{[(4-Carboxycyclohexyl)amino]carbonyl}-4-{3-[4-(4-phenoxybutoxy)phenyl]-propoxy}benzoesäure

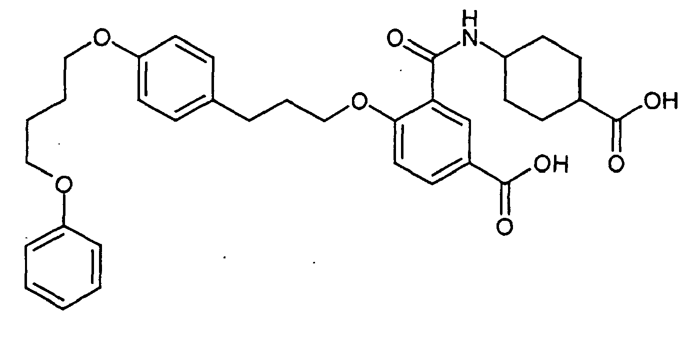

Analog dem unter Beispiel 11 beschriebenen Verfahren wird 3-{[(4-Carboxycyclohexyl)amino]carbonyl}-4-{3-[4-(4-phenoxybutoxy)phenyl]propoxy}benzoesäure aus 3-({[4-(Methoxycarbonyl)cyclohexyl]amino}carbonyl)-4-{3-[4-(4-phenoxybutoxy)phenyl]propoxy}benzoesäure-methylester hergestellt.
Schmelzpunkt: 163-165°C.
¹H NMR (500 MHz, DMSO-d₆, δ/ppm): 8.21 (d, 1H), 8.09 (d, 1H), 7.97 (dd, 2H), 7.30-7.22 (m, 2H), 7.16 (d, 1H), 7.11 (d, 2H), 6.99-6.82 (m, 5H), 4.12 (t, 2H), 4.02-3.95 (m, 5H), 2.68 (t, 2H), 2.28 (t, 1H), 2.10-2.00 (m, 2H), 1.90-1.76 (m, 6H), 1.72-1.60 (m, 6H).
MS: m/z = 590 (M+H⁺).

### Beispiel 17

### 4-{3-[4-(1,1'-Biphenyl-4-ylmethoxy)phenyl]propoxy}-3-{[cis-(4-carboxycyclohexyl)amino]carbonyl}benzoesäure

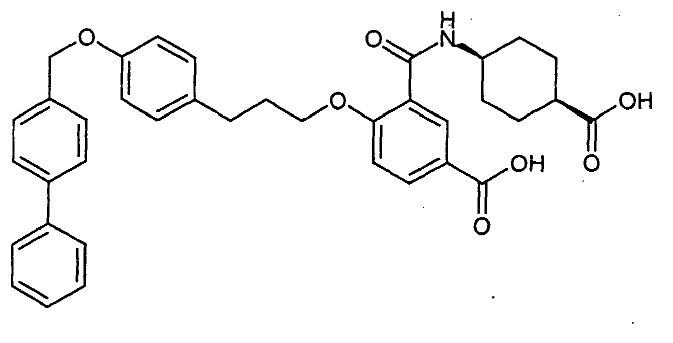

Analog dem unter Beispiel 11 beschriebenen Verfahren wird die Verbindung aus 4-{3-[4-(1,1'-Biphenyl-4-ylmethoxy)phenyl]propoxy}-3-({cis-[4-(methoxycarbonyl)-cyclohexyl]amino} carbonyl)benzoesäure-ethylester hergestellt.
HPLC (Methode 2): Rₜ: 5.04 min.
¹H-NMR (300MHz, DMSO-d₆, δ/ppm): 12.41 (s, breit, 2H), 8.21 (d, 1H), 8.09 (d, 1H), 7.96 (dd, 1H), 7.70-7.63 (m, 4H), 7.54-7.43 (m, 4H), 7.40-7.33 (m, 1H), 7.18-7.12 (m, 3H), 6:95 (d, 2H), 5.11 (s, 2H), 4.12 (t, 2H), 3.97 (m, breit, 1H), 2.69 (t, 2H), 2.41 (m, breit, 1H), 2.08 (m, 2H), 1.90-1.78 (m, 2H), 1.75-1.55 (m, 6H).
MS (ESI+): m/z = 608 (M+H⁺).

### Beispiel 18

### 3-{[4-(Carboxymethyl)-1-piperidinyl]carbonyl}-4-{3-[4-(4-phenoxybutoxy)phenyl]-propoxy}benzoesäure

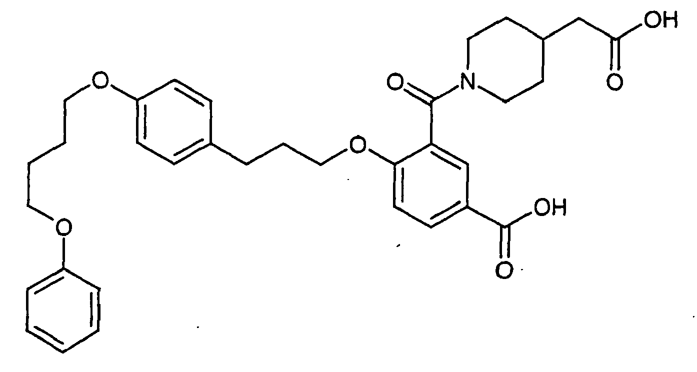

Analog dem unter Beispiel 11 beschriebenen Verfahren wird die Verbindung aus 3-{[4-(2-Ethoxy-2-oxoethyl)-1-piperidinyl]carbonyl}-4-{3-[4-phenoxybutoxy)phenyl]-propoxy}benzoesäure-ethylester hergestellt. Das erhaltene Rohprodukt wird durch HPLC [YMC GEL ODS-AQ-S 5/15 µm, Gradient: Acetonitril/ (Wasser + 0,2 % TFA) 10:90...95:5] gereinigt.
Schmelzpunkt: 192 °C.
LC-MS (Methode 3): Rₜ: 3.76 min.
¹H-NMR (300MHz, DMSO-d₆ δ/ppm): 12.40 (s, breit, 2H), 7.94-7.91 (m, 1), 7.68 (dd, 1H), 7.30-7.22 (m, 2H), 7.15-7.07 (m, 3H), 6.95-6.80 (m, 5H), 4.50 (m, 1H), 4.18-3.90 (m, 6H), 3.09-2.90 (m, 1H), 2.83-2.58 (m, 3H), 2.22-2.08 (m, 2H), 2.02-1.71 (m, 9H), 1.59 (m, 1H), 1.27-0.92 (m, 2H).
MS (ESI+): m/z = 590 (M+H⁺).

### Beispiel 19

### 3-({cis-[2-Carboxycyclohexyl]amino}carbonyl)-4-{3-[4-(4-phenoxybutoxy)phenyl]-propoxy}benzoesäure

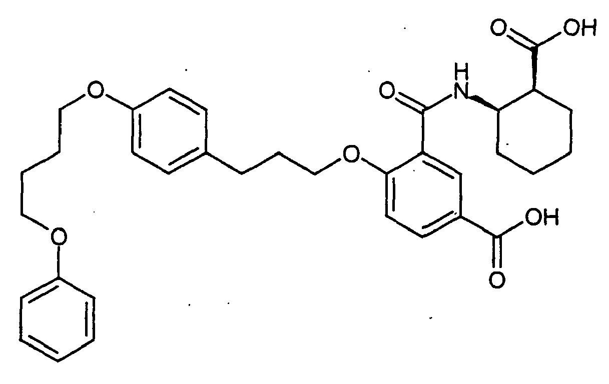

Analog dem unter Beispiel 11 beschriebenen Verfahren wird die Verbindung aus 3-({cis-[2-(Ethoxycarbonyl)cyclohexyl]amino}carbonyl)-4-{3-[4-(4-phenoxybutoxy)-phenyl]propoxy}benzoesäure-ethylester hergestellt.
HPLC (Methode 2): Rₜ. 5.16 min.
¹H-NMR (300MHz, DMSO-d₆, δ/ppm): 12.59 (s, breit, 2H), 8.50-8.42 (m, 2), 8.00 (dd, 1H), 7.30-7.18 (m, 3H), 7.13 (d,2H), 6.95-6.82 (m, 5H), 4.35 (m, 1H), 4.20 (m, 2H), 2.79-2.63 (m, 3H), 2.14 (m, 2H), 1.93-1.77 (m, 6H), 1.70-1.53 (m, 3H), 1.46-1.33 (m, 3H).
MS (ESI+): m/z = 590 (M+H⁺).

### Beispiel 20

### 1-(5-Carboxy-2-{3-[4-(4-phenoxybutoxy)phenyl]propoxy}benzoyl)-3-azetidincarbonsäure

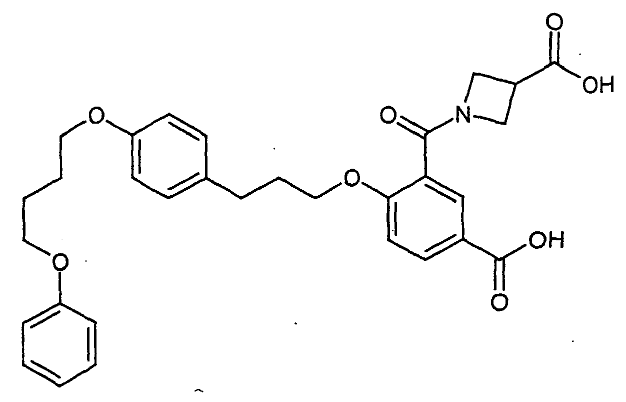

Analog dem unter Beispiel 11 beschriebenen Verfahren wird die Verbindung aus 1-(5-(Ethoxycarbonyl)-2-(3-[4-(4,phenoxybutoxy)phenyl]propoxy)benzoyl)-3-azetidincarbonsäure-methylester hergestellt.
¹H-NMR (300MHz, DMSO-d₆, δ/ppm): 12.73 (s, breit, 2H), 7.95 (dd, 1H), 7.82 (d, 1H), 7.27 (m, 2H), 7.16-7.11 (m, 3H), 6.93-6.83 (m, 5H), 4.21 (t, 1H), 4.13-3.95 (m, 9H), 3.49-3.37 (m, 1H), 2.68 (t, 2H), 2.01 (quint, 2H), 1.85 (m, 4H).
LC-MS (Methode 3): Rₜ: 3.67 min.
MS (ESI+): m/z = 548 (M+H⁺).

### Beispiel 21

### 4-{3-[4-(1,1'-Biphenyl-4-ylmethoxy)phenyl]propoxy}-3-({cis-[2-carboxycyclohexyl]amino}carbonyl)benzoesäure

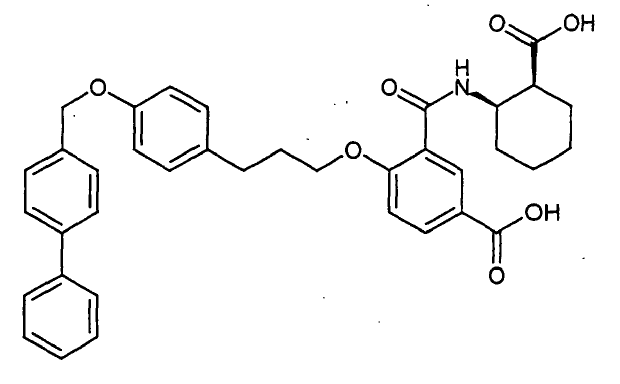

Analog dem unter Beispiel 11 beschriebenen Verfahren wird die Verbindung aus 4-{3-[4-(1,1'-Biphenyl-4-ylmethoxy)phenyl]propoxy}-3-({cis-[2-(ethoxycarbonyl)-cyclohexyl]amino}carbonyl)benzoesäure-ethylester hergestellt.
HPLC (Methode 2): Rₜ: 5.28 min.
¹H-NMR (200MHz, DMSO-d_{6,} δ/ppm): 12.66 (s, breit, 2H), 8.56-8.43 (m, 2H), 8.00 (dd, 1H), 7.72-7.62 (m, 4H), 7.56-7.31 (m, 5H), 7.27-7.12 (m, 3H), 6.95 (d, 2H), 5.1 (s, 2H), 4.42-4.09 (m, 3H), 3.57 (m, 3H), 2.72 (m, 3H), 2.25-2.03 (m, 2H), 1.97-1.29 (m, 5H).
MS (ESI+): m/z = 608 (M+H⁺).

### Beispiel 22

### 4-{3-[4-(3-Cyclohexylpropoxy)phenyl]propoxy}-3-({[3-(methoxycarbonyl)-cyclohexyl]amino}carbonyl)benzoesäure-methylester

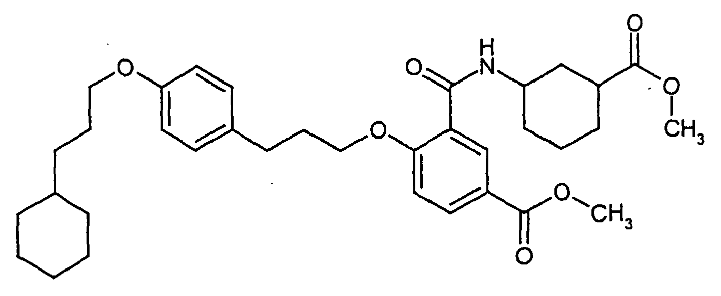

Eine Lösung von 17.88 g des (+)-B-Enantiomers von 4-Hydroxy-3-({[3(methoxycarbonyl)cyclohexyl]amino}carbonyl)benzoesäure-methylester (siehe Beispiel IX, Methode 2) und 19.9 g 1-(3-Brompropyl)-4-(3-cyclohexylpropoxy)benzol in 160 ml wasserfreiem DMF wird mit 20.85 g Cäsiumcarbonat versetzt und 3 Stunden auf ca. 50 bis 60 °C erwärmt. Nach dem Abkühlen auf Raumtemperatur wird der Ansatz auf 180 ml 0.001-molare Salzsäure gegossen und mit Ethylacetat extrahiert. Die organische Phase wird nacheinander mit Wasser und gesättigter Kochsalzlösung gewaschen, Trocknen über wasserfreiem Natriumsulfat, Filtrieren, Einrotieren. Es wird ein Öl erhalten, das mit wenig Ethylacetat und Cyclohexan versetzt wird. Es fällt ein Feststoff aus. Die Suspension wird in einem Eisbad 1 Stunde gekühlt. Anschliessend wird der Feststoff abgesaugt und mit wenig Cyclohexan nachgewaschen. Es werden 26.0 g eines Feststoffs erhalten.
DC: R_{f}-Wert: 0.43 (Cyclohexan/Ethylacetat 1:1).
HPLC (Methode 2): Rₜ: 6.75 min.
¹H-NMR (300MHz, DMSO-d₆, δ/ppm): 8.21 (d, 1H), 8.07 (d, 1H), 8.00 (dd, 1H), 7.21 (d, 1H), 7.11 (d, 2H), 6.82 (d, 2H), 4.13 (pseudo-t, 3H), 3.89 (t, 2H), 3.83 (s, 3H), 3.56 (s, 3H), 2.68 (pseudo-t, 3H), 2.07 (quint., 2H), 1.88-1.78 (m, 2H), 1.71-1.49 (m, 13H), 1.32-1.09 (m, 6H), 0.92-0.80 (m, 2H).
MS (DCI, NH₃): m/z = 594.3 (M+H⁺), 611.4 (M+NH₄⁺).

### Beispiel 23

### 3-{[(3-Carboxycyclohexyl)amino]carbonyl}-4-{3-[4-(3-cyclohexylpropoxy)phenyl]-propoxy}benzoesäure

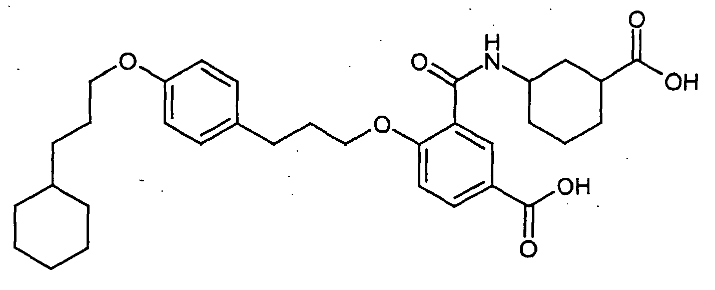

Eine Lösung von 90.73 g 4-{3-[4-(3-Cyclohexylpropoxy)phenyl]propoxy}-3-({[3-(methoxycarbonyl)cyclohexyl]amino}carbonyl)benzoesäure-methylester in 500 ml THF und 500 ml Methanol wird mit 300 ml 2-molarer Natronlauge versetzt und auf 60 °C erwärmt. Nach einer Stunde werden die organischen Lösemittel am Rotationsverdampfer weitestgehend entfernt. Der Rückstand wird unter Rühren mit 325 ml 2-molarer Salzsäure versetzt. Dabei fällt ein Niederschlag aus, der abgesaugt und mit Wasser gewaschen wird. Das Produkt wird durch Umkristallisieren aus einem Gemisch von 500 ml Aceton und 1000 ml Wasser gereinigt. Es werden 77.5 g Produkt erhalten.
Fp.: 160 °C.
HPLC (Methode 1): Rₜ: 5.61 min.
¹H-NMR (400MHz, DMSO-d₆, δ/ppm): 12.48 (s breit, 2H), 8.21 (d, 1H), 8.07 (d, 1H), 7.98 (dd, 1H), 7.18 (d, 1H), 7.12 (d, 2H), 6.82 (d, 2H), 4.12 (pseudo-t, 3H), 3.88 (t, 2H), 2.70 (pseudo-t, 2H), 2.62 (m, 1H), 2.07 (quint., 2H), 1.89-1.47 (m, 15H), 1.31-1.07 (m, 6H), 0.91-0.82 (m, 2H).
MS (ESI+): m/z = 566.5 (M+H⁺).

### Beispiel 24

### 4-(3-{4-[4-Cyclohexyloxy)butoxy]phenyl}propoxy)-3-({[3-(methoxycarbonyl)-cyclohexyl]amino}carbonyl)benzoesäure-methylester

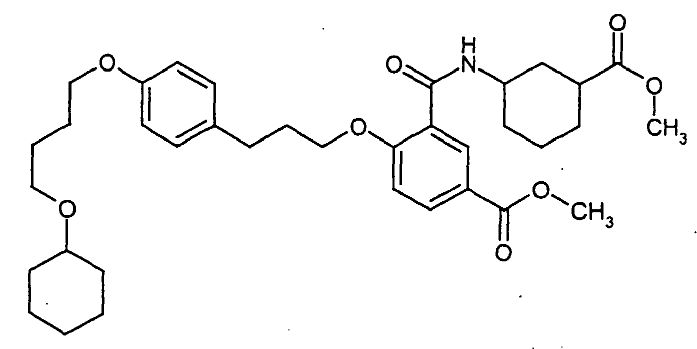

Die Darstellung erfolgt analog Beispiel 22 aus dem (+)-B-Enantiomer von 4-Hydroxy-3-({[3(methoxycarbonyl)cyclohexyl]amino}carbonyl)benzoesäure-methylester (siehe Beispiel IX, Methode 2) und 1-(3-Brompropyl)-4-[4-(cyclohexyloxy)butoxy]benzol.
DC: R_{f}-Wert: 0.46 (Cyclohexan/Ethylacetat 1:1).
HPLC (Methode 2): Rₜ: 6.08 min.
¹H-NMR (300MHz, DMSO-d₆, δ/ppm): 8.22 (d, 1H), 8.08 (d, 1H), 8.00 (dd, 1H), 7.21 (d, 1H), 7.11 (d, 2H), 6.82 (d, 2H), 4.13 (pseudo-t, 3H), 3.92 (t, 2H), 3.82 (s, 3H), 3.57 (s, 3H), 3.42 (t, 2H), 3.21 (m, 1H), 2.69 (pseudo-t, 3H), 2.07 (quint., 2H), 1.83-1.42 (m, 16H), 1.19 (m, 6H).
MS (DCI, NH₃): m/z = 624 (M+H⁺), 641 (M+NH₄⁺).

### Beispiel 25

### 3-{[(3-Carboxycyclohexyl)amino]carbonyl)-4-(3-{4-[4-(cyclohexyloxy)butoxy]-phenyl}propoxy)benzoesäure

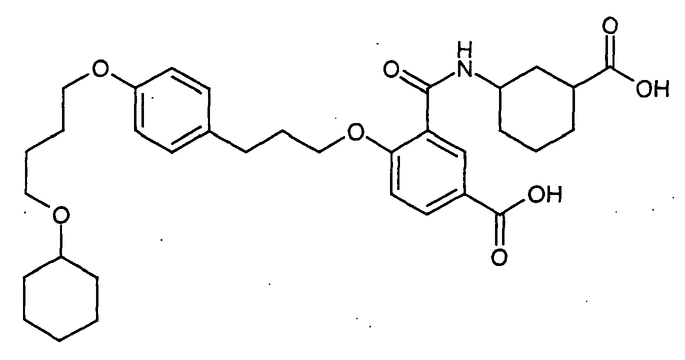

Die Darstellung erfolgt analog Beispiel 23 aus 4-(3-{4-[4-Cyclohexyloxy)butoxy]-phenyl}propoxy)-3-({[3-(methoxycarbonyl)cyclohexyl]-amino}carbonyl)benzoesäure-methylester.
Fp.: 161-162 °C.
HPLC (Methode 2): Rₜ: 5.27 min.
¹H-NMR (300MHz, DMSO-d₆, δ/ppm): 12.43 (s breit, 2H), 8.22 (d, 1H), 8.05 (d, 1H), 7.98 (dd, 1H), 7.18 (d, 1H), 7.11 (d, 2H), 6.82 (d, 2H), 4.13 (pseudo-t, 3H), 3.93 (t, 2H), 3.42 (t, 2H), 3.21 (m, 1H), 2.70 (pseudo-t, 2H), 2.62 (m, 1H), 2.07 (quint., 2H), 1.91-1.42 (m, 17H), 1.21 (m, 5H).
MS (ESI-): m/z = 594 (M-H⁺)⁻.

### Beispiel 26

### 3-({[3-(Methoxycarbonyl)cyclohexyl]amino}carbonyl)-4-{3-[4-(4-phenoxybutoxy)-phenyl]propoxy}benzoesäure-methylester

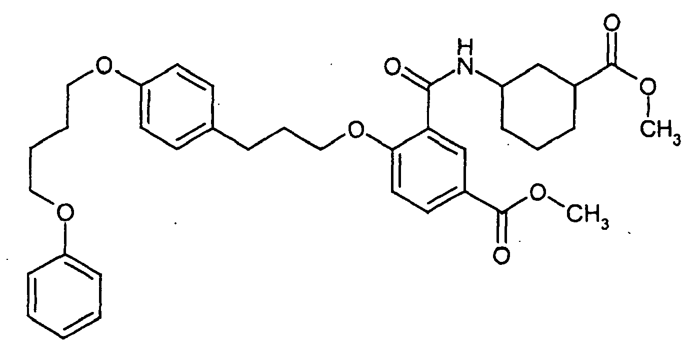

Die Darstellung erfolgt analog Beispiel 22 aus dem (+)-B-Enantiomer von 4-Hydroxy-3-({[3(methoxycarbonyl)cyclohexyl]amino}carbonyl)benzoesäure-methylester (siehe Beispiel IX, Methode 2) und 1-(3-Brompropyl)-4-[4-(phenoxy)-butoxy]benzol.
DC: R_{f}-Wert: 0.35 (Cyclohexan/Ethylacetat 1:1).
HPLC (Methode 2): Rₜ: 5.80 min.
¹H-NMR (200MHz, DMSO-d₆, δ/ppm): 8.20 (d, 1H), 8.12 (d, 1H), 8.01 (dd, 1H), 7.31-7.19 (m, 3H), 7.12 (d, 2H), 6.90-6.83 (m, 5H), 4.13 (pseudo-t, 3H), 3.99 (m, 4H), 3.83 (s, 3H), 3.57 (s, 3H), 2.70 (pseudo-t, 3H), 2.08 (quint., 2H), 1.85 (m, 6H), 1.68-1.49 (m, 6H).
MS (ESI+): m/z = 618 (M+H⁺), 640 (M+Na⁺).

### Beispiel 27

### 3-{[(3-Carboxycyclohexyl)amino)carbonyl}-4-{3-[4-(4-phenoxybutoxy)phenyl]-propoxy}benzoesäure

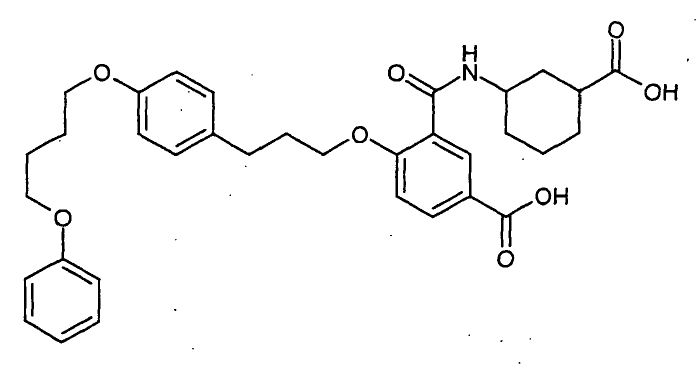

Die Darstellung erfolgt analog Beispiel 23 aus 3-({[3-(Methoxycarbonyl)-cyclohexyl]amino}carbonyl)-4-{3-[4-(4-phenoxybutoxy)phenyl]-propoxy}benzoesäure-methylester. Da beim Ansäuern das Produkt nicht ausgefallen ist, wird mit Ethylacetat extrahiert und der organische Extrakt über wasserfreiem Natriumsulfat getrocknet. Nach Filtrieren und Einrotieren wird das Produkt erhalten.
HPLC (Methode 2): Rₜ: 5.03 min.
¹H-NMR (300MHz, DMSO-d₆, δ/ppm): 12.39 (s breit, 2H), 8.21 (d, 1H), 8.05 (d, 1H), 7.98 (dd, 1H), 7.30-7.23 (dd, 2H), 7.18.(d, 1H), 7.12 (d, 2H), 6.94-6.82 (m, 5H), 4.13 (pseudo-t, 3H), 4.00 (m, 4H), 2.70 (pseudo-t, 2H), 2.62 (m, 1H), 2.07 (quint., 2H), 1.90-1.48 (m, 12H).
LC-MS (Methode 11): Rₜ: 3.80 min, m/z (ES+) = 590 (M+H⁺).

### Beispiel 28

### 4-{3-[4-(Biphenyl-4-ylmethoxy)phenyl]propoxy}-3-({[(1S,3R)-3-(methoxycarbonyl)cyclopentyl]amino}carbonyl)benzoesäure-ethylester

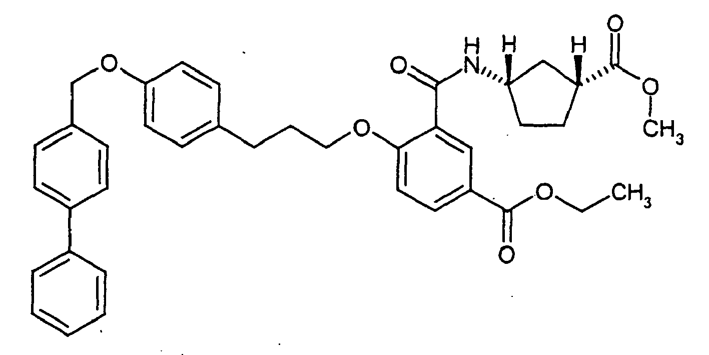

Die Herstellung erfolgt analog Beispiel 5 aus 2-{3-[4-(1,1'-Biphenyl-4-ylmethoxy)-phenyl]propoxy}-5-(ethoxycarbonyl)benzoesäure und (1R,3S)-3-Aminocyclopentancarbonsäure-methylester - Hydrochlorid.
HLPC (Methode 2): Rₜ: 5.95 min.
¹H-NMR (300 MHz, DMSO-d₆, δ/ppm): 8.22 (d, 1H), 8.18 (d, 1H), 8.00 (dd, 1H), 7.69-7.65 (m, 4H), 7.53-7.43 (m, 4H), 7.39-7.33 (m, 1H), 7.22 (d, 1H), 7.14 (d, 2H), 6.94 (d, 2H), 5.11 (s, 2H), 4.30 (quart., 2H), 4.14 (t, 2H), 3.55 (s, 3H), 2.88 (quint., 1H), 2.71 (pseudo-t, 2H), 2.32-2.22 (m, 1H), 2.08 (quint., 2H), 1.99-1.82 (m, 3H), 1.78-1.57 (m, 2H), 1.31 (t, 3H).
MS (ESI+): m/z = 636 (M+H⁺).

### Beispiel 29

### 4-{3-[4-(Biphenyl-4-ylmethoxy)phenyl]propoxy}-3-({[(1S,3R)-3-carboxycyclopentyl]amino}carbonyl)benzoesäure

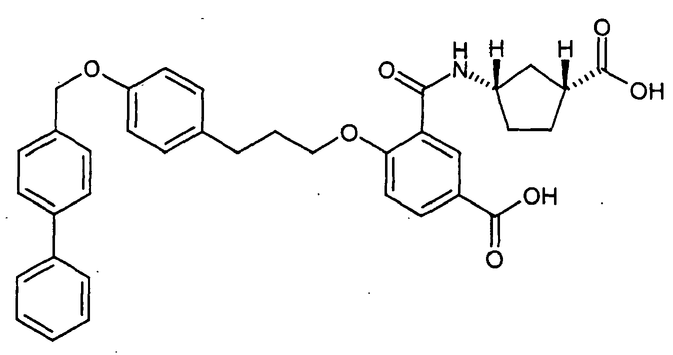

Die Darstellung erfolgt analog Beispiel 23 aus 4-{3-[4-(Biphenyl-4-ylmethoxy)phenyl]propoxy}-3-({[(1S,3R)-3-(methoxycarbonyl)cyclopentyl]amino}-carbonyl)benzoesäure-ethylester.
HLPC (Methode 12): Rₜ: 8.05 min.
¹H-NMR (300 MHz, DMSO-d₆, δ/ppm): 12.28 (s breit), 8.23 (d, 1H), 8.18 (d, 1H), 7.98 (dd, 1H), 7.69-7.64 (m, 4H), 7.53-7.43 (m, 4H), 7.39-7.35 (m, 1H), 7.18-7.13 (m, 3H), 6.94 (d, 2H), 5.11 (s, 2H), 4.31 (m, 1H), 4.14 (t, 2H), 2.78 (quint., 1H), 2.70 (pseudo-t, 2H), 2.29-2.20 (m, 1H), 2.08 (quint., 2H), 1.99-1.82 (m, 3H), 1.78-1.53 (m, 2H).
LC-MS (Methode 8): Rₜ: 3.55 min, m/z (ES+) =594 (M+H⁺).

### Beispiel 30

### 1-[2-{3-[4-(3-Cyclohexylpropoxy)phenyl]propoxy}-5-(methoxycarbonyl)benzoyl]-piperidin-4-carbonsäure-methylester

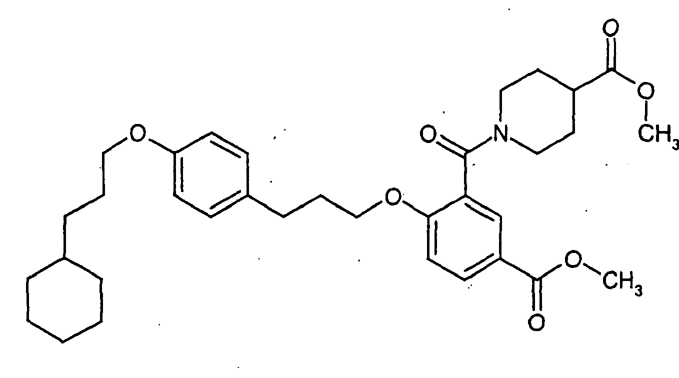

Eine Lösung von 6.47 g 1-[2-Hydroxy-5-(methoxycarbonyl)benzoyI]-4-piperidincarbonsäure-methylester und 8.2 g 1-(3-Brompropyl)-4-(3-cyclohexylpropoxy)-benzol werden in 82 ml Butyronitril vorgelegt, mit 4.18 g Kaliumcarbonat versetzt und 15 Stunden zum Rückfluss erhitzt. Anschliessend wird die Reaktionslösung zur Trockene eingeengt, der Rückstand mit Wasser aufgenommen und mit Ethylacetat extrahiert. Die organische Phase wird über Natriumsulfat getrocknet, filtriert und eingeengt. Das Rohprodukt wird durch Saugfiltration über Kieselgel mit Cyclohexan/Ethylacetat 5:1 nach Cyclohexan/Ethylacetat 1:1 als Laufmittel gereinigt. Es werden 10.8 g eines Öls erhalten.
DC: R_{f}-Wert: 0.36 (Cyclohexan/Ethylacetat 1:1).
HPLC (Methode 2): Rₜ: 6.07 min.
¹H-NMR (300MHz, DMSO-d₆, δ/ppm): 7.97 (dd, 1H), 7.76 und 7.69 (je d, zusammen 1H), 7.17 (d, 1H), 7.08 (d, 2H), 6.82 (d, 2H), 4.43 und 4.39 (zusammen 1H), 4.17-3.96 (m, 2H), 3.89 (t, 2H), 3.82 (s, 3H), 3.61 und 3.57 (je s, zusammen 3H), 3.11-2.88 (m, 2H), 2.63 (m, 3H), 1.96 (m, 3H), 1.77-1.50 (m, 10H), 1.32-1.09 (m, 7H), 0.92-0.80 (m, 2H).
MS (ESI): m/z = 580 (M+H⁺).

### Beispiel 31

### 1-(5-Carboxy-2-{3-[4-(3-cyclohexylpropoxy)phenyl]propoxy}benzoyl)piperidin-4-carbonsäure

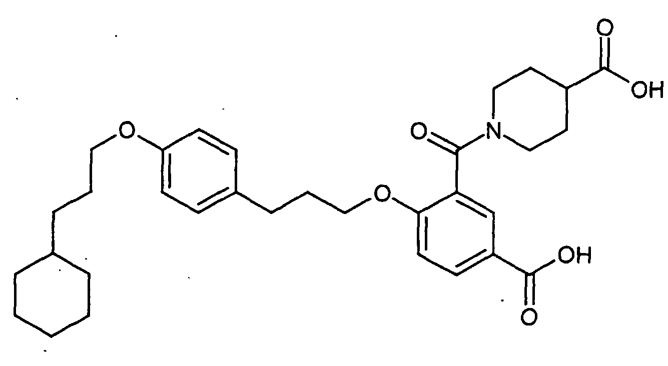

Eine Lösung von 10.4 g 1-[2-{3-[4-(3-Cyclohexylpropoxy)phenyl]propoxy}-5-(methoxycarbonyl)benzoyl]piperidin-4-carbonsäure-methylester in einem Gemisch aus 160 ml THF und 160 ml Methanol wird mit 160 ml 2-molarer Natronlauge versetzt und 1 Stunde bei einer Temperatur von 60 °C gerührt. Anschliessend werden die organischen Lösemittel am Rotationsverdampfer weitestgehend entfernt. Die verbleibende wässrige Lösung wird unter Rühren mit 165 ml 2-molarer Salzsäure versetzt. Es fällt ein Niederschlag aus. Die Fällung wird durch Rühren bei 0 °C vervollständigt. Der Niederschlag wird abgesaugt und mit Wasser gewaschen. Das Rohprodukt wird durch Umkristallisieren aus einem Lösemittelgemisch aus 250 ml Aceton und 200 ml Wasser gereinigt. Es werden 8.7 g eines Feststoffs erhalten, der nochmals in einem Gemisch aus 300 ml Wasser und 100 ml Aceton 6 Stunden zum Rückfluss erhitzt wird. Nach Abkühlen auf 0 °C, Filtrieren, Waschen mit Wasser und Trocknen werden 8.6 g eines Feststoffs erhalten.
Fp.: 183.4-184.5 °C.
HPLC (Methode 2): Rₜ: 5.32 min.
¹H-NMR (300MHz, DMSO-d₆, δ/ppm): 12.51 (s breit, 2H), 7.93 (dd, 1H), 7.72 und 7.67 (je d, zusammen 1H), 7.13 (d, 1H), 7.09 (d, 2H), 6.83 und 6.90 (je d, zusammen 2H), 4.43 und 4.38 (zusammen 1H), 4.15-3.94 (m, 2H), 3.88 (t, 2H), 3.11-2.88 (m, 2H), 2.62 (m, 2H), 2.54 (m, 1H), 1.96 (m, 3H), 1.78-1.47 (m, 10H), 1.37-1.09 (m, 7H), 0.93-0.81 (m, 2H).
MS (ESI): m/z = 552 (M+H⁺).

### Beispiel 32

### 4-(3-{4-[(4-Isopropoxybenzyl)oxy]phenyl}propoxy)-3-({[3-(methoxycarbonyl)-cyclohexyl]amino}carbonyl)benzoesäure-methylester

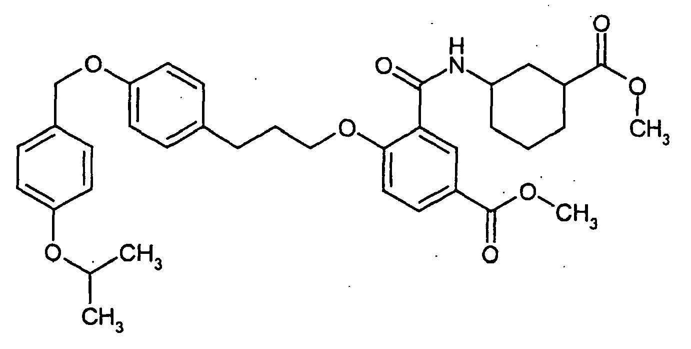

Die Darstellung erfolgt analog Beispiel 22 aus dem (+)-B-Enantiomer von 4-Hydroxy-3-({[3(methoxycarbonyl)cyclohexyl]amino}carbonyl)benzoesäure-methylester (siehe Beispiel IX, Methode 2) und 1-(3-Brompropyl)-4-[(4-isopropoxybenzyl)oxy]benzol.
DC: R_{f}-Wert: 0.43 (Cyclohexan/Ethylacetat 1:1):
HPLC (Methode 2): Rₜ: 5.69 min.
¹H-NMR (400MHz, DMSO-d₆, δ/ppm): 8.21 (d, 1H), 8.10 (d breit; 1H), 8.01 (dd, 1H), 7.32 (d, 2H), 7.22 (d, 1H), 7.13 (d, 2H), 6.91 (2 d, 4H), 4.95 (s, 2H), 4.60 (sep, 1H), 4.13 (pseudo-t, 3H), 3.83 (s, 3H), 3.57 (s, 3H), 2.69 (pseudo-t, 3H), 2.07 (pseudo-quint., 3H), 1.82 (m, 2H), 1.68-1.47 (m, 6H), 1.24 (d, 6H).
MS (DCI, NH₃): m/z = 618 (M+H⁺), 635 (M+NH₄⁺).

### Beispiel 33

### 3-{[(3-Carboxycyclohexyl)amino]carbonyl}-4-(3-{4-[(4-isopropoxybenzyl)oxy]-phenyl}propoxy)benzoesäure

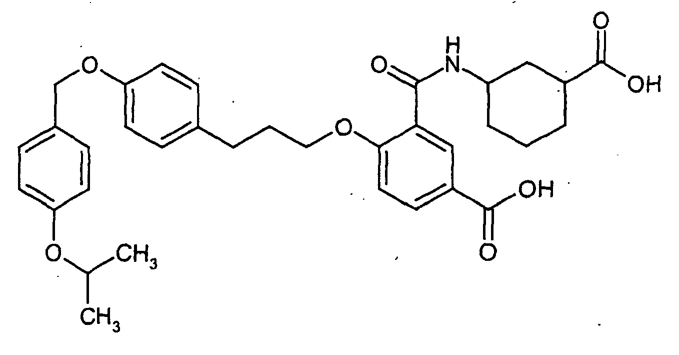

Die Darstellung erfolgt analog Beispiel 23 aus 4-(3-{4-[(4-Isopropoxybenzyl)-oxy]phenyl}propoxy)-3-({[3-(methoxycarbonyl)cyclohexyl]amino}carbonyl)-benzoesäure-methylester.
HPLC (Methode 2): Rₜ: 4.97 min.
¹H-NMR (300MHz, DMSO-d₆, δ/ppm): 12.47 (s breit, 2H), 8.22 (d, 1H), 8.07 (d breit, 1H), 7.98 (dd, 1H), 7.32 (d, 2H), 7.18 (d, 1H), 7.I3 (d, 2H), 6.91 (2 d, 4H), 4.96 (s, 2H), 4.59 (sep, 1H), 4.13 (pseudo-t, 3H), 2.71 (pseudo-t, 2H), 2.63 (m, 1H), 2.08 (pseudo-quint., 2H), 1.91-1.47 (m, 8H), 1.25 (d, 6H).
MS (ESI): m/z = 590 (M+H⁺).

### Beispiel 34

### 4-(3-{4-[3-(5,5-Dimethyl-1,3-dioxan-2-yl)propoxy]phenyl}propoxy)-3-({[3-(methoxycarbonyl)cyclohexyl]amino}carbonyl)benzoesäure-methylester

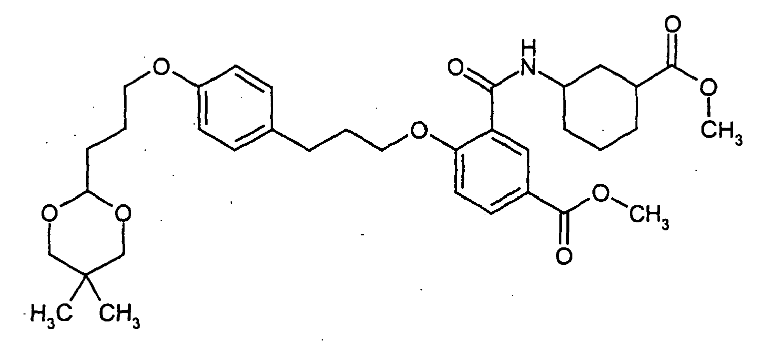

Eine Lösung von 130 mg 4-[3-(4-Hydroxyphenyl)propoxy]-3-({[3-(methoxycarbonyl)cyclohexyl]amino}carbonyl)benzoesäure-methylester und 85.9 mg 2-(3-Brompropyl)-5,5-dimethyl-1,3-dioxan in 1 ml wasserfreiem DMF wird mit 128.8 mg Cäsiumcarbonat versetzt und 15 Stunden lang bei 100 °C gerührt. Anschliessend wird das Reaktionsgemisch komplett auf eine RP-HPLC-Anlage aufgebracht und mit einem Wasser/Acetonitril-Gradienten chromatographiert. Nach Einrotieren der Produktfraktionen werden 87.5 mg eines Feststoffs erhalten.
DC (Cyclohexan/Ethylacetat 1:1): R_{f}: 0.37.
HPLC (Methode 2): Rₜ: 5.54 min.
¹H-NMR (300 MHz, DMSO-d₆, δ/ppm): 8.22 (d, 1H), 8.08 (d breit, 1H), 8.01 (dd, 1H), 7.22 (d, 1H), 7.11 (d, 2H), 6.83 (d, 2H), 4.48 (t, 1H), 4.14 (pseudo-t, 3H), 3.93 (t, 2H), 3.83 (s, 3H), 3.58 (s, 3H), 3.52 (d, 2H), 3.39 (d, 2H), 2.70 (m, 3H), 2.07 (quint, 2H), 1.84-1.72 (m, 4H), 1.68-1.50 (m, 8H), 1.09 (s, 3H), 0.68 (s, 3H).
LC-MS (Methode 13): Rₜ: 4.3 min, m/z (ES+) = 626 (M+H⁺).

### Beispiel 35

### 3-{[(3-Carboxycyclohexyl)amino]carbonyl}-4-(3-{4-[3-(5,5-dimethyl-1,3-dioxan-2-yl)propoxy]phenyl }propoxy)benzoesäure-methylester

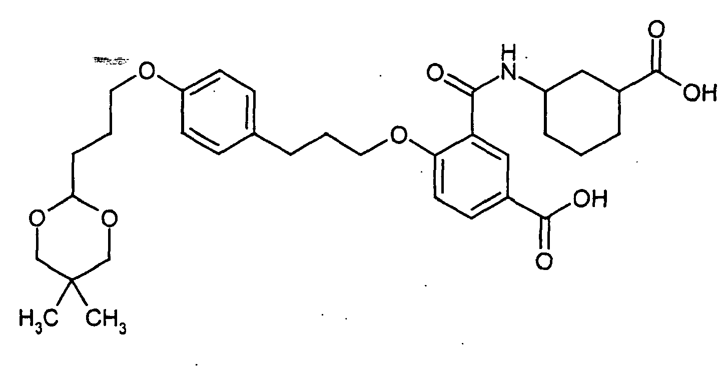

Eine Lösung von 70.0 mg 4-(3-{4-[3-(5,5-Dimethyl-1,3-dioxan-2-yl)propoxy]-phenyl} propoxy)-3-({[3-(methoxycarbonyl)cyclohexyl]amino }carbonyl)benzoesäure-methylester in jeweils 1 ml Methanol, THF, und 2-molarer Natronlauge wird 2 Stunden bei einer Temperatur von 60 °C gerührt. Anschliessend wird mit 1.1 ml 2-molarer Salzsäure versetzt und man lässt das Reaktionsgefäß 15 Stunden offen bei Raumtemperatur stehen. Es fällt ein Niederschlag aus, der abgesaugt und mit Wasser gewaschen wird. Sollte die Fällung nicht vollständig sein, so kann mit Ethylacetat extrahiert werden. Der organische Extrakt wird über wasserfreiem Natriumsulfat getrocknet und einrotiert. Es werden 53.3 mg eines Feststoffs erhalten.
HPLC (Methode 2): Rₜ: 4.86 min.
¹H-NMR (200 MHz, DMSO-d₆, δ/ppm): 12.52 (s breit, 2H), 8.21 (d, 1H), 8.09 (d breit, 1H), 7.98 (dd, 1H), 7.19 (d, 1H), 7.13 (d, 2H), 6.83 (d, 2H), 4.48 (t, 1H), 4.13 (pseudo-t, 3H), 3.92 (t, 2H), 3.52 (d, 2H), 3.39 (d, 2H), 2.73-2.60 (m, 3H), 2.07 (quint, 2H), 1.87-1.47 (m, 12H), 1.09 (s, 3H), 0.68 (s, 3H).
LC-MS (Methode 14): R,: 3.13 min, m/z (ES+) = 598 (M+H⁺).

### Beispiel 36

### 3-({[3-(Methoxycarbonyl)cyclohexyl]amino}carbonyl)-4-(3-{4-[(4-propoxybenzyl)-oxy]phenyl}propoxy)benzoesäure-methylester

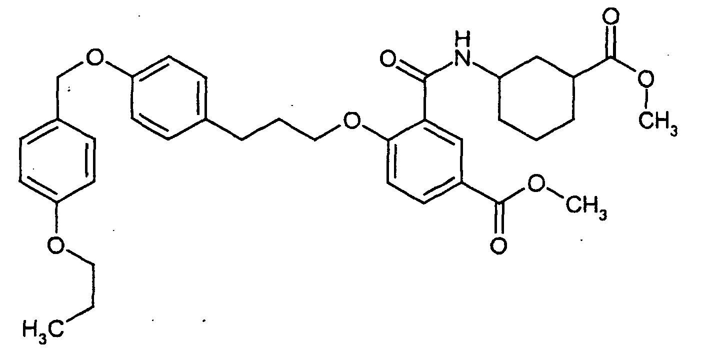

Analog dem unter Beispiel 34 beschriebenen Verfahren wird 3-({[3-(Methoxycarbonyl)cyclohexyl]amino}carbonyl)-4-(3-{4-[(4-propoxybenzyl)oxy]phenyl}-propoxy)benzoesäure-methylester aus 4-[3-(4-Hydroxyphenyl)propoxy]-3-({[3-(methoxycarbonyl)cyclohexyl]amino}carbonyl)benzoesäure-methylester und 1-(Chlormethyl)-4-propoxybenzol hergestellt.
DC (Cyclohexan/Ethylacetat 1:1): R_{f}: 0.39.
HPLC (Methode 2): Rₜ: 5.67 min.
MS (ESI): m/z = 618 (M+H⁺), 640 (M+Na⁺).

### Beispiel 37

### 3-{[(3-Carboxycyclohexyl)amino]carbonyl}-4-(3-{4-[(4-propoxybenzyl)oxy]-phenyl}propoxy)benzoesäure

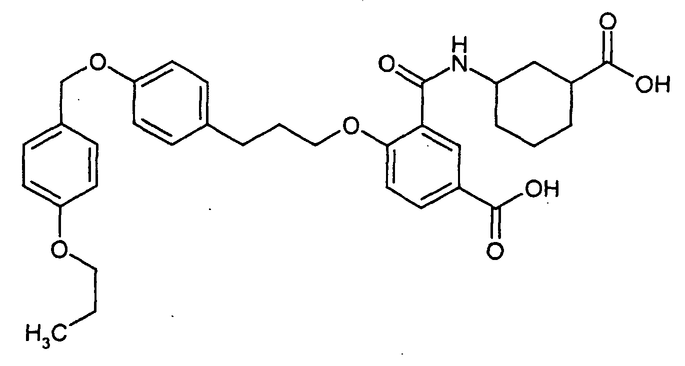

Analog dem unter Beispiel 35 beschriebenen Verfahren wird 3-{[(3-Carboxycyclohexyl)amino]carbonyl}-4-(3-{4-[(4-propoxybenzyl)oxy]phenyl}propoxy)-benzoesäure aus 3-({[3-(Methoxycarbonyl)cyclohexyl]amino}carbonyl)-4-(3-{4-[(4-propoxybenzyl)oxy]phenyl}propoxy)benzoesäure-methylester hergestellt.
HPLC (Methode 2): Rₜ: 5.05 min.
¹H-NMR (200 MHz, DMSO-d₆, δ/ppm): 12.52 (s breit, 2H), 8.20 (d, 1H), 8.09 (d breit, 1H), 7.98 (dd, 1H), 7.33 (d, 2H), 7.19 (d, 1H), 7.13 (d, 2H), 6.92 (d, 2H), 6.91 (d, 2H), 4.96 (s, 2H), 4.12 (pseudo-t, 3H), 3.91 (t, 2H), 2.73-2.58 (m, 3H), 2.06 (m, 2H), 1.87-1.48 (m, 10H), 0.97 (t, 3H).
MS (ESI): m/z = 588 (M-H⁺).

### Beispiel 38

### 4-[3-(4-{[4-(Cyclopropylmethoxy)benzyl]oxy}phenyl)propoxy]-3-({[3-(methoxycarbonyl)cyclohexyl]amino}carbonyl)benzoesäure-methylester

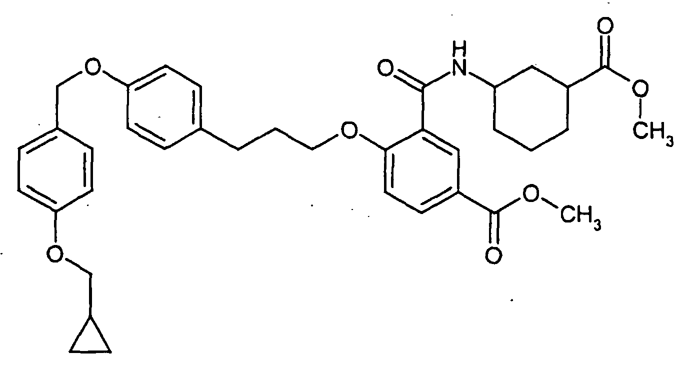

Analog dem unter Beispiel 34 beschriebenen Verfahren wird 4-[3-(4-{[4-(Cyclopropylmethoxy)benzyl]oxy}phenyl)propoxy]-3-({[3-(methoxycarbonyl)-cyclohexyl]amino}carbonyl)benzoesäure-methylester aus 4-[3-(4-Hydroxyphenyl)-propoxy]-3-({[3-(methoxycarbonyl)cyclohexyl]amino}carbonyl)benzoesäure-methylester und 1-(Chlormethyl)-4-(cyclopropylmethoxy)benzol hergestellt.
DC (Cyclohexan/Ethylacetat 1:1): R_{f}: 0.35.
HPLC (Methode 2): Rₜ: 5.58 min.
MS (ESI): m/z = 630 (M+H⁺), 652 (M+Na⁺).

### Beispiel 39

### 3-{[(3-Carboxycyclohexyl)amino]carbonyl}-4-[3-(4-{[4-(cyclopropylmethoxy)-benzyl]oxy}phenyl)propoxy]benzoesäure

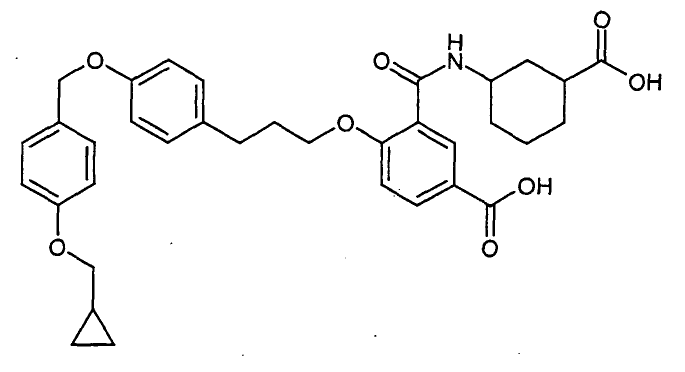

Analog dem unter Beispiel 35 beschriebenen Verfahren wird 3-{[(3-Carboxycyclohexyl)amino]carbonyl}-4-[3-(4-{[4-(cyclopröpylmethoxy)-benzyl]oxy}phenyl)propoxy]benzoesäure aus 4-[3-(4-{[4-(Cyclopropylmethoxy)benzyl]oxy}phenyl)propoxy]-3-({[3-(methoxycarbonyl)cyclohexyl]amino}carbonyl)benzoesäure-methylester hergestellt.
HPLC (Methode 2): Rₜ: 4.99 min.
¹H-NMR (200 MHz, DMSO-d₆, δ/ppm): 12.52 (s breit, 2H), 8.20 (d, 1H), 8.08 (d breit, 1H), 7.98 (dd, 1H), 7.33 (d, 2H), 7.18 (d, 1H), 7.13 (d, 2H), 6.91 (2 d, 4H), 4.95 (s, 2H), 4.12 (pseudo-t, 3H), 3.80 (d, 2H), 2.73-2.57 (m, 3H), 2.07 (m, 2H), 1.87-1.46 (m, 8H), 1.30-1.13 (m, 2H), 0.57 (m, 2H), 0.32 (m, 2H).
MS (ESI): m/z = 600 (M-H⁺).

### Beispiel 40

### 3-({[3-(Methoxycarbonyl)cyclohexyl]amino}carbonyl)-4-(3-{4-[(4-phenoxybenzyl)-oxy]phenyl}propoxy)benzoesäure-methylester

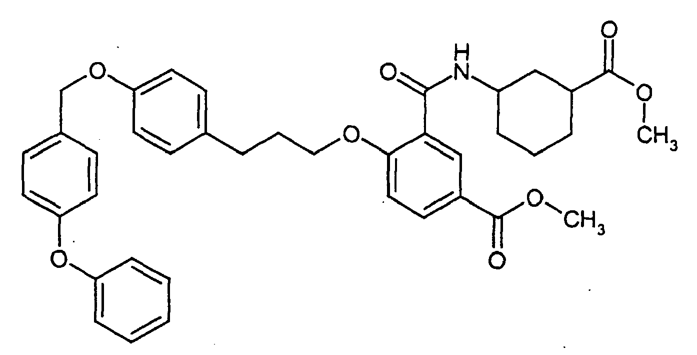

Analog dem unter Beispiel 34 beschriebenen Verfahren wird 3-({[3-(Methoxycarbonyl)cyclohexyl]amino}carbonyl)-4-(3-{4-[(4-phenoxybenzyl)-oxy]phenyl}propoxy)benzoesäure-methylester aus 4-[3-(4-Hydroxyphenyl)propoxy]-3-({[3-(methoxycarbonyl)cyclohexyl]amino}carbonyl)benzoesäure-methylester und 1-(Chlormethyl)-4-phenoxybenzol hergestellt.
DC (Cyclohexan/Ethylacetat 1:1): R_{f}: 0.36.
HPLC (Methode 2): Rₜ: 5.86 min.
MS (ESI): m/z = 652 (M+H⁺), 674 (M+Na⁺).

### Beispiel 41

### 3-{[(3-Carboxycyclohexyl)amino]carbonyl}-4-(3-{4-[(4-phenoxybenzyl)oxy]-phenyl}propoxy)benzoesäure

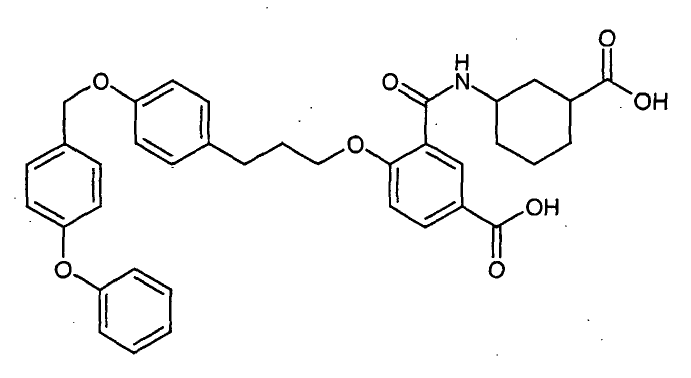

Analog dem unter Beispiel 35 beschriebenen Verfahren wird 3-{[(3-Carboxycyclohexyl)amino]carbonyl}-4-(3-{4-[(4-phenoxybenzyl)oxy]phenyl}-propoxy)benzoesäure aus 3-({[3-(Methoxycarbonyl)cyclohexyl]amino}carbonyl)-4-(3-(4-[(4-phenoxybenzyl)oxy]phenyl) propoxy)benzoesäure-methylester hergestellt.
HPLC (Methode 2): Rₜ: 5.19 min.
¹H-NMR (300 MHz, DMSO-d₆, δ/ppm): 12.45 (s breit, 2H), 8.21 (d, 1H), 8.04 (d breit, 1H), 7.98 (dd, 1H), 7.40 (d, 1H), 7.38 (d, 2H), 7.21-6.88 (m, 11H), 5.05 (s, 2H), 4.13 (pseudo-t, 3H), 2.07 (t, 2H), 2.61 (m, 1H), 2.07 (m, 2H), 1.90-1.47 (m, 8H).
LC-MS (Methode 8): Rₜ: 3.62 min, m/z (ES+) = 624 (M+H⁺).

### Beispiel 42

### 4-(3-{4-[(4-Isobutoxybenzyl)oxy]phenyl}propoxy)-3-({[3-(methoxycarbonyl)-cyclohexyl]amino}carbonyl)benzoesäure-methylester

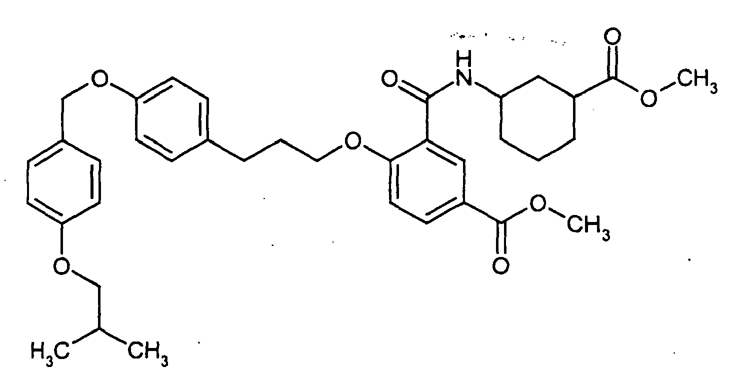

Analog dem unter Beispiel 34 beschriebenen Verfahren wird 4-(3-{4-[(4-Isobutoxybenzyl)oxy]phenyl}propoxy)-3-({[3-(methoxycarbonyl)cyclohexyl]-amino}carbonyl)benzoesäure-methylester aus 4-[3-(4-Hydroxyphenyl)propoxy]-3-({[3-(methoxycarbonyl)cyclohexyl]amino}carbonyl)benzoesäure-methylester und 1-(Chlormethyl)-4-isobutoxybenzol hergestellt.
DC (Cyclohexan/Ethylacetat 1:1): R_{f}: 0.37.
HPLC (Methode 2): Rₜ: 5.91 min.
MS (ESI): m/z = 632 (M+H⁺), 654 (M+Na⁺).

### Beispiel 43

### 3-{[(3-Carboxycyclohexyl)amino]carbonyl}-4-(3-{4-[(4-isobutoxybenzyl)-oxy]phenyl}propoxy)benzoesäure

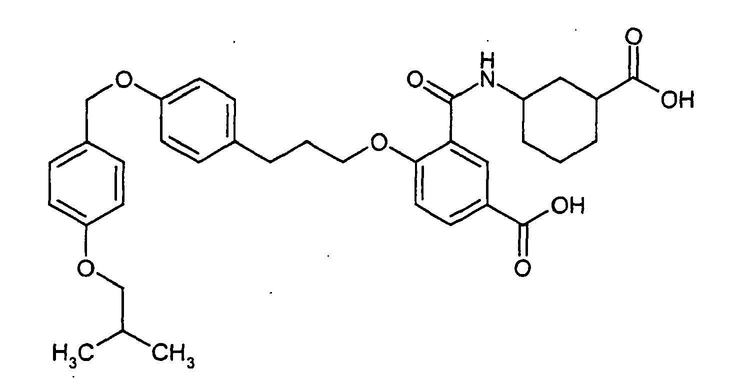

Analog dem unter Beispiel 35 beschriebenen Verfahren wird 3-{[(3-Carboxycyclohexyl)amino]carbonyl}-4-(3-{4-[(4-isobutoxybenzyl)oxy]phenyl}propoxy)-benzoesäure aus 4-(3-{4-[(4-Isobutoxybenzyl)oxy]phenyl}propoxy)-3-({[3-(methoxycarbonyl)cyclohexyl]amino}carbonyl)benzoesäure-methylester hergestellt.
HPLC (Methode 2): Rₜ: 5.26 min.
¹H-NMR (200 MHz, DMSO-d₆, δ/ppm): 12.51 (s breit, 2H), 8.20 (d, 1H), 8.08 (d breit, 1H), 7.98 (dd, 1H), 7.33 (d, 2H), 7.18 (d, 1H), 7.13 (d, 2H), 6.91 (d, 2H), 6.90 (d, 2H), 4.97 (s, 2H), 4.12 (pseudo-t, 3H), 3.73 (d, 2H), 2.73-2.58 (m, 3H), 2.10-1.90 (m, 3H), 1.86-1.68 (m, 3H), 1.63-1.47 (m, 5H), 0.97 (d, 6H).
LC-MS (Methode 8): Rₜ: 3.66 min, m/z (ES+) = 604 (M+H⁺).

### Beispiel 44

### 3-({[3-(Methoxycarbonyl)cyclohexyl]amino}carbonyl)-4-{3-[4-(5,6,7,8-tetrahydronaphthalin-2-ylmethoxy)phenyl]propoxy}benzoesäure-methylester

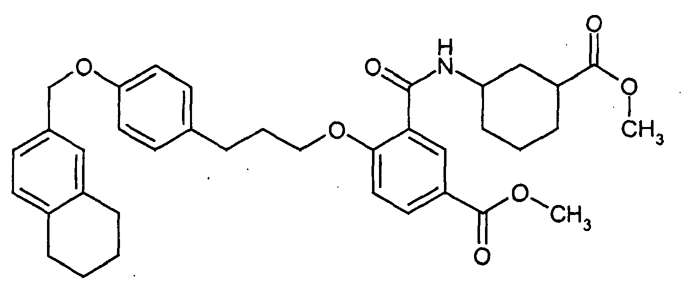

Analog dem unter Beispiel 34 beschriebenen Verfahren wird 3-({[3-(Methoxycarbonyl)cyclohexyl]amino}carbonyl)-4-{3-[4-(5,6,7,8-tetrahydronaphthalin-2-ylmethoxy)phenyl]propoxy}benzoesäure-methylester aus 4-[3-(4-Hydroxyphenyl)propoxy]-3-({[3-(methoxycarbonyl)cyclohexyl]amino}carbonyl)-benzoesäure-methylester und 6-(Chlormethyl)-1,2,3,4-tetrahydronaphthalin hergestellt.
DC (Cyclohexan/Ethylacetat 1:1): R_{f}: 0.47.
HPLC (Methode 2): Rₜ: 5.97 min.
MS (ESI): m/z = 614 (M+H⁺), 636 (M+Na⁺).

### Beispiel 45

### 3-{[(3-Carboxycyclohexyl)amino]carbonyl}-4-{3-[4-(5,6,7,8-tetrahydronaphthalin-2-ylmethoxy)phenyl]propoxy}benzoesäure

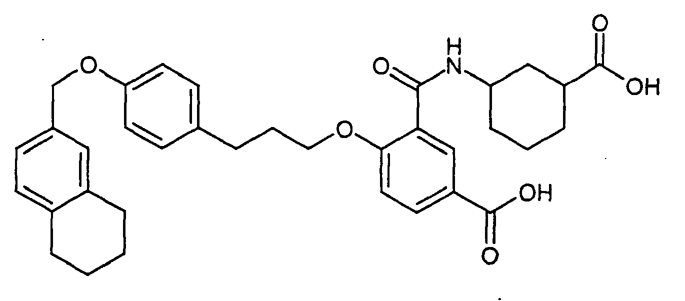

Analog dem unter Beispiel 35 beschriebenen Verfahren wird 3-{[(3-Carboxycyclohexyl)amino]carbonyl}-4-{3-[4-(5,6,7,8-tetrahydronaphthalin-2-yl-methoxy)phenyl]propoxy}benzoesäure aus 3-({[3-(Methoxycarbonyl)cyclohexyl]-amino} carbonyl)-4- {3-[4-(5,6,7,8-tetrahydronaphthalin-2-ylmethoxy)phenyl]-propoxy}benzoesäure-methylester hergestellt.
HPLC (Methode 2): Rₜ: 5.26 min.
¹H-NMR (200 MHz, DMSO-d₆, δ/ppm): 12.51 (s breit, 2H), 8.19 (d, 1H), 8.08 (d breit, 1H), 7.98 (dd, 1H), 7.22-6.88 (m, 8H), 4.97 (pseudo-d, 2H), 4.12 (pseudo-t, 3H), 2.72 (m, 7H), 2.08 (m, 2H), 1.87-1.48 (m, 12H).
LC-MS (Methode 8): Rₜ: 3.65 min, m/z (ES+) = 584 (M+H⁺).

### Beispiel 46

### 4-(3-{4-[(4-Cyclohexylbenzyl)oxy]phenyl}propoxy)-3-({[3-(methoxycarbonyl)-cyclohexyl]amino}carbonyl)benzoesäure-methylester

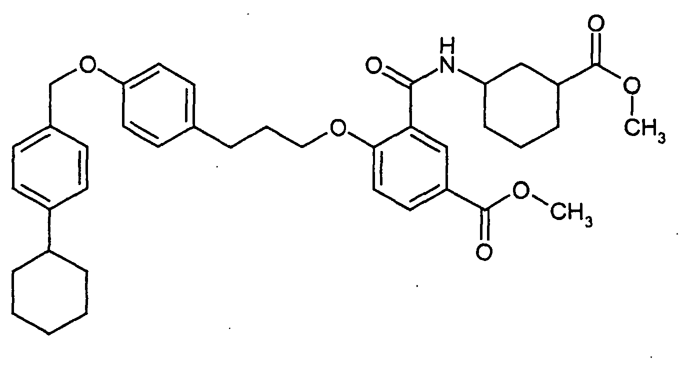

Analog dem unter Beispiel 34 beschriebenen Verfahren wird 4-(3-{4-[(4-Cyclohexylbenzyl)oxy]phenyl}propoxy)-3-({[3-(methoxycarbonyl)cyclohexyl]-amino}carbonyl)benzoesäure-methylester aus 4-[3-(4-Hydroxyphenyl)propoxy]-3-({[3-(methoxycarbonyl)cyclohexyl]amino} carbonyl)benzoesäure-methylester und 1-(Chlormethyl)-4-cyclohexylbenzol hergestellt.
DC (Cyclohexan/Ethylacetat 1:1): R_{f}: 0.47.
HPLC (Methode 2): Rₜ: 6.49 min.
LC-MS (Methode 13): Rₜ: 4.9 min, m/z (ES+) = 642 M+H⁺).

### Beispiel 47

### 3-{[(3-Carboxycyclohexyl)amino]carbonyl}-4-(3-{4-[(4-cyclohexylbenzyl)oxy]-phenyl}propoxy)benzoesäure

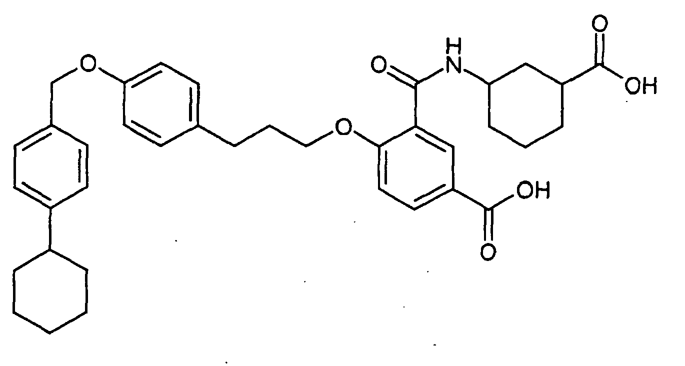

Analog dem unter Beispiel 35 beschriebenen Verfahren wird 3-{[(3-Carboxycyclohexyl)amino]carbonyl}-4-(3-{4-[(4-cyclohexylbenzyl)oxy]phenyl}-propoxy)benzoesäure aus 4-(3-{4-[(4-Cyclohexylbenzyl)oxy]phenyl}propoxy)-3-({[3-(methoxycarbonyl)cyclohexyl]amino} carbonyl)benzoesäure-methylester hergestellt.
HPLC (Methode 2): Rₜ: 5.66 min.
¹H-NMR (200 MHz, DMSO-d₆, δ/ppm): 12.51 (s breit, 2H), 8.19 (d, 1H), 8.08 (d breit, 1H), 7.97 (dd, 1H), 7.33 (d, 2H), 7.23-7.11 (m, 5H), 6.91 (d, 2H), 5.01 (s, 2H), 4.12 (pseudo-t, 3H), 2.73-2.56 (m, 3H), 2.07 (m, 2H), 1.87-1.14 (m, 19H).
LC-MS (Methode 14): Rₜ: 3.57 min, m/z (ES+) = 614 (M+H⁺).

### Beispiel 48

### 4-{3-[4-(Biphenyl-4-ylmethoxy)phenyl]propoxy}-3-({[(1R,3S)-3-(methoxycarbonyl)cyclopentyl]amino}carbonyl)benzoesäure-ethylester

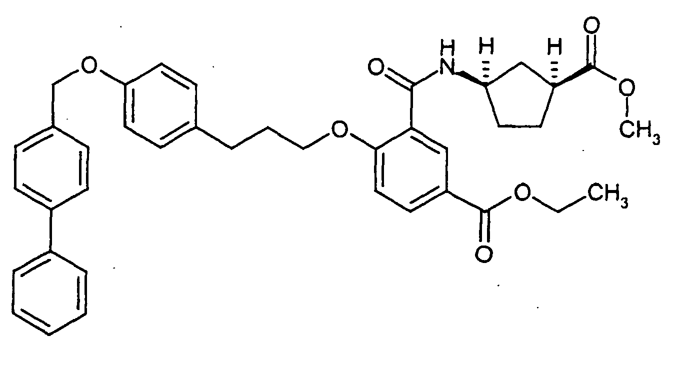

Die Herstellung erfolgt analog Beispiel 5 aus 2-{3-[4-(1,1'-Biphenyl-4-ylmethoxy)-phenyl]propoxy}-5-(ethoxycarbonyl)benzoesäure und (1S,3R)-3-Aminocyclopentancarbonsäure-methylester - Hydrochlorid.
HLPC (Methode 2): Rₜ: 6.02 min.
¹H-NMR (200 MHz, DMSO-d₆, δ/ppm): 8.23 (d breit, 1H), 8.21 (d, 1H), 8.00 (dd, 1H), 7.69-7.65 (m, 4H), 7.53-7.43 (m, 4H), 7.39-7.33 (m, 1H), 7.22 (d, 1.H), 7.14 (d, 2H), 6.94 (d, 2H), 5.11 (s, 2H), 4.30 (quart., 2H), 4.14 (t, 2H), 3.55 (s, 3H), 2.88 (quint., 1H), 2.71 (pseudo-t, 2H), 2.32-2.22 (m, 1H), 2.08 (quint., 2H), 1.99-1.82 (m, 3H), 1.78-1.57 (m, 2H), 1.31 (t, 3H).
MS (ESI+): m/z = 636 (M+H⁺).

### Beispiel 49

### 4-{3-[4-(Biphenyl-4-ylmethoxy)phenyl]propoxy}-3-({[(1R,3S)-3-carboxycyclopentyl]amino} carbonyl)benzoesäure

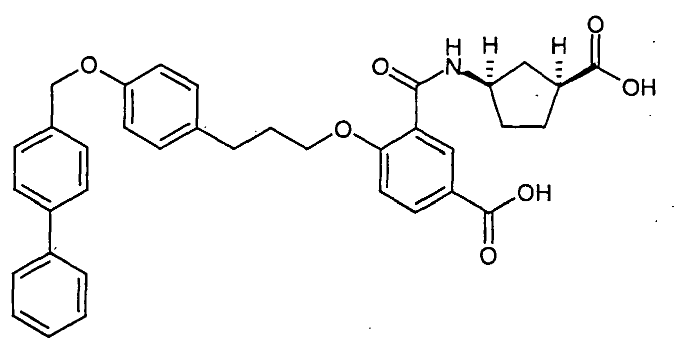

Die Darstellung erfolgt analog Beispiel 23 aus 4-{3-[4-(Biphenyl-4-ylmethoxy)phenyl]propoxyl}-3-({[(1R,3S)-3-(methoxycarbonyl)cyclopentyl]amino}-carbonyl)benzoesäure-ethylester.
¹H-NMR (300 MHz, DMSO-d₆, δ/ppm): 11 (s breit), 8.23 (d, 1H), 8.19 (d, 1H), 7.98 (dd, 1H), 7.70-7.65 (m, 4H), 7.53-7.33 (m, 5H), 7.18-7.13 (m, 3H), 6.94 (d, 2H), 5.11 (s, 2H), 4.30 (m, 1H), 4.22 (t, 2H), 2.78 (quint., 1H), 2.70 (t, 2H), 2.23 (m, 1H), 2.08 (quint., 2H), 1.99-1.82 (m, 3H), 1.73 (m, 1H), 1.59 (m, 1H).
LC-MS (Methode 8): Rₜ: 3.56 min, m/z (ES+) = 594 (M+H⁺).

### Beispiel 50

### 3-({[3-(Methoxycarbonyl)cyclohexyl]amino}carbonyl)-4-[3-(4-{[4-(trifluormethoxy)benzyl]oxy} phenyl)propoxy]benzoesäure-methylester

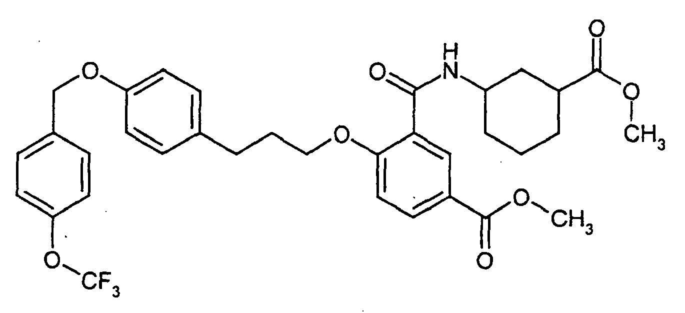

Analog dem unter Beispiel 34 beschriebenen Verfahren wird 3-({[3-(Methoxycarbonyl)cyclohexyl]amino}carbonyl)-4-[3-(4-{[4-(trifluormethoxy)-benzyl]oxy}phenyl)propoxy]benzoesäure-methylester aus 4-[3-(4-Hydroxyphenyl)-propoxy]-3-({[3-(methoxycarbonyl)cyclohexyl]amino}carbonyl)benzoesäure-methylester und 1-(Chlormethyl)-4-trifluormethoxybenzol hergestellt.
DC (Cyclohexan/Ethylacetat 1:1): R_{f}: 0.36.
HPLC (Methode 2): Rₜ: 6.62 min.
MS (ESI): m/z = 644 (M+H⁺).

### Beispiel 51

### 3-{[(3-Carboxycyclohexyl)amino]carbonyl}-4-[3-(4-{[4-(trifluormethoxy)benzyl]-oxy}phenyl)propoxy]benzoesäure

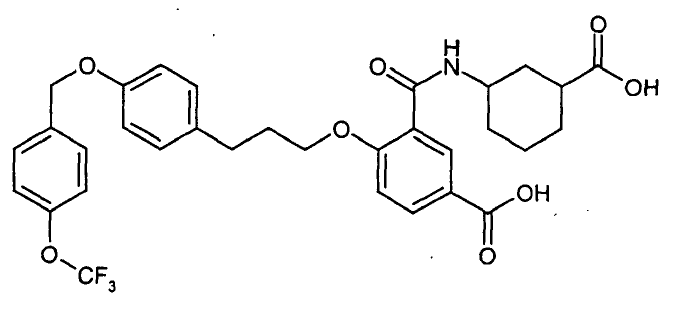

Analog dem unter Beispiel 35 beschriebenen Verfahren wird 3-{[(3-Carboxycyclohexyl)amino]carbonyl}-4-[3-(4-{[4-(trifluormethoxy)benzyl]oxy}phenyl)-propoxy]benzoesäure aus 3-({[3-(Methoxycarbonyl)cyclohexyl]amino}carbonyl)-4-[3-(4-{[4-(trifluormethoxy)benzyl)oxy}phenyl)propoxy]benzoesäure-methylester hergestellt.
HPLC (Methode 2): Rₜ: 5.04 min.
¹H-NMR (200 MHz, DMSO-d₆, δ/ppm): 12.37 (s breit, 2H), 8.19 (d, 1H), 8.08 (d breit, 1H), 7.98 (dd, 1H), 7.57 (d, 2H), 7.38 (d, 2H), 7.18 (d, 2H), 7.17 (d, 2H), 6.93 (d, 2H), 5.01 (s, 2H), 4.12 (pseudo-t, 3H), 2.74-2.56 (m, 3H), 2.08 (m, 2H), 1.87-1.43 (m, 8H).
MS (ESI): m/z = 614 (M-H⁺).

### Beispiel 52

### 4-(3-{4-[3-(4-Chlorphenyl)propoxy]phenyl}propoxy)-3-({[3-(methoxycarbonyl)-cyclohexyl]amino}carbonyl)benzoesäure-methylester

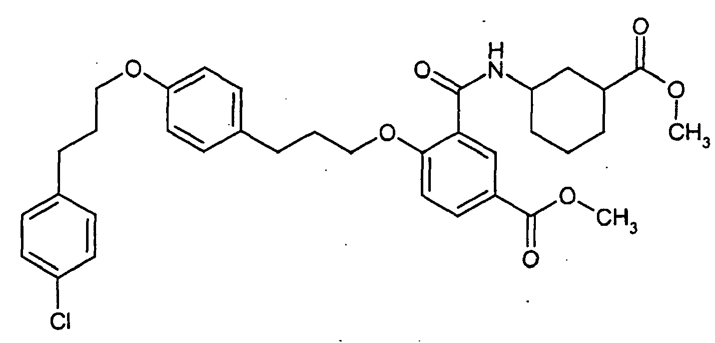

Analog dem unter Beispiel 34 beschriebenen Verfahren wird 4-(3-{4-[3-(4-Chlorphenyl)propoxy]pheny]}propoxy)-3-({[3-(methoxycarbonyl)cyclohexyl]-amino}carbonyl)benzoesäure-methylester aus 4-[3-(4-Hydroxyphenyl)propoxy]-3-({[3-(methoxycarbonyl)cyclohexyl]amino}carbonyl)benzoesäure-methylester und 1-(3-Brompropyl)-4-chlorbenzol hergestellt.
DC (Cyclohexan/Ethylacetat 1:1): R_{f}: 0.44.
HPLC (Methode 2): Rₜ: 5.90 min.
LC-MS (Methode 13): Rₜ: 4.5 min, m/z (ES+) = 622 und 623 (M+H⁺).

### Beispiel 53

### 3-{[(3-Carboxycyclohexyl)amino]carbonyl}-4-(3-{4-[3-(4-chlorphenyl)propoxy]-phenyl}propoxy)benzoesäure

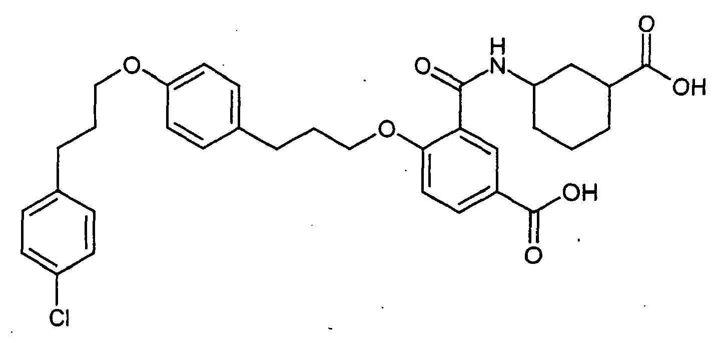

Analog dem unter Beispiel 35 beschriebenen Verfahren wird 3-{[(3-Carboxycyclohexyl)amino]carbonyl}-4-(3-{4-[3-(4-chlorphenyl)propoxy]phenyl}propoxy)-benzoesäure aus 4-(3-{4-[3-(4-Chlorphenyl)propoxy]phenyl}propoxy)-3-({[3-(methoxycarbonyl)cyclohexyl]amino}carbonyl)benzoesäure-methylester hergestellt.
HPLC (Methode 2): Rₜ: 5.21 min.
LC-MS (Methode 14): Rₜ: 3.37 min, m/z (ES+) = 594 und 596 (M+H⁺).

### Beispiel 54

### 4-{3-[4-(3-Cyclohexylpropoxy)phenyl]propoxy}-3-{[3-(2-ethoxy-2-oxoethyl)-azetidin-1-yl]carbonyl}benzoesäure-methylester

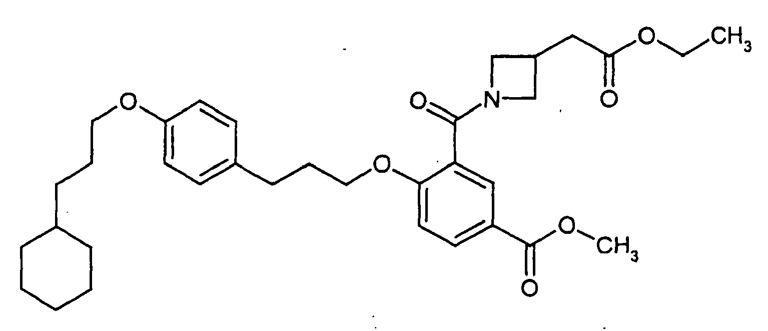

Die Herstellung erfolgt analog dem in Beispiel 22 beschriebenen Verfahren aus 3-{[3-(2-Ethoxy-2-oxoethyl)azetidin-1-yl]carbonyl}-4-hydroxybenzoesäuremethylester und 1-(3-Brompropyl)-4-(3-cyclohexylpropoxy)benzol.
HPLC (Methode 5): Rₜ: 5.83 min.
¹H-NMR (300 MHz, DMSO-d₆, δ/ppm): 7.97 (dd, 1H), 7.83 (d, 1H), 7.20-7.09 (m, 3H), 6.83 (d, 2H), 4.19-3.94 (m, 6H), 3.94-3.86 (m, 2H), 3.82 (s, 3H), 3.75-3.67 (m, 1H), 3.63-3.54 (m, 1H), 2.95-2.82 (m, 1H), 2.74-2.62 (m, 4H), 2.05-1.96 (m, 2H), 1.75-1.55 (m, 7H), 1.33-1.08 (m, 9H), 0.96-0.78 (m, 2H).
MS (ESI+): m/z = 580 (M+H⁺).

### Beispiel 55

### 3-{[3-(Carboxymethyl)azetidin-1-yl]carbonyl-4-{3-[4-(3-cyclohexylpropoxy)-phenyl]-propoxy}benzoesäure

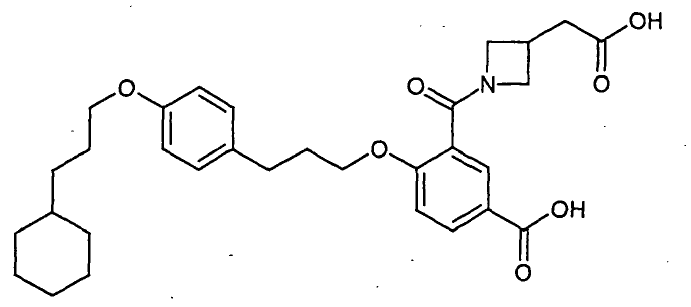

Die Herstellung erfolgt analog Beispiel 11 aus 4-{3-[4-(3-Cyclohexylpropoxy)-phenyl]-propoxy}-3-{[3-(2-ethoxy-2-oxoethyl)azetidin-1-yl]carbonyl}benzoesäure-methylester.
HPLC (Methode 5): Rₜ: 5.05 min.
¹H-NMR (300 MHz, DMSO-d₆, δ/ppm): 12.48 (s, breit, 2H), 7.94 (dd, 1H), 7.81 (d, 1H), 7.13 (pseudo-d, 3H), 6.82 (d, 2H), 4.19-4.04 (m, 3H), 3.98 (t, 1H), 3.89 (t, 2H), 3.70 (q, 1H), 3.57 (q, 1H), 2.93-2.77 (m, 1H), 2.75-2.54 (m, 4H), 2.09-1.95 (m, 2H), 1.76-1.55 (m, 7H), 1.35-1.04 (m, 6H), 0.97-0.79 (m, 2H).
MS (ESI+): m/z = 538 (M+H⁺).

### Beispiel 56

### 3-({[3-(Methoxycarbonyl)cyclohexyl]amino}carbonyl)-4-{3-[4-(5-phenoxypentyl)-phenyl]propoxy}benzoesäure-methylester

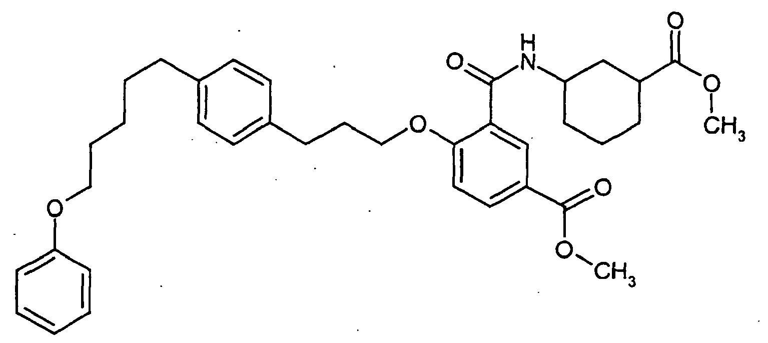

Die Herstellung erfolgt analog Beispiel 22 aus dem (+)-B-Enantiomer von 4-Hydroxy-3-({[3(methoxycarbonyl)cyclohexyl]amino}carbonyl)benzoesäure-methylester (siehe Beispiel IX, Methode 2) und 1-(3-Iodpropyl)-4-(5-phenoxypentyl)benzol.
HPLC (Methode 5): Rₜ: 5.57 min.
¹H-NMR (300 MHz, CDCl₃, δ/ppm): 8.87 (d, 1H), 8.09 (dd, 1H), 7.86 (d, 1H), 7.30-7.22 (m, 2H), 7.14-7.06 (m, 4H), 6.98-6.84 (m, 4H), 4.43-4.34 (m, 1H), 4.19 (t, 2H), 3.95 (t, 2H), 3.89 (s, 3H), 3.65 (s, 3H), 2.81 (t, 2H), 2.65-2.54 (m; 3H), 2.30-2.19 (m, 2H), 2.01-1.94 (m, 2H), 1.87-1.62 (m, 9H), 1.56-1.47 (m, 3H).
LC-MS (Methode 9): Rₜ: 3.19 min, m/z (EI+) = 615.

### Beispiel 57

### 3-{[(3-Carboxycyclohexyl)amino]carbonyl}-4-{3-[4-(5-phenoxypentyl)phenyl]-propoxy}-benzoesäure

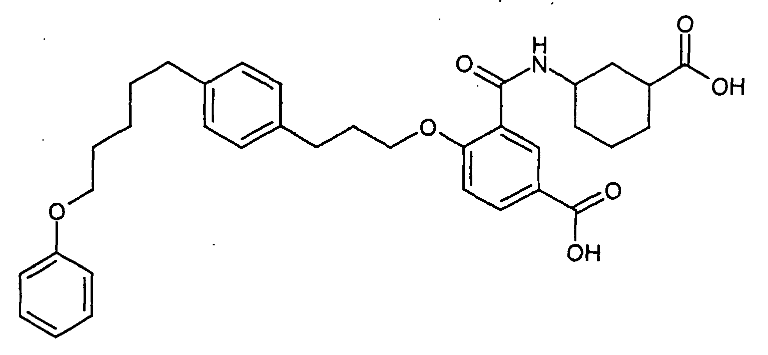

Die Herstellung erfolgt analog Beispiel 11 aus 3-({[3-(Methoxycarbonyl)-cyclohexyl]amino}-carbonyl)-4-{3-[4-(5-phenoxypentyl)phenyl]propoxy}-benzoesäure-methylester.
HPLC (Methode 5): Rₜ: 4.94 min.
¹H-NMR (300 MHz, CDCl₃, δ/ppm): 8.92 (d, 1H), 8.12 (dd, 1H), 7.86 (d, 1H), 7.29-7.22 (m, 2H), 7.12-7.08 (m, 4H), 6.98-6.85 (m, 4H), 4.48-4.34 (m, 1H), 4.18 (t, 2H), 3.94 (t, 2H), 2.82 (t, 2H), 2.72-2.56 (m, 3H), 2.30-1.23 (m, 18H).
LC-MS (Methode 9): Rₜ: 2.75 min, m/z (EI+) = 587.

### Beispiel 58

### 3-{[3-(2-Ethoxy-2-oxoethyl)azetidin-1-yl]carbonyl}-4-{3-[4-(4-phenoxybutoxy)-phenyl]-propoxy}benzoesäure-ethylester

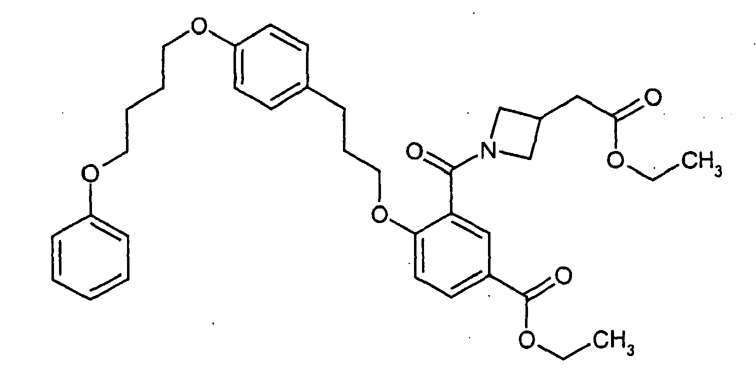

Die Herstellung erfolgt analog Beispiel IX, Methode 3, aus 3-(2-Ethoxy-2-oxoethyl)-azetidiniumchlorid und 5-(Ethoxycarbonyl)-2-{3-[4-(4-phenoxybutoxy)phenyl]-propoxy}benzoesäure.
HPLC (Methode 5): Rₜ: 5.19 min.
¹H-NMR (300 MHz, CDCl₃, δ/ppm): 8.07-8.00 (m, 2H), 7.31-7.23 (m, 2H), 7.10 (d, 2H), 6.96-6.81 (m, 6H), 4.40-4.30 (m, 4H), 4.17-3.98 (m, 8H), 3.90-3.83 (m, 1H), 3.70-3.62 (m, 1H), 3.05-2.93 (m, 1H), 2.79-2.70 (m, 2H), 2.70-2.55 (m, 2H), 2.17-2.07 (m, 2H), 2.00-1.93 (m, 4H), 1.37 (t, 3H), 1.22 (t, 3H).
MS (ESI+): m/z = 618 (M+H⁺).

### Beispiel 59

### 3-{[3-(Carboxymethyl)azetidin-1-yl]carbonyl}-4-{3-[4-(4-phenoxybutoxy)phenyl]-propoxy}benzoesäure

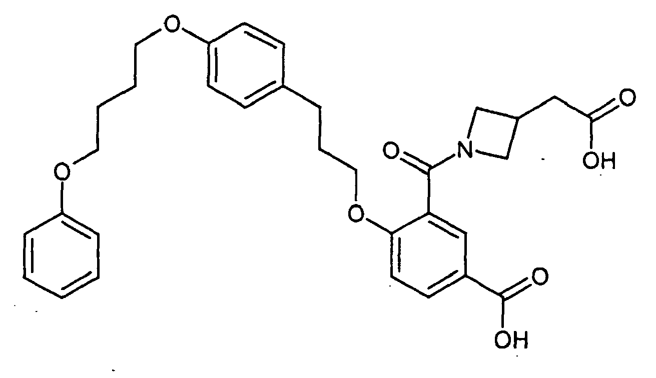

Die Herstellung erfolgt analog Beispiel 11 aus 3-{[3-(2-Ethoxy-2-oxoethyl)azetidin-1-yl]carbonyl}-4-{3-[4-(4-phenoxybutoxy)phenyl]-propoxy}benzoesäureethylester.
HPLC (Methode 5): Rₜ: 4.39 min.
¹H-NMR (300 MHz, DMSO-d₆, δ/ppm): 12.48 (s, breit, 2H), 7.94 (dd, 1H), 7.81 (d, 1H), 7.31-7.24 (m, 2H), 7.14 (pseudo-d, 3H), 6.95-6.82 (m, 5H), 4.18-3.95 (m, 8H), 3.69 (dd, 1H), 3.62-3.56 (m, 1H), 2.92-2.80 (m, 1H), 2.69 (t, 2H), 2.61-2.55 (m, 2H), 2.08-1.96 (m, 2H), 1.89-1.81 (m, 4H).
LC-MS (Methode 6): Rₜ: 2.32 min, m/z (EI+) = 561.

### Beispiel 60

### 4-{2-[4-(Biphenyl-4-ylmethoxy)phenoxy]ethoxy}-3-({[3-(methoxycarbonyl)-cyclohexyl]-amino}carbonyl)benzoesäure-methylester

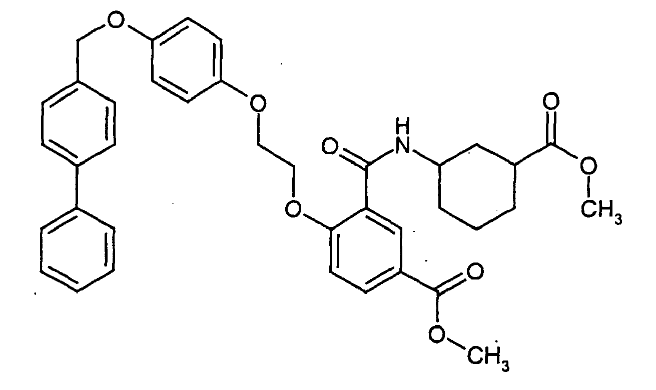

Die Herstellung erfolgt analog Beispiel 22 aus dem (+)-B-Enantiomer von 4-Hydroxy-3-({[3(methoxycarbonyl)cyclohexyl]amino}carbonyl)benzoesäure-methylester (siehe Beispiel IX, Methode 2) und 4-{[4-(2-Bromethoxy)phenoxy]-methyl}biphenyl.
HPLC (Methode 5): Rₜ: 5.22 min.
¹H-NMR (200 MHz, CDCl₃, δ/ppm): 8.90 (d, 1H), 8.15 (dd, 1H), 7.91 (d, 1H), 7.65-7.34 (m, 8H), 7.08-6.82 (m, 6H), 5.06 (s, 2H), 4.53-4.45 (m, 2H), 4.42-4.32 (m, 3H), 3.91 (s, 3H), 3.61 (s, 3H), 2.52-2.38 (m, 1H), 1.84 (t, 2H), 1.76-1.24 (m, 6H).
MS (ESI+): m/z = 638 (M+H⁺).

### Beispiel 61

### 4-{2-[4-(Biphenyl-4-ylmethoxy)phenoxy]ethoxy}-3-{[(3-carboxycyclohexyl)amino]-carbonyl}benzoesäure

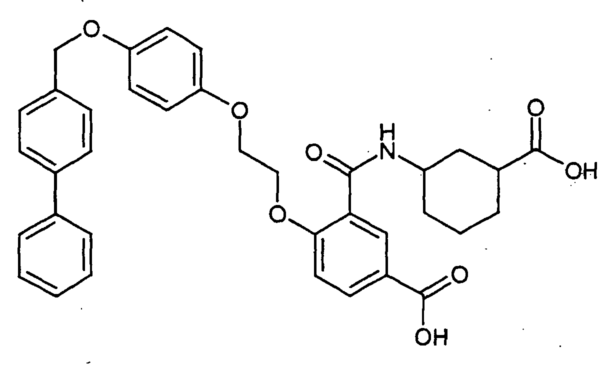

Die Herstellung erfolgt analog Beispiel 11 aus 4-{2-[4-(Biphenyl-4-ylmethoxy)-phenoxy]-ethoxy}-3-({[3-(methoxycarbonyl)cyclohexyl]-amino}carbonyl)-benzoesäure-methylester.
HPLC (Methode 5): Rₜ: 4.66 min.
¹H-NMR (300 MHz, DMSO-d₆, δ/ppm): 8.39 (d, 1H), 8.06-7.99 (m, 2H), 7.69-7.65 (m, 4H), 7.54-7-44 (m, 4H), 7.39-7.27 (m, 2H), 7.00-6.90 (m, 4H), 5.10 (s, 2H), 4.55-4.49 (m, 2H), 4.37-4.33 (m, 2H), 4.14-4.04 (m, 1H), 2.47-2.38 (m, 1H), 1.80-1.22 (m, 8H).
LC-MS (Methode 9): Rₜ: 2.53 min, m/z (EI+) = 609.

### Beispiel 62

### 4-{3-[4-(2-Biphenyl-4-ylethyl)phenyl]propoxy}-3-({[3-(methoxycarbonyl)-cyclohexyl]-amino}carbonyl)benzoesäure-methylester

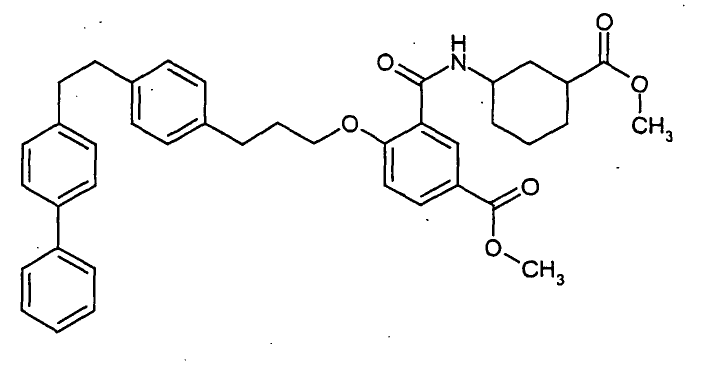

Die Herstellung erfolgt analog Beispiel 22 aus dem (+)-B-Enantiomer von 4-Hydroxy-3-({[3(methoxycarbonyl)cyclohexyl]amino}carbonyl)benzoesäure-methylester (siehe Beispiel IX, Methode 2) und 4-{2-[4-(3-Iodpropyl)phenyl]-ethyl}biphenyl.
HPLC (Methode 5): Rₜ: 5.64 min.
¹H-NMR (300 MHz, CDCl₃, δ/ppm): 8.87 (d, 1H), 8.09 (dd, 1H), 7.85 (d, 1H), 7.61-7.55 (m, 2H), 7.54-7.48 (m, 2H), 7.46-7.39 (m, 3H), 7.36-7.22 (m, 2H), 7.18-7.08 (m, 4H), 6.96 (d, 1H), 4.44-4.30 (m, 1H), 4.19 (t, 2H), 3.89 (s, 3H), 3.65 (s, 3H), 2.83 (t, 2H), 2.69-2.55 (m, 1H), 2.31-2.19 (m, 2H), 2.02-1.95 (m, 2H), 1.81-1.62 (m, 5H), 1.40-1.22 (m, 5H).
LC-MS (Methode 9): Rₜ: 3.24 min, m/z (EI+) = 633.

### Beispiel 63

### 4-{3-[4-(2-Biphenyl-4-ylethyl)phenyl]propoxy}-3-{[(3-carboxycyclohexyl)amino]-carbonyl}benzoesäure

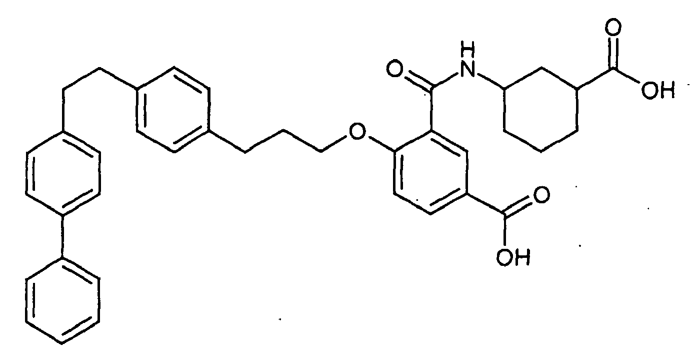

Die Herstellung erfolgt analog Beispiel 11 aus 4-{3-[4-(2-Biphenyl-4-ylethyl)-phenyl]-propoxy}-3-({[3-(methoxycarbonyl)cyclohexyl]-amino}carbonyl)-benzoesäure-methylester.
HPLC (Methode 5): Rₜ: 5.03 min.
LC-MS (Methode 9): Rₜ: 2.82 min, m/z (EI+) = 605.

### Beispiel 64

### 3-({[3-(Methoxycarbonyl)cyclohexyl]amino}carbonyl)-4-{2-[4-(4-phenoxybutoxy)-phenoxy]ethoxy}benzoesäure-methylester

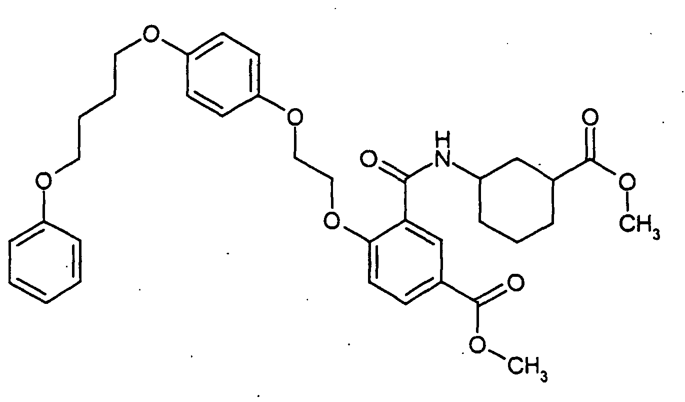

Die Herstellung erfolgt analog Beispiel 22 aus dem (+)-B-Enantiomer von 4-Hydroxy-3-({[3(methoxycarbonyl)cyclohexyl]amino}carbonyl)benzoesäure-methylester (siehe Beispiel IX, Methode 2) und 1-(2-Bromethoxy)-4-(4-phenoxybutoxy)benzol.
HPLC (Methode 5): Rₜ: 5.11 min.
¹H-NMR (200 MHz, CDCl₃, δ/ppm): 8.90 (d, 1H), 8.15 (dd, 1H), 7.92 (d, 1H), 7.33-7.22 (m, 2H), 7.07-6.81 (m, 8H), 4.55-4.46 (m, 2H), 4.42-4.32 (m, 2H), 4.07-3.95 (m, 4H), 3.90 (s, 3H), 3.62 (s, 3H), 2.53-2.36 (m, 1H), 2.04-1.78 (m, 6H), 1.70-1.25 (m, 7H).
MS (ESI+): m/z = 620 (M+H⁺).

### Beispiel 65

### 3-{[(3-Carboxycyclohexyl)amino]carbonyl}-4-{2-[4-(4-phenoxybutoxy)phenoxy]-ethoxy}benzoesäure

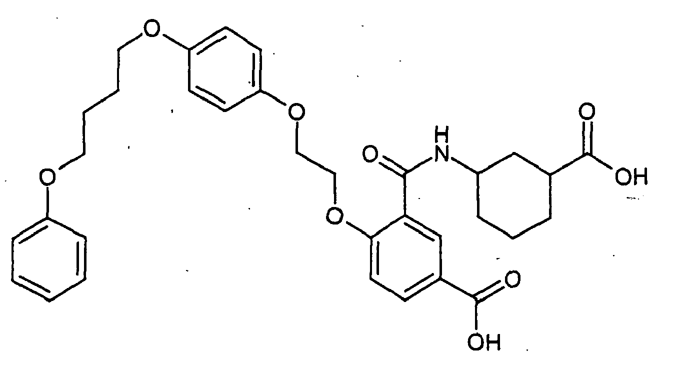

Die Herstellung erfolgt analog Beispiel 11 aus 3-({[3-(Methoxycarbonyl)-cyclohexyl]amino}-carbonyl)-4-{2-[4-(4-phenoxybutoxy)-phenoxy]ethoxy}-benzoesäure-methylester.
HPLC (Methode 5): Rₜ: 4.52 min.
¹H-NMR (300 MHz, DMSO-d₆, δ/ppm): 12.45 (s, breit, 2H); 7.39 (d, 1H), 8.07-7.98 (m, 2H), 7.34-7.22 (m, 3H), 6.95-6.83 (m, 7H), 4.56-4.49 (m, 2H), 4.37-4.32 (m, 2H), 4.15-3.93 (m, 5H), 2.48-2.39 (m, 1H), 1.89-1.22 (m, 12H).
LC-MS (Methode 9): Rₜ: 2.42 min, m/z (EI+) = 591.

### Beispiel 66

### 3-({[3-(Methoxycarbonyl)cyclohexyl]amino}carbonyl)-4-(2-{4-[(1Z)-5-phenoxypent-1-en-1-yl]phenoxy}ethoxy)benzoesäure-methylester

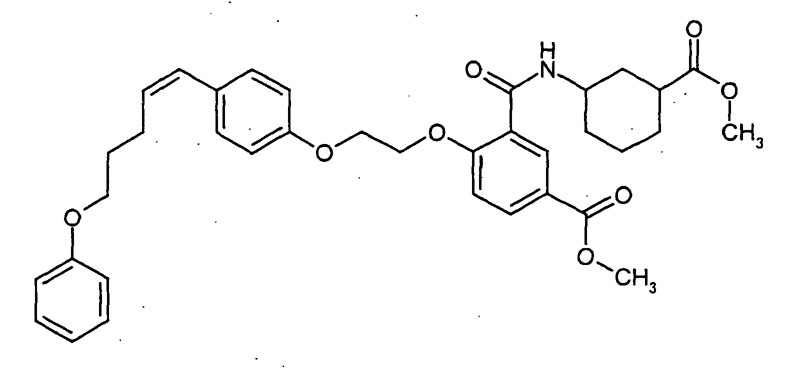

Die Herstellung erfolgt analog Beispiel 22 aus dem (+)-B-Enantiomer von 4-Hydroxy-3-({[3(methoxycarbonyl)cyclohexyl]amino}carbonyl)benzoesäure-methylester (siehe Beispiel IX, Methode 2) und 1-(2-Iodethoxy)-4-[(1Z)-5-phenoxypent-1-en-1-yl]benzol.
HPLC (Methode 5): Rₜ: 5.33 min.
¹H-NMR (300 MHz, CDCl₃, δ/ppm): 8.90 (d, 1H), 8.14 (dd, 1H), 7.85 (d, 1H), 7.29-7.21 (m, 4H), 7.04 (d, 1H), 6.94-6.83 (m, 5H), 6.39 (d, 1H), 5.64 (dt, 1H), 4.56-4.50 (m, 2H), 4.44-4.30 (m, 2H), 4.39-4.30 (m, 1H), 3.99 (t, 2H), 3.91 (s, 3H), 3.61 (s, 3H), 2.54-2.32 (m, 3H), 2.10-1.7 (m, 5H), 1.48-1.23 (m, 5H).
LC-MS (Methode 14): Rₜ: 3.63 min, m/z (EI+) = 615.

### Beispiel 67

### 3-{[(3-Carboxycyclohexyl)amino]carbonyl}-4-(2-{4-[(1Z)-5-phenoxypent-1-en-1-yl]-phenoxy}ethoxy)benzoesäure

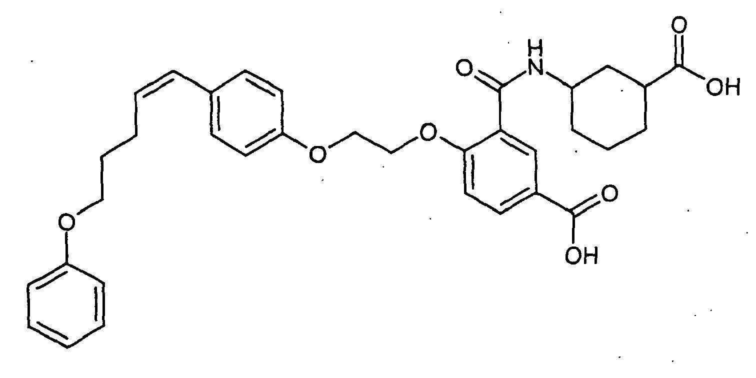

Die Herstellung erfolgt analog Beispiel 11 aus 3-({[3-(Methoxycarbonyl)-cyclohexyl]amino}-carbonyl)-4-(2-{4-[(1Z)-5-phenoxypent-1-en-1-yl]phenoxy}-ethoxy)benzoesäure-methylester.
HPLC (Methode 5): Rₜ: 4.67 min.
¹H-NMR (300 MHz, CDCl₃, δ/ppm): 8.88 (s, 1H), 8.10 (d, 1H), 7.88 (d, 1H), 7.30-7.16 (m, 3H), 7.01-6.81 (m, 7H), 6.36 (d, 1H), 5.65-5.54 (m, 1H), 4.52-4.28 (m, 5H), 4.01-3.91 (m, 2H), 2.54-2.42 (m, 2H), 2.03-1.18 (m, 11H).
LC-MS (Methode 9): Rₜ: 2.54 min, m/z (EI+) = 587.

### Beispiel 68

### 1-[2-{2-[4-(Biphenyl-4-ylmethoxy)phenoxy]ethoxy}-5-(methoxycarbonyl)benzoyl]-piperidin-4-carbonsäure-methylester

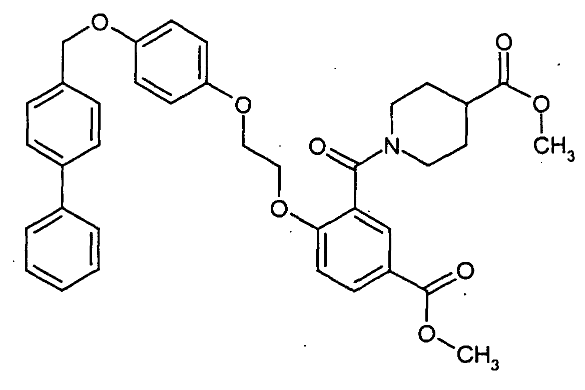

Die Herstellung erfolgt analog Beispiel 22 aus 1-[2-Hydroxy-5-(methoxycarbonyl)-benzoyl]piperidin-4-carbonsäuremethylester und 4-{[4-(2-Bromethoxy)phenoxy]-methyl}biphenyl.
HPLC (Methode 5): Rₜ: 5.02 min.
¹H-NMR (300 MHz, CDCl₃, δ/ppm): 8.05 (d, 1H), 7.95 (dd, 1H), 7.64-7.55 (m, 4H), 7.52-7.40 (m, 4H), 7.38-7.33 (m, 1H), 7.03-6.90 (m, 3H), 6.88-6.81 (m, 2H), 5.06 (s, 2H), 4.54-4.18 (m, 5H), 3.89 (s, 3H), 3.68 und 3.57 (s, zusammen 3H), 3.53-3.37 (m, 1H), 3.15-2.88 (m, 2H), 2.56-2.39 (m, 1H), 2.01-1.88 (m, 1H), 1.87-1.60 (m, 3H).
LC-MS (Methode 9): Rₜ: 2.79 min, m/z (EI+) = 623.

### Beispiel 69

### 1-(2-{2-[4-(Biphenyl-4-ylmethoxy)phenoxy]ethoxy}-5-carboxybenzoyl)piperidin-4-carbonsäure

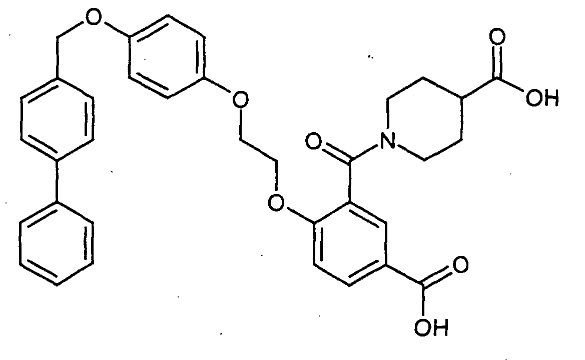

Die Herstellung erfolgt analog Beispiel 11 aus 1-[2-{2-[4-(Biphenyl-4-ylmethoxy)-phenoxy]-ethoxy}-5-(methoxycarbonyl)benzoyl]-piperidin-4-carbonsäuremethylester.
HPLC (Methode 5): Rₜ: 4.42 min.
¹H-NMR (300 MHz, DMSO-d₆, δ/ppm): 12.52 (s, breit, 2H), 7.96 (dd, 1H); 7.75-7.64 (m, 5H), 7.56-7.43 (m, 4H), 7.40-7.33 (m, 1H), 7.25 (d, 1H), 7.01-6.86 (m, 4H), 5.08 (d; 2H); 4.48-4.38 (m, 2H), 4.34-4.21 (m, 3H), 3.15-2.75 (m, 2H), 2.48-2.37(m, 1H), 1.91-1..26 (m, 4H).

### Beispiel 70

### 3-({[3-(Methoxycarbonyl)cyclohexyl]amino}carbonyl)-4-({3-[4-(4-phenoxybutoxy)-phenyl]prop-2-in-1-yl}oxy)benzoesäure-methylester

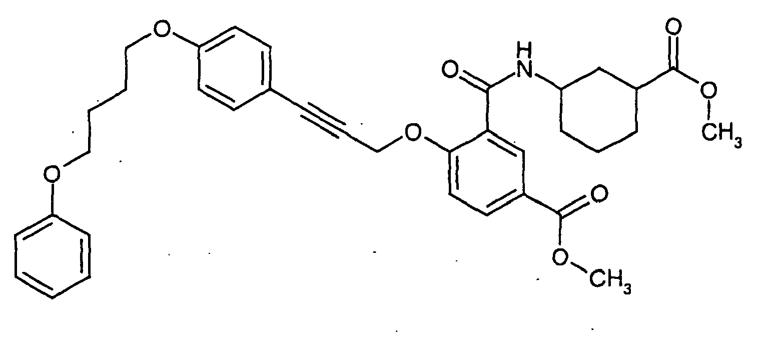

Die Herstellung erfolgt analog Beispiel 22 aus dem (+)-B-Enantiomer von 4-Hydroxy-3-({[3(methoxycarbonyl)cyclohexyl]amino}carbonyl)benzoesäure-methylester (siehe Beispiel IX, Methode 2) und 1-(3-Bromprop-1-in-1-yl)-4-(4-phenoxybutoxy)benzol.
HPLC (Methode 5): Rₜ: 5.23 min.
¹H-NMR (400 MHz, CDCl₃, δ/ppm): 8.9 (d, 1H), 8.15 (dd, 1H), 7.98 (d, 1H), 7.38 (d, 2H), 7.31-7.25 (m, 2H), 7.10 (d, 1H), 6.94 (t, 1H), 6.89 (d, 2H), 6.84 (d, 2H), 5.09 (s, 2H), 4.47-4.40 (m, 1H), 4.08-4.01. (m, 4H), 3.91 (s, 3H), 3.62 (s, 3H), 2.64-2.57 (m, 1H), 2.11-1.95 (m, 5H), 1.88-1.80 (m, 1H), 1.72-1.49 (m, 6H).
MS (CI+): m/z = 614 (M+H⁺).

### Beispiel 71

### 3-{[(3-Carboxycyclohexyl)amino]carbonyl}-4-({3-[4-(4-phenoxybutoxy)phenyl]-prop-2-in-1-yl}oxy)benzoesäure

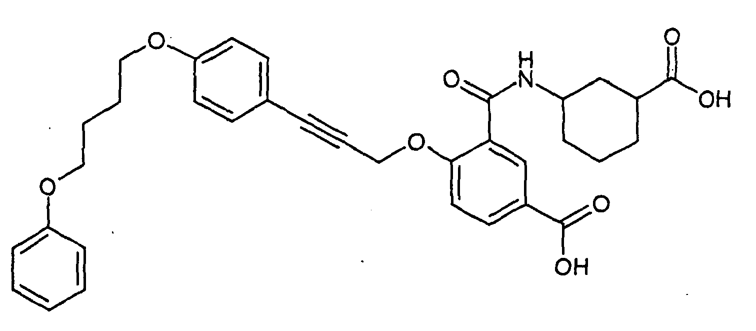

Die Herstellung erfolgt analog Beispiel 11 aus 3-({[3-(Methoxycarbonyl)-cyclohexyl]amino}-carbonyl)-4-({3-[4-(4-phenoxybutoxy)-phenyl]prop-2-in-1-yl} oxy)benzoesäure-methylester.
¹H-NMR (400 MHz, DMSO-d₆, δ/ppm): 12.55 (s, breit, 2H), 8.23 (d, 1H), 8.13 (d, 1H), 8.04 (dd, 1H), 7.40 (d, 2H), 7.34 (d, 1H), 7.26 (dd, 2H), 6.98-6.88 (m, 5H), 5.22 (s, 2H), 4.18-3.97 (m, 5H), 2.62-2.55 (m, 1H), 1.92-1.43 (m, 12H).
LC-MS (Methode 6): Rₜ: 2.59 min, m/z (EI+) = 585.

### Beispiel 72

### 3-({[3-(Methoxycarbonyl)cyclohexyl]amino}carbonyl)-4-(3-{4-[(4-phenoxybut-2-in-1-yl)oxy]phenyl}propoxy)benzoesäure-methylester

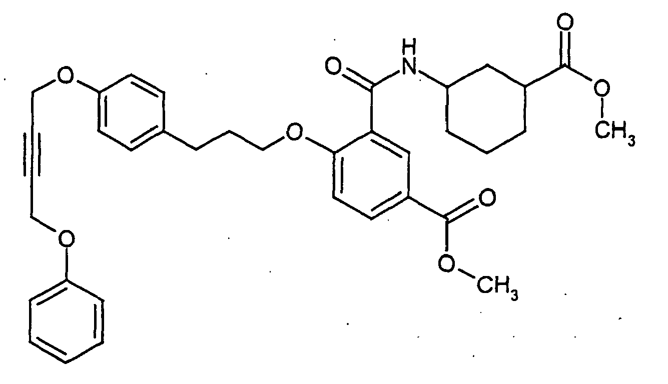

Die Herstellung erfolgt analog Beispiel 22 aus dem (+)-B-Enantiomer von 4-Hydroxy-3-({[3(methoxycarbonyl)cyclohexyl]amino}carbonyl)benzoesäure-methylester (siehe Beispiel IX, Methode 2) und 1-(3-Brompropyl)-4-[(4-phenoxybut-2-in-1-yl)oxy]benzol.
HPLC (Methode 5): Rₜ: 5.00 min.
¹H-NMR (200 MHz, CDCl₃, δ/ppm): 8.87 (d, 1H), 8.09 (dd, 1H), 7.86 (d, 1H), 7.35-7.21 (m, 1H), 7.10 (d, 2H), 7.04-6.81 (m, 7H), 4.71 (s, 4H), 4.46-4.32 (m, 1H), 4.18 (t, 2H), 3.89 (s, 3H), 3.65 (s, 3H), 2.70 (t, 2H), 2.72-2.52 (m, 2H), 2.32-2.14 (m, 2H), 1.98 (t, 2H), 1.88-1.45 (m, 5H).
LC-MS (Methode 6): Rₜ: 3.14 min, m/z (EI+) = 613.

### Beispiel 73

### 3-{[(3-Carboxycyclohexyl)amino]carbonyl}-4-(3-{4-[(4-phenoxybut-2-in-1-yl)oxy]-phenyl}propoxy)benzoesäure

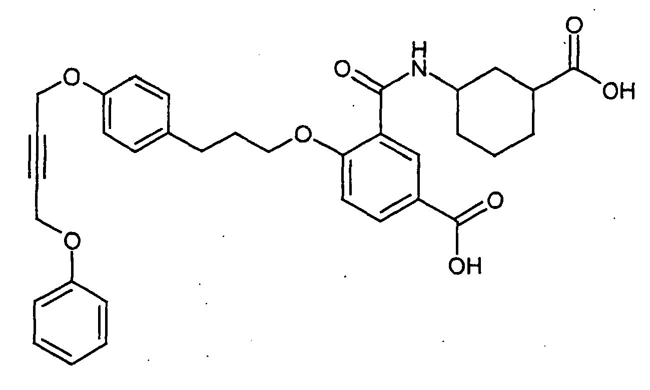

Die Herstellung erfolgt analog Beispiel 11 aus 3-({[3-(Methoxycarbonyl)-cyclohexyl]amino}carbonyl)-4-(3-{4-[(4-phenoxybut-2-in-1-yl)oxy]phenyl}-propoxy)benzoesäure-methylester.
HPLC (Methode 5): Rₜ: 4.39 min.
¹H-NMR (400 MHz, CDCl₃, δ/ppm): 8.68 (d, 1H), 8.06 (dd, 1H), 7.94 (d, 1H), 7.27 (t, 2H), 7.10 (d, 2H), 7.03 (d, 1H), 6.98-6.82 (m, 5H), 4.74 (d, 4H), 4.38-4.29 (m, 1H), 4.23-4.15 (m, 2H), 2.79 (t, 2H), 2.27-2.18 (m, 2H), 2.03-1.85 (m, 2H), 1.84-1.52 (m, 7H).
LC-MS (Methode 7): Rₜ: 2.39 min, m/z (EI+) = 585.

### Beispiel 74

### 3-({[3-(Methoxycarbonyl)cyclohexyl]amino}carbonyl)-4-(3-{4-[(1Z)-5-phenoxypent-1-en-1-yl]phenyl}propoxy)benzoesäure-methylester

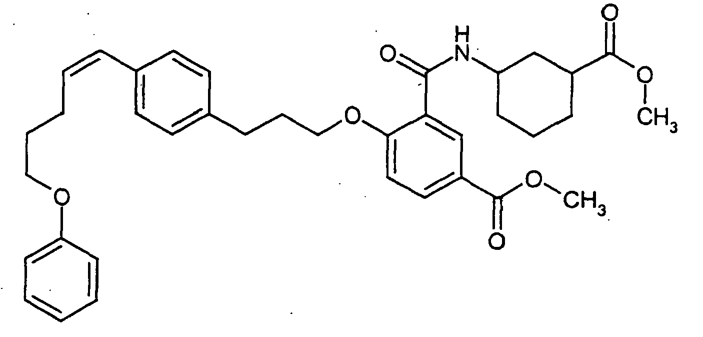

Die Herstellung erfolgt analog Beispiel 22 aus dem (+)-B-Enantiomer von 4-Hydroxy-3-({[3(methoxycarbonyl)cyclohexyl]amino}carbonyl)benzoesäure-methylester (siehe Beispiel IX, Methode 2) und (4Z)-5-[4-(3-Brompropyl)phenyl]-pent-4-en-1-yl-phenylether.
HPLC (Methode 5): Rₜ: 5.42 min.
¹H-NMR (300 MHz, CDCl₃, δ/ppm): 8.87 (d, 1H), 8.08 (dd, 1H), 7.84 (d, 1H), 7.30-7.20 (m, 4H), 7.13 (d, 2H), 6.97-6.84 (m, 4H), 6.43 (d, 1H), 5.68 (dq, 1H), 4.43-4.34 (m, 1H), 4.19 (t, 2H), 4.02-3.94 (m, 2H), 3.89 (s, 3H), 3.65 (s, 3H), 3.42 (t, 2H), 2.84 (t, 2H), 2.64-2.54 (m, 1H), 2.53 (dq, 2H), 2.33-2.20 (m, 2H), 2.04-1.60 (m, 8H).
LC-MS (Methode 7): Rₜ: 3.40 min, m/z (EI+) = 613.

### Beispiel 75

### 3-{[(3-Carboxycyclohexyl)amino]carbonyl}-4-(3-{4-[(1Z)-5-phenoxypent-1-en-1-yl]-phenyl}propoxy)benzoesäure

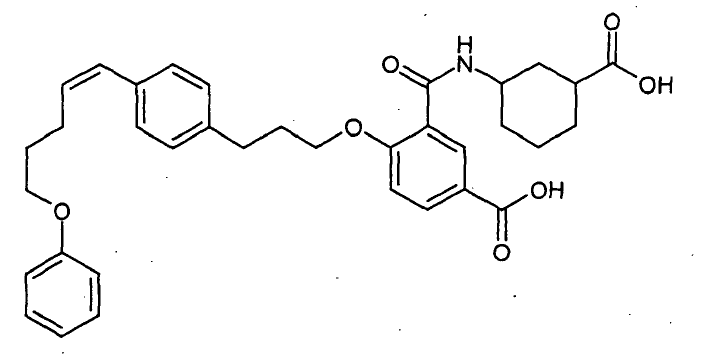

Die Herstellung erfolgt analog Beispiel 11 aus 3-({[3-(Methoxycarbonyl)-cyclohexyl]amino}-carbonyl)-4-(3-{4-[(1Z)-5-phenoxypent-1-en-1-yl]phenyl}-propoxy)benzoesäure-methylester.
¹H-NMR (300 MHz, CDCl₃, δ/ppm): 8.91 (d, 1H), 8.11 (dd, 1H), 7.84 (d, 1H), 7.30-7.18 (m, 4H), 7.13 (d, 2H), 6.97-6.83 (m, 4H), 6.43 (d, 1H), 5.67 (dq, 1H), 4.48-4.35 (m, 1H), 4.19 (t, 2H), 3.98 (t, 2H), 2.84 (t, 2H), 2.73-2.61 (m, 1H), 2.51 (dq, 2H), 2.33-2.18 (m, 2H), 2.15-2.04 (m, 1H), 2.02-1.52 (m, 9H).
LC-MS (Methode 7): Rₜ: 2.68 min, m/z (EI+) = 585.

### Beispiel 76

### 3-({[3-(Methoxycarbonyl)cyclohexyl]amino}carbonyl)-4-(3-{4-[(1E)-5-phenoxypent-1-en-1-yl]phenyl}propoxy)benzoesäure-methylester

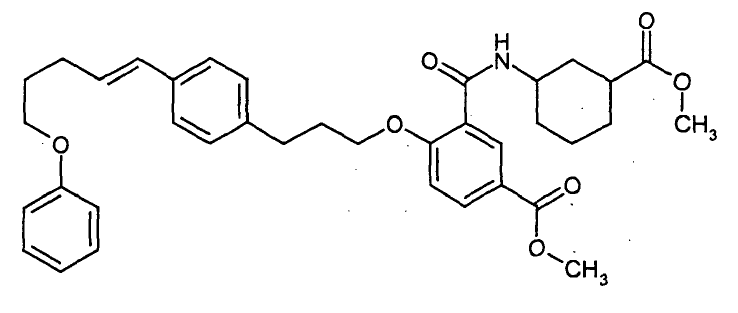

Die Herstellung erfolgt analog Beispiel 22 aus dem (+)-B-Enantiomer von 4-Hydroxy-3-({[3 (methoxycarbonyl)cyclohexyl]amino} carbonyl)benzoesäure-methylester (siehe Beispiel IX, Methode 2) und (4E)-5-[4-(3-Brompropyl)phenyl]-pent-4-en- 1 -yl-phenylether.
HPLC (Methode 5): Rₜ: 5.44 min.
¹H-NMR (300 MHz, CDCl₃, δ/ppm): 8.86 (d, 1H), 8.09 (d, 1H), 7.84 (d, 1H), 7.32-7.23 (m, 4H), 7.11 (d, 2H), 6.97-6.86 (m, 4H), 6.40 (d, 1H), 6.28-6.16 (m, 1H), 4.42-4.35 (m, 1H), 4.18 (t, 2H), 4.02 (t, 2H), 3.89 (s, 3H), 3.65 (s, 3H), 2.83 (t, 2H), 2.73-2.54 (m, 2H), 2.44-2.35 (m, 2H) 2.30-2.17 (m, 2H), 2.03-1.90 (m, 4H), 1.85-1.22 (m, 5H).
LC-MS (Methode 7): Rₜ: 3.22 min, m/z (EI+) = 613.

### Beispiel 77

### 3-{[(3-Carboxycyclohexyl)amino]carbonyl}-4-(3-{4-[(1E)-5-phenoxypent-1-en-1-yl]-phenyl}propoxy)benzoesäure

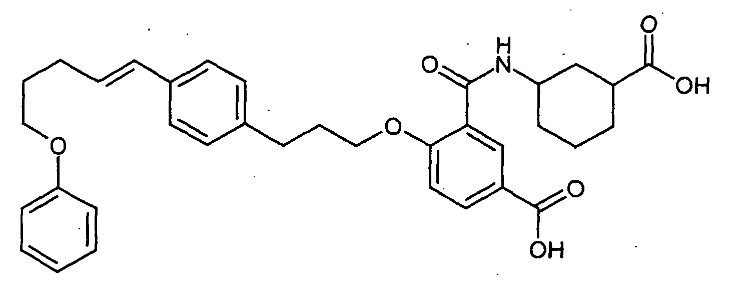

Die Herstellung erfolgt analog Beispiel 11 aus 3-({[3-(Methoxycarbonyl)-cyclohexyl]amino}-carbonyl)-4-(3- {4-[(1E)-5-phenoxypent-1-en-1-yl]phenyl}-propoxy)benzoesäure-methylester.
¹H-NMR (300 MHz, CDCl₃, δ/ppm): 8.91 (d, 1H), 8.12 (dd, 1H), 7.84 (d, 1H), 7.32-7.20 (m, 5H), 7.11 (d, 1H), 6.98-6.86 (m, 4H), 6.40 (d, 1H), 6.22 (dt, 1H), 4.48-4.36 (m, 1H), 4.17 (t, 2H), 4.00 (t, 2H), 2.86 (t, 2H), 2.74-2.63 (m, 1H), 2.44-2.33 (m, 2H) 2.30-2.18 (m, 2H), 2.17-2.03 (m, 1H), 2.02-1.22 (m, 9H).
LC-MS (Methode 7): Rₜ: 2.72 min, m/z (EI+) =585.

### Beispiel 78

### 3-({[3-(Methoxycarbonyl)cyclohexyl]amino}carbonyl)-4-({(2E)-3-[4-(4-phenoxybutoxy)phenyl]prop-2-en-1-yl}oxy) benzoesäure-methylester

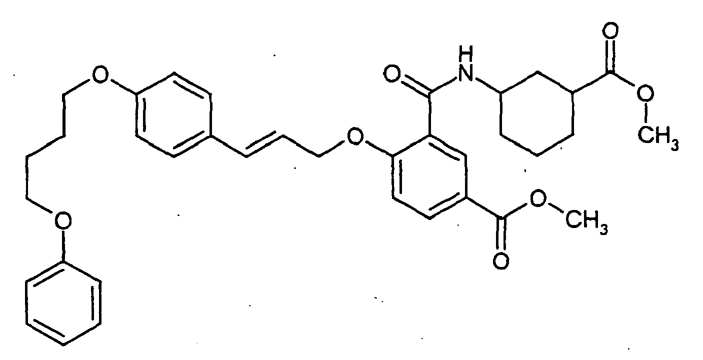

Die Herstellung erfolgt analog Beispiel 22 aus dem (+)-B-Enantiomer von 4-Hydroxy-3-({[3(methoxycarbonyl)cyclohexyl]amino}carbonyl)benzoesäure-methylester (siehe Beispiel IX, Methode 2) und 1-[(1E)-3-Bromprop-1-en-1-yl]-4-(4-phenoxybutoxy)benzol.
¹H-NMR (300 MHz, CDCl₃, δ/ppm): 8.94 (d, 1H), 8.17 (dd, 1H), 8.08 (d, 1H), 7.37 (d, 2H), 7.33-7.24 (m, 2H), 7.07 (d, 1H), 6.96-6.84 (m, 5H), 6.78 (d, 1H), 6.35 (dt, 1H), 4.83 (d, 2H), 4.48-4.39 (m, 1H), 4.08-4.00 (m, 4H), 3.90 (s, 3H), 3.60 (s, 3H), 2.53-2.40 (m, 1H), 2.10-1.93 (m, 5H), 1.83-1.37 (m, 7H).
MS (ESI+): m/z = 638 (M+Na⁺)

### Beispiel 79

### 3-{[(3-Carboxycyclohexyl)amino]carbonyl}-4-({(2E)-3-[4-(4-phenoxybutoxy)-phenyl]prop-2-en-1-yl}oxy)benzoesäure

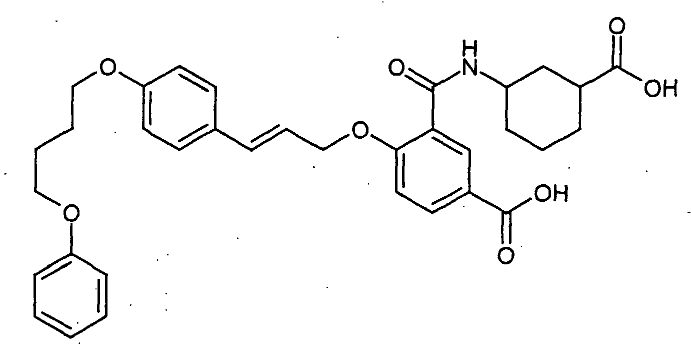

Die Herstellung erfolgt analog Beispiel 11 aus 3-({[3-(Methoxycarbonyl)-cyclohexyl]amino}carbonyl)-4-{(2E)-3-[4-(4-phenoxybutoxy)-phenyl]prop-2-en-1-yl} oxy)benzoesäure-methylester.
HPLC (Methode 5): Rₜ: 4.66 min.
¹H-NMR (300 MHz, CDCl₃, δ/ppm): 8.95 (d, 1H), 8.17 (dd, 1H), 8.08 (d, 1H), 7.36 (d, 2H), 7.32-7.22 (m, 2H), 7.07 (d, 1H), 6.96-6.74 (m, 6H), 6.34 (dt, 1H), 4.83 (d, 2H), 4.50-4.40 (m, 1H), 4.08-3.96 (m, 4H), 2.58-2.46 (m, 1H), 2.08-1.46 (m, 12H).. LC-MS (Methode 7): Rₜ: 2.76 min, m/z (EI+) = 587.

### Beispiel 80

### 3-[({[2-(Methoxycarbonyl)cyclohexyl]methyl}amino)carbonyl]-4-{3-[4-(4-phenoxybutoxy)phenyl]propoxy}benzoesäure-ethylester

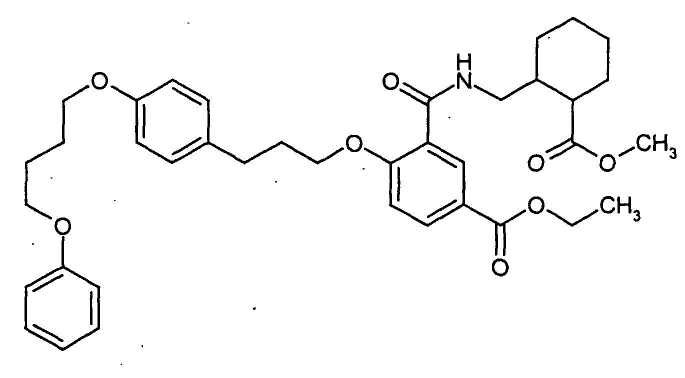

Die Herstellung erfolgt analog Beispiel IX, Methode 3, aus [2-(Methoxycarbonyl)-cyclohexyl]-methanammoniumbromid und 5-(Ethoxycarbonyl)-2-{3-[4-(4-phenoxybutoxy)phenyl]propoxy}benzoesäure.
HPLC (Methode 5): Rₜ: 5.62 min.
¹H-NMR (300 MHz, CDCL₃, δ/ppm): 8.86 (d, 1H), 8.09 (dd, 1H), 7.93 (t, 1H), 7.31-7.25 (m, 2H), 7.10 (d, 2H), 6.97-6.82 (m, 6H), 4.35 (q, 2H), 4.16 (t, 2H), 4.07-3.98 (m, 4H), 3.70-3.59 (m, 1H), 3.58 (s, 3H), 3.41-3.30 (m, 1H), 2.80-2.68 (m, 3H), 2.27-2.11 (m, 3H), 2.01-1.83 (m, 5H), 1.74-1.55 (m, 5H), 1.44-1.33 (m, 2H), 1.37 (t, 3H).
MS (ESI+): m/z = 646 (M+H⁺).

### Beispiel 81

### 3-({[(2-Carboxycyclohexyl)methyl]amino}carbonyl)-4-{3-[4-(4-phenoxybutoxy)-phenyl]propoxy}benzoesäure

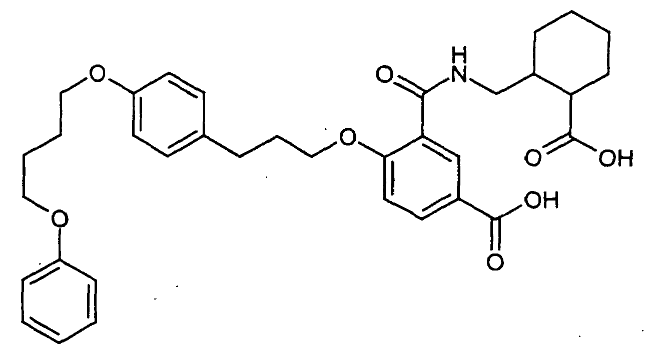

Die Herstellung erfolgt analog Beispiel 11 aus 3-[({[2-(Methoxycarbonyl)-cyclohexyl]-methyl} amino)carbonyl]-4-{3-[4-(4-phenoxybutoxy)phenyl]propoxy}-benzoesäure-ethylester.
HPLC (Methode 5): Rₜ: 4.64 min.
¹H-NMR (300 MHz, DMSO-d₆, δ/ppm): 8.23 (d, 1H), 8.12 (t, 1H), 7.98 (dd, 1H), 7.31-7.25 (m, 2H), 7.21-7.10 (m, 3H), 6.95-9.82 (m, 5H), 4.14 (t, 2H), 4.05-3.96 (m, 4H), 3.6-3.2 (m, 2H, unter Wasser-Signal), 2.68 (t, 2H), 2.62-2.56 (m, 1H), 2.12 - 1.93 (m, 3H), 1.88-1.82 (m, 4H), 1.82-1.12 (m, 8H).
MS (ESI-): m/z = 602 (M-H⁺)

Analog zu Verfahren, die in den obigen Beispielen beschrieben sind, werden außerdem folgende Verbindungen hergestellt. (Wenn nicht anders angegeben, sind alle Derivate, die das 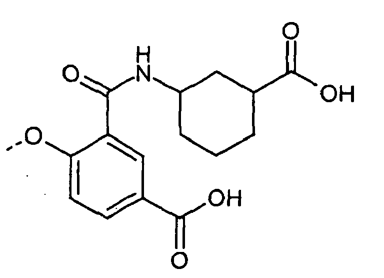 oder

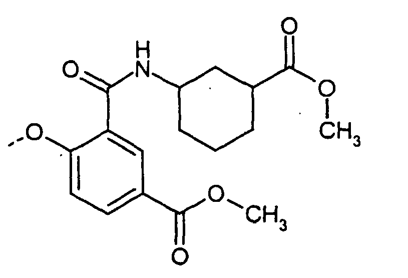

Fragment enthalten, aus dem (+)-B-Enantiomer der Verbindung aus Beispiel IX, Methode 2, hergestellt.)

| Bsp. -Nr. | Struktur | Molmasse [g/mol] | HPLC Methode | HPLC Rₜ [min] | MS oder LC-MS (Methode) |
|---|---|---|---|---|---|
| **82** | 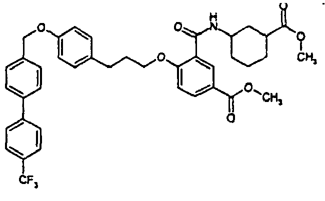 | 703 | 2 | 6.04 | 4.7 min, 704 (13) |
| **83** | 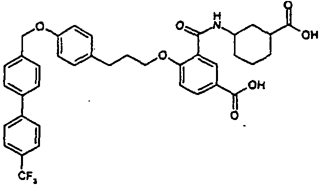 | 675 | 2 | 5.40 | 3.47 min, 675 (14) |
| **84** | 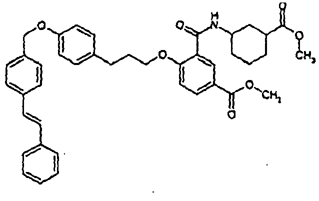 | 661 | 2 | 6.06 | 662 (ESI) |
| **85** | 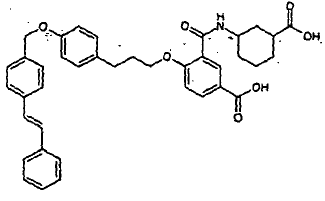 | 633 | 2 | 5.39 | 3.71 min, 633 (8) |
| **86** | 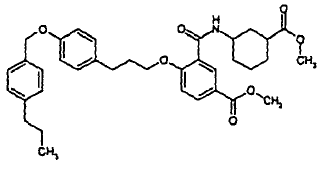 | 601 | 2 | 5.87 | 602 (ESI) |
| **87** | 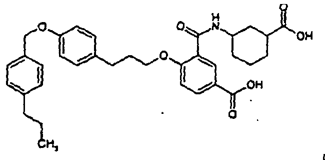 | 573 | 2 | 5.24 | 572 (ESI) |
| **88** | 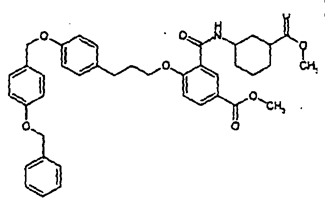 | 665 | 2 | 5.82 | 666 (ESI) |
| **89** | 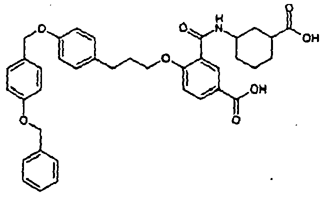 | 637 | 2 | 5.17 | 3.62 min, 637 (8) |
| **90** | 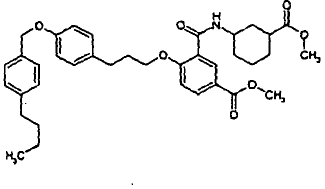 | 615 | 2 | 6.17 | 616(ESI) |
| **91** | 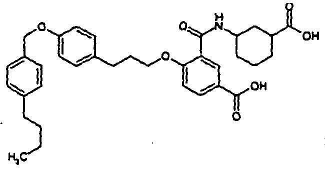 | 5.87 | 1 | 5.46 | 586 (ESI) |
| **92** | 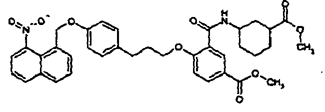 | 654 | 2 | 5.56 | 655 (ESI) |
| **93** | 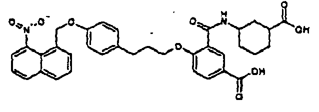 | 626 | 2 | 4.91 | 3.46 min, 626 (8) |
| **94** | 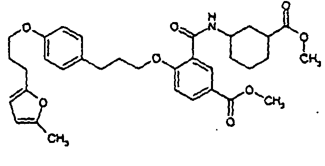 | 591 | 2 | 5.65 | 4.34 min, 592/614 (13) |
| **95** | 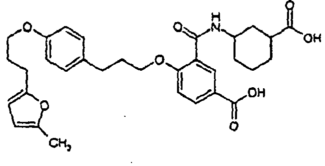 | 563 | 2 | 5.0 | 3.24 min, 563 (14) |
| **96** | 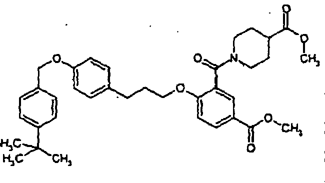 | 601 | 2 | 5.76 | 602 (DCI) |
| **97** | 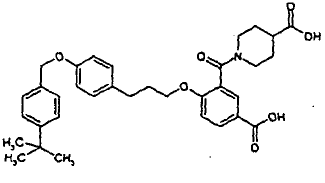 | 573 | 2 | 5.03 | 572 (ESI) |
| **98** | 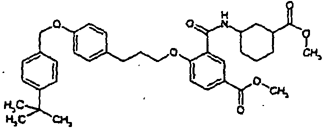 | 615 | 2 | 6.01 | 616 (ESI) |
| **99** | 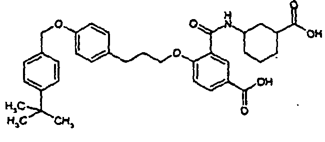 | 587 | 1 | 5.29 | 586 (ESI) |
| **100** | 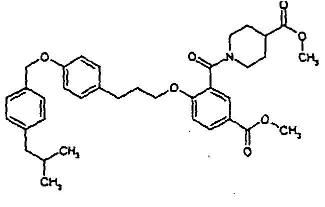 | 601 | 2 | 5.86 | 602 (DCI) |
| **101** | 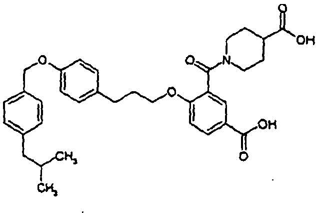 | 573 | 2 | 5.12 | 572 (ESI) |
| **102** | 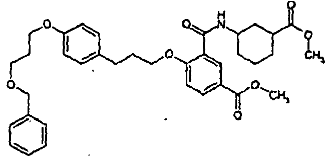 | 617 | 2 | 5.58 | 4.3 min, 617(13) |
| **103** | 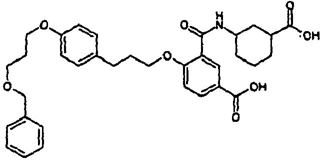 | 589 | 2 | 4.93 | 3.18 min, 589 (14) |
| **104** | 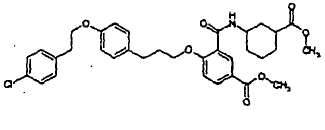 | 608 | 2 | 5.72 | 608 (ESI) |
| **105** | 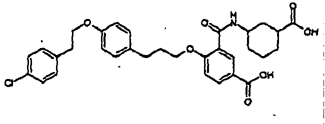 | 580 | 2 | 5.03 | 3.25 min, 579(14) |
| **106** | 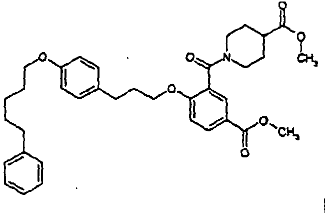 | 601 | 2 | 5.7 | 602 (ESI) |
| **107** | 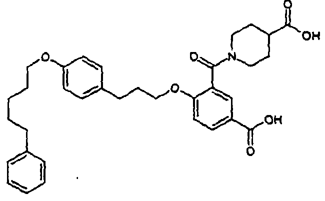 | 573 | 2 | 5.04 | 572 (ESI) |
| **108** | 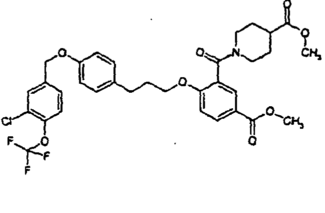 | 664 | 2 | 5.67 | 664 (ESI) |
| **109** | 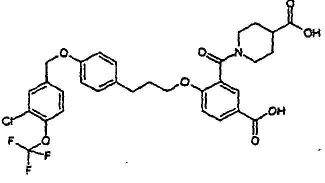 | 636 | 2 | 5.01 | 3.49 min, 635 (8) |
| **110** | 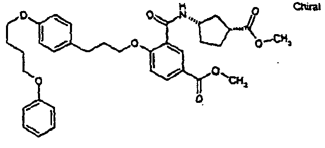 | 617 | 2 | 5.81 | 618 (ESI) |
| **111** | 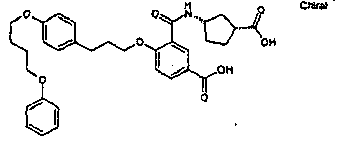 | 575 | 12 | 7.7 | 3.44 min, 575 (8) |
| **112** | 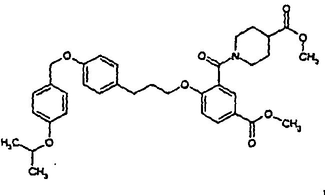 | 603 | 2 | 5.41 | 604 (DCI) |
| **113** | 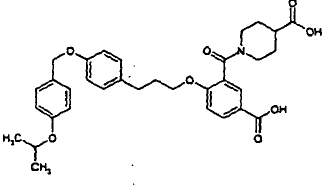 | 575 | 2 | 4.7 | 574 (ESI) |
| **114** | 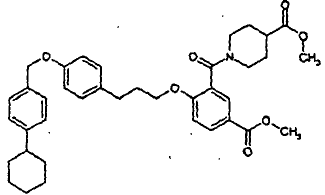 | 627 | 2 | 6.15 | 628 (DCI) |
| **115** | 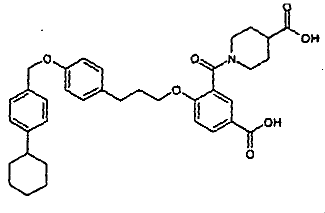 | 599 | 2 | 5.35 | 598 (ESI) |
| **116** | 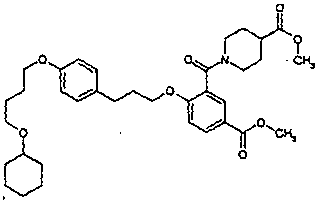 | 609 | 2 | 5.73 | 610 (DCI) |
| **117** | 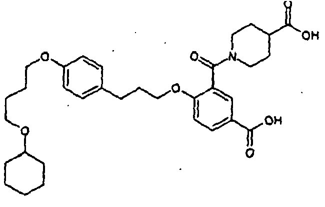 | 581 | 2 | 4.9 | 580 (ESI) |
| **118** | 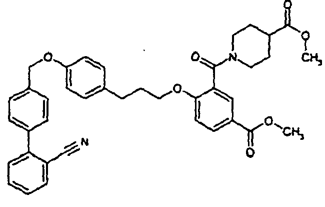 | 646 | 2 | 5.36 | 647 (DCI) |
| **119** | 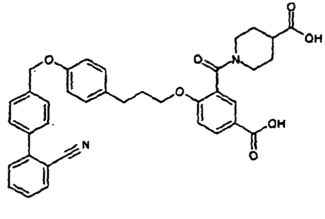 | 618 | 2 | 5.15 | 3.29 min, 619 (8) |
| **120** | 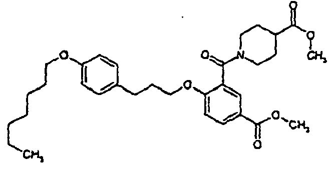 | 553 | 2 | 5.85 | 554 (ESI) |
| **121** | 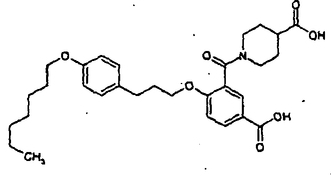 | 525 | 2 | 5.11 | 524 (ESI) |
| **122** | 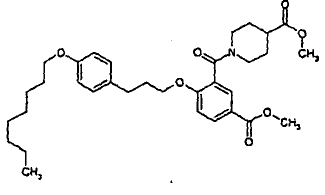 | 567 | 2 | 6.08 | 568 (ESI) |
| **123** | 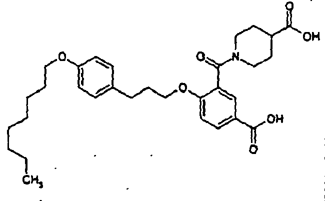 | 539 | 2 | 5.31 | 538 (ESI) |
| **124** | 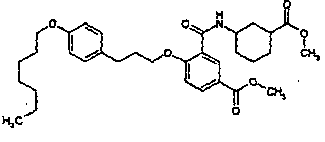 | 567 | 2 | 6.18 | 568 (ESI) |
| **125** | 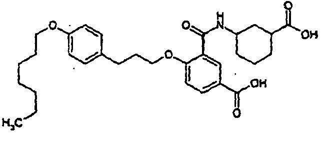 | 539 | 2 | 5.45 | 538 (ESI) |
| **126** | 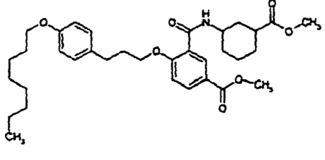 | 581 | 2 | 6.50 | 582 (ESI) |
| **127** | 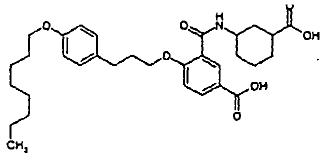 | 553 | 2 | 5.66 | 552 (ESI) |
| **128** | 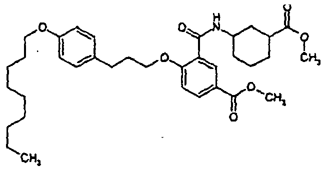 | 595 | 2 | 7.08 | 5.1 min, 595 (13) |
| **129** | 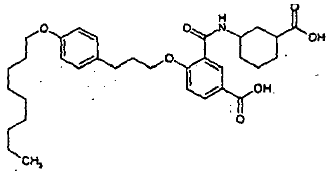 | 567 | 2 | 5.86 | 3.64 min, 567 (14) |
| **130** | 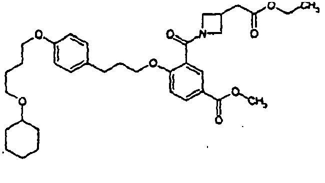 | 609 | - | - | 3.08 min, 610 (6) |
| **131** | 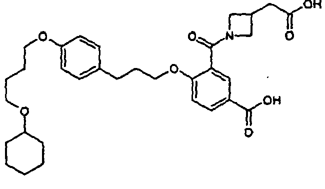 | 567 | - | - | 2.60 min, 568 (7) |
| **132** | 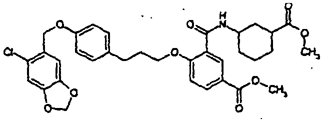 | 638 | 2 | 5.63 | 638 (ESI) |
| **133** | 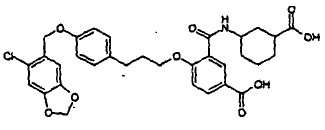 | 610 | 2 | 4.94 | 3.46 min, 609 (8) |
| **134** | 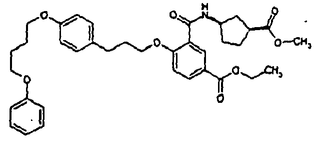 | 617 | 2 | 5.8 | 618 (ESI) |
| **135** | 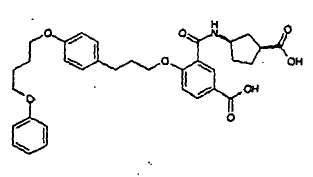 | 575 | - | - | 3.45 min, 575 (8) |
| **136** | 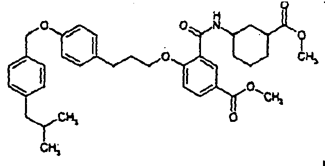 | 615 | 2 | 6.13 | 616 (ESI) |
| **137** | 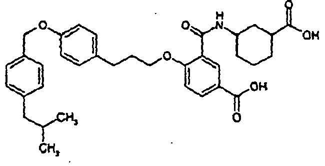 | 587 | 1 | 5.43 | 586 (ESI) |
| **138** | 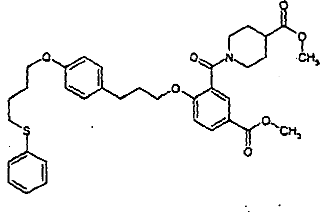 | 619 | 2 | 5.59 | 620 (DCI) |
| **139** | 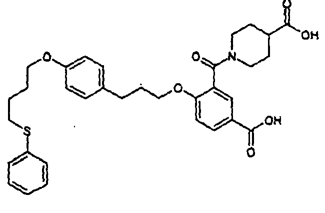 | 591 | 2 | 4.89 | 590 (ESI) |
| **140** | 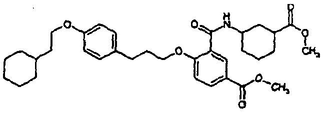 | 579 | 2 | 6.30 | 580.(ESI) |
| **141** | 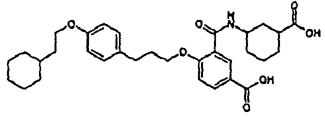 | 551 | 1 | 5.35 | 550 (ESI) |
| **142** | 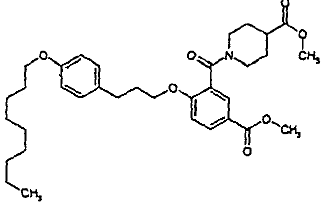 | 581 | 2 | 6.49 | 582 (DCI) |
| **143** | 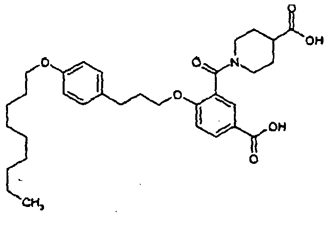 | 553 | 2 | 5.51 | 552 (ESI) |
| **144** | 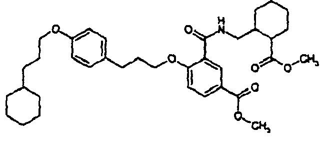 | 607 | - | - | 608 (ESI) |
| **145** | 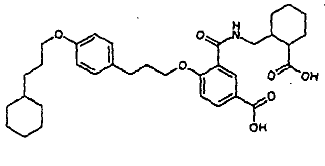 | 579 | - | - | 3.24 min, 579 (7) |
| **146** | 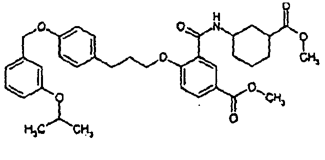 | 617 | 2 | 5.60 | 618 (ESI) |
| **147** | 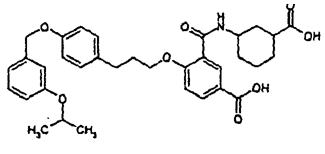 | 589 | 2 | 4.9 | 2.52 min, 589 (9) |
| **148** | 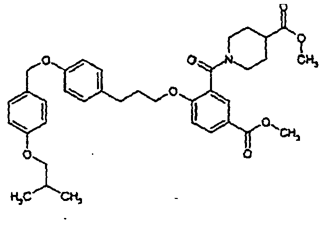 | 617 | 2 | 5.69 | 618 (DCI) |
| **149** | 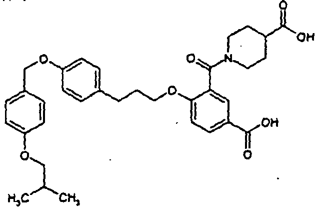 | 589 | 2 | 4.99 | 588 (ESI) |
| **150** | 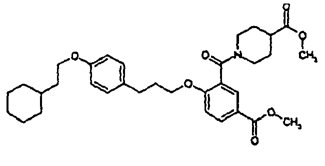 | 565 | 2 | 5.83 | 566 (DCI) |
| **151** | 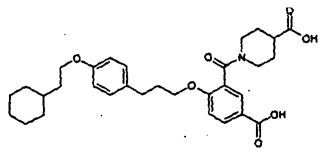 | 537 | 2 | 5.06 | 538 (ESI) |
| **152** | 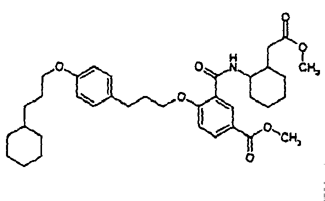 | 607 | 2 | 6.60 | 608 (ESI) |
| **153** | 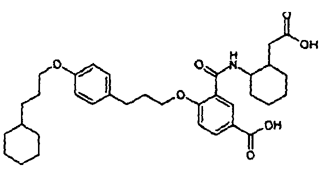 | 579 | 2 | 5.69 | 578 (ESI) |
| **154** | 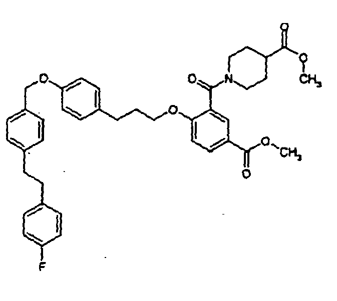 | 667 | 2 | 4.32 | 456 (ESI) |
| **155** | 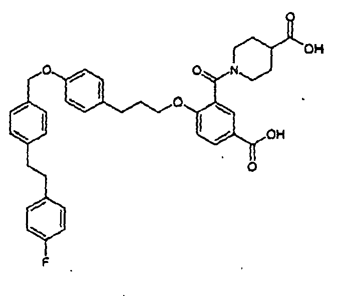 | 639 | 2 | 4.74 | 3.6 min, 639 (8) |
| **156** | 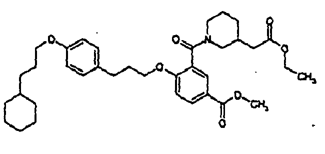 | 607 | 1 | 6.47 | 608 (ESI) |
| **157** | 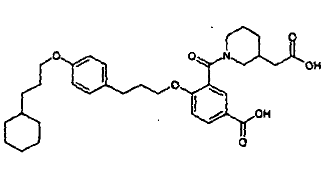 | 565 | 1 | 5.45 | 564 (ESI) |
| **158** | 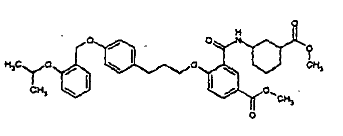 | 617 | 2 | 5.69 | 618 (ESI) |
| **159** | 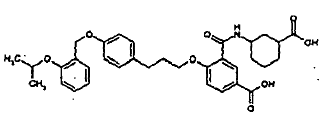 | 589 | 2 | 5.06 | 588 (ESI) |
| **160** | 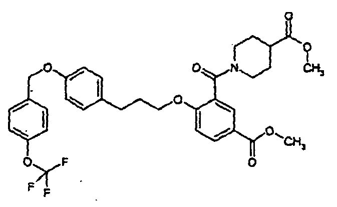 | 629 | 2 | 5.49 | 630 (ESI) |
| **161** | 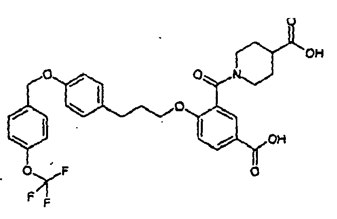 | 601 | 2 | 4.80 | 602 (ESI) |
| **162** | 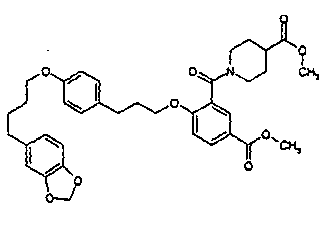 | 631 | -- | -- | -- |
| **163** | 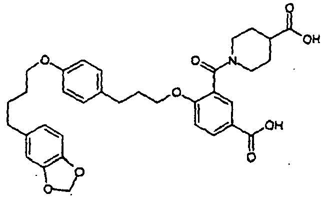 | 603 | -- | -- | -- |
| **164** | 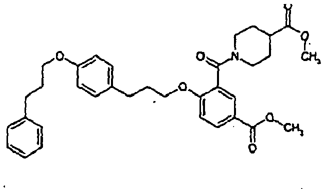 | 573 | 2 | 5.45 | 574 (ESI) |
| **165** | 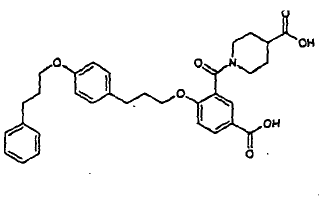 | 545 | 2 | 4.77 | 3.27 min, 545 (8) |
| **166** | 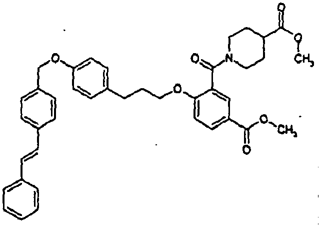 | 647 | 2 | 5.82 | 648 (DCI) |
| **167** | 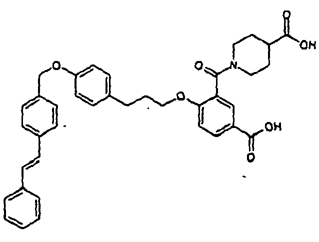 | 619 | 2 | 5.1 | 618 (ESI) |
| **168** | 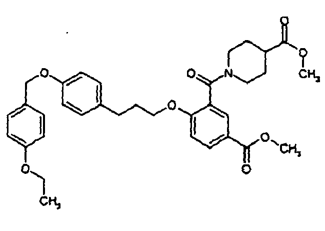 | 589 | 2 | 5.3 | 590 (DCI) |
| **169** | 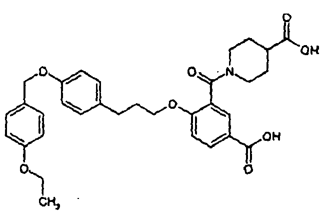 | 561 | 2 | 4.62 | 560 (ESI) |
| **170** | 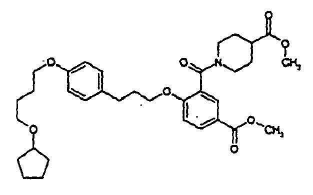 | 595 | 2 | 5.57 | 596 (DCI) |
| **171** | 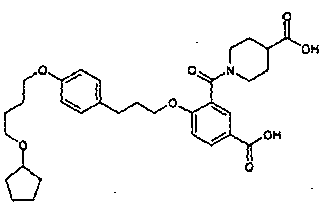 | 567 | 2 | 4.8 | 566 (ESI) |
| **172** | 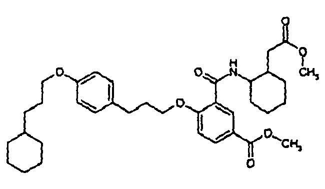 | 607 | 2 | 6.63 | 608 (ESI) |
| **173** | 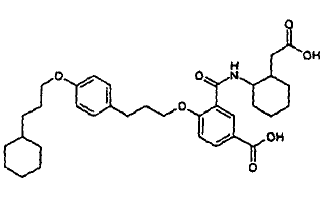 | 579 | 2 | 5.74 | 578 (ESI) |
| **174** | 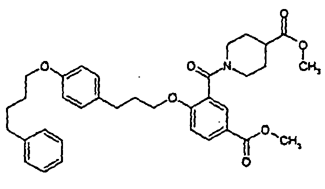 | 587 | 2 | 5.56 | 588 (ESI) |
| **175** | 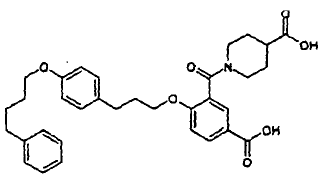 | 559 | 2 | 4.89 | 560 (ESI) |
| **176** | 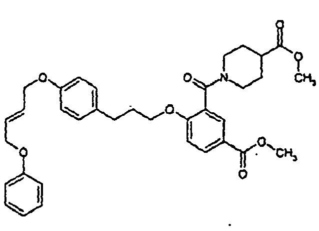 | 601 | 2 | 5.27 | 602 (ESI) |
| **177** | 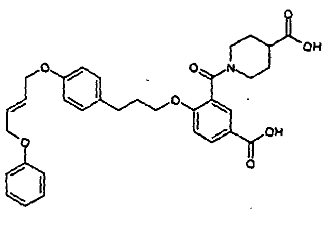 | 573 | 2 | 4.67 | 574 (ESI) |
| **178** | 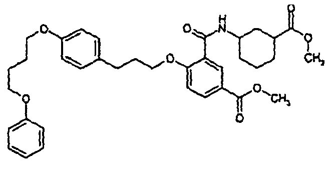 | 617 | 1 | 5.69 | 618 (ESI) |
| **179** | 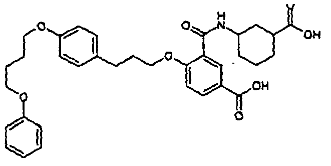 | 589 | 1 | 4.99 | 3.96 min, 589 (8) |
| | aus (-)-B-Enantiomer (Beispiel IX, Methode 2) hergestellt | | | | |
| **180** | 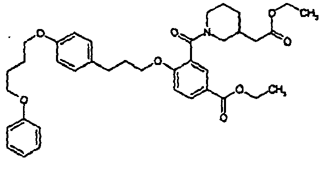 | 645 | 4 | 5.78 | 646 (ESI) |
| **181** | 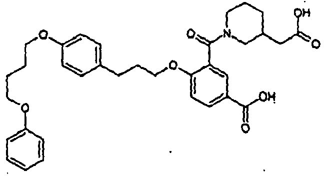 | 589 | 2 | 4.89 | 588 (ESI) |
| **182** | 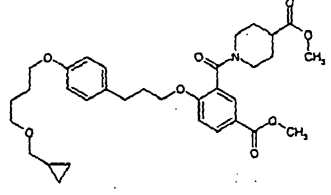 | 581 | 2 | 5.26 | 582 (DCI) |
| **183** | 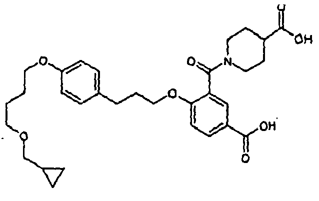 | 553 | 1 | 4.52 | 554 (ESI) |
| **184** | 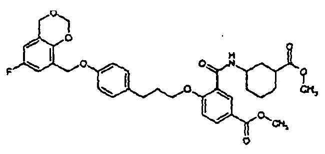 | 635 | 2 | 5.37 | 636 (ESI) |
| **185** | 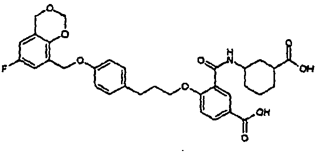 | 607 | 2 | 4.66 | 3.22 min, 607 (8) |
| **186** | 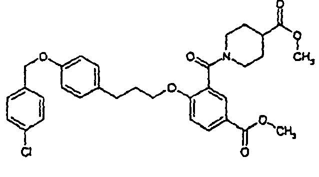 | 580 | 2 | 5.39 | 580 (DCI) |
| **187** | 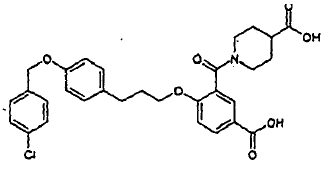 | 552 | 2 | 4.71 | 550 (ESI) |
| **188** | 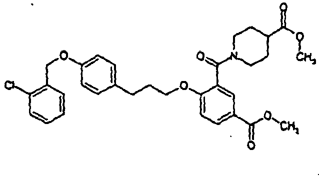 | 580 | 2 | 5.41 | 580 (ESI) |
| **189** | 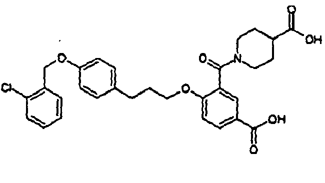 | 552 | 2 | 4.6 | 550 (ESI) |
| **190** | 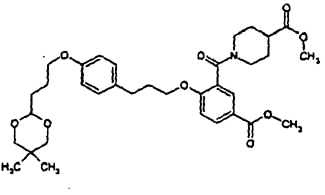 | 611 | 2 | 5.27 | 612 (DCI) |
| **191** | 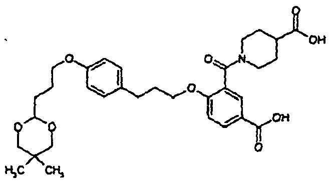 | 583 | 2 | 4.56 | 582 (ESI) |
| **192** | 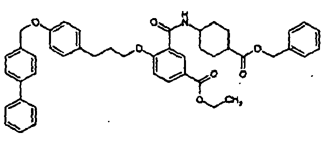 | 725 | 4 | 6.13 | 726 (ESI) |
| | cis/trans Gemisch | | | | |
| **193** | 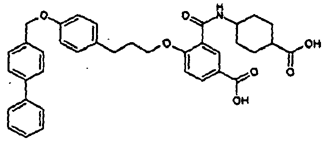 | 607 | 2 | 5.02 | 608 (EI+) |
| | cis/trans Gemisch | | | | |
| **194** | 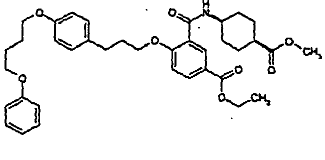 | 631 | 2 | 5.78 | 632 (ESI) |
| **195** | 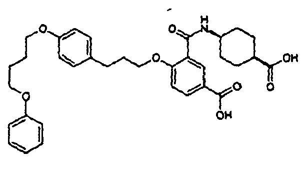 | 589 | 2. | 4.83 | 590 (ESI) |
| **196** | 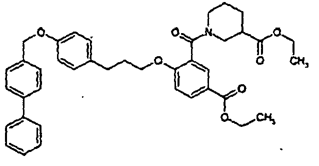 (+)-Enantiomer | 649 | 2 | 5.88 | 650 (ESI) |
| **197** | 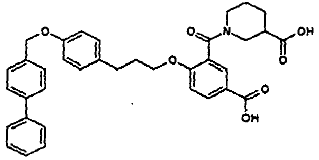 (+)-Enantiomer | 593 | 2 | 5.05 | 3.92 min, 594 (8, EI+) |
| **198** | 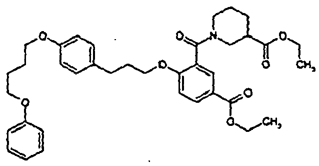 (+)-Enantiomer | 631 | 2 | 5.73 | 632 (ESI) |
| **199** | 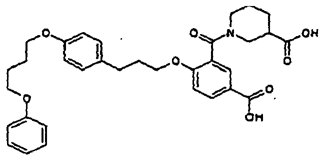 (+)-Enantiomer | 575 | 2 | 4.90 | 3.82 min, 576 (8, EI+) |
| **200** | 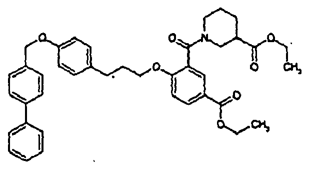 | 649 | 1 | 5.92 | 650 (ESI) |
| **201** | 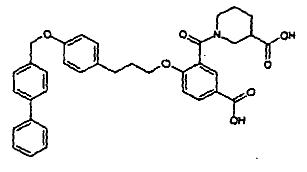 | 593 | 1 | 5.01 | 594 (DCI, NH₃) |
| **202** | 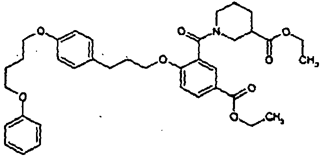 | 631 | 1 | 5.77 | 632 (ESI) |
| **203** | 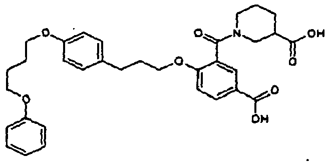 | 575 | 1 | 4.88 | 576 (DCI, NH₃) |

### B. Bewertung der physiologischen Wirksamkeit

### Abkürzungen:

- DMEM: Dulbecco's Modified Eagle Medium
- FCS: Fetal Calf Serum
- HEPES: 4-(2-hydroxyethyl)-1-piperazinethanesulfonic acid

### 1. Zellulärer in vitro-Test zur Bestimmung der CysLT2-Aktivität

Die Identifizierung von Antagonisten des humanen Cysteinyl-Leukotrien 2 Rezeptors (CysLT2R) sowie die Quantifizierung der Wirksamkeit der hier beschriebenen Substanzen erfolgt mit Hilfe einer rekombinanten Zelllinie. Die Zelle leitet sich ursprünglich von einer Ovarepithelzelle des Hamsters (Chinese Hamster Ovary, CHO K1, ATCC: American Type Culture Collection, Manassas, VA 20108, USA) ab. Die Testzelllinie exprimiert konstitutiv das Calcium-sensitive Photoprotein Aequorin, das nach Rekonstitution mit dem Co-Faktor Coelenterazin bei Erhöhungen der cytoplasmatischen Calcium-Konzentration Licht emittiert (Rizzuto R, Simpson AW, Brini M, Pozzan T.; Nature 358 (1992) 325-327). Zusätzlich ist die Zelle stabil transfiziert mit dem humanen CysLT2 Rezeptor (Heise et. al., JBC 275 (2000) 30531-30536). Die resultierende CysLT2R-Testzelle reagiert auf Stimulation des rekombinanten CysLT2 Rezeptors (Agonisten: Leukotrien D4 (LTD4) und Leukotrien C4 (LTC4)) mit einer intrazellulären Freisetzung von Calcium-Ionen, die durch die resultierende Aequorin-Lumineszenz mit einem geeignetem Luminometer quantifiziert werden kann (Milligan G, Marshall F, Rees S, Trends in Pharmacological Sciences 17 (1996) 235-237). Vorinkubation mit Antagonisten des CysLT2 Rezeptors vermindert die durch die Agonisten LTD4 bzw. LTC4 induzierte Calciumfreisetzung und damit die gemessene Lichtmenge.

Testablauf: Die Zellen werden zwei Tage vor dem Test in Kulturmedium (DMEM/F12 mit Glutamax, Gibco Cat.# 61965-026; 10% FCS, Gibco Cat.# 10270-106; 1,4 mM Natriumpyruvat, Gibco Cat.# 11360-039; 1,8 mM Natriumbicarbonat, Gibco Cat.# 25080-060; 10 mM HEPES, Gibco Cat.# 15290-026; gehört jetzt zu: Invitrogen GmbH, 76131 Karlsruhe) in 384- (oder 1536-) Loch-Mikrotiterplatten ausplattiert und in einem Zellinkubator (96% Luftfeuchtigkeit, 5% v/v CO₂, 37°C) gehalten. Am Testtag wird das Kulturmedium durch eine Tyrodelösung (in mM: 140 NaCl, 5 KCl, 1 MgCl₂, 2 CaCl₂, 20 Glucose, 20 HEPES), die zusätzlich den Co-Faktor Coelenterazin (50 µM) enthält, ausgetauscht und die Mikrotiterplatte anschließend für weitere 3-4 Stunden inkubiert. 15 Minuten nach Übertragung der Testsubstanzen in die Löcher der Mikrotiterplatte wird das resultierende Lichtsignal nach Gabe von LTD4 (3 x 10⁻⁸ M) im Luminometer gemessen. Die Ergebnisse sind in Tabelle 1 gezeigt.

### 2. Zellulärer in vitro-Test zur Bestimmung der CysLT1-Aktivität

Die Identifizierung von Antagonisten des humanen Cysteinyl-Leukotrien 1 Rezeptors (CysLT1R) sowie die Quantifizierung der Wirksamkeit der hier beschriebenen Substanzen erfolgt mit Hilfe einer rekombinanten Zelllinie. Die Zelle leitet sich ursprünglich von einer Ovarepithelzelle des Hamsters (Chinese Hamster Ovary, CHO K1, ATCC: American Type Culture Collection, Manassas, VA 20108, USA) ab. Die Testzelllinie exprimiert konstitutiv das calcium-sensitive Photoprotein mtAequorin, das nach Rekonstitution mit dem Co-Faktor Coelenterazin bei Erhöhungen der cytoplasmatischen Calcium-Konzentration Licht emittiert (Rizzuto R, Simpson AW, Brini M, Pozzan T.; Nature 358 (1992) 325-327). Zusätzlich ist die Zelle stabil transfiziert mit dem humanen CysLT1 Rezeptor (Lynch et.al., Nature 399 (1999) 789-793). Die resultierende CysLT1R-Testzelle reagiert auf Stimulation des rekombinanten CysLT1 Rezeptors (Agonist: Leukotrien D4 (LTD4)) mit einer intrazellulären Freisetzung von Calcium-Ionen, die durch die resultierende Aequorin-Lumineszenz mit einem geeignetem Luminometer quantifiziert werden kann (Milligan G, Marshall F, Rees S, Trends in Pharmacological Sciences 17 (1996) 235-237). Vorinkubation mit Antagonisten des CysLT1 Rezeptors vermindert die durch den Agonisten LTD4 induzierte Calciumfreisetzung und damit die gemessene Lichtmenge.

Testablauf: Die Zellen werden zwei Tage vor dem Test in Kulturmedium (DMEM/F12 mit Glutamax, Gibco Cat.# 61965-026; 10% FCS, Gibco Cat.# 10270-106; 1,4 mM Natriumpyruvat, Gibco Cat.# 11360-039; 1,8 mM Natriumbicarbonat, Gibco Cat.# 25080-060; 10 mM HEPES, Gibco Cat.# 15290-026; gehört jetzt zu: Invitrogen GmbH, 76131 Karlsruhe) in 384- (oder 1536-) Loch-Mikrotiterplatten ausplattiert und in einem Zellinkubator (96% Luftfeuchtigkeit, 5% v/v CO₂, 37°C) gehalten. Am Testtag wird das Kulturmedium durch eine Tyrodelösung (in mM: 140 NaCl, 5 KCl, 1 MgCl₂, 2 CaCl₂, 20 Glucose, 20 HEPES), die zusätzlich den Co-Faktor Coelenterazin (50 µM) enthält, ausgetauscht und die Mikrotiterplatte anschließend für weitere 3-4 Stunden inkubiert. 15 Minuten nach Übertragung der Testsubstanzen in die Löcher der Mikrotiterplatte wird das resultierende Lichtsignal nach Gabe von LTD4 (3 x 10⁻⁹ M) im Luminometer gemessen. Die Ergebnisse sind in Tabelle 1 gezeigt.

**Tabelle 1: Vergleich CysLT1-Aktivität / CysLT2-Aktivität**

| Beispiel Nr. | CysLT1: IC₅₀ (nM) | CysLT2: IC₅₀ (nM) |
|---|---|---|
| 11 | > 10000 | 35 |
| 23 | 2000 | 5 |
| 25 | >10000 | 6 |
| 31 | 2500 | 14 |
| 33 | > 10000 | 15 |

### 3. in vivo-Test zum Nachweis der kardiovaskulären Wirkung: Langendorff-Herz Meerschweinchen

Narkotisierten Meerschweinchen wird nach Eröffnung des Brustkorbes das Herz entnommen und in eine konventionelle Langendorff-Apparatur eingeführt. Die Koronararterien werden volumenkonstant (10 ml/min) perfundiert und der dabei auftretende Perfusionsdruck wird über einen entsprechenden Druckaufnehmer registriert. Eine Abnahme des Perfusionsdrucks in dieser Anordnung entspricht einer Relaxation der Koronararterien. Gleichzeitig wird über einen in die linke Herzkammer eingeführten Ballon und einen weiteren Druckaufnehmer der Druck gemessen, der vom Herzen während jeder Kontraktion entwickelt wird (der linksventrikuläre Druck). Die Frequenz des spontan schlagenden Herzens wird rechnerisch aus der Anzahl der Kontraktionen pro Zeiteinheit ermittelt.

Für den Nachweis der Wirkung von CysLT2 Rezeptor-Antagonisten wird die Perfusion mit dem Agonisten LTC4 (10⁻⁸ M) 15 Minuten vor Gabe aufsteigender Konzentration der Testsubstanz (10⁻⁸ bis 10⁻⁶ M) gestartet.

**Tabelle 2: Veränderung des Perfusionsdrucks in isolierten Meerschweinchen-Herzen nach Gabe von LTC4 in Abwesenheit und Gegenwart unterschiedlicher Konzentrationen der Testsubstanzen**

| **Bsp- Nr.** | **Perfusionsdruck relativ zur Kontrolle ohne LTC4 [%]** | | | |
|---|---|---|---|---|
| | LTC4 (10⁻⁸M) ohne Testsub. | LTC4 (10⁻⁸M) + Testsub. (10⁻⁸M) | LTC4 (10⁻⁸M) + Testsub. (10⁻⁷M) | LTC4 (10⁻⁸M) + Testsub. (10⁻⁶M) |
| **11** | 127 | 125 | 112 | 108 |
| **23** | 134 | 133 | 127 | 111 |
| **25** | 131 | 128 | 123 | 111 |
| **31** | 130 | 128 | 123 | 112 |
| **33** | 127 | 122 | 121 | 108 |

**Tabelle 3: Veränderung des linksventrikulären Drucks in isolierten Meerschweinchen-Herzen nach Gabe von LTC4 in Abwesenheit und Gegenwart unterschiedlicher Konzentrationen der Testsubstanzen**

| **Bsp- Nr.** | **Linksventrikuläre Druck relativ zur Kontrolle ohne LTC4 [%]** | | | |
|---|---|---|---|---|
| | LTC4 (10⁻⁸M) ohne Testsub. | LTC4 (10⁻⁸M)+ Testsub. (10⁻⁸M) | LTC4 (10⁻⁸M)+ Testsub. (10⁻⁷M) | LTC4 (10⁻⁸M)+ Testsub. (10⁻⁶M) |
| **11** | 47 | 44 | 60 | 79 |
| **23** | 59 | 72 | 84 | 97 |
| **25** | 46 | 55 | 71 | 89 |
| **31** | 54 | 67 | 81 | 87 |
| **33** | 44 | 44 | 57 | 84 |

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 11, 50 mg Lactose (Monohydrat), 50 mg mikrokristalline Cellulose, 10 mg Polyvinylpyrrolidon (PVP) (Fa. BASF, Ludwigshafen, Deutschland), 10 mg quervernetzte Na-Carboxymethyl-Cellulose und 2 mg Magnesiumstearat.
Tablettengewicht 222 mg. Durchmesser 8 mm, Wölbungsradius 12 mm.

### Herstellung:

Die Mischung aus Wirkstoff, Lactose und Cellulose wird mit einer 5%-igen Lösung (m/m) des PVPs in Wasser granuliert. Das Granulat wird nach dem Trocknen mit der quervernetzten Na-Carboxymethyl-Cellulose und dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 11, 1000 mg Ethanol (96%), 400 mg Xanthan Gummi (Fa. FMC, Pennsylvania, USA) und 97.6 g Wasser. Einer Einzeldosis von 100 mg der erfindungsgemäßen Verbindung entsprechen 10 g orale Suspension.

### Herstellung:

Der Xanthan Gummi wird in Ethanol suspendiert, der Wirkstoff wird der Suspension zugefügt. Unter Rühren erfolgt die Zugabe des Wassers. Bis zum Abschluss der Quellung des Xanthan Gummis wird ca. 6h gerührt.

### Oral applizierbare Lösung:

### Zusammensetzung

500 mg der Verbindung von Beispiel 11, 2.5 g Polysorbat und 97 g Polyethylenglycol 400. Einer Einzeldosis von 100 mg der erfindungsgemäßen Verbindung entsprechen 20 g orale Lösung.

### Herstellung

Der Wirkstoff wird in der Mischung aus Polyethylenglycol und Polysorbat unter Rühren suspendiert. Der Rührvorgang wird bis zur vollständigen Auflösung des Wirkstoffes fortgesetzt.

### Intravenös applizierbare Lösung:

Der Wirkstoff wird in einer Konzentration unterhalb der Sättigungslöslichkeit in einem physiologisch verträglichen Lösungsmittel (siehe Beispiele) gelöst. Die Lösung wird sterilfiltriert und in sterile und pyrogenfreie Injektions-/ Infusionsbehältnisse abgefüllt.

### Zusammensetzung I:

100 mg der Verbindung von Beispiel 11, 15 g Polyethylenglykol 400 und 250 g einer 2 %igen wässrigen Natriumhydrogencarbonat-Lösung für Injektionszwecke.

### Herstellung:

Die Verbindung von Beispiel 11 wird zusammen mit Polyethylenglykol 400 in der 2 %igen wässrigen Natriumhydrogencarbonat-Lösung unter Rühren gelöst. Die Lösung wird sterilfiltriert (Porendurchmesser - 0.22 µm) und unter aseptischen Bedingungen in hitzesterilisierte Infusionsflaschen abgefüllt. Diese werden mit Infusionsstopfen und Bördelkappen verschlossen.

### Zusammensetzung II:

100 mg der Verbindung von Beispiel 11 und 250 ml einer wässrigen Lösung von 0.31 g wasserfreier Zitronensäure und 5.66 g Natriummonohydrogenphosphat-Dihydrat.

### Herstellung:

Die Verbindung von Beispiel 11 wird in der wässrigen Lösung unter Rühren gelöst. Die Lösung wird sterilfiltriert und unter aseptischen Bedingungen in sterile und pyrogenfreie Injektions-/Infusionsbehältnisse abgefüllt.

### Zusammensetzung III:

100 mg der Verbindung von Beispiel 11 und 250 ml einer wässrigen Lösung von 0.044 g wasserfreier Zitronensäure, 0.81 g Natriummonohydrogenphosphat-Dihydrat und 1.87 g Natriumchlorid.

### Herstellung:

Die Verbindung von Beispiel 11 wird in der wässrigen Lösung unter Rühren gelöst. Die Lösung wird sterilfiltriert und unter aseptischen Bedingungen in sterile und pyrogenfreie Injektions-/Infusionsbehältnisse abgefüllt.

## Patentansprüche

1. Verbindung der Formel 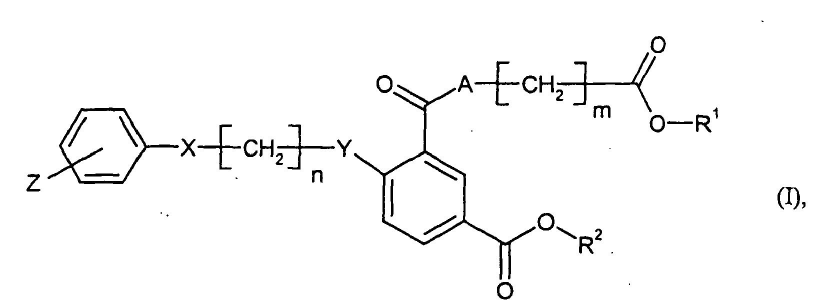 worin
A einen 4- bis 7-gliedrigen stickstoffhaltigen gesättigten Heterocyclus, der über das Stickstoffatom an die Ketogruppe gebunden ist und der gegebenenfalls in Nachbarschaft zu einem Stickstoffatom eine Carbonylgruppe trägt,
oder
einen Rest 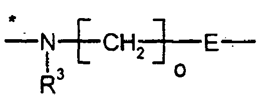 bedeutet,
worin
E für (C₃-C₇)-Cycloalkandiyl, (C₅-C₇)-Cycloalkendiyl oder für 5-bis 10-gliedriges Heterocyclyl, das über ein Kohlenstoffatom an die [CH₂]ₒ-Gruppe gebunden ist, steht,
o für 0, 1 oder 2 steht,
R³ für Wasserstoff oder (C₁-C₆)-Alkyl steht und
* für die Anknüpfstelle an die Ketogruppe steht,
m 0, 1 oder 2 bedeutet,
n 1, 2, 3 oder 4 bedeutet,
R¹ Wasserstoff oder (C₁-C₆)-Alkyl bedeutet,
R² Wasserstoff oder (C₁-C₆)-Alkyl bedeutet,
X eine Bindung, -CH=CH-, -C≡C- oder O bedeutet,
Y 0, *-NH-C(=O)- oder NH bedeutet,
worin
* für die Anknüpfstelle an den Phenylring steht
und
Z sich in meta oder para Stellung zum Substituenten X befindet und entweder (C₆-C₁₀)-Alkoxy, das 1 oder 2 weitere Sauerstoffatome in der Kette enthalten kann,
oder
einen Rest 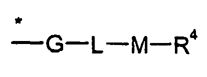 bedeutet,
worin
G für eine Bindung, O oder S steht,
L für (C₁-C₆)-Alkandiyl, (C₃-C₆)-Alkendiyl oder (C₃-C₆)-Alkindiyl steht,
M für eine Bindung, O oder S steht,
R⁴ für (C₆-C₁₀)-Aryl, Biphenyl, Phenoxyphenyl, Benzyloxyphenyl, (E)-Phenylvinylphenyl, 2-Phenylethylphenyl, Tetrahydronaphthyl, Benzyl, Heteroaryl, 5- bis 10-gliedriges Heterocyclyl, (C₃-C₇)-Cycloalkyl oder (C₃-C₇)-Cycloalkylmethyl steht, wobei Aryl, Biphenyl, Phenoxyphenyl, Benzyloxyphenyl, (E)-Phenylvinylphenyl, 2-Phenylethylphenyl, Tetrahydronaphthyl, Benzyl, Heteroaryl, Heterocyclyl, Cycloalkyl und Cycloalkylmethyl ihrerseits bis zu dreifach unabhängig voneinander durch Halogen, Cyano, Nitro, Trifluormethyl, Trifluormethoxy, (C₁-C₆)-Alkyl, (C₁-C₆)-Alkoxy, (C₂-C₆)-Alkenyl, (C₃-C₇)-Cycloalkyl, (C₃-C₇)-Cycloalkylmethoxy, (C₅-C₇)-Cycloalkenyl, (C₃-C₇)-Cycloalkoxy oder (C₅-C₇)-Cycloalkenyloxy substituiert sein können, und
* für die Anknüpfstelle an den Phenylring steht,
und ihre Salze, Hydrate, Hydrate der Salze und Solvate.

2. Verbindung der Formel (I) nach Anspruch 1,
worin
A einen 4- bis 6-gliedrigen stickstoffhaltigen gesättigten Heterocyclus, der über das Stickstoffatom an die Ketogruppe gebunden ist,
oder
einen Rest 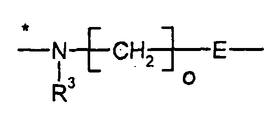 bedeutet,
worin
E für (C₅-C₆)-Cycloalkandiyl steht,
o für 0 oder 1 steht,
R³ für Wasserstoff steht und
* für die Anknüpfstelle an die Ketogruppe steht,
m 0 oder 1 bedeutet,
n 1, 2 oder 3 bedeutet,
R¹ Wasserstoff bedeutet,
R² Wasserstoff bedeutet,
X eine Bindung oder O bedeutet,
Y O oder *-NH-C(=O)- bedeutet,
worin
* für die Anknüpfstelle an den Phenylring steht
und
Z sich in meta oder para Stellung zum Substituenten X befindet und entweder (C₇-C₉)-Alkoxy, das 1 weiteres Sauerstoffatom in der Kette enthalten kann,
oder
einen Rest 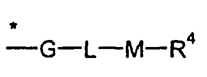 bedeutet,
worin
G für eine Bindung oder 0 steht,
L für (C₁-C₆)-Alkandiyl oder (C₃-C₆)-Alkendiyl steht,
M für eine Bindung, O oder S steht,
R⁴ für Phenyl, Naphthyl, Biphenyl, Phenoxyphenyl, Benzyloxyphenyl, (E)-Phenylvinylphenyl, 2-Phenylethylphenyl, Tetrahydronaphthyl, Benzyl, 1,3-Dioxanyl, 1,4-Dioxanyl, Dimethyl-1,3-dioxanyl, Tetrahydro-2H-pyranyl, (C₃-C₇)-Cycloalkyl oder (C₃-C₇)-Cycloalkylmethyl steht, wobei Phenyl, Naphthyl, Biphenyl, Phenoxyphenyl, Benzyloxyphenyl, (E)-Phenylvinylphenyl, 2-Phenylethylphenyl, Tetrahydronaphthyl, Benzyl, Cycloalkyl und Cycloalkylmethyl ihrerseits bis zu dreifach unabhängig voneinander durch Halogen, Cyano, Nitro, Trifluormethyl, Trifluormethoxy, (C₁-C₆)-Alkyl, (C₁-C₆)-Alkoxy, (C₃-C₇)-Cycloalkyl, (C₃-C₇)-Cycloalkylmethoxy oder (C₃-C₇)-Cycloalkoxy substituiert sein können, und
* für die Anknüpfstelle an den Phenylring steht,
und ihre Salze, Hydrate, Hydrate der Salze und Solvate.

3. Verbindung der Formel (I) nach Anspruch 1,
worin
A-[CH₂]ₘ-CO₂R¹
einen Rest 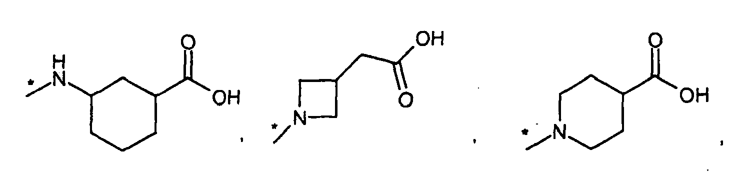 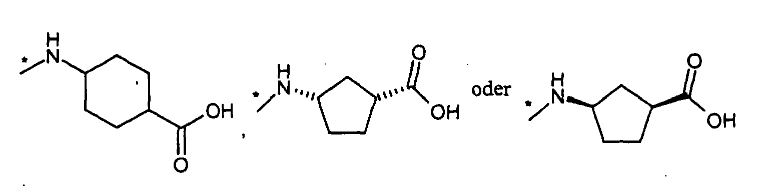 bedeutet,
worin
* für die Anknüpfstelle an die Ketogruppe steht,
n 3 bedeutet,
R² Wasserstoff bedeutet,
X eine Bindung bedeutet,
Y O bedeutet,
und
Z sich in para Stellung zum Substituenten X befindet und entweder n-Octyloxy, n-Heptyloxy,
oder
einen Rest 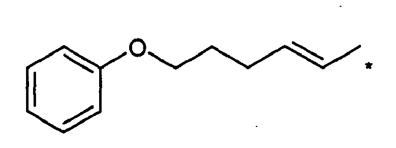 worin
* fiir die Anknüpfstelle an den Phenylring steht
oder
einen Rest 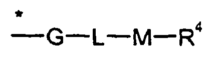 bedeutet,
worin
G für O steht,
L für Methandiyl, n-Propandiyl oder n-Butandiyl steht,
M für eine Bindung oder O steht,
R⁴ für Phenyl, 4-Biphenyl, 4-Phenoxyphenyl, 4-Benzyloxyphenyl, 1,2,3,4-Tetrahydronaphth-6-yl, 5,5-Dimethyl-1,3-dioxan-2-yl oder Cyclohexyl steht, wobei Phenyl seinerseits einmal durch Halogen, Trifluormethoxy, (C₃-C₄)-Alkyl, (C₃-C₄)-Alkoxy, Cyclopentyl, Cyclohexyl oder (C₃-C₆)-Cycloalkylmethoxy substituiert sein kann, und
* für die Anknüpfstelle an den Phenylring steht,
und ihre Salze, Hydrate, Hydrate der Salze und Solvate.

4. Verbindung der Formel (I) nach Anspruch 1,
worin
A-[CH₂]ₘ-CO₂R¹
einen Rest 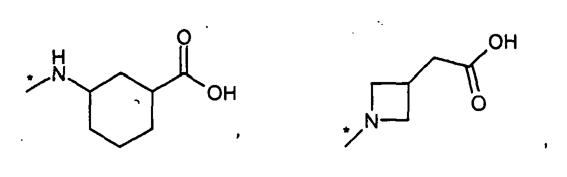 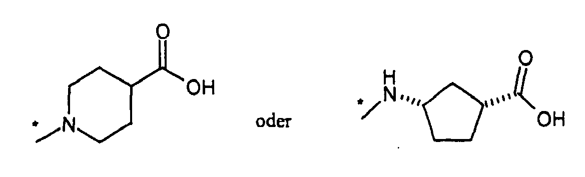 bedeutet,
worin
* für die Anknüpfstelle an die Ketogruppe steht,
n 3 bedeutet,
R² Wasserstoff bedeutet,
X eine Bindung bedeutet,
Y O bedeutet,
und
Z sich in para Stellung zum Substituenten X befindet und entweder n-Octyloxy, n-Heptyloxy,
oder
einen Rest 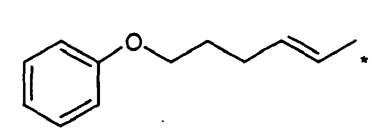 worin
* für die Anknüpfstelle an den Phenylring steht
oder
einen Rest
*-O-CH₂-R⁴,
worin
R⁴ für Phenyl, 4-Biphenyl, 4-Phenoxyphenyl, 4-Benzyloxyphenyl oder 1,2,3,4-Tetrahydronaphth-6-yl, wobei Phenyl seinerseits einmal durch Trifluormethoxy, n-Propyl, n-Butyl, tert-Butyl, n-Propyloxy, iso-Propyloxy, iso-Butyloxy, Cyclohexyl oder Cyclopropylmethoxy substituiert sein kann, und
* für die Anknüpfstelle an den Phenylring steht,
oder
einen Rest
*-O-CH₂-CH₂-CH₂-R⁴,
worin
R⁴ für 4-Chlorphenyl, 5,5-Dimethyl-1,3-dioxan-2-yl oder Cyclohexyl steht und
* für die Anknüpfstelle an den Phenylring steht,
oder
einen Rest
*-O-CH₂-CH₂-CH₂-CH₂-O-R⁴
bedeutet,
worin
R⁴ für Phenyl oder Cyclohexyl steht und
* für die Anknüpfstelle an den Phenylring steht,
und ihre Salze, Hydrate, Hydrate der Salze und Solvate.

5. Verbindung der Formel (I) nach Anspruch 1,
worin
A-[CH₂]ₘ-CO₂R¹
einen Rest 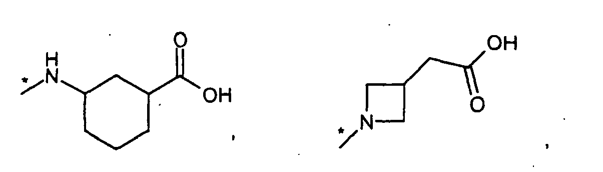 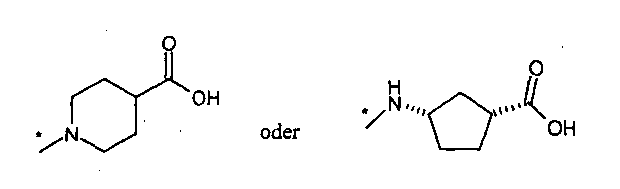 bedeutet,
worin
* für die Anknüpfstelle an die Ketogruppe steht,
n 3 bedeutet,
R² Wasserstoff bedeutet,
X eine Bindung bedeutet,
Y O bedeutet,
und
Z sich in para Stellung zum Substituenten X befindet und einen Rest 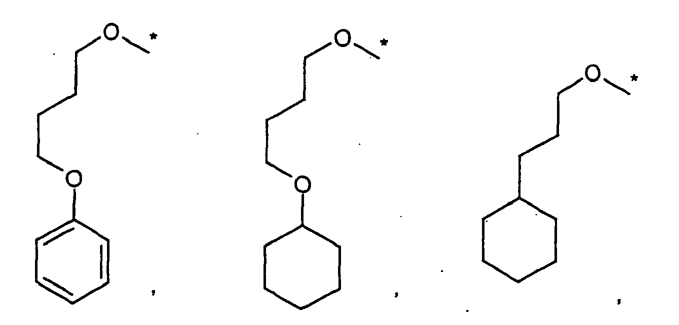 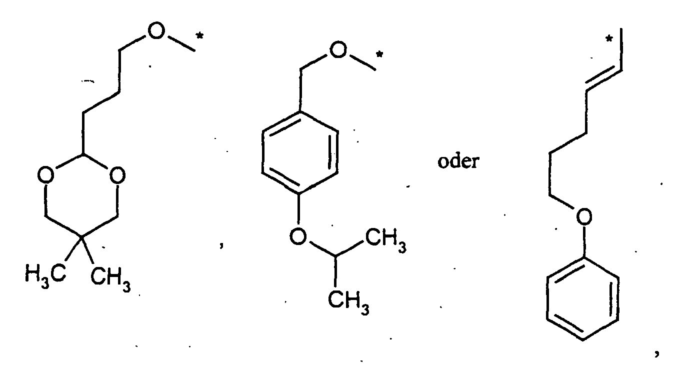 bedeutet,
worin
* für die Anlmüpfstelle an den Phenylring steht,
und ihre Salze, Hydrate, Hydrate der Salze und Solvate.

6. Verbindung der Formel (I) nach Anspruch 1:
3-{[(3-Carboxycyclohexyl)amino]carbonyl]-4-{3-[4-(4-phenoxybutoxy)-phenyl]-propoxy}benzoesäure,
3-{[(3-Carboxycyclohexyl)amino]carbonyl}-4-{3-[4-(3-cyclohexylpropoxy)-phenyl]propoxyl}benzoesäure,
3-{[(3-Carboxycyclohexyl)amino]carbonyl}-4-(3-{4-[4-(cyclohexyloxy)-butoxy]phenyl}propoxy)benzoesäure,
1-(5-Carboxy-2-{3-[4-(3-cylohexylpropoxy)phenyl]propoxy}benzoyl)-piperidin-4-carbonsäure,
3-{[(3-Carboxycyclohexyl)amino]carbonyl}-4-(3-{4-[(4-isopropoxybenzyl)-oxy]phenyl}propoxy)benzoesäure,
3-{[3-(Carboxymethyl)azetidin-1-yl]carbonyl}-4-{3-[4-(3-cyclohexylpropoxy)phenyl]-propoxy}benzoesäure oder
3-{[(3-Carboxycyclohexyl)amino]carbonyl}-4-(3-{4-[(1E)-5-phenoxypent-1-en-1-yl]-phenyl}propoxy)benzoesäure
und ihre Salze, Hydrate, Hydrate der Salze und Solvate.

7. Verfahren zur Herstellung von Verbindungen der Formel (I), wie in Anspruch 1 definiert, **dadurch gekennzeichnet, dass** man
entweder
[**A**] Verbindungen der Formel (II) 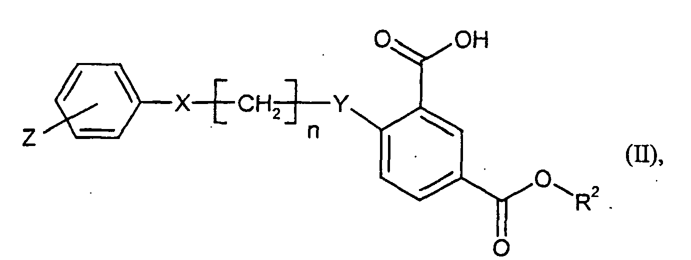 worin
R² für (C₁-C₆)-Alkyl steht und
n, X, Y und Z die in Anspruch 1 angegebene Bedeutung haben,
mit Verbindungen der Formel (III) 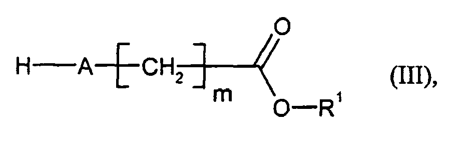 worin
R¹ für (C₁-C₆)-Alkyl steht und
m und A die in Anspruch 1 angegebene Bedeutung haben,
oder
[**B1**] Verbindungen der Formel (IVa) 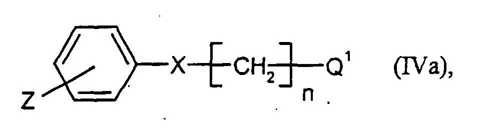 worin
Q¹ für eine Abgangsgruppe steht und
n, X, und Z die in Anspruch 1 angegebene Bedeutung haben,
mit Verbindungen der Formel (Va) 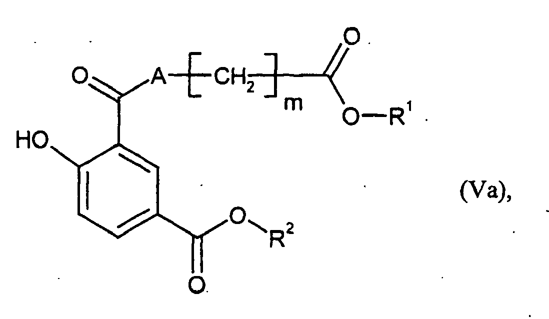 worin
R¹ und R² für (C₁-C₆)-Alkyl stehen und
A und m die in Anspruch 1 angegebene Bedeutung haben,
oder
[**B2**] Verbindungen der Formel (IVb) 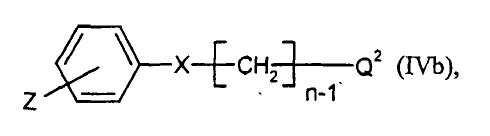 worin
Q² für eine Säurechloridgruppe steht und
n, X, und Z die in Anspruch 1 angegebene Bedeutung haben,
mit Verbindungen der Formel (Vb) 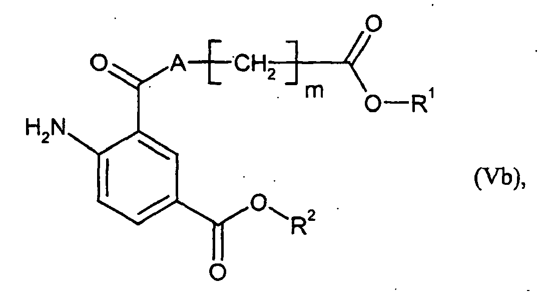 worin
R¹ und R² für (C₁-C₆)-Alkyl stehen und
A und m die in Anspruch 1 angegebene Bedeutung haben,
oder
[**B3**] Verbindungen der Formel (IVa) 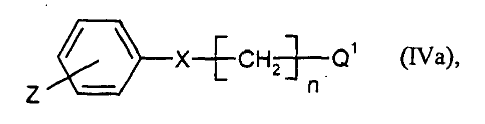 worin
Q¹ für eine Abgangsgruppe steht und
n, X, und Z die in Anspruch 1 angegebene Bedeutung haben,
mit Verbindungen der Formel (Vb) 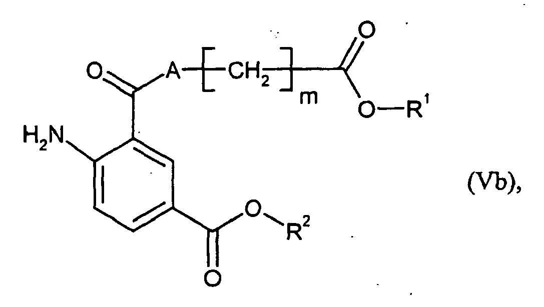 worin
R¹ und R² für (C₁-C₆)-Alkyl stehen und
A und m die in Anspruch 1 angegebene Bedeutung haben,
oder
[**C**] Verbindungen der Formel (XII) 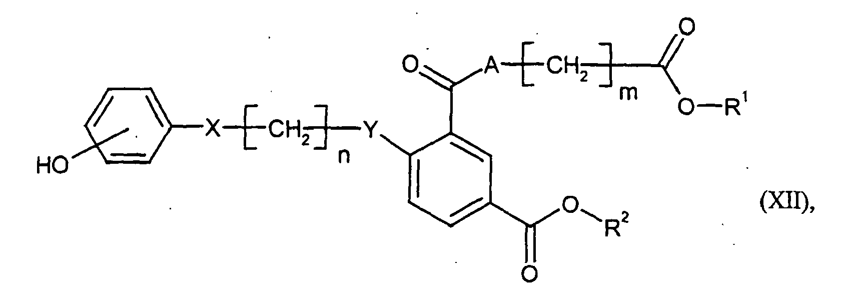 worin
R¹ und R² für (C₁-C₆)-Alkyl stehen und
n, m, X, Y und A die in Anspruch 1 angegebene Bedeutung haben,
mit Verbindungen der Formel (XIII)
R⁴-M-L-Q³ (XIII),
worin
Q³ für eine Abgangsgruppe steht und
R⁴, M und L die in Anspruch 1 angegebene Bedeutung haben,
umsetzt
oder
[**D**] in Verbindungen, die gemäß Verfahrensschritt [A], [B1], [B2], [B3] oder [C] hergestellt wurden, die beiden Estergruppen verseift.

8. Verbindung der Formel (I), wie in Anspruch 1 definiert, zur Behandlung und/oder Prophylaxe von Erkrankungen.

9. Arzneimittel enthaltend mindestens eine Verbindung der Formel (I), wie in Anspruch 1 definiert, und mindestens einen weiteren Hilfsstoff.

10. Arzneimittel enthaltend mindestens eine Verbindung der Formel (I), wie in Anspruch 1 definiert, und mindestens einen weiteren Wirkstoff.

11. Verwendung von Verbindungen der Formel (I), wie in Anspruch 1 definiert, zur Herstellung von Arzneimitteln zur Behandlung und/oder Prophylaxe von kardiovaskulären Erkrankungen.

12. Verwendung nach Anspruch 11 zur Behandlung und/oder Prophylaxe von instabiler Angina pectoris oder Myokardinfarkt.

## Claims

1. Compound of the formula 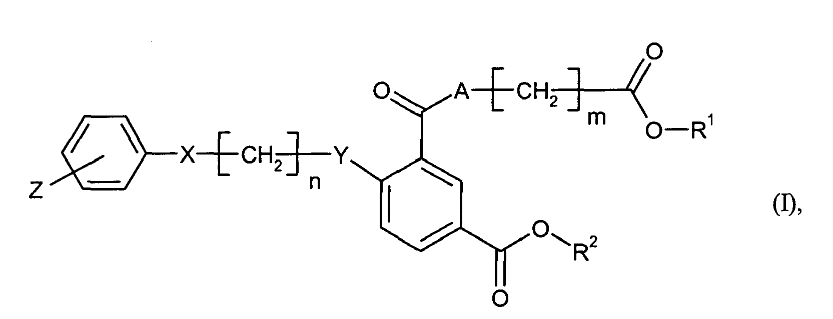 in which
A is a 4- to 7-membered nitrogen-containing saturated heterocycle which is bonded via the nitrogen atom to the keto group and which optionally has a carbonyl group adjacent to a nitrogen atom,
or
a radical 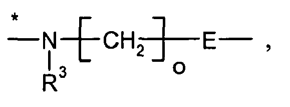 in which
E is (C₃-C₇)-cycloalkanediyl, (C₅-C₇)-cycloalkenediyl or is 5- to 10-membered heterocyclyl which is bonded via a carbon atom to the [CH₂]ₒ group,
o is 0, 1 or 2,
R³ is hydrogen or (C₁-C₆)-alkyl, and
* is the point of linkage to the keto group,
m 0, 1 or 2,
n is 1, 2, 3 or 4,
R¹ is hydrogen or (C₁-C₆)-alkyl,
R² is hydrogen or (C₁-C₆)-alkyl,
X is a bond, -CH=CH-, -C≡C- or O,
Y is O, *-NH-C(=O)- or NH,
in which
* is the point of linkage to the phenyl ring,
and
Z is located in the position meta or para to the substituent X and is either (C₆-C₁₀)-alkoxy which may comprise 1 or 2 further oxygen atoms in the chain,
or
a radical 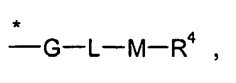 in which
G is a bond, O or S,
L is (C₁-C₆)-alkanediyl, (C₃-C₆)-alkenediyl or (C₃-C₆)-alkynediyl,
M is a bond, O or S,
R⁴ is (C₆-C₁₀)-aryl, biphenyl, phenoxyphenyl, benzyloxyphenyl, (E)-phenylvinylphenyl, 2-phenylethylphenyl, tetrahydronaphthyl, benzyl, heteroaryl, 5- to 10-membered heterocyclyl, (C₃-C₇)-cycloalkyl or (C₃-C₇)-cycloalkylmethyl, where aryl, biphenyl, phenoxyphenyl, benzyloxyphenyl, (E)-phenylvinylphenyl, 2-phenylethylphenyl, tetrahydronaphthyl, benzyl, heteroaryl, heterocyclyl, cycloalkyl and cycloalkylmethyl in turn may be substituted up to three times independently of one another by halogen, cyano, nitro, trifluoromethyl, trifluoromethoxy, (C₁-C₆)-alkyl, (C₁-C₆)-alkoxy, (C₂-C₆)-alkenyl, (C₃-C₇)-cycloalkyl, (C₃-C₇)-cycloalkylmethoxy, (C₅-C₇)-cycloalkenyl, (C₃-C₇)-cycloalkoxy or (C₅-C₇)-cycloalkenyloxy, and
* is the point of linkage to the phenyl ring,
and the salts, hydrates, hydrates of the salts and solvates thereof.

2. Compound of the formula (I) according to Claim 1,
in which
A is a 4- to 6-membered nitrogen-containing saturated heterocycle which is bonded via the nitrogen atom to the keto group,
or
a radical 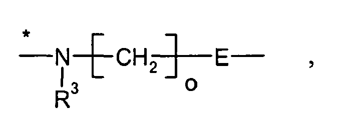 in which
E is (C₅-C₆)-cycloalkanediyl,
o is 0 or 1,
R³ is hydrogen, and
* is the point of linkage to the keto group,
m is 0 or 1,
n is 1, 2 or 3,
R¹ is hydrogen,
R² is hydrogen,
X is a bond or O,
Y is O or *-NH-C(=O)-,
in which
* is the point of linkage to the phenyl ring,
and
Z is located in the position meta or para to the substituent X and is either (C₇-C₉)-alkoxy, which may comprise 1 further oxygen atom in the chain,
or
a radical 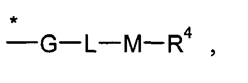 in which
G is a bond or O,
L is (C₁-C₆)-alkanediyl or (C₃-C₆)-alkenediyl,
M is a bond, O or S,
R⁴ is phenyl, naphthyl, biphenyl, phenoxyphenyl, benzyloxyphenyl, (E)-phenylvinylphenyl, 2-phenylethylphenyl, tetrahydronaphthyl, benzyl, 1,3-dioxanyl, 1,4-dioxanyl, dimethyl-1,3-dioxanyl, tetrahydro-2H-pyranyl, (C₃-C₇)-cycloalkyl or (C₃-C₇)-cycloalkylmethyl, where phenyl, naphthyl, biphenyl, phenoxyphenyl, benzyloxyphenyl, (E)-phenylvinylphenyl, 2-phenylethylphenyl, tetrahydronaphthyl, benzyl, cycloalkyl and cycloalkylmethyl in turn may be substituted up to three times independently of one another by halogen, cyano, nitro, trifluoromethyl, trifluoromethoxy, (C₁-C₆)-alkyl, (C₁-C₆)-alkoxy, (C₃-C₇)-cycloalkyl, (C₃-C₇)-cycloalkylmethoxy or (C₃-C₇)-cycloalkoxy, and
* is the point of linkage to the phenyl ring,
and the salts, hydrates, hydrates of the salts and solvates thereof.

3. Compound of the formula (I) according to Claim 1,
in which
A-[CH₂]ₘ-CO₂R¹
is a radical 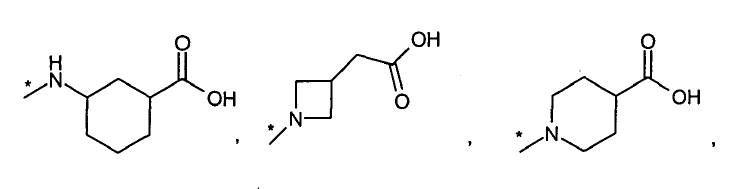 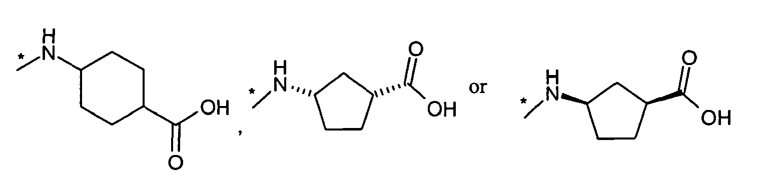 in which
* is the point of linkage to the keto group,
n is 3,
R² is hydrogen,
X is a bond,
Y is O,
and
Z is located in the position para to the substituent X and is either n-octyloxy, n-heptyloxy,
or
a radical 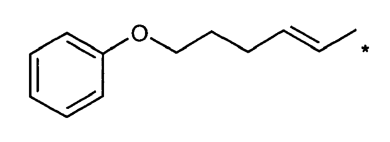 in which
* is the point of linkage to the phenyl ring,
or
a radical 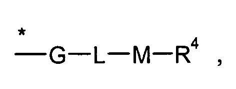 in which
G is O,
L is methanediyl, n-propanediyl or n-butanediyl,
M is a bond or O,
R⁴ is phenyl, 4-biphenyl, 4-phenoxyphenyl, 4-benzyloxyphenyl, 1,2,3,4-tetrahydronaphth-6-yl, 5,5-dimethyl-1,3-dioxan-2-yl or cyclohexyl, where phenyl in turn may be substituted once by halogen, trifluoromethoxy, (C₃-C₄)-alkyl, (C₃-C₄)-alkoxy, cyclopentyl, cyclohexyl or (C₃-C₆)-cycloalkylmethoxy, and
* is the point of linkage to the phenyl ring,
and the salts, hydrates, hydrates of the salts and solvates thereof.

4. Compound of the formula (I) according to Claim 1,
in which
A-[CH₂]ₘ-CO₂R¹
is a radical 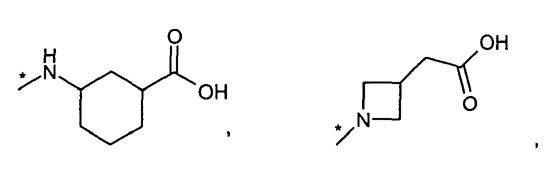 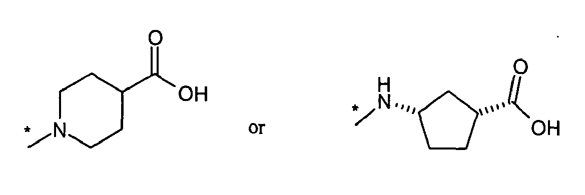 in which
* is the point of linkage to the keto group,
n is 3,
R² is hydrogen,
X is a bond,
Y is O,
and
Z is located in the position para to the substituent X, and is either n-octyloxy, n-heptyloxy,
or
a radical 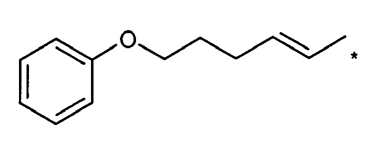 in which
* is the point of linkage to the phenyl ring,
or
a radical
*-O-CH₂-R⁴,
in which
R⁴ is phenyl, 4-biphenyl, 4-phenoxyphenyl, 4-benzyloxyphenyl or 1,2,3,4-tetrahydronaphth-6-yl, where phenyl in turn may be substituted once by trifluoromethoxy, n-propyl, n-butyl, tert-butyl, n-propyloxy, isopropyloxy, isobutyloxy, cyclohexyl or cyclopropylmethoxy, and
* is the point of linkage to the phenyl ring,
or
a radical
*-O-CH₂-CH₂-CH₂-R⁴,
in which
R⁴ is 4-chlorophenyl, 5,5-dimethyl-1,3-dioxan-2-yl or cyclohexyl, and
* is the point of linkage to the phenyl ring,
or
a radical
*-O-CH₂-CH₂-CH₂-CHz-O-R⁴,
in which
R⁴ is phenyl or cyclohexyl, and
* is the point of linkage to the phenyl ring,
and the salts, hydrates, hydrates of the salts and solvates thereof.

5. Compound of the formula (I) according to Claim 1,
in which
A-[CH₂]ₘ- CO₂R¹
is a radical 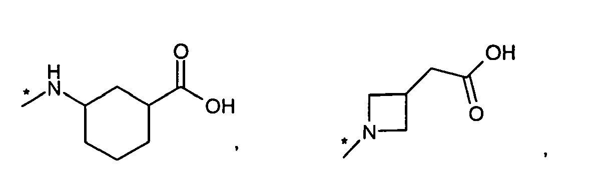 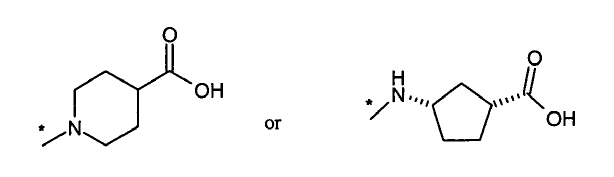 in which
* is the point of linkage to the keto group,
n is 3,
R² is hydrogen,
X is a bond,
Y is O,
and
Z is located in the position para to the substituent X and is a radical 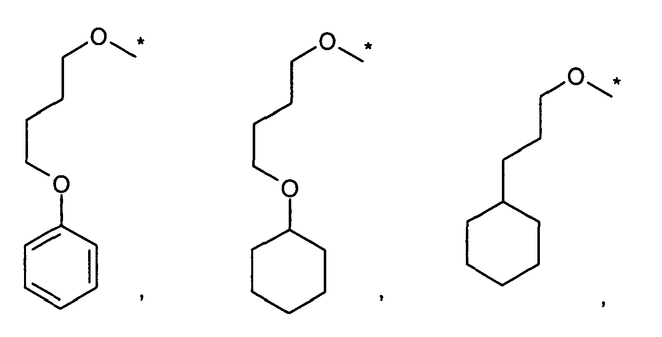 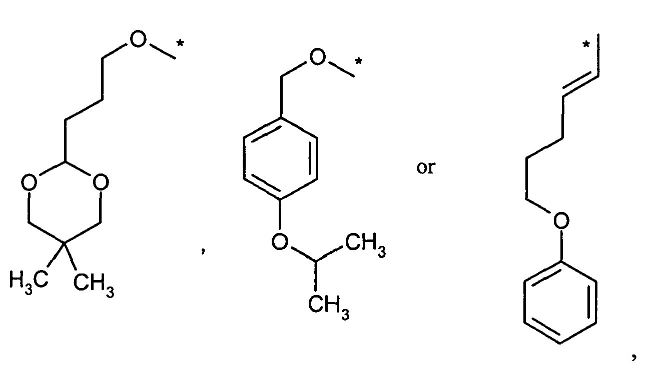 in which
* is the point of linkage to the phenyl ring,
and the salts, hydrates, hydrates of the salts and solvates thereof.

6. Compound of the formula (I) according to Claim 1:
3-{[(3-carboxycyclohexyl)amino]carbonyl}-4-{3-[4-(4-phenoxybutoxy)-phenyl]propoxy}benzoic acid,
3-{[(3-carboxycyclohexyl)amino]carbonyl}-4-{3-[4-(3-cyclohexylpropoxy)-phenyl]propoxy}benzoic acid,
3-{[(3-carboxycyclohexyl)amino]carbonyl}-4-(3-{4-[4-(cyclohexyloxy)-butoxy]phenyl}propoxy)benzoic acid,
1-(5-carboxy-2-{3-[4-(3-cyclohexylpropoxy)phenyl]propoxy}benzoyl)-piperidine-4-carboxylic acid,
3-{[(3-carboxycyclohexyl)amino]carbonyl}-4-(3-{4-[(4-isopropoxybenzyl)-oxy]phenyl}propoxy)benzoic acid,
3-{[3-(carboxymethyl)azetidin-1-yl]carbonyl}-4-{3-[4-(3-cyclohexylpropoxy)phenyl]propoxy}benzoic acid or
3-{[(3-carboxycyclohexyl)amino]carbonyl}-4-(3-{4-[(1E)-5-phenoxypent-1-en-1-yl]phenyl}propoxy)benzoic acid
and the salts, hydrates, hydrates of the salts and solvates thereof.

7. Process for preparing compounds of the formula (I) as defined in Claim 1, **characterized in that**
either
[**A**] compounds of the formula (II) 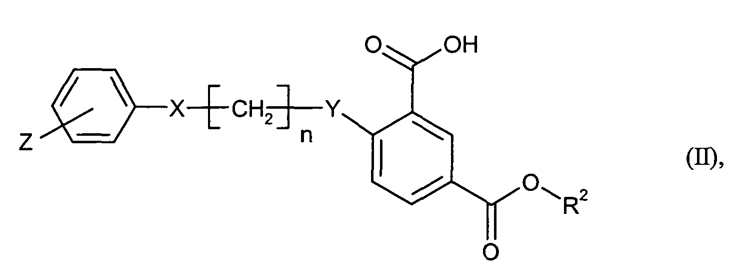 in which
R² is (C₁-C₆)-alkyl and
n, X, Y and Z have the meaning indicated in Claim 1,
are reacted with compounds of the formula (III) 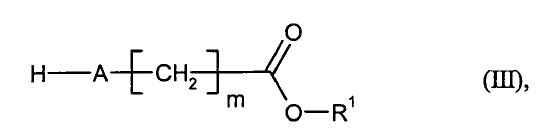 in which
R¹ is (C₁-C₆)-alkyl, and
m and A have the meaning indicated in Claim 1,
or
[**B1**] compounds of the formula (IVa) 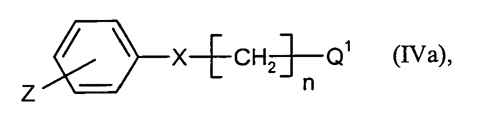 in which
Q¹ is a leaving group and
n, X and Z have the meaning indicated in Claim 1,
are reacted with compounds of the formula (Va) 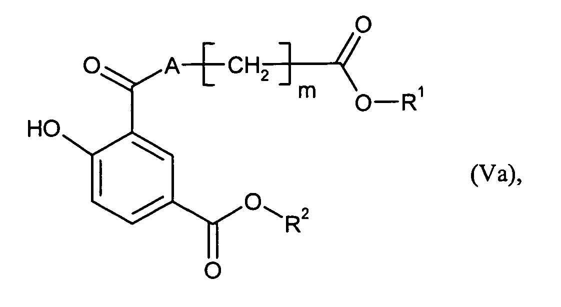 in which
R¹ and R² are (C₁-C₆)-alkyl, and
A and m have the meaning indicated in Claim 1,
or
[**B2**] compounds of the formula (IVb) 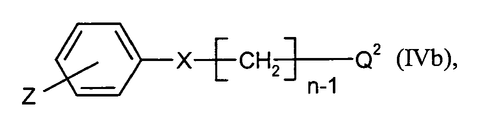 in which
Q² is an acid chloride group, and
n, X and Z have the meaning indicated in Claim 1,
are reacted with compounds of the formula (Vb) 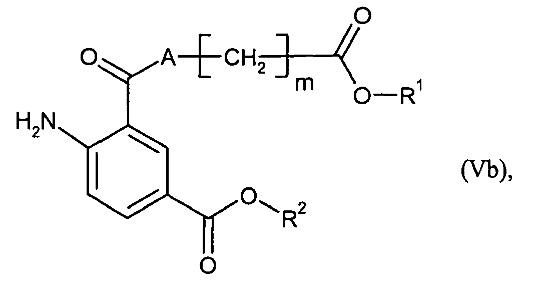 in which
R¹ and R² are (C₁-C₆)-alkyl, and
A and m have the meaning indicated in Claim 1,
or
[**B3**] compounds of the formula (IVa) 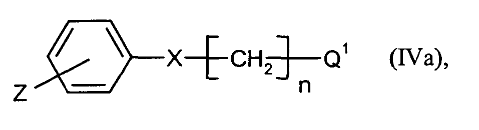 in which
Q¹ is a leaving group and
n, X and Z have the meaning indicated in Claim 1,
are reacted with compounds of the formula (Vb) 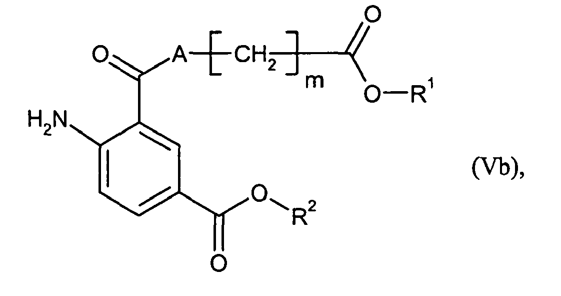 in which
R¹ and R² are (C₁-C₆)-alkyl, and
A and m have the meaning indicated in Claim 1,
or
[**C**] compounds of the formula (XII) 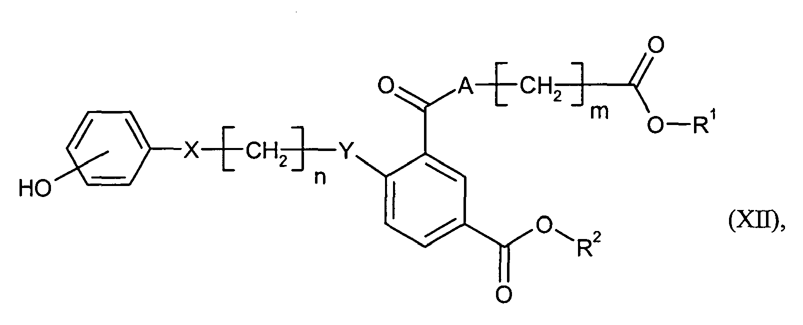 in which
R¹ and R² are (C₁-C₆)-alkyl, and
n, m, X, Y and A have the meaning indicated in Claim 1,
are reacted with compounds of the formula (XIII)
R⁴-M-L-Q³ (XIII),
in which
Q³ is a leaving group and
R⁴, M and L have the meaning indicated in Claim 1,
or
[**D**] the two ester groups in compounds prepared by process step [A], [B1], [B2], [B3] or [C] are hydrolysed.

8. Compound of the formula (I) as defined in Claim 1 for the treatment and/or prophylaxis of disorders.

9. Medicament comprising at least one compound of the formula (I) as defined in Claim 1 and at least one further excipient.

10. Medicament comprising at least one compound of the formula (I) as defined in Claim 1 and at least one further active ingredient.

11. Use of compounds of the formula (I) as defined in Claim 1 for producing medicaments for the treatment and/or prophylaxis of cardiovascular disorders.

12. Use according to Claim 11 for the treatment and/or prophylaxis of unstable angina pectoris or myocardial infarction.

## Revendications

1. Composé de formule 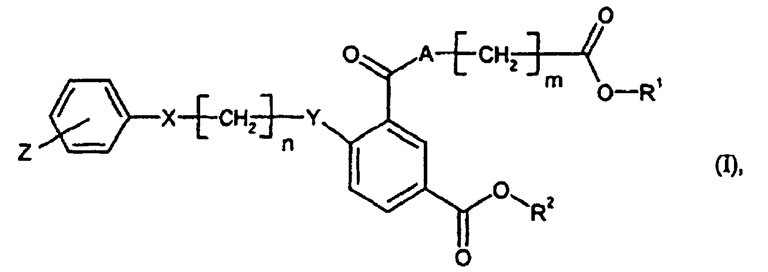 dans laquelle
A représente un hétérocycle saturé tétra- à heptagonal contenant de l'azote, qui est lié au groupe céto par l'intermédiaire de l'atome d'azote et qui porte éventuellement un groupe carbonyle au voisinage d'un atome d'azote,
ou bien
un reste 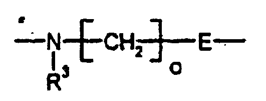 dans lequel
E représente un reste cycloalcanediyle en C₃ à C₇, cycloalcènediyle en C₅ à C₇ ou un hétérocycle penta- à décagonal qui est lié au groupe [CH₂]ₒ par l'intermédiaire d'un atome de carbone,
o a la valeur 0, 1 ou 2,
R³ représente l'hydrogène ou un reste alkyle en C₁ à C₆ et
* indique le site de rattachement au groupe céto,
m a la valeur 0, 1 ou 2,
n a la valeur 1, 2, 3 ou 4,
R¹ représente l'hydrogène ou un reste alkyle en C₁ à C₆,
R² représente l'hydrogène ou un reste alkyle en C₁ à C₆,
X désigne une liaison, un groupe -CH=CH-, -C≡C- ou O,
Y représente O, un groupe *-NH-C(=O)- ou NH,
où
* désigne le site de rattachement au noyau phényle
et
Z se trouve en position méta ou para par rapport au substituant X et désigne ou bien un reste alkoxy en C₆ à C_{10,} qui peut contenir 1 ou 2 autres atomes d'oxygène dans sa chaîne,
ou bien
un reste 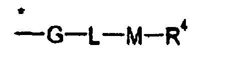 dans lequel
G désigne une liaison, O ou S,
L représente un groupe alcanediyle en C₁ à C₆, alcènediyle en C₃ à C₆ ou alcynediyle en C₃ à C₆,
M représente une liaison, O ou S,
R⁴ représente un reste aryle en C₆ à C₁₀, biphényle, phénoxyphényle, benzyloxyphényle, (E)-phénylvinylphényle, 2-phényléthylphényle, tétrahydronaphtyle, benzyle, hétéroaryle, hétérocyclyle penta- à décagonal, cycloalkyle en C₃ à C₇ ou (cycloalkyle en C₃ à C₇) méthyle, les restes aryle, biphényle, phénoxyphényle, benzyloxyphényle, (E)-phénylvinylphényle, 2-phényléthylphényle, tétrahydronaphtyle, benzyle, hétéroaryle, hétérocyclyle, cycloalkyle et cycloalkylméthyle pouvant eux-mêmes porter jusqu'à trois substituants, indépendamment les uns des autres, halogéno, cyano, nitro, trifluorométhyle, trifluorométhoxy, alkyle en C₁ à C₆, alkoxy en C₁ à C₆, alcényle en C₂ à C₆, cycloalkyle en C₃ à C₇, (cycloalkyle en C₃ à C₇)méthoxy, cycloalcényle en C₅ à C₇, cycloalkoxy en C₃ à C₇ ou cycloalcényloxy en C₅ à C₇,
et
* représente le site de rattachement au noyau phényle,
et ses sels, ses hydrates, les hydrates des sels et ses produits de solvatation.

2. Composé de formule (I) suivant la revendication 1, dans laquelle
A représente un hétérocycle saturé tétra- à hexagonal contenant de l'azote, qui est lié au groupe céto par l'intermédiaire de l'atome d'azote,
ou bien
un reste 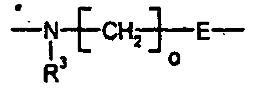 dans lequel
E désigne un reste cycloalcanediyle en C₅ ou C₆,
o a la valeur 0 ou 1,
R³ représente l'hydrogène et
* désigne le site de rattachement au groupe céto,
m a la valeur 0 ou 1,
n a la valeur 1, 2 ou 3,
R¹ représente l'hydrogène,
R² représente l'hydrogène,
X représente une liaison ou O,
Y représente O ou un groupe *-NH-C(=O)-,
où
* désigne le site de rattachement au noyau phényle
et
Z se trouve en position méta ou para par rapport au substituant X et représente ou bien un reste alkoxy en C₇ à C₉, qui peut contenir dans sa chaîne 1 autre atome d'oxygène,
ou bien
un reste 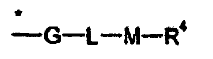 dans lequel
G désigne une liaison ou 0,
L représente un reste alcanediyle en C₁ à C₆ ou alcènediyle en C₃ à C₆,
M représente une liaison, O ou S,
R⁴ représente un reste phényle, naphtyle, biphényle, phénoxyphényle, benzyloxyphényle, (E)-phénylvinylphényle, 2-phényléthylphényle, tétrahydronaphtyle, benzyle, 1,3-dioxannyle, 1,4-dioxannyle, diméthyl-1,3-dioxannyle, tétrahydro-2H-pyrannyle, cycloalkyle en C₃ à C₇ ou (cycloalkyle en C₃ à C₇)méthyle, les restes phényle, naphtyle, biphényle, phénoxyphényle, benzyloxyphényle, (E)-phénylvinylphényle, 2-phényléthylphényle, tétrahydronaphtyle, benzyle, cycloalkyle et cycloalkylméthyle pouvant porter eux-mêmes jusqu'à trois substituants, indépendamment les uns des autres, halogéno, cyano, nitro, trifluorométhyle, trifluorométhoxy, alkyle en C₁ à C₆, alkoxy en C₁ à C₆, cycloalkyle en C₃ à C₇, (cycloalkyle en C₃ à C₇)méthoxy ou cycloalkoxy en C₃ à C₇,
et
* désigne le site de rattachement au noyau phényle,
et ses sels, ses hydrates, les hydrates des sels et ses produits de solvatation.

3. Composé de formule (I) suivant la revendication 1, dans lequel
A-[CH₂]ₘ-CO₂R¹
représente un reste 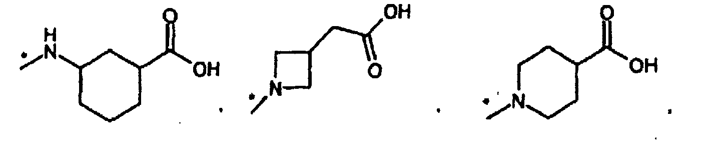 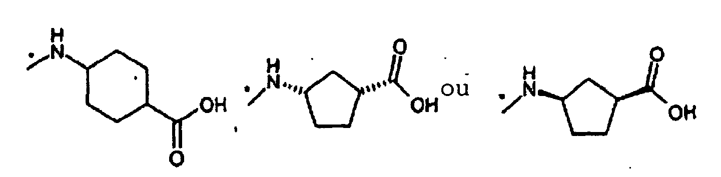 dans lequel
* désigne le site de rattachement au groupe céto,
n est égal à 3,
R² représente l'hydrogène,
X représente une liaison,
Y représente O,
et
Z se trouve en position para par rapport au substituant X et représente ou bien un reste n-octyloxy, n-heptyloxy,
ou bien
un reste 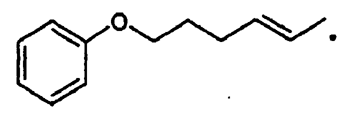 dans lequel
* désigne le site de rattachement au noyau phényle,
ou bien
un reste 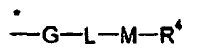 dans lequel
G représente O,
L représente un reste méthanediyle, n-propanediyle ou n-butanediyle,
M représente une liaison ou O,
R⁴ est un reste phényle, 4-biphényle, 4-phénoxyphényle, 4-benzyloxyphényle, 1,2,3,4-tétrahydronapht-6-yle, 5,5-diméthyl-1,3-dioxanne-2-yle ou cyclohexyle, le reste phényle pouvant lui-même porter un substituant halogéno, trifluorométhoxy, alkyle en C₃ ou C₄, alkoxy en C₃ ou C₄, cyclopentyle, cyclohexyle ou (cycloalkyle en C₃ à C₆)méthoxy, et
* désigne le site de rattachement au noyau phényle,
et ses sels, ses hydrates, les hydrates des sels et ses produits de solvatation.

4. Composé de formule (I) suivant la revendication 1, dans lequel
A-[CH₂]ₘCO₂R¹
représente un reste 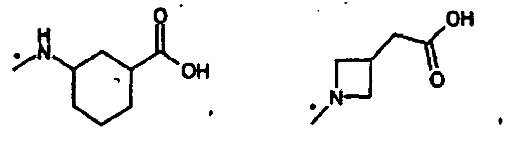 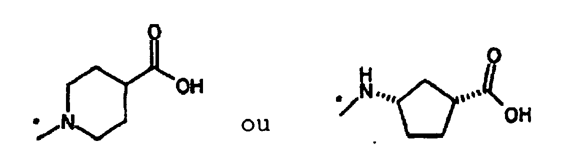 dans lequel
* désigne le site de rattachement au groupe céto,
n est égal à 3,
R² représente l'hydrogène,
X représente une liaison,
Y représente 0,
et
Z se trouve en position para par rapport au substituant X et représente ou bien un reste n-octyloxy, n-heptyloxy,
ou bien
un reste 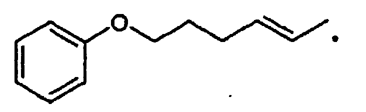 dans lequel
* désigne le site de rattachement au noyau phényle,
ou bien
un reste
*-O-CH₂-R⁴,
dans lequel
R⁴ représente un reste phényle, 4-biphényle, 4-phénoxyphényle, 4-benzyloxyphényle ou 1,2,3,4-tétrahydronapht-6-yle, le reste phényle pouvant lui-même porter un substituant trifluorométhoxy, n-propyle, n-butyle, tertiobutyle, n-propyloxy, isopropyloxy, isobutyloxy, cyclohexyle ou cyclopropylméthoxy, et
* désigne le site de rattachement au noyau phényle,
ou bien
un reste
*-O-CH₂-CH₂-CH₂-R⁴,
dans lequel
R⁴ représente un reste 4-chlorophényle, 5,5-diméthyl-1,3-dioxanne-2-yle ou cyclohexyle et
* désigne le site de rattachement au noyau phényle,
ou bien
un reste
*-O-CH₂-CH₂-CH₂-CH₂-O-R⁴,
dans lequel
R⁴ représente un reste phényle ou cyclohexyle et
* désigne le site de rattachement au noyau phényle,
et ses sels, ses hydrates, les hydrates des sels et ses produits de solvatation.

5. Composé de formule (I) suivant la revendication 1, dans lequel
A-[CH₂]ₘ-CO₂R¹
représente un reste 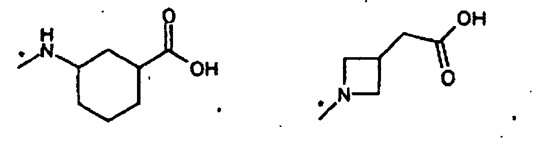 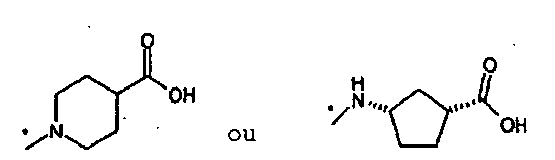 dans lequel
* désigne le site de rattachement au groupe céto,
n est égal à 3,
R² représente l'hydrogène,
X représente une liaison,
Y représente O,
et
Z se trouve en position para par rapport au substituant X et représente un reste 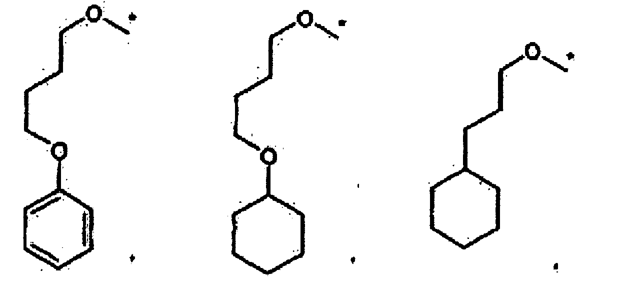 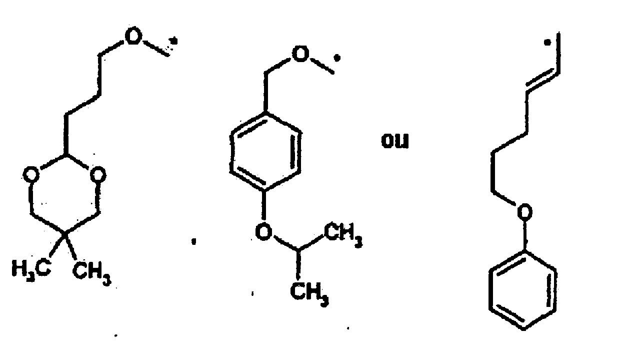 dans lequel
* désigne le site de rattachement au noyau phényle,
et ses sels, ses hydrates, les hydrates des sels et ses produits de solvatation.

6. Composé de formule (I) suivant la revendication 1 :
acide 3-{[(3-carboxycyclohexyl)amino]carbonyl}-4-{3-[4-(4-phénoxybutoxy)-phényl]-propoxy}benzoïque,
acide 3-{[(3-carboxycyclohexyl)amino]carbonyl}-4-{3-[4-(3-cyclohexylpropoxy)-phényl]-propoxy}benzoïque,
acide 3-{[(3-carboxycyclohexyl)amino]carbonyl}-4-(3-{4-[4-(cyclohexyloxy)-butoxy]-phényl}-propoxy)benzoïque,
acide 1-(5-carboxy-2-{3-[4-(3-cyclohexylpropoxy)phényl]-propoxy}benzoyl)-pipéridine-4-carboxylique,
acide 3-{[(3-carboxycyclohexyl)amino]carbonyl}-4-(3-{4-[(4-isopropoxybenzyl)-oxy]phényl}-propoxy)benzoïque,
acide 3-{[(3-carboxyméthyl)azétidine-1-yl]carbonyl}-4-{3-[4-(3-cyclohexylpropoxy)phényl]-propoxy}benzoïque ou
acide 3-{[3-(carboxycyclohexyl)amino]carbonyl}-4-(3-{4-[(1E)-5-phénoxypent-1-ène-1-yl]-phényl}propoxy)benzoïque,
et ses sels, ses hydrates, les hydrates des sels et ses produits de solvatation.

7. Procédé de production de composés de formule (I), telle que définie dans la revendication 1, **caractérisé en ce que**
[A] on fait réagir des composé de formule (II) 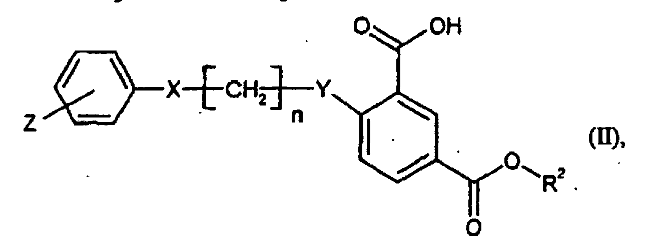 dans laquelle
R² représente un reste alkyle en C₁ à C₆ et
n, X, Y et Z ont la définition indiquée dans la revendication 1,
avec des composés de formule (III) 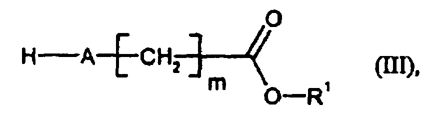 dans laquelle
R¹ est un reste alkyle en C₁ à C₆ et
m et A ont la définition indiquée dans la revendication 1,
ou bien
[B1] on fait réagir des composés de formule (IVa) 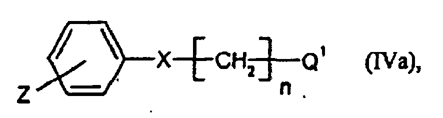 dans laquelle
Q¹ représente un groupe partant et
n, X et Z ont la définition indiquée dans la revendication 1,
avec des composés de formule (Va) 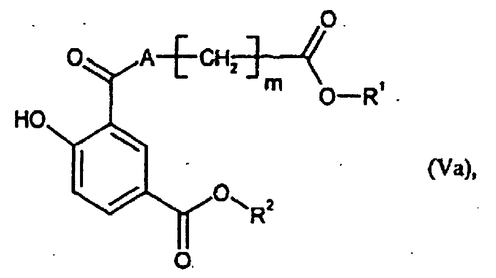 dans laquelle
R¹ et R² représentent un reste alkyle en C₁ à C₆ et
A et m ont la définition indiquée dans la revendication 1,
ou bien
[B2] on fait réagir des composés de formule (IVb) 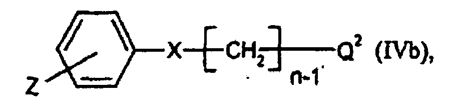 dans laquelle
Q² représente un groupe chlorure d'acide et
n, X et Z ont la définition indiquée dans la revendication 1,
avec des composés de formule (Vb) 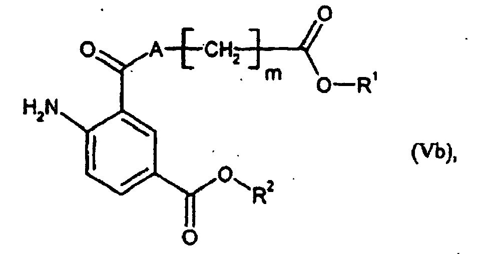 dans laquelle
R¹ et R² représentent un reste alkyle en C₁ à C₆ et
A et m ont la définition indiquée dans la revendication 1,
ou bien
[B3] on fait réagir des composés de formule (IVa) 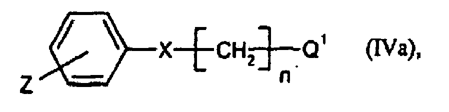 dans laquelle
Q¹ représente un groupe partant et
n, X et Z ont la définition indiquée dans la revendication 1,
avec des composés de formule (Vb) 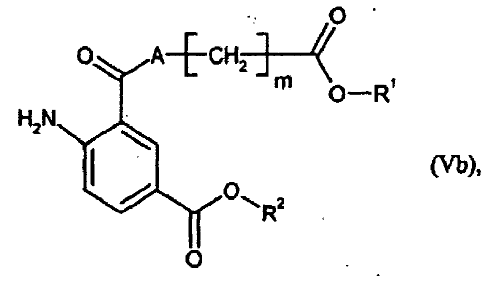 dans laquelle
R¹ et R² représentent un groupe alkyle en C₁ à C₆ et
A et m ont la définition indiquée dans la revendication 1,
ou bien
[C] on fait réagir des composés de formule (XII) 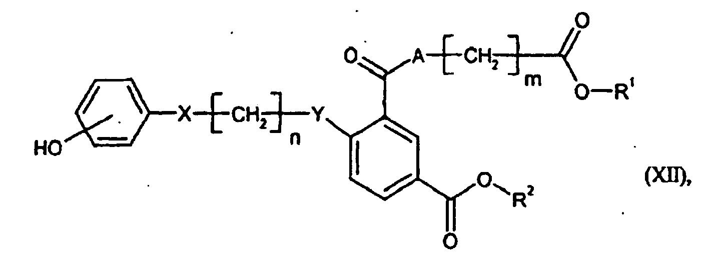 dans laquelle
R¹ et R² représentent un reste alkyle en C₁ à C₆ et
n, m, X, Y et A ont la définition indiquée dans la revendication 1,
avec des composés de formule (XIII)
R⁴-M-L-Q³ (XIII)
dans laquelle
Q³ représente un groupe partant et
R⁴, M et L ont la définition indiquée dans la revendication 1,
ou bien
[D] on saponifie les deux groupes ester dans des composés qui ont été préparés conformément au mode opératoire [A], [B1], [B2], [B3] ou [C].

8. Composé de formule (I), tel que défini dans la revendication 1, destiné au traitement et/ou à la prophylaxie de maladies.

9. Médicament contenant au moins un composé de formule (I), tel que défini dans la revendication 1, et au moins une autre substance auxiliaire.

10. Médicament contenant au moins un composé de formule (I), tel que défini dans la revendication 1, et au moins une autre substance active.

11. Utilisation de composés de formule (I), telle que définie dans la revendication 1, pour la préparation de médicaments destinés au traitement et/ou à la prophylaxie de maladies cardio-vasculaires.

12. Utilisation suivant la revendication 11, pour le traitement et/ou la prophylaxie de l'angor instable ou de l'infarctus du myocarde.
